# EUROPEAN PATENT APPLICATION

(11) **EP 4 421 175 A1**
(43) Date of publication of application: **28.08.2024**
(21) Application number: 22882841.4
(22) Date of filing: 18.10.2022
(51) Int. Cl.: C12N 15/113, A61K 31/713, A61K 47/54, A61P 9/12

(54) **AGT INHIBITOR AND USE THEREOF**

(30) Priority: 20.10.2021 CN 202111222428
(71) Applicant: Kylonova (Xiamen) Biopharma Co., Ltd., Xiamen, Fujian 361022 (CN)
(72) Inventor: CUI, Kunyuan, Xiamen, Fujian 361022 (CN); WANG, Shengjun, Xiamen, Fujian 361022 (CN); CHEN, Qingyan, Xiamen, Fujian 361022 (CN)
(74) Representative: Witthoff Jaekel Steinecke Patentanwälte PartG mbB
(86) International application number: PCT/CN2022/125877
(87) International publication number: WO 2023/066236

(57) **Abstract**

This invention relates to an RNAi agent comprising a carrier structure and an interfering nucleic acid in its structure or pharmaceutically acceptable salt thereof. The invention also relates to a method of using the RNAi agent to inhibit AGT gene expression and a method of using the RNAi agent to prevent and treat AGT related diseases.

## Description

### Technical field

The invention relates to the field of biomedicine, in particular to an RNAi agent for inhibiting AGT gene expression and its application.

### Background

### RNAi

RNAi (RNA interference) was discovered by Andrew Z. Fire et al. during antisense RNA inhibition experiments in Caenorhabditis elegans in 1998, and this process was called RNAi. This discovery was rated as one of the top ten scientific advances in 2001 by Science magazine, and ranked first among the top ten scientific advances in 2002. Since then, siRNA with RNAi as its mechanism of action has received widespread attention as a potential gene therapy drug. In 2006, Andrew Z. Fire and Craig C. Mello won the Nobel Prize in physiology or medicine for their contributions in the study of RNAi mechanism. RNAi is triggered by double stranded RNA (dsRNA) in many organisms, including animals, plants and fungi. In the process of RNAi, an endonuclease called "Dicer" cleaves or "dices" long dsRNA into small segments of 21-25 nucleotides long. These small segments are known as small interfering RNAs (siRNAs), in which the antisense strand is loaded onto Argonaute protein (AGO2). AGO2 loading occurs in the RISC-loading complex, a ternary complex consisting of Argonaute protein, Dicer, and dsRNA binding protein (TRBP for short). During loading, the sense strand is cleaved by AGO2 and discharged. Then, AGO2 uses the antisense strand to bind to mRNA containing fully complementary sequences, and then catalyzes the cleavage of these mRNA, resulting in mRNA cleavage to loss the role of translation template, thereby preventing the synthesis of related proteins. After cleavage, the cleaved mRNA is released, and the RISC-loading complex loaded with the antisense strand is recycled for another round of cleavage.

According to statistics, about more than 80% of the disease-related proteins in the human body cannot be targeted by the current conventional small molecule drugs and biomacromolecule preparations, and are undruggable proteins. Gene therapy, which aims to treat diseases through gene expression, silencing and other functions, is considered by the industry to be the third generation of therapeutic drugs after chemical small molecule drugs and biological macromolecular drugs. This therapy can treat diseases at the gene level and is not restricted by undruggable proteins. As the most mainstream type of gene therapy, RNAi technology is to treat diseases at the mRNA level, which has higher efficiency as compared with chemical small molecule drugs and biological macromolecular drugs at the protein level. Using RNAi technology, we can design the sense strand and antisense strand sequences of siRNAs with high specificity and good inhibition effect according to the specific gene sequence. These single strand sequences are synthesized through solid-phase synthesis, and then the sense strand and antisense strand are paired into siRNA in a specific annealing buffer according to the principle of base pairing. Finally, it is delivered to the corresponding target in the body through the vehicle system, degrade the target mRNA, and destroy the function of the target mRNA as a translation template, thus preventing the synthesis of related proteins.

### Delivery system of siRNA

siRNA is unstable in blood and tissues and easy to be degraded by nucleases. In order to improve the stability of siRNA, the sense strand and / or antisense strand of siRNA can be modified, but these chemical modifications only provide limited protection from nuclease degradation and may ultimately affect the activity of siRNA. Therefore, a corresponding delivery system is also needed to ensure that siRNA can safely and efficiently cross cell membrane. Due to its large molecular weight, large amount of negative charge, and high water solubility, siRNA itself cannot smoothly cross cell membrane to enter the cell.

Liposome is basically composed of hydrophilic core and phospholipid bilayer. It has a phospholipid bilayer structure similar to biofilm and has high biocompatibility, so liposome once became the most popular and widely used siRNA vehicle. Liposome-mediated siRNA delivery mainly encapsulates siRNA into liposomes, protects siRNA from degradation by nucleases, improves the efficiency of siRNA through cell membrane barriers, and thus promotes cell absorption. Examples of liposomes include, for example, anionic liposomes, pH sensitive liposomes, immunoliposomes, fusogenic liposomes, and cationic lipids. Despite some progress, liposomes themselves are prone to trigger inflammatory reactions, a variety of antihistamines and hormones such as cetirizine and dexamethasone must be used before administration to reduce the possible acute inflammatory reactions. Therefore, they are not suitable for all treatment fields in actual clinical application, especially for diseases with long treatment cycles such as chronic hepatitis B, where the possible accumulated toxicity of long-term use is a potential safety hazard. Therefore, a safer and more effective carrier system is needed to deliver siRNA.

Asialoglycoprotein receptor (ASGPR) in the liver is a receptor for hepatocyte-specific expression and is a highly efficient endocytic receptor. Due to the fact that under physiological conditions, various glycoproteins are hydrolyzed by enzymes or acids to remove their sialic acid, exposing the galactose residues at the secondary terminal end, the sugar specifically bound by ASGPR is galactosyl, so it is also called galactose-specific receptor. Monosaccharide and polysaccharide molecules such as galactose, galactosamine, and N-acetylgalactosamine all have high affinity for ASGPR. ASGPR mainly has the physiological function of mediating the clearance of asialoglycoprotein, lipoprotein and other substances in the blood, and it is closely related to the occurrence and development of liver diseases such as viral hepatitis, liver cirrhosis, liver cancer and so on. The discovery of this characteristic of ASGPR plays an important role in the diagnosis and treatment of hepatogenic diseases (Ashwell G, Harford J, Carbohydrate specific Receptors of the Liver, Ann Rev Biochem 1982 51:531-554). The therapeutic drug for hepatogenic diseases containing galactose or galactosamine and their derivatives in the structure can have specific affinity with ASGPR, so it actively targets liver and does not need other vehicle systems to deliver.

### Angiotensinogen (AGT) and hypertension

Blood pressure refers to the pressure of blood in the circulatory system on the blood vessel wall. Blood pressure is mainly caused by the beating of the animal heart. During each heartbeat, blood pressure varied between maximum (systolic) blood pressure (SBP) and minimum (diastolic) blood pressure (DBP). Mean arterial pressure (MAP) is the mean arterial pressure during the heartbeat cycle. Blood pressure can be measured by a sphygmomanometer (i.e. a blood pressure monitor). Normal blood pressure at rest is in the range of 100-140mmHg systolic and 60-90mmHg diastolic, and is usually expressed as systolic blood pressure (highest reading) / diastolic blood pressure (lowest reading) mmHg.

Hypertension was defined as systolic blood pressure (SBP) ≥ 140 mmHg and / or diastolic blood pressure (DBP) ≥ 90 mmHg without antihypertensive drugs. According to the level of elevated blood pressure, hypertension was divided into grade 1, grade 2 and grade 3. According to blood pressure levels, cardiovascular risk factors, target organ damage, clinical complications and diabetes mellitus, cardiovascular risk was stratified into four levels: low-risk, medium-risk, high-risk and very high-risk. Blood pressure is classified and defined as follows:

| | classification | SBP (mmHg) | DBP (mmHg) |
|---|---|---|---|
| Normal blood pressure | | < 120 and | < 80 |
| Normal high value | | 120∼139 and / or | 80∼89 |
| hypertension | | ≥ 140 and / or | ≥ 90 |
| | Grade 1 hypertension (mild) | 140∼159 and / or | 90∼99 |
| | Grade 2 hypertension (moderate) | 160∼179 and / or | 100∼109 |
| | Grade 3 hypertension (severe) | ≥ 180 and / or | ≥ 110 |
| Isolated systolic hypertension | | ≥ 140 and | < 90 |

According to the cause of the disease, hypertension can also be divided into primary hypertension and secondary hypertension. Primary hypertension is hypertension caused by many factors, or hypertension caused by unknown reasons. There are various reasons such as genetic, geographical or sodium water retention, sympathetic excitation, RAS activation, etc., so primary hypertension can only be controlled, not cured. Secondary hypertension refers to the increase of blood pressure caused by a certain disease. Hypertension is one of the clinical symptoms of primary disease, accounting for 95%. Generally, secondary hypertension is common in renal hypertension, renal artery stenosis, primary aldosteronism, pheochromocytoma, multiple Takayasu arteritis, and so on.

There is a close causal relationship between blood pressure level and the risk of morbidity and mortality of cardio-cerebrovascular disease. Some studies have found that the baseline blood pressure from 115 / 75 mmHg to 185 / 115 mmHg, with an average follow-up of 12 years shows that the SBP or DBP in the consulting room has a continuous, independent, and direct positive correlation with the risk of stroke, coronary heart disease events, and cardiovascular death. For every 20 mmHg increase in SBP or 10 mmHg increase in DBP, the risk of cardiovascular and cerebrovascular diseases doubled. Heart failure and stroke are the two complications most closely related to blood pressure. At the same time, it was also found that the incidence of end-stage renal disease (ESRD) also increased significantly. In severe hypertension, the incidence of ESRD is more than 11 times that of normotensive patients, and even if the blood pressure is at the normal high level, it is 1.9 times. Therefore, the fundamental goal of hypertension treatment is to effectively control the disease process of hypertension, and prevent the occurrence of severe hypertension such as hypertensive emergencies and sub emergencies by reducing blood pressure, effectively preventing or delaying the occurrence of complications such as stroke, myocardial infarction, heart failure, renal insufficiency.

Renin-angiotensin-aldosterone system", abbreviated as RAAS or RAS (renin-angiotensin system), is a system in the human body that regulates cardiovascular function. Under the leadership of the sympathetic nervous system, angiotensin secreted has the effect of constricting blood vessels. Excessive stimulation or activity of RAS pathway is one of the reasons for the formation of hypertension.

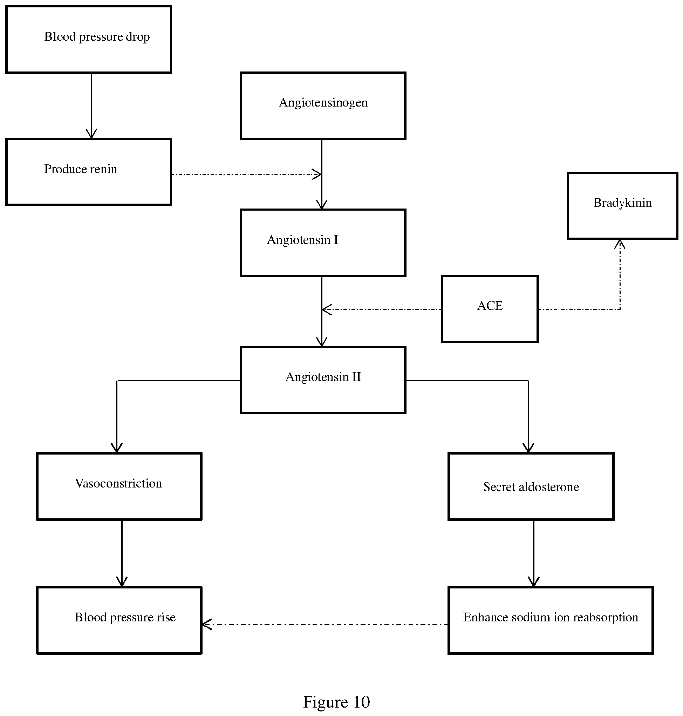

Angiotensinogen (AGT), a member of serpin family, also known as SERPINA8, is encoded by AGT gene and is the only precursor of all angiotensin peptides in RAS. Human AGT has 485 amino acids, including a 33-amino acid signal peptide, which is mainly produced in the liver and released into the systemic circulation, during which renin converts it to angiotensin I. Angiotensin I is subsequently converted to angiotensin II by angiotensin converting enzyme (ACE). Angiotensin I can stimulate the adrenal medulla to secrete epinephrine, but the effect of direct vasoconstriction is not obvious; Angiotensin II can make the systemic arterioles contract and elevate blood pressure. In addition, it can also promote the secretion of aldosterone from the adrenal cortex. Aldosterone acts on renal tubules, and plays a role in sodium retention, water retention and potassium excretion, thus causing an increase in blood volume and blood pressure. The mechanism of action is shown in Figure 10.

There are five main categories of antihypertensive drugs in the clinical front line. The commonly used antihypertensive drugs are divided into five categories, which are generally represented by the letter ABCD. A includes angiotensin-converting enzyme inhibitor ACEI (such as -prils) and angiotensin II antagonist ARB (such as sartans), B means β1-receptor antagonists (such as -lols), C refers to dihydropyridine calcium channel antagonists (such as -dipines), D refers to diuretics (such as -thiazides). The mechanism of action of these five antihypertensive drugs is not exactly the same. According to the different ages and blood pressure levels of patients, one drug is administrated first for the control of blood pressure in clinical practice, and if the control effect of one drug on blood pressure is not good enough, two or even three antihypertensive drugs are administrated in combination. If the combination of three antihypertensive drugs can control the blood pressure to the normal range, it is called refractory hypertension. Refractory hypertension is a common clinical problem in the treatment of hypertension, and it is also a thorny problem in the treatment.

At present, these five kinds of drugs commonly used in clinical practice all need long-term uninterrupted daily administration. Some patients have poor compliance, and the side effects are the main reason for poor compliance . Both ACEI and ARB can cause dry cough and edema, and can lead to renal dysfunction in severe cases; The side effects of category CB drugs can be seen as heartbeat acceleration, blushing, headache, and foot swelling; category B can also cause fatigue and affect blood glucose and lipid metabolism; category D drugs can cause body weakness, cramps, and even lead to gout in severe cases.

Therefore, there is a need for more effective treatment in this field for those with new mechanism of action that are different from existing clinical drugs and with fewer side effects.

### Summary

This invention provides an AGT RNAi agent, which can prevent and / or treat diseases related to RAS pathway, such as hypertension, including "non treatment-resistant hypertension" and "treatment-resistant hypertension", by specifically interfering with the mRNA of AGT gene, destroying its function as a translation template and preventing the expression of angiotensinogen AGT protein.

The RNAi agent of this invention can be formed by base pairing of sense strand and antisense strand. The sense strand and antisense strand are at least 80% base complementary to each other, and 2' positions of part or all of the nucleotide glycosyls can be fluoro or methoxy, and the phosphate bond among at least three consecutive nucleotides at the end of the strand can be thioated.

The structure of the RNAi agent of this invention can also contain the structures 5'MVIP and 3'MVIP that make the RNAi agent have liver targeting specificity, wherein 5'MVIP can be conjugated at the 5' end of the sense strand and / or antisense strand of the RNAi agent, 3'MVIP can be conjugated at the 3' end of the antisense strand and / or sense strand of the RNAi agent, and both 5'MVIP and 3'MVIP can contain (liver targeting specificity) targeting unit X, branched chain L, linker B and linking chain D. The 5'MVIP can also include a transition point R₁ connected with the 5' end of the sense strand or antisense strand of the RNAi agent, and the 3'MVIP can also include a transition point R₂ connected with the 3' end of the sense strand or antisense strand of the RNAi agent. The targeting unit X, branched chain L, linker B and linking chain D can be the same or different within the respective 5'MVIP and 3'MVIP or between the 5'MVIP and 3'MVIP. According to the in vivo and in vitro pharmacodynamic experiments, this RNAi agent can directly degrade AGT mRNA, continuously and efficiently inhibit AGT gene expression, and can be used to treat or / and prevent AGT gene-mediated diseases, such as hypertension.

In an aspect, the invention provides an RNAi agent or pharmaceutically acceptable salt thereof. The structure of the RNAi agent contains a carrier structure and an interfering nucleic acid, as shown in formula IIIa, IIIb or IIIc: 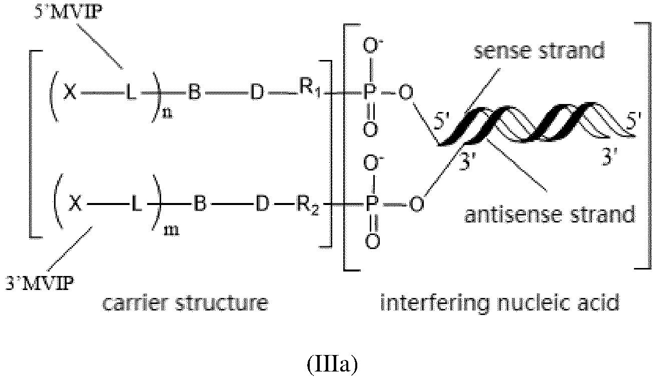 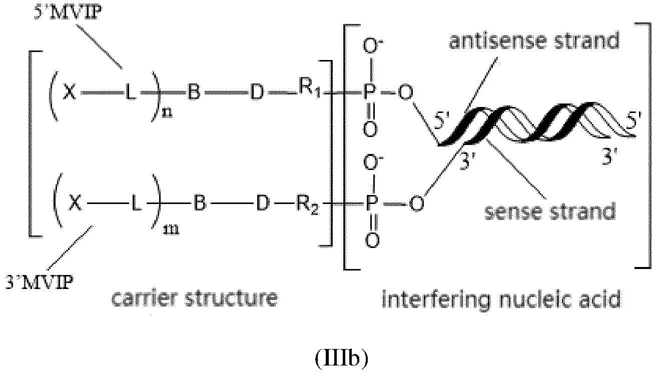 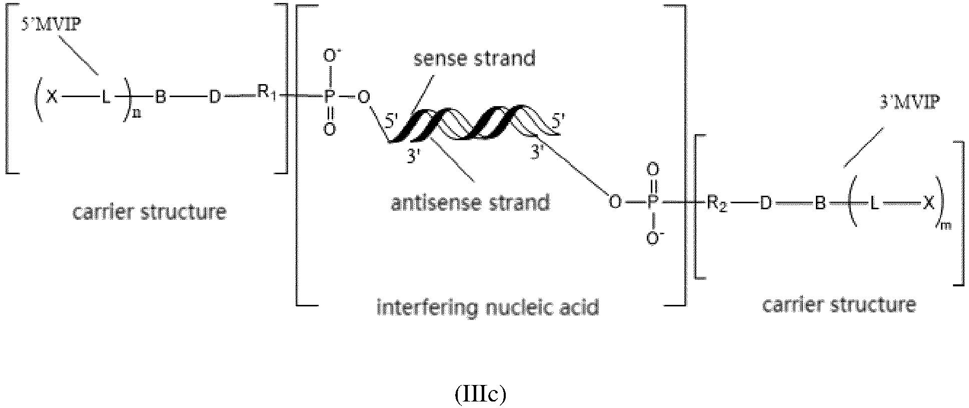 wherein,
The interfering nucleic acid targets an AGT gene, which includes an antisense strand and a sense strand;
The carrier structure includes a 5'MVIP (5'MultiValent Import Platform) and / or 3 'MVIP (3'MultiValent Import Platform);
The 5'MVIP is composed of a transition point Ri, a linking chain D, a linker B, a branched chain L and a liver targeting specific ligand X. The 3'MVIP is composed of a transition point R₂, a linking chain D, a linker B, a branched chain L and a liver targeting specific ligand X. The 5'MVIP is connected with the 5' end of sense strand or the 5' end of antisense strand through transition point R₁. The 3'MVIP is connected with the 3' end of sense strand or the 3' end of antisense strand through transition point R₂. n and m are each independently any integer from 0 to 4.

In some embodiments, the interfering nucleic acid is used to inhibit AGT gene expression.

In some embodiments, the interfering nucleic acid includes siRNA or miRNA.

In some embodiments, n+m is an integer of 2-6, preferably n+m=2, 3 or 4, more preferably 4.

In some embodiments, the 5'MVIP is selected from any one of 5'MVIP01 to 5'MVIP22 in Table 10, and / or the 3'MVIP is selected from any one of 3'MVIP01 to 3'MVIP27 in Table 11.

In some embodiments, the sense strand is essentially homologous to any one of SEQ ID NO: 1, SEQ ID NO: 7, SEQ ID NO: 8, SEQ ID NO: 10, SEQ ID NO: 11, SEQ ID NO: 12, SEQ ID NO: 17 and SEQ ID NO: 18 or a sequence that differs from any of the above by no more than 3 nucleotides.

In some embodiments, the antisense strand comprises any one of the following nucleotide sequences: SEQ ID NO: 19, SEQ ID NO: 25, SEQ ID NO: 26, SEQ ID NO: 28, SEQ ID NO: 29, SEQ ID NO: 30, SEQ ID NO: 35 and SEQ ID NO: 36 or a sequence that differs from any of the above by no more than 3 nucleotides.

In some embodiments, the sense strand comprises any one of SEQ ID NO: 37, SEQ ID NO: 43, SEQ ID NO: 44, SEQ ID NO: 46, SEQ ID NO: 47, SEQ ID NO: 48, SEQ ID NO: 53 and SEQ ID NO: 54 or a sequence that differs from any of the above by no more than 3 nucleotides, and the antisense strand comprises any one of SEQ ID NO: 55, SEQ ID NO: 61, SEQ ID NO: 62, SEQ ID NO: 64, SEQ ID NO: 65, SEQ ID NO: 66, SEQ ID NO: 71 and SEQ ID NO: 72 or a sequence that differs from any of the above by no more than 3 nucleotides.

In some embodiments, the interfering nucleic acid includes any one or more of Kylo-09-DS01, Kylo-09-DS07, Kylo-09-DS08, Kylo-09-DS10, Kylo-09-DS11, Kylo-09-DS12, Kylo-09-DS 17, Kylo-09-DS18, Kylo-09-DS37- Kylo-09-DS54.

In some embodiments, the RNAi agent or pharmaceutically acceptable salt thereof includes any one or more of Kylo-09-DS 122, Kylo-09-DS 131 to Kylo-09-DS 147 in table 18.

In some embodiments, the RNAi agent or pharmaceutically acceptable salt thereof includes any one or more of Kylo-09-DS 122, Kylo-09-DS131, Kylo-09-DS141, Kylo-09-DS 142, and Kylo-09-DS 147 in Table 19.

In another aspect, the present invention provides an RNAi agent or pharmaceutically acceptable salt thereof for inhibiting AGT gene expression, which comprises an antisense strand, wherein the antisense strand comprises at least 12 consecutive nucleotides that are substantially complementary to the nucleotides selected from the corresponding positions of the following sequences or a sequence that differs from them by no more than 3 nucleotides: at least 12 consecutive nucleotides with starting positions of 1854-1874, 1907-1927, 1895-1915, 1352-1372, 1903-1923, 2019-2039, 1853-1873 and 1818-1838 in AGT mRNA NM_001382817.3 or a sequence that differs from any of the above by no more than 3 nucleotides, or at least 12 consecutive nucleotides with starting positions of 1822-1842, 1875-1895, 1863-1883, 1320-1340, 1871-1891, 1987-2007, 1821-1841, and 1786-1806 in NM_001384479.1 or a sequence that differs from any of the above by no more than 3 nucleotides.

In some embodiments, the RNAi agent includes single stranded or double stranded nucleic acid molecules.

In some embodiments, the RNAi agent includes siRNA or miRNA.

In some embodiments, the RNAi agent or pharmaceutically acceptable salt thereof for inhibiting AGT gene expression also comprises a sense strand, wherein the complementary region formed by the antisense strand and the sense strand comprises at least 12 consecutive nucleotides.

In some embodiments, one or more nucleotides on the sense strand and / or antisense strand are modified to form modified nucleotides.

In some embodiments, the RNAi agent or pharmaceutically acceptable salt thereof for inhibiting AGT gene expression further comprises a ligand, which is conjugated to the sense strand and / or antisense strand through a carrier structure.

In yet another aspect, the present invention provides a cell comprising the aforementioned RNAi agent or pharmaceutically acceptable salt thereof, or the aforementioned RNAi agent or pharmaceutically acceptable salt thereof for inhibiting AGT gene expression.

In yet another aspect, the present invention provides a pharmaceutical composition comprising the aforementioned RNAi agent or pharmaceutically acceptable salt thereof, or the aforementioned RNAi agent or pharmaceutically acceptable salt thereof for inhibiting AGT gene expression, and an optional pharmaceutically acceptable excipient, vehicle, and / or diluent.

In yet another aspect, the present invention provides a method for reducing AGT mRNA or protein expression in a cell or tissue, which includes contacting the cell or tissue with an effective amount of the aforementioned RNAi agent or pharmaceutically acceptable salt thereof, the aforementioned RNAi agent or pharmaceutically acceptable salt thereof for inhibiting AGT gene expression, and / or the aforementioned pharmaceutical composition.

In yet another aspect, the present invention provides the use of the aforementioned RNAi agent or pharmaceutically acceptable salt thereof, the aforementioned RNAi agent or pharmaceutically acceptable salt thereof for inhibiting AGT gene expression, or the aforementioned pharmaceutical composition in the preparation of drugs for preventing and / or treating diseases or conditions or reducing the risk of diseases or conditions.

In yet another aspect, the invention provides a method for preventing and / or treating diseases or conditions, which includes administering an effective amount of the aforementioned RNAi agent or pharmaceutically acceptable salt thereof, the aforementioned RNAi agent or pharmaceutically acceptable salt thereof for inhibiting AGT gene expression, and / or the aforementioned pharmaceutical composition to a subject in need thereof.

In yet another aspect, the present invention provides a kit, which comprises the aforementioned RNAi agent or pharmaceutically acceptable salt thereof, the aforementioned RNAi agent or pharmaceutically acceptable salt thereof for inhibiting AGT gene expression, or the aforementioned pharmaceutical composition.

The AGT RNAi agent of this invention can interfere with AGT mRNA, destroy its function as a translation template, adjust the expression level of AGT protein (the only precursor of all angiotensin peptides of RAS) in the systemic circulation, regulate blood pressure from the genetic level and the source of RAS system, and provide a brand-new treatment mode to combat various types of hypertension, including primary and secondary, as well as treatment-resistant hypertension (TRH), make drugs available to those patients with treatment-resistant hypertension (TRH) or chronic heart failure with reduced ejection fraction. The AGT RNAi agent of this invention also has unique long-term performance, and it may be administered once a quarter or half a year, which has incomparable advantages over current small molecule hypertension therapeutics.

Those skilled in the art can easily perceive other aspects and advantages of the invention from the following detailed description. Only exemplary embodiments of the present invention are shown and described in the following detailed description. As those skilled in the art will recognize, the content of the present invention enables those skilled in the art to make changes to the disclosed specific embodiments without departing from the spirit and scope of the invention involved in the present application. Accordingly, the descriptions in the drawings and specification of the present invention are merely exemplary and not restrictive.

### Brief description of the Drawings

The specific features of the invention involved in this application are shown in the appended claims. The features and advantages of the invention covered by the present application can be better understood by referring to the exemplary embodiments and drawings described in detail below. A brief description of the attached drawings is as follows:
Figure 1A shows the AGT mRNA level in Hep 3B cells after RNAi interference in Example 2 of this application;
Figure 1B shows the AGT mRNA level in HepG 2 cells after RNAi interference in Example 2 of this application;
Figure 2A-2D shows the HPLC diagram of stability detection of RNAi agent in different periods of time in Example 3 of the application;
Figure 3 shows the high-resolution mass spectrum of ERCd-01-c2 synthesized in Example 4.1.1.5 of the application;
Figure 4 shows the high-resolution mass spectrum of 3'MVIP17-c1 synthesized in Example 4.1.2.6 of the application;
Figure 5 shows the high-resolution mass spectrum of 5'MVIP09-ERCd-PFP-c2 synthesized in Example 4.2.1.2 of this application;
Figure 6 shows the average levels of hAGT in transgenic mice after administration in Example 6 of the application;
Figure 7 shows the average levels of hAGT in the serum of transgenic mice after administration in Example 7 of this application.
Figure 8 shows the average levels of AGT in the serum of cynomolgus monkeys after administration in Example 8 of the application.
Figures 9A-9E show the structural formulas of Kylo-09-DS122, Kylo-09-DS131, Kylo-09-DS141, Kylo-09-DS142 and Kylo-09-DS147.
Figure 10 shows the mechanism of action of angiotensinogen (AGT).

### Embodiments

The implementation of the invention of the application will be described below with specific examples. Those skilled in the art can easily understand other advantages and effects of the invention of the application from the contents disclosed in the specification.

### Definition of terms

In this application, the term "angiotensinogen" can be used interchangeably with the term "AGT". Examples of AGT mRNA sequences are easily obtained using published databases, such as GenBank, UniProt, OMIM, and Macaca genome project website. The term "AGT" includes human AGT, whose mRNA sequence can be found in, for example, GenBank NM_001382817.3 or GenBank NM_001384479.1; cynomolgus monkey AGT, whose amino acids and complete coding sequence can be found in, for example, GenBank accession number GI:90075391(AB170313 .1); mouse (Mus musculus) AGT, whose amino acid and complete coding sequence can be found in, for example, GenBank accession number GI:113461997(NM_007428 .3); and rat (Rattus norvegicus) AGT, whose amino acids and complete coding sequence can be found in, for example, GenBank accession number GI:51036672(NM_134432). The term "AGT" used herein also refers to the naturally occurring DNA sequence variations of the AGT gene, such as single nucleotide polymorphisms (SNP) in the AGT gene.

In this application, the terms "iRNA", "RNAi agent", "iRNA agent" and "RNA interfering agent" can be used interchangeably, and generally refer to agents containing RNA as defined by the terms herein, which can mediate the targeted cleavage of RNA transcripts through the RNA-induced silencing complex (RISC) pathway. iRNA guides the sequence specific degradation of mRNA via a process called RNA interference (RNAi). iRNA regulates (e.g., inhibits) the expression of AGT genes in a cell (e.g., cell in a subject such as a mammalian subject).

In some embodiments, RNAi agents may be single stranded siRNAs (ssRNAi) introduced into a cell or organism to inhibit target mRNA. Single stranded RNAi agents bind the RISC endonuclease Argonaute 2, which then cleaves the target mRNA. Single stranded siRNAs are generally 15 to 30 nucleotides and chemically modified. The design and test of single stranded siRNA are described in U.S. Patent No. 8,101,348 and Lima et al. (2012) Cell 150:883-894, and their complete contents are incorporated herein by reference. Any antisense nucleotide sequence described herein can be used as a single stranded siRNA described herein or chemically modified by the method described in Lima et al. (2012) Cell 150:883-894.

In some embodiments, the "iRNA" used in this application is a double stranded RNA, and is referred to herein as "double stranded RNAi agent", "double stranded RNA (dsRNA) molecule", "dsRNA agent" or "dsRNA". The term "dsRNA" refers to a complex of ribonucleic acid molecules with a duplex structure containing two antiparallel and essentially complementary nucleic acid strands, known as having a "sense" and "antisense" orientation relative to the target RNA (i.e., AGT gene). In some embodiments of the present application, double stranded RNA (dsRNA) triggers the degradation of target RNA (e.g., mRNA) through a post transcriptional gene silencing mechanism (herein referred to as RNA interference or RNAi).

The duplex structure can be any length that allows the required target RNA to be specifically degraded through the RISC pathway, and can have a length in range of about 19 to 36 base pairs, for example, the length of about 19-30 base pairs, for example, the length of about 19, 20, 21, 22, 23, 24, 25, 26, 27, 28, 29, 30, 31, 32, 33, 34, 35 or 36 base pairs. The ranges and lengths within the above ranges and lengths are also included as part of this application. In some embodiments, the iRNA agent of the present application is a dsRNA containing 15-23 nucleotides in each strand, which interacts with the target RNA sequence (e.g., AGT gene) to guide the cleavage of the target RNA. In some embodiments, the iRNA of the present application is a dsRNA containing 24-30 nucleotide, which interacts with the target RNA sequence (e.g., AGT target mRNA sequence) to guide the cleavage of the target RNA.

Generally, most nucleotides of each chain of dsRNA molecule are ribonucleotides, but as detailed herein, each chain or both chains can also include one or more non-ribonucleotides, such as deoxyribonucleotides or modified nucleotides. In addition, the "iRNA" used in this specification may include ribonucleotides with chemical modifications; iRNA can include substantial modifications at multiple nucleotides. The term "modified nucleotide" used herein refers to a nucleotide that independently has a modified sugar moiety, a modified internucleotide linkage or a modified nucleobase, or any combination thereof. Therefore, the term "modified nucleotide" covers the substitution, addition, or removal of functional groups or atoms of internucleotide linkages, sugar moieties, or nucleobases. The modifications applicable to the agent of the invention include all types of modifications disclosed herein or known in the art.

In this application, the terms "nucleic acid" and "polynucleotide" can be used interchangeably and refer to the polymeric form of nucleotides (deoxyribonucleotides or ribonucleotides or analogues thereof) of any length. Polynucleotides can have any three-dimensional structure and can perform any function. The following are non-limiting examples of polynucleotides: genes or gene segments (such as probes, primers, EST or SAGE tags), exons, introns, messenger RNA (mRNA), transfer RNA, ribosomal RNA, ribozymes, cDNA, recombinant polynucleotides, branched polynucleotides, plasmids, vectors, isolated DNA of any sequence, isolated RNA of any sequence, nucleic acid probes, siRNA, miRNA, shRNA, RNAi reagents and primers. Polynucleotides may be modified or substituted at one or more bases, sugars, and / or phosphates with any of the various modifications or substitutions described in this application or known in the art. Polynucleotides can contain modified nucleotides, such as methylated nucleotides and nucleotide analogues. If present, the nucleotide structure can be modified before or after polymer assembly. Nucleotide sequences can be blocked by non-nucleotide components. Polynucleotides can be modified after polymerization, for example by conjugating with labeled components. The term can be double stranded and single stranded molecules. Unless otherwise stated or required, any embodiment as a polynucleotide of the present application includes a double stranded form and each of the two complementary single stranded forms known or predicted to constitute the double stranded form.

In the present application, the term "target nucleic acid" or "target sequence" generally refers to the consecutive part of the nucleotide sequence of the mRNA molecule formed during the transcription of the AGT gene, including mRNA as the RNA processing product of the main transcription product. The target part of the sequence should be at least long enough to serve as a substrate to guide iRNA cleavage during AGT gene transcription. In one embodiment, the target sequence is within the protein coding region of AGT. The target sequence can be about 19-36 nucleotides in length, for example, it is preferred to be about 19-30 nucleotides in length. The ranges and lengths within the above ranges and lengths are also included as part of this application.

In this application, the term "nucleotide sequence" usually refers to a series or a certain sequence of nucleobases, nucleotides and / or nucleosides, whether modified or unmodified, described by a sequence of letters using standard nucleotide nomenclature and a list of symbols for modified nucleotides described in this application.

In this application, the term "oligonucleotide" generally refers to a polymer composed of multiple nucleotide residues (deoxyribonucleotide or ribonucleotide, or related structural variant or synthetic analog thereof) connected by phosphodiester bond (or related structural variant or synthetic analog thereof). Therefore, although the term "oligonucleotide" generally refers to the nucleotide polymer in which the nucleotide residues and the linkage between them are naturally occurred, it should be understood that the scope of the term also includes various analogues, including but not limited to: peptide nucleic acid (PNA), aminophosphate, thiophosphate, methyl phosphonate, 2-o-methyl ribonucleic acid, etc. The exact size of the molecule may depend on the specific application. Oligonucleotides are generally short in length, usually about 10-30 nucleotide residues, but the term can also refer to molecules of any length, although the term "polynucleotide" or "nucleic acid" is generally used for larger oligonucleotides.

In some embodiments, oligonucleotides comprise one or more unmodified ribonucleosides (RNA) and / or unmodified deoxyribonucleosides (DNA) and / or one or more modified nucleosides. The term "modified oligonucleotide" generally refers to an oligonucleotide containing at least one modified nucleoside and / or at least one modified internucleoside linkage.

In the present application, the term "modified nucleoside" generally means a nucleoside containing at least one chemical modification compared with a naturally occurring RNA or DNA nucleoside. Modified nucleosides contain modified sugar moieties and / or modified nucleobases.

In the present application, the term "nucleobase" generally refers to a heterocyclic pyrimidine or purine compound, which is a component of all nucleic acids and includes adenine (A), guanine (G), cytosine (C), thymine (T) and uracil (U). Nucleotides may include modified nucleotides or nucleotide mimics, abasic sites (Ab or X), or the part substituted by the substituent. As used in this application, "nucleobase sequence" generally means the sequence of consecutive nucleobases that does not depend on any sugar, linkage or nucleobase modification. The terms "unmodified nucleobase" or "naturally occurring nucleobase" generally mean naturally occurring heterocyclic nucleobases of RNA or DNA: purine bases adenine (A) and guanine (G); and the pyrimidine bases thymine (T), cytosine (C) (including 5-methyl C) and uracil (U). "Modified nucleobase" usually means any nucleobase that is not a naturally occurring nucleobase.

In the present application, the term "sugar moiety" generally refers to the naturally occurring sugar moiety or modified sugar moiety of a nucleoside. The term "naturally occurring sugar moiety" generally refers to a ribofuranosyl group as found in naturally occurring RNA or a deoxyribofuranosyl group as found in naturally occurring DNA. "Modified sugar moiety" means a substituted sugar moiety or sugar substitute.

In the present application, the term "internucleoside linkage" generally refers to the covalent linkage between adjacent nucleosides in oligonucleotides. "Naturally occurring internucleoside linkage" means 3' to 5' phosphodiester linkage. "Modified internucleoside linkage" means any internucleoside linkage other than the naturally occurring internucleoside linkage.

In the present application, the term "antisense oligonucleotide" refers to a single stranded oligonucleotide molecule with a nucleobase sequence complementary to the corresponding segment of the target nucleic acid (e.g., the target genome sequence, mRNA precursor, or mRNA molecule). In some embodiments, the antisense oligonucleotide is 12 to 30 nucleobases in length. In some embodiments, the antisense oligonucleotide is an unmodified or modified nucleic acid with a nucleotide sequence complementary to the sequence of target nucleic acid (such as AGT polynucleotide).

In the present application, the term "antisense strand" generally refers to the strand of RNAi agent (such as dsRNA) that includes a region that are substantially complementary to the target sequence. When used herein, the term "complementary region" generally refers to a region on the antisense strand that is substantially complementary to the sequence (e.g., target sequence) defined in the application. When the complementary region is not fully complementary to the target sequence, the mismatch can be in the inner or terminal region of the molecule. In general, the most tolerated mismatches are in the terminal region, for example, within 5, 4, 3, or 2 nucleotides at the 5' and / or 3' ends.

In the present application, the term "sense strand" (s) generally refers to such a strand of RNAi agents, which includes a region that is substantially complementary to a region of the antisense strand as the term defined herein. The "sense" strand is sometimes referred to as the "passenger" strand or "anti-guide" strand. With their sequences, the antisense strand targets the desired mRNA, while the sense strand targets different targets. Therefore, if the antisense strand is incorporated into RISC, the correct target is targeted. The incorporation of sense strand can lead to off-target effects. These off-target effects can be limited by the use of modifications or 5' end caps on the sense strand.

In the present application, the term "complementary" when used to describe the first nucleotide sequence (such as RNAi agent sense strand or AGT mRNA) in terms of the second nucleotide sequence (such as RNAi agent antisense strand) refers to the ability of oligonucleotides or polynucleotides containing the first nucleotide sequence to hybridize (form base pair hydrogen bonds) with oligonucleotides or polynucleotides containing the second nucleotide sequence and form duplex or double helix structures under certain conditions. Complementary sequences include Watson-Crick base pairs or non-Watson-Crick base pairs, and include natural or modified nucleotides or nucleotide mimics, as long as the above requirements for their hybridization ability are realized. "Complementation" does not require nucleobase complementarity on each nucleoside. On the contrary, some mismatches can be tolerated.

In the present application, the term "fully complementary" generally means that all (100%) bases in the consecutive sequence of the first polynucleotide will hybridize with the same number of bases in the consecutive sequence of the second polynucleotide. The consecutive sequence may comprise all or part of the first or second nucleotide sequence. As used in this application, "partially complementary" generally means that in the hybridized nucleobase sequence pairs, at least about 70% of the bases in the consecutive sequence of the first polynucleotide will hybridize with the same number of bases in the consecutive sequence of the second polynucleotide. As used in this application, "substantially complementary" generally means that in the hybridized nucleobase sequence pairs, at least about 90% of the bases in the consecutive sequence of the first polynucleotide will hybridize with the same number of bases in the consecutive sequence of the second polynucleotide. The terms "complementary", "fully complementary" and "substantially complementary" as used in this application can be used in terms of base matching between the sense strand and antisense strand of RNAi agent or between the antisense strand of RNAi agent and the sequence of AGT mRNA. Sequence identity or complementarity does not depend on modification. For the purpose of determining identity or complementarity, for example, a and fA are complementary to U (or T) and identical to A.

In the present application, the term "homologous" or "homology" generally refers to the number of nucleotides of the subject nucleic acid sequence that have matched the same nucleotide of the reference nucleic acid sequence, which is usually determined by sequence analysis programs (for example, Karlin and Altschul, 1990, PNAS 87:2264-2268; Karlin and Altschul, 1993, PNAS 90:5873-5877), or by visual inspection. As used herein, the term "complete homology" or "fully homologous" generally refers to complete (100%) homology or "identity" between the reference sequence and the subject nucleic acid sequence. As used herein, the term "substantially homologous" or "substantial homology" generally refers to the fact that nucleotides at the same nucleotide positions in the subject sequence and the reference sequence are at least 50% (e.g., at least 55%, 60%, 65%, 70%, 75%, 80%, 85%, 90%, 95%, 96%, 97%, 98% or 99%) homologous.

In the present application, the term "ligand" generally refers to any compound or molecule that can be covalently or otherwise chemically bound to bioactive substances (such as oligonucleotides). In some embodiments, the ligand is able to interact directly or indirectly with another compound such as a receptor, the receptor interacting with the ligand may exist on the cell surface, or alternatively may be an intracellular and / or intercellular receptor, the interaction of the ligand with the receptor may lead to a biochemical reaction, or may simply be a physical interaction or binding.

In the present application, the terms "induce", "inhibit", "strengthen", "elevate", "increase", "decrease", "reduce" and the like usually indicate quantitative differences between two states. For example, "the amount that effectively inhibits the activity or expression of AGT" means that the level of AGT activity or expression in the treated samples will be lower than that in the untreated samples. The terms described apply, for example, to expression levels and activity levels. The terms "decrease" and "reduce" are used interchangeably and usually indicate any changes less than the original. "Decrease" and "reduce" are relative terms that need to be compared before and after measurement. "Decrease" and "reduce" include complete depletion.

In some embodiments, the term "reduce" may refer to an overall reduction of about 5%, 10%, 20%, 25%, 30%, 40%, 50%, 60%, 70%, 80%, 85%, 90%, 95%, or 100% in the expression level/amount of a gene/gene product such as protein or biomarker in a first sample compared to the expression level/amount of a corresponding gene/gene product such as protein or biomarker in a second sample, as detected by standard methods known in the art, such as those described in the present application. In some embodiments, the term "reduce" refers to a reduction in the expression level / amount of a gene or biomarker in the first sample, wherein the reduction is at least about 0.9 times, 0.8 times, 0.7 times, 0.6 times, 0.5 times, 0.4 times, 0.3 times, 0.2 times, 0.1 times, 0.05 times, or 0.01 times the expression level / amount of the corresponding gene or biomarker in the second sample. In some embodiments, the first sample is a sample obtained from a subject, while the second sample is a reference sample.

In the present application, the term "expression" generally refers to the process by which a gene eventually produces a protein. The expression includes, but is not limited to, transcription, post transcriptional modifications (e.g., splicing, polyadenylation, addition of 5 '- caps), and translation.

In the present application, the term "pharmaceutically acceptable" generally refers to one or more non-toxic substances that do not interfere with the biological activity and effectiveness of the active ingredient. Such preparations may generally contain salts, excipients, buffers, preservatives, compatible carriers, and optionally other therapeutic agents. Such pharmaceutically acceptable preparations may also generally contain compatible solid or liquid fillers, diluents, or encapsulation materials suitable for administration to humans. When used in medicine, salts should be pharmaceutically acceptable salts, but non-pharmaceutically acceptable salts can be conveniently used to prepare pharmaceutically acceptable salts, which cannot be excluded from the scope of this application. Such pharmacologically and pharmaceutically acceptable salts include but are not limited to those prepared from the following acids: hydrochloric acid, hydrobromic acid, sulfuric acid, nitric acid, phosphoric acid, maleic acid, acetic acid, salicylic acid, citric acid, boric acid, formic acid, malonic acid, succinic acid, etc. Pharmaceutically acceptable salts can also be prepared into alkali metal salts or alkaline earth metal salts, such as sodium salt, potassium salt or calcium salt.

In the present application, the term "lipid nanoparticles" or "LNP" generally refers to vesicles containing a lipid layer encapsulating pharmacologically active molecules, such as nucleic acid molecules, for example, iRNA or plasmid from which iRNA is transcribed. LNP is described in, for example, Chinese patent No. CN103189057B, the complete contents of which are incorporated herein by reference.

In the present application, the term "prevention and / or treatment" not only includes the prevention and / or treatment of disease, but also generally includes the prevention of the onset of disease, slowing or reversing the progression of disease, preventing or slowing the onset of one or more symptoms related to disease, reducing and / or alleviating one or more symptoms related to disease, reducing the severity and / or duration of the disease and / or any symptom associated with it and / or prevent further increases in the severity of the disease and / or any symptom associated with it, preventing, reducing or reversing any physiological damage caused by the disease, and any pharmacological effects that are generally beneficial to the patient being treated. The RNAi agent or pharmaceutical composition of the application does not need to achieve complete cure or eradication of any symptoms or manifestations of the disease to form a feasible therapeutic agent. As recognized in relevant fields, drugs used as therapeutic agents can reduce the severity of a given disease state, but do not need to eliminate every manifestation of the disease to be considered as useful therapeutic agents. Similarly, treatments administered prophylactically to constitute viable prophylactic agents do not need to be completely effective in preventing the onset of the condition. Simply reducing the impact of disease in subjects (for example, by reducing the numbers or severity of their symptoms, or by improving the effectiveness of another treatment, or by producing another beneficial effect), or reducing the likelihood of disease occurrence or exacerbation is sufficient.

In the present application, the term "disease" or "condition" can be used interchangeably, and generally refers to any deviation from the normal state of the subject, such as any change in the state of the body or some organs, which hinders or disturbs the performance of the function, and / or causes symptoms such as discomfort, dysfunction, pain or even death in the person who is ill or in contact with him. Disease or condition can also be called distemper, ailing, ailment, malady, disorder, sicknesses, illnesses, complaints, inderdispositions or affectation.

In the present application, the term "angiotensinogen related disease" or "AGT related disease" generally refers to a disease or disorder caused by or related to the activation of renin-angiotensin-aldosterone system (RAAS), or whose symptoms or progression respond to the inactivation of RAAS. The term "angiotensinogen related disease" includes a disease, disorder, or condition that benefits from reduced AGT expression. Such diseases are usually associated with high blood pressure. Non limiting examples of angiotensinogen related diseases include hypertension, for example, borderline hypertension (also known as prehypertension), primary hypertension (also known as essential hypertension or idiopathic hypertension), secondary hypertension (also known as non-essential hypertension), isolated systolic or diastolic hypertension, pregnancy related hypertension (e.g., preeclampsia, eclampsia, and postpartum preeclampsia), diabetic hypertension, treatment-resistant hypertension, refractory hypertension, paroxysmal hypertension, renovascular hypertension (also known as renal hypertension), Goldblatt's hypertension, ocular hypertension, glaucoma, pulmonary hypertension, portal hypertension, systemic venous hypertension, systolic hypertension, unstable hypertension; hypertensive heart disease, hypertensive nephropathy, atherosclerosis, arteriosclerosis, vascular disease (including peripheral vascular disease), diabetic nephropathy, diabetic retinopathy, chronic heart failure, cardiomyopathy, diabetic cardiomyopathy, glomerulosclerosis, main artery constriction, aortic aneurysm, ventricular fibrosis, sleep apnea, heart failure (for example, left ventricular systolic dysfunction), myocardial infarction, angina, stroke, renal disease (e.g., chronic kidney disease or diabetic nephropathy, optionally in the case of pregnancy), renal failure (e.g., chronic renal failure), and systemic sclerosis (e.g., scleroderma renal crisis). In some embodiments, AGT related diseases include intrauterine growth retardation (IUGR) or fetal growth restriction. In some embodiments, AGT related disorders also include obesity, hepatic steatosis / fatty liver, for example, non-alcoholic steatohepatitis (NASH) and non-alcoholic fatty liver disease (NAFLD), glucose intolerance, type 2 diabetes mellitus (non-insulin-dependent diabetes), and metabolic syndrome. In some embodiments, hypertension includes hypertension associated with low plasma renin activity or plasma renin concentration.

In the present application, the term "administration" generally refers to the introduction of the pharmaceutical preparation of this application into the subject's body through any route of introduction or delivery. Any method known to those skilled in the art for contacting a cell, organ or tissue with the drug can be adopted. The administration may include, but is not limited to, intravenous, intra-arterial, intranasal, intra-abdominal, intramuscular, subcutaneous transdermal, or oral administration. The daily dose can be divided into one, two or more suitable forms of dose to be administered at one, two or more times during a certain time period.

In the present application, the term "contact" generally refers to the contact of two or more different types of substances in any order, in any way and for any duration. Contact can occur in vivo, ex vivo, or in vitro. In some embodiments, it may mean that the RNAi agent or composition of the present application directly contacts a cell or tissue. In other embodiments, the term refers to making the RNAi agent or composition of the present application contact a cell or tissue indirectly. For example, the method of the present application includes a method in which the subject is exposed to the RNAi agent or composition of the present application, and then the RNAi agent or composition contacts the cell or tissue through diffusion or any other active or passive transport process known in the art through which the compound circulates in the body.

In the present application, the term "effective amount" or "effective dose" generally refers to an amount sufficient to achieve or at least partially achieve the desired effect. The "therapeutically effective amount" or "therapeutically effective dose" of a drug or therapeutic agent is generally any amount of drug that, when used alone or in combination with another therapeutic agent, promotes disease regression (as evidenced by a decrease in the severity of disease symptoms, an increase in the frequency and duration of asymptomatic periods of disease, or prevention of impairment or disability due to the disease). The "preventive effective amount" or "preventive effective dose" of a drug usually refers to the amount of drug that inhibits the development or recurrence of disease when administered alone or in combination with another therapeutic agent to subjects at risk of disease development or disease recurrence. A variety of methods known to those skilled in the art can be used to evaluate the ability of therapeutic or preventive agents to promote disease regression or inhibit disease development or recurrence, such as predicting efficacy in humans in human subjects during clinical trials and in animal model systems, or determining the activity of the agent in in vitro assays. In some embodiments, "effective amount" refers to the amount of RNAi agent that produces the expected pharmacological, therapeutic or preventive results.

In the present application, the term "subject" generally refers to humans or non-human animals (including mammals) that need diagnosis, prognosis, improvement, prevention and / or treatment of diseases, such as humans, non-human primates (apes, gibbons, gorillas, chimpanzees, orangutans, macaques), domestic animals (dogs and cats), farm animals (poultry such as chickens and ducks, horses, cattle, goats, sheep, pigs) and experimental animals (mice, rats, rabbits, guinea pigs). Human subjects include fetal, newborn, infant, adolescent and adult subjects. Subjects include animal disease models.

In the present application, the terms "include", "comprise", "have", "may/can", "contain" and their variants are generally intended to be open-ended transitional phrases, terms or words, which do not exclude the possibility of additional actions or structures. The term "consisting of/composed of" usually means that no other component (or similarly, feature, integer, step, etc.) can exist. Unless otherwise specified in the context, the singular forms such as "a", "an", "the" in English generally include the plural form of the thing referred to.

In the present application, the term "about" usually means approximately, in the region of, roughly, or around. When the term "about" is used to refer to the range of values, the cut-off value or specific value is used to indicate that the stated value may differ by up to 10% from the enumerated value. Therefore, the term "about" can be used to cover variation from a specific value of ± 10% or less, ± 5% or less, ± 1% or less, ± 0.5% or less, or ± 0.1% or less.

It should be understood that the term "at least" before a number or series of numbers includes the number adjacent to the term "at least", and all subsequent numbers or integers logically included, as defined from the context. For example, the number of nucleotides in a nucleic acid molecule must be an integer. For example, "at least 19 nucleotides in a nucleic acid molecule of 21 nucleotides" means that 19, 20, or 21 nucleotides have the indicated properties. When "at least" appears before a series of numbers or ranges, it should be understood that "at least" can modify each number in the series or ranges.

It should be understood that "no more than" or "less than" used herein refers to a value or integer that is adjacent to the phrase and logically lower, e.g., to zero in contextual logic. For example, duplexes with protruding end of "no more than 3 nucleotides" have protruding end of 3, 2, 1, or 0 nucleotides. When "no more than" appears before a series of numbers or ranges, it should be understood that "no more than" can modify each number in the series or ranges. The scope used herein includes both upper and lower limits.

### Detailed description of the Invention

### RNAi agent

In an aspect, the present invention provides an RNAi agent inhibiting AGT gene expression.

In some embodiments, RNAi agents include single stranded oligonucleotides or double stranded ribonucleic acid (dsRNA) molecules used to inhibit the expression of the AGT gene in a cell, such as that of a subject (e.g., a mammal, such as a person prone to AGT related disorders such as hypertension). The dsRNA includes an antisense strand with a complementary region complementary to at least part of the mRNA formed in the expression of the AGT gene. The complementary region is about 12-30 nucleotides in length (e.g., about 30, 29, 28, 27, 26, 25, 24, 23, 22, 21, 20, 19, 18, 17, 16, 15, 14, 13, or 12 nucleotides in length).

dsRNA includes two RNA strands, which can be complementary and hybridize to form a duplex structure (complementary region) under the conditions used by dsRNA. One strand (antisense strand) of dsRNA includes a complementary region that is substantially and often completely complementary to the target sequence. The target sequence can be derived from a sequence that forms mRNA during the expression of the AGT gene. The other strand (sense strand) includes a region complementary to the antisense strand, so that when combined under appropriate conditions, the two strands can hybridize and form a duplex structure. Typically, duplex structures are 12 to 30 base pairs in length. Similarly, the length of the region complementary to the target sequence is 12 to 30 nucleotides.

In some embodiments, the dsRNA is about 19 to about 23 nucleotides in length, or about 24 to about 30 nucleotides in length. In general, the dsRNA is long enough to be used as a substrate for Dicer enzymes. For example, it is known in the art that dsRNA with a length greater than about 21-23 nucleotides can be used as a substrate for Dicer. Those skilled in the art also understand that the region of RNA targeted for cleavage is usually part of larger RNA molecules (usually mRNA molecules). A "part" of the target is the consecutive nucleotide of the mRNA target, which is long enough to allow it to be a substrate for RNAi-guided cleavage (i.e., cleavage via RISC pathway).

Those skilled in the art should also understand that the duplex region is the main functional part of dsRNA, for example, the duplex region of about 19 to about 30 base pairs, such as, about 19-30, 19-29, 19-28, 19-27, 19-26, 19-25, 19-24, 19-23, 19-22, 19-21, 19-20 base pairs. Therefore, in one embodiment, in order to achieve the degree of becoming a functional duplex (for example, 15-30 base pairs) that targets the desired RNA for cleavage, RNA molecules or complexes of RNA molecules with duplex regions of more than 30 base pairs are dsRNA.

In one aspect, the RNAi agent or pharmaceutically acceptable salt thereof of the present invention comprises an antisense strand, wherein the antisense strand comprises at least 12 consecutive nucleotides that are substantially complementary to the nucleotides selected from the corresponding positions of the following sequences or a sequence that differs from them by no more than 3 nucleotides: at least 12 consecutive nucleotides with starting positions of 1854-1874, 1907-1927, 1895-1915, 1352-1372, 1903-1923, 2019-2039, 1853-1873 and 1818-1838 in AGT mRNA NM_001382817.3 or a sequence that differs from any of the above by no more than 3 nucleotides, or at least 12 consecutive nucleotides with starting positions of 1822-1842, 1875-1895, 1863-1883, 1320-1340, 1871-1891, 1987-2007, 1821-1841, and 1786-1806 in NM_001384479.1 or a sequence that differs from any of the above by no more than 3 nucleotides.

In some embodiments, the duplex structure (complementary region) formed by the antisense strand and the sense strand comprises at least 12, 13, 14, 15, 16, 17, 18, 19, 20 or 21 consecutive nucleotides.

In some embodiments, the sense strand of the RNAi agent has substantial homology with the target sequences in Table 1.

**Table 1 Target sequences**

| SEQ ID NO. | Single strand code | Target sequence 5'→ 3' | Scope in NM_001382817.3 | Scope in NM_001384479.1 |
|---|---|---|---|---|
| 1 | S1 | gcttgtttgtgaaacaaaaaa | 1854-1874 | 1822-1842 |
| 2 | S2 | cccttgtgttagtaataaacg | 2103-2123 | 2171-2191 |
| 3 | S3 | gttttaaaattaaagtataca | 1904-1924 | 1876-1896 |
| 4 | S4 | gggttttaaaattaaagtata | 1906-1926 | 1874-1894 |
| 5 | S5 | gcttgtgatttttgaacaata | 2023-2043 | 1990-2010 |
| 6 | S6 | aattgggttttaaaattaaag | 1902-1922 | 1870-1890 |
| 7 | S7 | ggttttaaaattaaagtatac | 1907-1927 | 1875-1895 |
| 8 | S8 | gaacaaaaattgggttttaaa | 1895-1915 | 1863-1883 |
| 9 | S9 | ggtgctagtcgctgcaaaact | 336-356 | 304-324 |
| 10 | S10 | gcattttttttgagcttgaag | 1352-1372 | 1320-1340 |
| 11 | S11 | attgggttttaaaattaaagt | 1903-1923 | 1871-1891 |
| 12 | S12 | gatgcttgtgatttttgaaca | 2019-2039 | 1987-2007 |
| 13 | S13 | gagtctacccaacagcttaac | 1393-1403 | 1361-1381 |
| 14 | S14 | cgaccagcttgtttgtgaaac | 1848-1868 | 1816-1836 |
| 15 | S15 | ggagtgacatccaggacaact | 1037-1057 | 1005-1025 |
| 16 | S16 | ccgtgtagtgtctgtaatacc | 1983-2003 | 1951-1971 |
| 17 | S17 | agcttgtttgtgaaacaaaaa | 1853-1873 | 1821-1841 |
| 18 | S18 | cgggactactgttccaaaaag | 1818-1838 | 1786-1806 |

Where, g= guanylate, a= adenylate, t= thymoside, and c= cytidylate.

**Table 2 Antisense strand sequences**

| SEQ ID NO. | Single strand code | Antisense strand sequence 5'→3' |
|---|---|---|
| 19 | AS1 | uuuuguuucacaaacaagcun |
| 20 | AS2 | uuuauuacuaacacaagggan |
| 21 | AS3 | uauacuuuaauuuuaaaaccn |
| 22 | AS4 | uacuuuaauuuuaaaacccan |
| 23 | AS5 | uuguucaaaaaucacaagcan |
| 24 | AS6 | uuaauuuuaaaacccaauuun |
| 25 | AS7 | auacuuuaauuuuaaaacccn |
| 26 | AS8 | uaaaacccaauuuuuguucun |
| 27 | AS9 | uuuugcagcgacuagcaccan |
| 28 | AS10 | ucaagcucaaaaaaaaugcun |
| 29 | AS11 | uuuaauuuuaaaacccaauun |
| 30 | AS12 | uucaaaaaucacaagcaucun |
| 31 | AS13 | uaagcuguuggguagacucun |
| 32 | AS14 | uucacaaacaagcuggucggu |
| 33 | AS15 | uuguccuggaugucacuccan |
| 34 | AS16 | uauuacagacacuacacggan |
| 35 | AS17 | uuuguuucacaaacaagcugn |
| 36 | AS18 | uuuuggaacaguagucccgcn |

Where n is a or u or g or c, g= guanylate, a= adenylate, u= uridylate, c= cytidylate.

In some screening embodiments, the sense and antisense strands of the RNAi agent are selected from the sequences in Table 3 or differ by one, two or three nucleotides from each sequence in Table 3.

**Table 3 Screening RNAi agent sequences**

| SEQ ID NO. | Single strand code | Sense strand sequence 5'→ 3' | SEQ ID NO. | Single strand code | Antisense strand sequence 5'→ 3' | Double strand code |
|---|---|---|---|---|---|---|
| 37 | S19 | gcuuguuugugaaacaaaaaa | 55 | AS19 | uuuuguuucacaaacaagcug | Kylo-09-DS01 |
| 38 | S20 | cccuuguguuaguaauaaacg | 56 | AS20 | uuuauuacuaacacaagggag | Kylo-09-DS02 |
| 39 | S21 | guuuuaaaauuaaaguauaca | 57 | AS21 | uauacuuuaauuuuaaaaccc | Kylo-09-DS03 |
| 40 | S22 | ggguuuuaaaauuaaaguaua | 58 | AS22 | uacuuuaauuuuaaaacccan | Kylo-09-DS04 |
| 41 | S23 | gcuugugauuuuugaacaaua | 59 | AS23 | uuguucaaaaaucacaagcau | Kylo-09-DS05 |
| 42 | S24 | aauuggguuuuaaaauuaaag | 60 | AS24 | uuaauuuuaaaacccaauuuu | Kylo-09-DS06 |
| 43 | S25 | gguuuuaaaauuaaaguauac | 61 | AS25 | auacuuuaauuuuaaaaccca | Kylo-09-DS07 |
| 44 | S26 | gaacaaaaauuggguuuuaaa | 62 | AS26 | uaaaacccaauuuuuguucuc | Kylo-09-DS08 |
| 45 | S27 | ggugcuagucgcugcaaaacu | 63 | AS27 | uuuugcagcgacuagcaccag | Kylo-09-DS09 |
| 46 | S28 | gcauuuuuuuugagcuugaag | 64 | AS28 | ucaagcucaaaaaaaaugcug | Kylo-09-DS10 |
| 47 | S29 | auuggguuuuaaaauuaaagu | 65 | AS29 | uuuaauuuuaaaacccaauuu | Kylo-09-DS11 |
| 48 | S30 | gaugcuugugauuuuugaaca | 66 | AS30 | uucaaaaaucacaagcaucug | Kylo-09-DS12 |
| 49 | S31 | gagucuacccaacagcuuaac | 67 | AS31 | uaagcuguuggguagacucug | Kylo-09-DS13 |
| 50 | S32 | cgaccagcuuguuugugaaac | 68 | AS32 | uucacaaacaagcuggucggu | Kylo-09-DS14 |
| 51 | S33 | ggagugacauccaggacaacu | 69 | AS33 | uuguccuggaugucacuccag | Kylo-09-DS15 |
| 52 | S34 | ccguguagugucuguaauacc | 70 | AS34 | uauuacagacacuacacggag | Kylo-09-DS16 |
| 53 | S35 | agcuuguuugugaaacaaaaa | 71 | AS35 | uuuguuucacaaacaagcugg | Kylo-09-DS17 |
| 54 | S36 | cgggacuacuguuccaaaaag | 72 | AS36 | uuuuggaacaguagucccgcg | Kylo-09-DS18 |

Wherein, g= guanylate, a= adenylate, u= uridylate, and c= cytidylate.

It should be understood that although the sequences in Table 3 are not described as modified or conjugated sequences, the RNA of the iRNA (for example, dsRNA) of the present application may contain any of the sequences shown in Table 3, or the modified sequences of Tables 4, 5 or 6, or the coupled sequences of Tables 13, 14, 16 or 17. In other words, the present application covers unmodified, unconjugated, modified or conjugated dsRNA as described herein.

In some embodiments, the RNAi agent can be added into the cell line through liposome-nucleic acid nanoparticles for sequence screening. Patents US9233971B2, US9080186B2, CN102985548B and CN103189057B related to methods for preparing lipid compounds and liposome-nucleic acid nanoparticles are incorporated into this specification in their entirety.

In some embodiments, the amphoteric lipids in the lipid compounds described therein are preferred macrocyclic lipids D1C1, T1C1, T1C6, T4C4, B2C1, B2C6, B2C7 and M10C1.

The person skilled in the art know that dsRNA with a duplex structure of about 20 to 23 base pairs, for example, 21 base pairs, has been considered to be particularly effective in inducing RNA interference (Elbashir et al., EMBO 2001, 20:6877-6888). However, others have found that shorter or longer RNA duplex structures are also effective (Chu and Rana (2007)RNA14:1714-1719; Kim et al (2005) Nat Biotech 23:222-226). It is reasonable to expect that duplexes that subtract or add a few nucleotides at one or both ends from a sequence in Tables 1, 2, and 3 can be similarly effective compared with the dsRNA. Therefore, dsRNA having a sequence of at least 18, 19, 20, 21 or more consecutive nucleotides derived from one of the sequences in Tables 1, 2 and 3 and having a difference of no more than about 5, 10, 15, 20, 25 or 30% in the ability to inhibit AGT gene expression compared to dsRNA comprising the full sequence are all included within the scope of this invention.

In addition, the RNA provided in Tables 1, 2, and 3 identify sites prone to RISC-mediated cleavage in AGT transcripts. Therefore, the present application further contains an iRNA targeting one of such sites. If iRNA promotes the cleavage of transcripts at any position within the specific site, then iRNA, as used herein, is referred to as targeting RNA transcripts within the specific site. Such iRNA typically includes at least about 12, 13, 14, 15, 16, 17, 18, or 19 consecutive nucleotides from a sequence provided in Tables 1, 2, and 3.

The dsRNA described in this application may further include protruding end of one or more single stranded nucleotide, for example, 1, 2, 3 or 4 nucleotides. dsRNAs with protruding end of at least one nucleotide can have superior inhibitory properties than their blunt ended counterparts. The nucleotide protruding end may comprise nucleotide / nucleoside analogues or their combination, including deoxynucleotides / nucleosides. The protruding end may be on the sense strand, the antisense strand, or any combination thereof. In addition, the nucleotides on protruding end can exist at the 5'- end, 3'- end or both ends of the antisense or sense strand of dsRNA. The protruding end can be caused by one strand being longer than the other, or by two strands of the same length interleaving. The protruding end may form a mismatch with the target mRNA or it may be complementary to the targeted gene sequence or may be another sequence.

dsRNA can also contain only a single protruding end, which can enhance the interference activity of RNAi agents without affecting its overall stability. For example, the single strand protruding end can be located at the 3'- end of the sense strand, or alternatively, at the 3'- end of the antisense strand. RNAi agents can also have blunt end, located at the 5'- end of the antisense strand (or the 3'- end of the sense strand), and vice versa. Generally, the antisense strand of RNAi agents has a nucleotide protruding end at the 3'- end and a blunt end at the 5'- end. Although it is not desirable to be bound by theory, the asymmetric blunt end of the 5'- end of the antisense strand and protruding end of the 3'- end of the antisense strand are conducive to guide the strand into the RISC process.

In some embodiments, the protruding end exists at the 3'- end of the sense strand, the antisense strand, or both strands. In some embodiments, this 3'- protruding end is present in the antisense strand. In some embodiments, this 3'- protruding end exists in the sense strand.

In some embodiments, the dsRNA has a length of 21 nucleotide and is blunt ended on both ends.

In some embodiments, the dsRNA has a length of 21 nucleotides, and both the sense strand and antisense strand have protruding end of 2 nucleotide at the 3 'end.

### Modified RNAi agents

In order to enhance the stability of the RNAi agent described in the present application in vivo, the sense strand and antisense strand of the RNAi agent can be modified without affecting its activity or even enhancing its activity, and the nucleotide therein can have a modification group to modify the whole strand or part of it. In some embodiments, one or more nucleotides on the sense strand and / or antisense strand are modified to form modified nucleotides.

In some embodiments, the RNA of the RNAi agent (e.g., dsRNA) of the present application is unmodified and does not include, for example, chemical modification or conjugation known in the art and described herein. In other embodiments, the RNA of the RNAi agent (e.g., dsRNA) of the present application is chemically modified to enhance stability or other favorable properties. In other embodiments of the application, all nucleotides or basically all nucleotides of the RNAi agent of the application are modified, that is, no more than 5, 4, 3, 2 or 1 unmodified nucleotide exists in the strand of the RNAi agent.

Nucleic acids described in the present application can be synthesized and / or modified by methods well known in the art, such as those described in "Current protocols in nucleic acid chemistry", Beaucage, S.L. et al. (eds.), JohnWiley & Sons, Inc, New York, NY, USA, which is incorporated herein by reference. Modification includes, for example, end modification, such as, 5'-end modification (phosphorylation, conjugation, reverse ligation) or 3'- end modifications (conjugation, DNA nucleotide, reverse ligation, etc.); base modification, such as replacing, removing bases (abasic nucleotides) or conjugating bases with stabilized bases, destabilized bases, or bases paired with an expanded repertoire of partners; sugar modification (e.g., 2'- or 4' - position) or sugar substitution; or backbone modifications, including modification or substitution of phosphodiester linkages. In the RNAi agent provided by this application, both the sense strand and antisense strand of the RNAi agent do not need uniform modification, and one or more modifications can be incorporated into a single nucleotide therein.

In some embodiments, the modified nucleotides are selected from the group consisting of deoxyribonucleotides, nucleotide mimics, abasic nucleotides, 2'- modified nucleotides, 3' -3'- linkage (inverted) nucleotides, nucleotides containing unnatural bases, bridged nucleotides, peptide nucleic acids (PNA), unlocked nucleobase analogues, locked nucleotides, 3'-O-methoxy (2' inter-nucleoside linkage) nucleotides, 2'-F-arabinose nucleotides, 5'-Me / 2'- Fluoro- nucleotides, morpholino nucleotides, vinyl phosphonate deoxyribonucleotides, nucleotides containing vinyl phosphonate and nucleotides containing cyclopropyl phosphonate.

In some embodiments, the 2'- modified nucleotide includes: 2'-O-methyl nucleotide, 2'-deoxy-2'-fluoronucleotide, 2'-deoxynucleotide, 2'-methoxyethyl nucleotide, 2'-amino nucleotide and / or 2'-alkyl nucleotide.

In some embodiments, at least one 2' positions of the nucleotide glycosyls at positions 7, 12 and 14 from the 5' end of the antisense strand is fluorine. For example, the 2' positions of nucleotide glycosyls at positions 7, 12 and 14 from the 5' end of the antisense strand are all fluorine.

In some embodiments, except for the nucleotides at positions 7, 12, and 14 starting from the 5' end of the antisense strand, at least one of the 2' positions of the remaining nucleotide glycosyls is methoxy.

In some embodiments, at least one 2' positions of the nucleotide glycosyls at positions 5, 7, 8 and 9 starting from the 5' end of the sense strand is fluorine. For example, the 2' positions of nucleotide glycosyls at positions 5, 7, 8 and 9 from the 5' end of the sense strand are all fluorine.

In some embodiments, except for the nucleotides at positions 5, 7, 8 and 9 starting from the 5' end of the sense strand, at least one of the 2' positions of the remaining nucleotide glycosyls is methoxy.

For example, some or all of the -OH at 2' positions of the nucleotide glycosyls of the sense strand or antisense strand can be substituted, wherein the substituent group is fluorine or methoxy, preferably 2' positions of the nucleotides at positions 5, 7, 8 and 9 from the 5' end of the sense strand are fluorine, and 2' positions of the nucleotides at positions 7, 12 and 14 from the 5' end of the antisense strand are fluorine, and 2' positions of the remaining nucleotides are methoxy.

For example, the 2' positions of nucleotide glycosyls at positions 7, 12 and 14 from the 5' end of the antisense strand are all fluorine, and the 2' positions of the remaining nucleotide glycosyls are all methoxy.

For example, the 2' positions of nucleotide glycosyls at positions 5, 7, 8, and 9 from the 5' end of the sense strand are all fluorine, and the 2' positions of the remaining nucleotide glycosyls are all methoxy.

In some embodiments, there are at least two consecutive phosphorothioate linkages among the nucleotides of the sense strand and / or antisense strand.

In some embodiments, there are at least two consecutive phosphorothioate linkages among three consecutive nucleotides at the end of the sense strand and / or the end of the antisense strand.

For example, there are at least two consecutive phosphorothioate linkages among three consecutive nucleotides at the 5' and 3' ends of the sense and antisense strands.

For another example, the 2' positions of nucleotides at positions 5, 7, 8, and 9 starting from the 5' end of the sense strand are fluorine, and the 2' positions of nucleotides at positions 7, 12, and 14 starting from the 5' end of the antisense strand are fluorine, and 2' positions of the remaining nucleotides are methoxy, and there are at least two consecutive phosphorothioate linkages among the 3 consecutive nucleotides at the 5' end and the 3 'end of the sense strand and the antisense strand.

In some screening embodiments, the sense and antisense strands of the RNAi agent are selected from the sequences in Table 4 or differ by one, two or three nucleotides from each sequence in Table 4.

**Table 4 Screening RNA inhibitor sequences**

| SEQ ID NO. | Single strand code | Sense strand sequence 5'→ 3' | SEQ ID NO. | Single strand code | Antisense strand sequence 5'→ 3' | Double strand code |
|---|---|---|---|---|---|---|
| 73 | S37 | GcuuGuuuGuGAAACAAAAAA | 91 | AS37 | UUUuGUUUcAcAAAcAAGCuG | Kylo-09-DS19 |
| 74 | S38 | cccuuGuGuuAGuAAuAAAcG | 92 | AS38 | UUuAUuACuAAcACAAGGGAG | Kylo-09-DS20 |
| 75 | S39 | GuuuuAAAAuuAAAGuAuAcA | 93 | AS39 | uAuACUUUAAUUUUAAAACCC | Kylo-09-DS21 |
| 76 | S40 | GGGuuuuAAAAuuAAAGuAuA | 94 | AS40 | uACUUuAAUUUuAAAACCCAA | Kylo-09-DS22 |
| 77 | S41 | GCuuGuGAuuuuuGAACAAuA | 95 | AS41 | UuGUUcAAAAAUcAcAAGcAU | Kylo-09-DS23 |
| 78 | S42 | AAuuGGGuuuuAAAAuuAAAG | 96 | AS42 | UuAAUUUuAAAACCcAAUUUU | Kylo-09-DS24 |
| 79 | S43 | GuuuuAAAAuuAAAGuAuAc | 97 | AS43 | AuACUUuAAUUUuAAAACCCA | Kylo-09-DS25 |
| 80 | S44 | GAAcAAAAAuuGGGuuuuAAA | 98 | AS44 | uAAAACCcAAUUUUuGUUCUC | Kylo-09-DS26 |
| 81 | S45 | GuGcuAGucGcuGcAAAAcu | 99 | AS45 | UUUuGcAGCGACuAGcACcAG | Kylo-09-DS27 |
| 82 | S46 | GcAuuuuuuuuGAGcuuGAAG | 100 | AS46 | UcAAGCUcAAAAAAAAuGCuG | Kylo-09-DS28 |
| 83 | S47 | AuuGGGuuuuAAAAuuAAAGu | 101 | AS47 | UUuAAUUUuAAAACCcAAUUU | Kylo-09-DS29 |
| 84 | S48 | GAuGcuuGuGAuuuuuGAAcA | 102 | AS48 | UUcAAAAAuCAcAAGcAUCuG | Kylo-09-DS30 |
| 85 | S49 | GAGucuAcccAAcAGcUUAAc | 103 | AS49 | UAAGCUGUUGGGUAGACUCUG | Kylo-09-DS31 |
| 86 | S50 | cGAccAGcuuGuuuGuGAAAc | 104 | AS50 | UUcAcAAAcAAGCUGGUCGGU | Kylo-09-DS32 |
| 87 | S51 | GGAGuGAcAuccAGGAcAAcu | 105 | AS51 | UuGUCCuGGAuGUCACUCCAG | Kylo-09-DS33 |
| 88 | S52 | ccGuGuAGuGuCuGuAAuAcc | 106 | AS52 | uAUuAcAGACACuAcACGGAG | Kylo-09-DS34 |
| 89 | S53 | AGcuuGuuuGuGAAAcAAAAA | 107 | AS53 | UUuGUUUcAcAAACAAGCuGG | Kylo-09-DS35 |
| 90 | S54 | cGGGAcuAcuGuuccAAAAAG | 108 | AS54 | UUUuGGAAcAGuAGUCCCGCG | Kylo-09-DS36 |

wherein, G=2'-methoxyguanylate, A=2'-methoxyadenylate, U=2'-methoxyuridylate, C=2'-methoxycytidylate; u= uridylate, and c= cytidylate.

In some embodiments, the antisense strand in the RNAi agent is selected from the sequences in Table 5 below. The 2' positions of nucleotides at positions 7, 12, and 14 starting from the 5' end of the antisense strand are fluorine, and 2' positions of the remaining nucleotides are methoxy, and the phosphate bond among at least three adjacent nucleotides at the end of the antisense strand can be thioated.

**Table 5 Modified sequences of antisense strand**

| SEQ ID NO. | Single strand code | Antisense strand sequence 5'→ 3' |
|---|---|---|
| SEQ ID NO. | 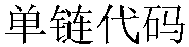 | 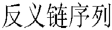 5'→3' |
| 109 | AS55 | UsUsUUGUfUUCACfAAfACAAGCsUsG |
| 110 | AS56 | UsUsUAUUfACUAAfCAfCAAGGGsAsG |
| 111 | AS57 | UsAsUACUfUUAAUfUUfUAAAACsCsC |
| 112 | AS58 | UsAsCUUUfAAUUUfUAfAAACCCsAsA |
| 113 | AS59 | UsUsGUUCfAAAAAfUCfACAAGCsAsU |
| 114 | AS60 | UsUsAAUUfUUAAAfACfCCAAUUsUsU |
| 115 | AS61 | AsUsACUUfUAAUUfUUfAAAACCsCsA |
| 116 | AS62 | UsAsAAACfCCAAUfUUfUUGUUCsUsC |
| 117 | AS63 | UsUsUUGCfAGCGAfCUfAGCACCsAsG |
| 118 | AS64 | UsCsAAGCfUCAAAfAAfAAAUGCsUsG |
| 119 | AS65 | UsUsUAAUfUUUAAfAAfCCCAAUsUsU |
| 120 | AS66 | UsUsCAAAfAAUCAfCAfAGCAUCsUsG |
| 121 | AS67 | UsAsAGCUfGUUGGfGUfAGACUCsUsG |
| 122 | AS68 | UsUsCACAfAACAAfGCfUGGUCGsGsU |
| 123 | AS69 | UsUsGUCCfUGGAUfGUfCACUCCsAsG |
| 124 | AS70 | UsAsUUACfAGACAfCUfACACGGsAsG |
| 125 | AS71 | UsUsUGUUfUCACAfAAfCAAGCUsGsG |
| 126 | AS72 | UsUsUUGGfAACAGfUAfGUCCCGsCsG |
| 127 | AS73 | AsAsGAAGfUUGGCfCAfGCAUsCsC |
| 128 | AS74 | UsAsUACGfGAAGCfCCfAAGAsAsG |
| 129 | AS75 | CsUsGUGCfAUGCCfAUfAUAUsAsC |
| 130 | AS76 | CsAsUGGAfCCACGfCCfCCAUsAsG |
| 131 | AS77 | AsAsAGACfAGCCGfUUfGGGGsAsG |
| 132 | AS78 | UsCsUUGUfCCACCfCAfGAACsUsC |
| 133 | AS79 | AsGsACCCfUCCACfCUfUGUCsCsA |
| 134 | AS80 | AsGsUGAGfACCCUfCCfACCUsUsG |
| 135 | AS81 | AsAsAGUGfAGACCfCUfCCACsCsU |
| 136 | AS82 | GsUsUGAGfGGAGUfUUfUGCUsGsG |
| 137 | AS83 | AsGsUUGAfGGGAGfUUfUUGCsUsG |
| 138 | AS84 | UsCsCAGUfUGAGGfGAfGUUUsUsG |
| 139 | AS85 | UsCsUUCAfUCCAGfUUfGAGGsGsA |
| 140 | AS86 | UsUsCUUCfAUCCAfGUfUGAGsGsG |
| 141 | AS87 | AsGsUUUCfUUCAUfCCfAGUUsGsA |
| 142 | AS88 | UsUsGCUCfAAUUUfUUfGCAGsGsU |
| 143 | AS89 | CsAsUUGCfUCAAUfUUfUUGCsAsG |
| 144 | AS90 | UsCsAUUGfCUCAAfUUfUUUGsCsA |
| 145 | AS91 | GsUsCAUUfGCUCAfAUfUUUUsGsC |
| 146 | AS92 | UsGsUGGGfCUCUCfUCfUCAUsCsC |
| 147 | AS93 | UsUsGAUCfAUACAfCAfGCAAsAsC |
| 148 | AS94 | UsUsUGAUfCAUACfACfAGCAsAsA |
| 149 | AS95 | AsAsAGGUfGGGAGfACfUGGGsGsG |
| 150 | AS96 | CsAsUUAGfAAGAAfAAfGGUGsGsG |
| 151 | AS97 | UsCsAUUAfGAAGAfAAfAGGUsGsG |
| 152 | AS98 | CsUsCAUUfAGAAGfAAfAAGGsUsG |
| 153 | AS99 | UsCsGGUUfGGAAUfUCfUUUUsUsG |
| 154 | AS100 | AsAsACAAfGCUGGfUCfGGUUsGsG |
| 155 | AS101 | UsCsACAAfACAAGfCUfGGUCsGsG |
| 156 | AS102 | UsUsCACAfAACAAfGCfUGGUsCsG |
| 157 | AS103 | UsUsUCACfAAACAfAGfCUGGsUsC |
| 158 | AS104 | UsUsUUGUfUUCACfAAfACAAsGsC |
| 159 | AS105 | UsUsUUUGfUUUCAfCAfAACAsAsG |
| 160 | AS106 | UsUsUUUUfGUUUCfACfAAACsAsA |
| 161 | AS107 | AsCsUUUUfUUGUUfUCfACAAsAsC |
| 162 | AS108 | AsCsACUUfUUUUGfUUfUCACsAsA |
| 163 | AS109 | AsAsAAGGfGAACAfCUfUUUUsUsG |
| 164 | AS110 | UsCsUCAAfCUUGAfAAfAGGGsAsA |
| 165 | AS111 | UsGsUUCUfCAACUfUGfAAAAsGsG |
| 166 | AS112 | AsAsCCCAfAUUUUfUGfUUCUsCsA |
| 167 | AS113 | UsAsAAACfCCAAUfUUfUUGUsUsC |
| 168 | AS114 | UsUsUUAAfAACCCfAAfUUUUsUsG |
| 169 | AS115 | AsUsUCAAfGACACfUAfAAUAsCsA |
| 170 | AS116 | UsCsUUACfAUUCAfAGfACACsUsA |
| 171 | AS117 | UsCsAUGUfUCUUAfCAfUUCAsAsG |
| 172 | AS118 | GsUsCAUGfUUCUUfACfAUUCsAsA |
| 173 | AS119 | AsUsCUGUfGGAAAfAAfACUAsAsG |
| 174 | AS120 | AsAsAUCAfCAAGCfAUfCUGUsGsG |
| 175 | AS121 | AsAsAAAUfCACAAfGCfAUCUsGsU |
| 176 | AS122 | CsGsGACAfAAUCAfGCfGAUGUsGsU |
| 177 | AS123 | CsCsAAAAfAGAAUfUCfCAAUUsGsA |
| 178 | AS124 | AsCsCGACfCAGCUfUGfUUUGUsGsA |
| 179 | AS125 | AsGsCGCGfGGACUfACfUGUUCsCsA |
| 180 | AS126 | GsCsGCGGfGACUAfCUfGUUCCsAsA |
| 181 | AS127 | AsAsCCGAfCCAGCfUUfGUUUGsUsG |
| 182 | AS128 | UsGsUUCCfCUUUUfCAfAGUUGsAsG |
| 183 | AS129 | UsCsCCUUfUUCAAfGUfUGAGAsAsC |
| 184 | AS130 | UsAsUACUfCUCAUfUGfUGGAUGsAsC |

wherein, G=2'-O-methylguanylate, A=2'-O-methyladenylate, U=2'-O-methyluridylate, C=2'-O-methylcytidylate, fG=2'-fluoroguanylate, fA=2'-fluoroadenylate, fU=2'-fluorouridylate, fC=2'-fluorocytidylate, Gs=2'-O-methyl-3'-thioguanylate, As=2'-O-methyl-3'-thioadenosine, Us=2'-O-methyl-3'-thiouridylate, Cs=2'-O-methyl-3'-thiocytidylate.

In some embodiment schemes, the sense strand in the RNAi agent is selected from the sequences in Table 6 below. The 2' positions of nucleotides at positions 5, 7, 8, and 9 starting from the 5' end of the sense strand are fluorine, and the 2' positions of remaining nucleotides are methoxy, and the phosphate bond among at least three adjacent nucleotides at the end of the antisense strand can be thioated.

**Table 6 Modified sequences of sense strand**

| SEQ ID NO. | Single strand code | Sense strand sequence 5'→ 3' |
|---|---|---|
| 185 | S55 | GsCsUUfGUfUfUfGUGAAACAAAAsAsA |
| 186 | S56 | CsCsCUfUGfUfGfUUAGUAAUAAAsCsG |
| 187 | S57 | GsUsUUfUAfAfAfAUUAAAGUAUAsCsA |
| 188 | S58 | GsGsGUfUUfUfAfAAAUUAAAGUAsUsA |
| 189 | S59 | GsCsUUfGUfGfAfUUUUUGAACAAsUsA |
| 190 | S60 | AsAsUUfGGfGfUfUUUAAAAUUAAsAsG |
| 191 | S61 | GsGsUUfUUfAfAfAAUUAAAGUAUsAsC |
| 192 | S62 | GsAsACfAAfAfAfAUUGGGUUUUAsAsA |
| 193 | S63 | GsGsUGfCUfAfGfUCGCUGCAAAAsCsU |
| 194 | S64 | GsCsAUfUUfUfUfUUUGAGCUUGAsAsG |
| 195 | S65 | AsUsUGfGGfUfUfUUAAAAUUAAAsGsU |
| 196 | S66 | GsAsUGfCUfUfGfUGAUUUUUGAAsCsA |
| 197 | S67 | GsAsGUfCUfAfCfCCAACAGCUUAsAsC |
| 198 | S68 | CsGsACfCAfGfCfUUGUUUGUGAAsAsC |
| 199 | S69 | GsGsAGfUGfAfCfAUCCAGGACAAsCsU |
| 200 | S70 | CsCsGUfGUfAfGfUGUCUGUAAUAsCsC |
| 201 | S71 | AsGsCUfUGfUfUfUGUGAAACAAAsAsA |
| 202 | S72 | CsGsGGfACfUfAfCUGUUCCAAAAsAsG |
| 203 | S73 | GsGsAUfGCfUfGfGCCAACUUCsUsU |
| 204 | S74 | CsUsUCfUUfGfGfGCUUCCGUAsUsA |
| 205 | S75 | GsUsAUfAUfAfUfGGCAUGCACsAsG |
| 206 | S76 | CsUsAUfGGfGfGfCGUGGUCCAsUsG |
| 207 | S77 | CsUsCCfCCfAfAfCGGCUGUCUsUsU |
| 208 | S78 | GsCsUGfUGfAfCfAGGAUGGAAsGsA |
| 209 | S79 | UsGsGAfCAfAfGfGUGGAGGGUsCsU |
| 210 | S80 | CsAsAGfGUfGfGfAGGGUCUCAsCsU |
| 211 | S81 | AsGsGUfGGfAfGfGGUCUCACUsUsU |
| 212 | S82 | CsCsAGfCAfAfAfACUCCCUCAsAsC |
| 213 | S83 | CsAsGCfAAfAfAfCUCCCUCAAsCsU |
| 214 | S84 | CsAsAAfACfUfCfCCUCAACUGsGsA |
| 215 | S85 | UsCsCCfUCfAfAfCUGGAUGAAsGsA |
| 216 | S86 | CsCsCUfCAfAfCfUGGAUGAAGsAsA |
| 217 | S87 | UsCsAAfCUfGfGfAUGAAGAAAsCsU |
| 218 | S88 | AsCsCUfGCfAfAfAAAUUGAGCsAsA |
| 219 | S89 | CsUsGCfAAfAfAfAUUGAGCAAsUsG |
| 220 | S90 | UsGsCAfAAfAfAfUUGAGCAAUsGsA |
| 221 | S91 | GsCsAAfAAfAfUfUGAGCAAUGsAsC |
| 222 | S92 | GsGsAUfGAfGfAfGAGAGCCCAsCsA |
| 223 | S93 | GsUsUUfGCfUfGfUGUAUGAUCsAsA |
| 224 | S94 | UsUsUGfCUfGfUfGUAUGAUCAsAsA |
| 225 | S95 | CsCsCCfCAfGfUfCUCCCACCUsUsU |
| 226 | S96 | CsCsCAfCCfUfUfUUCUUCUAAsUsG |
| 227 | S97 | CsCsACfCUfUfUfUCUUCUAAUsGsA |
| 228 | S98 | CsAsCCfUUfUfUfCUUCUAAUGsAsG |
| 229 | S99 | CsAsAAfAAfGfAfAUUCCAACCsGsA |
| 230 | S100 | CsCsAAfCCfGfAfCCAGCUUGUsUsU |
| 231 | S101 | CsCsGAfCCfAfGfCUUGUUUGUsGsA |
| 232 | S102 | CsGsACfCAfGfCfUUGUUUGUGsAsA |
| 233 | S103 | GsAsCCfAGfCfUfUGUUUGUGAsAsA |
| 234 | S104 | GsCsUUfGUfUfUfGUGAAACAAsAsA |
| 235 | S105 | CsUsUGfUUfUfGfUGAAACAAAsAsA |
| 236 | S106 | UsUsGUfUUfGfUfGAAACAAAAsAsA |
| 237 | S107 | GsUsUUfGUfGfAfAACAAAAAAsGsU |
| 238 | S108 | UsUsGUfGAfAfAfCAAAAAAGUsGsU |
| 239 | S109 | CsAsAAfAAfAfGfUGUUCCCUUsUsU |
| 240 | S110 | UsUsCCfCUfUfUfUCAAGUUGAsGsA |
| 241 | S111 | CsCsUUfUUfCfAfAGUUGAGAAsCsA |
| 242 | S112 | UsGsAGfAAfCfAfAAAAUUGGGsUsU |
| 243 | S113 | GsAsACfAAfAfAfAUUGGGUUUsUsA |
| 244 | S114 | CsAsAAfAAfUfUfGGGUUUUAAsAsA |
| 245 | S115 | UsGsUAfUUfUfAfGUGUCUUGAsAsU |
| 246 | S116 | UsAsGUfGUfCfUfUGAAUGUAAsGsA |
| 247 | S117 | CsUsUGfAAfUfGfUAAGAACAUsGsA |
| 248 | S118 | UsUsGAfAUfGfUfAAGAACAUGsAsC |
| 249 | S119 | CsUsUAfGUfUfUfUUUCCACAGsAsU |
| 250 | S120 | CsCsACfAGfAfUfGCUUGUGAUsUsU |
| 251 | S121 | AsCsAGfAUfGfCfUUGUGAUUUsUsU |
| 252 | S122 | AsCsACfAUfCfGfCUGAUUUGUCsCsG |
| 253 | S123 | UsCsGGfUUfGfGfAAUUCUUUUUsGsG |
| 254 | S124 | UsCsACfAAfAfCfAAGCUGGUCGsGsU |
| 255 | S125 | UsGsGAfACfAfGfUAGUCCCGCGsCsU |
| 256 | S126 | UsUsGGfAAfCfAfGUAGUCCCGCsGsC |
| 257 | S127 | CsAsCAfAAfCfAfAGCUGGUCGGsUsU |
| 258 | S128 | CsUsCAfACfUfUfGAAAAGGGAAsCsA |
| 259 | S129 | GsUsUCfUCfAfAfCUUGAAAAGGsGsA |
| 260 | S130 | GsUsCAfUCfCfAfCAAUGAGAGUAsCsA |

In some embodiments, the sense strand of the RNAi agent described in this application differs from each sequence in Table 6 by one, two or three nucleotides.

In some embodiments, the combination of sense strand and antisense strand in the RNAi agent is selected from Table 7.

**Table 7 RNAi agents**

| Sense strand code | Antisense strand code | Double strand code |
|---|---|---|
| S55 | AS55 | Kylo-09-DS37 |
| S56 | AS56 | Kylo-09-DS38 |
| S57 | AS57 | Kylo-09-DS39 |
| S58 | AS58 | Kylo-09-DS40 |
| S59 | AS59 | Kylo-09-DS41 |
| S60 | AS60 | Kylo-09-DS42 |
| S61 | AS61 | Kylo-09-DS43 |
| S62 | AS62 | Kylo-09-DS44 |
| S63 | AS63 | Kylo-09-DS45 |
| S64 | AS64 | Kylo-09-DS46 |
| S65 | AS65 | Kylo-09-DS47 |
| S66 | AS66 | Kylo-09-DS48 |
| S67 | AS67 | Kylo-09-DS49 |
| S68 | AS68 | Kylo-09-DS50 |
| S69 | AS69 | Kylo-09-DS51 |
| S70 | AS70 | Kylo-09-DS52 |
| S71 | AS71 | Kylo-09-DS53 |
| S72 | AS72 | Kylo-09-DS54 |
| S73 | AS73 | Kylo-09-DS55 |
| S74 | AS74 | Kylo-09-DS56 |
| S75 | AS75 | Kylo-09-DS57 |
| S76 | AS76 | Kylo-09-DS58 |
| S77 | AS77 | Kylo-09-DS59 |
| S78 | AS78 | Kylo-09-DS60 |
| S79 | AS79 | Kylo-09-DS61 |
| S80 | AS80 | Kylo-09-DS62 |
| S81 | AS81 | Kylo-09-DS63 |
| S82 | AS82 | Kylo-09-DS64 |
| S83 | AS83 | Kylo-09-DS65 |
| S84 | AS84 | Kylo-09-DS66 |
| S85 | AS85 | Kylo-09-DS67 |
| S86 | AS86 | Kylo-09-DS68 |
| S87 | AS87 | Kylo-09-DS69 |
| S88 | AS88 | Kylo-09-DS70 |
| S89 | AS89 | Kylo-09-DS71 |
| S90 | AS90 | Kylo-09-DS72 |
| S91 | AS91 | Kylo-09-DS73 |
| S92 | AS92 | Kylo-09-DS74 |
| S93 | AS93 | Kylo-09-DS75 |
| S94 | AS94 | Kylo-09-DS76 |
| S95 | AS95 | Kylo-09-DS77 |
| S96 | AS96 | Kylo-09-DS78 |
| S97 | AS97 | Kylo-09-DS79 |
| S98 | AS98 | Kylo-09-DS80 |
| S99 | AS99 | Kylo-09-DS81 |
| S100 | AS100 | Kylo-09-DS82 |
| S101 | AS101 | Kylo-09-DS83 |
| S102 | AS102 | Kylo-09-DS84 |
| S103 | AS103 | Kylo-09-DS85 |
| S104 | AS104 | Kylo-09-DS86 |
| S105 | AS105 | Kylo-09-DS87 |
| S106 | AS106 | Kylo-09-DS88 |
| S107 | AS107 | Kylo-09-DS89 |
| S108 | AS108 | Kylo-09-DS90 |
| S109 | AS109 | Kylo-09-DS91 |
| S110 | AS110 | Kylo-09-DS92 |
| S111 | AS111 | Kylo-09-DS93 |
| S112 | AS112 | Kylo-09-DS94 |
| S113 | AS113 | Kylo-09-DS95 |
| S114 | AS114 | Kylo-09-DS96 |
| S115 | AS115 | Kylo-09-DS97 |
| S116 | AS116 | Kylo-09-DS98 |
| S117 | AS117 | Kylo-09-DS99 |
| S118 | AS118 | Kylo-09-DS100 |
| S119 | AS119 | Kylo-09-DS101 |
| S120 | AS120 | Kylo-09-DS102 |
| S121 | AS121 | Kylo-09-DS103 |
| S122 | AS122 | Kylo-09-DS104 |
| S123 | AS123 | Kylo-09-DS105 |
| S124 | AS124 | Kylo-09-DS106 |
| S125 | AS125 | Kylo-09-DS107 |
| S126 | AS126 | Kylo-09-DS108 |
| S127 | AS127 | Kylo-09-DS109 |
| S128 | AS128 | Kylo-09-DS110 |
| S129 | AS129 | Kylo-09-DS111 |
| S130 | AS130 | Kylo-09-DS112 |

### RNAi agents conjugated to carrier

Another aspect of the RNAi agent of the present application relates to a method to conjugate interfering nucleic acids to carrier to enhance the stability, activity, cellular distribution or cellular uptake of RNAi agents.

In some embodiments, the distribution, targeting or stability of RNAi agents are changed by introducing ligands of target tissue receptors into the carrier. For example, specific ligands can provide enhanced affinity for selected targets, such as molecules, cells or cell types, compartments (such as cell or organ compartments, body tissues, organs, or regions), compared with species without ligands.

Ligands can include naturally occurring substances, such as proteins (such as human serum albumin (HSA), low-density lipoprotein (LDL), or globulin); carbohydrates (such as dextran, pullulan, chitin, chitosan, inulin, cyclodextrin, N-acetylglucosamine, N-acetylgalactosamine, or hyaluronic acid); or lipids. Ligands can also be recombinant or synthetic molecules, such as synthetic polymers, such as synthetic polyamino acids.

Ligands may also include targeting groups, such as cell or tissue targeting agents that bind to a specified cell type such as kidney cells, such as lectins, glycoproteins, lipids, or proteins, such as antibodies. The targeting groups can be thyrotropin, melanotropin, lectin, glycoprotein, surfactant protein A, mucin carbohydrate, multivalent lactose, multivalent galactose, N-acetyl-galactosamine, N-acetyl-glucosamine multivalent mannose, multivalent fucose, glycosylated polyamino acids, multivalent galactose, transferrin, bisphosphonates, polyglutamic acid, polyaspartic acid, lipids, cholesterol, steroids, cholic acid, folic acid, vitamin B 12, vitamin A, biotin, or RGD peptide or RGD peptide mimetics. In some embodiments, the ligand is a multivalent galactose, for example, N-acetyl-galactosamine.

The sense strand and antisense strand contained in the RNAi agent of the application can be conveniently and routinely prepared by the well-known technology of solid-phase synthesis. Any other methods known in the art for such synthesis, such as liquid-phase synthesis or fermentation, may be used additionally or alternatively. It is also known to use similar techniques to prepare other oligonucleotides, such as phosphorothioates and alkylated derivatives.

In some embodiments, in addition to standard nucleoside phosphoramidite monomers and non-standard nucleoside phosphoramidite monomers that are commercially available and routinely used in oligonucleotide synthesis, the oligonucleotides or linked nucleotides of the present application can be synthesized by an automated synthesizer using phosphoramidite method derived from the carrier-nucleoside phosphoramidite monomer.

In some embodiments, the ligand conjugation method described in the present invention is conjugated to the 5' and / or 3' ends of the antisense strand, and / or the 5' end and / or the 3' end of the sense strand.

For example, the carrier structure can be conjugated to the 5' end and / or 3' end of the sense strand; or the carrier structure can be conjugated to the 5' end of the antisense strand and the carrier structure is coupled to the 3' end of the sense strand; or the carrier structure can be conjugated to the 3' end of the antisense strand, and the ligand is conjugated to the 5' end of the sense strand; or the carrier structure is conjugated to the 5' and 3' ends of the sense strand.

In some embodiments, the carrier structure includes 5'MVIP and 3'MVIP, wherein the 5'MVIP is conjugated at the 5' end of the sense strand and / or antisense strand, the 3'MVIP is conjugated at the 3' end of the antisense strand and / or sense strand, the structure of the 5'MVIP is shown in formula I, the structure of the 3'MVIP is shown in formula II,

**(X-L)n-B-D-R₁⁻,** I

**(X-L)m-B-D-R₂⁻**, II

wherein,
X is the targeting specific ligand;
L is the branched chain;
B is the linker;
D is the linking chain;
R₁ and R₂ are transition points;

The 5'MVIP is connected with the 5' end of the sense strand or the 5' end of the antisense strand through the transition point Ri, and the 3'MVIP is connected with the 3' end of the sense strand or the 3' end of the antisense strand through the transition point R₂. n and m are each independently any integer from 0 to 4.

In some embodiments, the X is a tissue-specific targeting ligand.

In some embodiments, the X is liver targeting specific ligand.

In some embodiments, the R₁ or R₂ is connected to the sense or antisense strand through a phosphate or modified phosphate, preferably through a phosphate or phosphorothioate.

In some embodiments, n+m is an integer of 2-6, preferably n+m=2, 3 or 4, more preferably 4.

In some embodiments, m or n can be 0, that is, there is no 3'MVIP or 5'MVIP.

In some embodiments, when n=0 (that is, there is no 5'MVIP), the structure of the 3'MVIP can be:

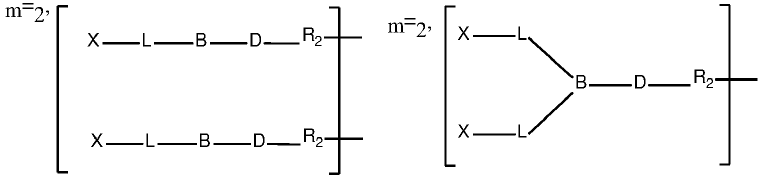

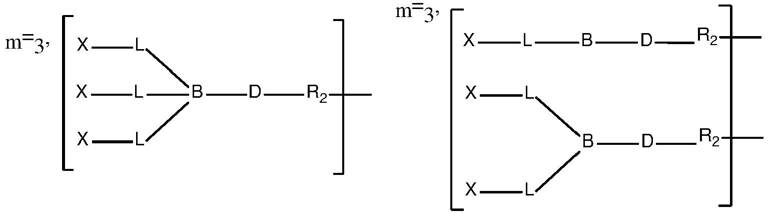

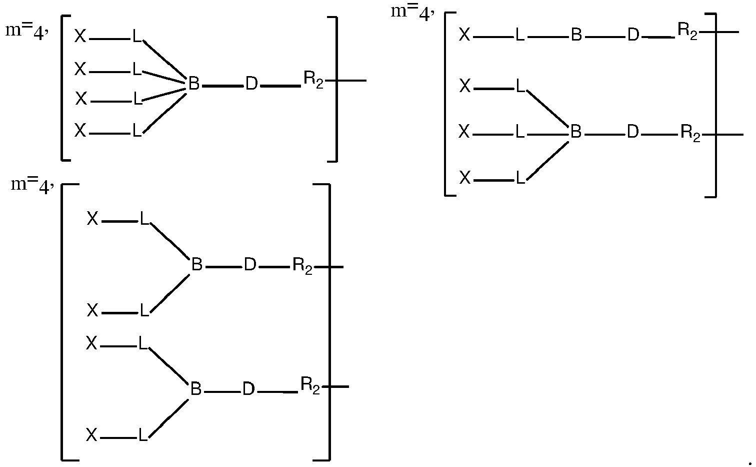

In some embodiments, when n=1, the structure of the 3'MVIP can be:

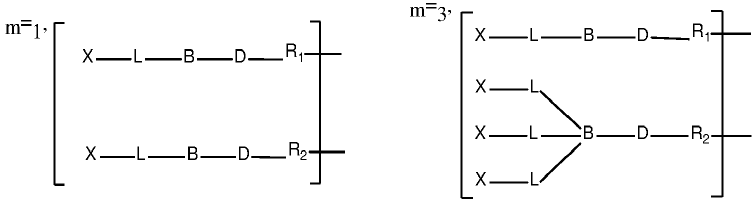

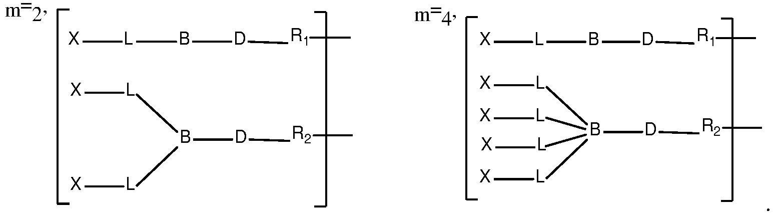

In some embodiments, when n=2, the structure of the 3'MVIP can be:

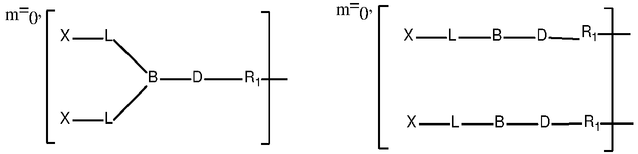

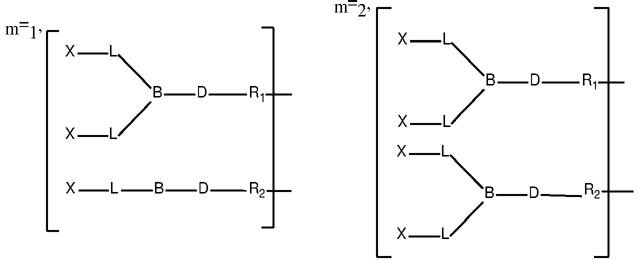

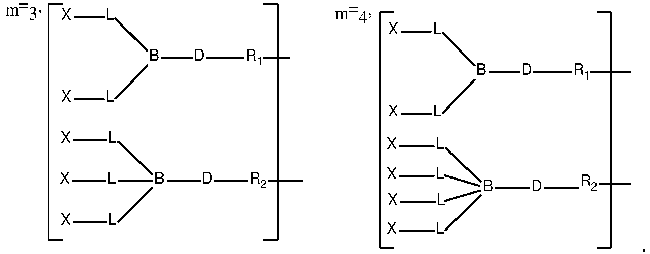

In some embodiments, when n=3, the structure of the 3'MVIP can be:

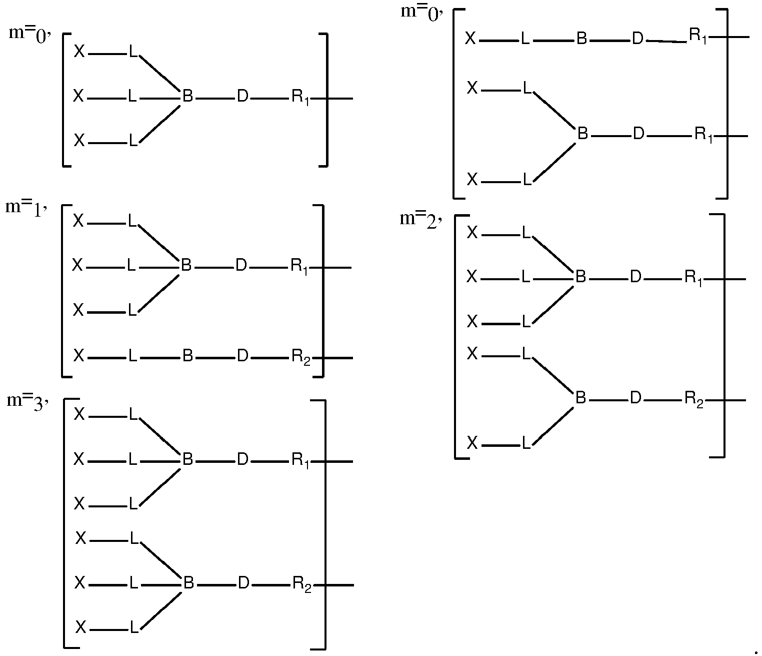

In some embodiments, when n=4, the structure of the 3'MVIP can be:

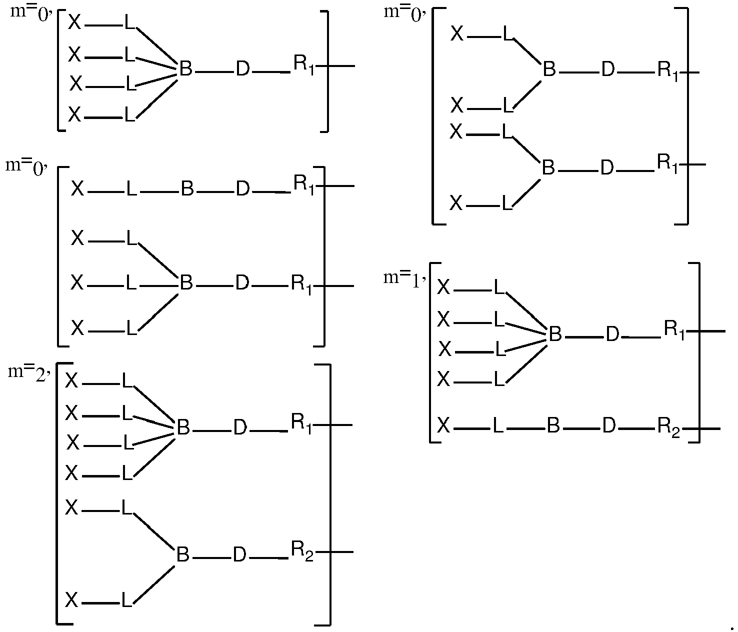

In some embodiments, the n refers to the sum of n in the 5'MVIP at 5' end of the sense strand and antisense strands of the RNAi agent at the same time, and the m refers to the sum of m in the 3'MVIP at 3' end of the sense strand and antisense strands of the RNAi agent at the same time.

In some embodiments, the R₁ and R₂ contain -NH-, -S-, and / or -O- in their structures, R₁ and R₂ are respectively connected with the linking chain D and the 5' and 3' ends of the sense strand and / or antisense strand through -NH-, -S-, or -O-, and R₁ and R₂ are the same or different.

In some embodiments, the R₁ and R₂ are optionally straight chains, or straight or cyclic structures with amido, carboxyl, or alkyl branched chains, and the cyclic structure includes saturated or unsaturated aliphatic carbocyclyl, or five or six membered heterocyclyl or aromatic hydrocarbyl containing sulfur, oxygen, or nitrogen atoms.

In some embodiments, the R₁ and / or R2 are -E₁(CH₂)ₓCH₂E₂-, where x is any integer of 3-12, and the groups E₁ and E₂ can be -NH-, -S-, or -O-, respectively.

In some embodiments, the R₁ and / or R₂ are -E₁(CH₂)ₓ₁CH(OH)(CH₂)ₓ₂E₂-, where X₁ or X₂ are each independently any integer of 3-10, and E₁ and E₂ can be -NH-, -S-, or -O-, respectively.

In some embodiments, the R₁ is a heterocyclic or carbocyclic structure containing N, S or O:

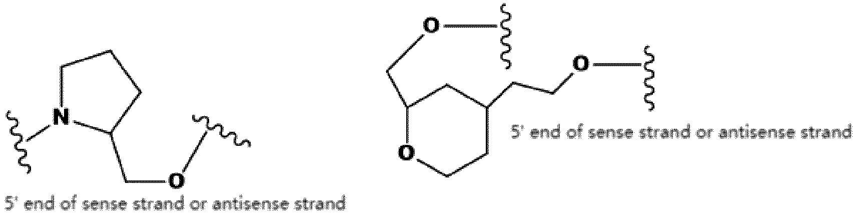

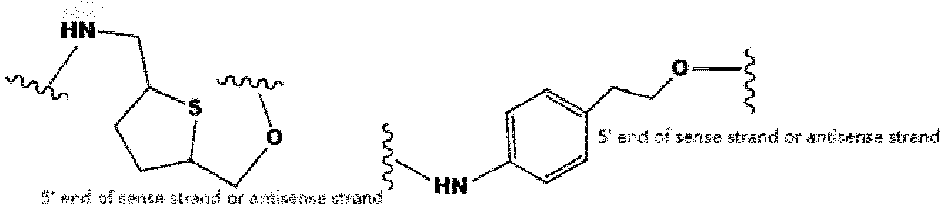

In some embodiments, the transition point R₁ is -NH(CH₂)ₓCH₂O-, where x is any integer of 3-12, preferably any integer of 4-6, which can be introduced by the following two phosphoramidite monomers:
i. One of -O- or -S- is used for the synthesis of R₁ phosphoramidite monomer, which is connected to the 5' end of the sense strand or antisense strand of RNAi agent by solid-phase synthesis. In the structure, -NH-, -S-, or -O- are used to connect with the linking chain D in the 5'MVIP, thereby introducing the liver targeting specific ligand X at the 5' end of the sense strand or antisense strand of the RNAi agent. An exemplary structure of a monomer introduced into the 5'end of the sense or antisense strand of the RNAi agent is as follows: 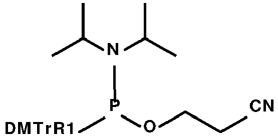 In some embodiments, the following structure is preferred: 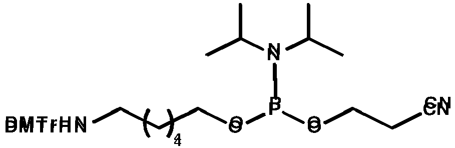
ii. In R₁ structure, -NH-, -S-, or -O- is first connected with the linking chain D, and another - NH-, -S-, or -O- is used to form ester with phosphoramidite in the synthesis of 5'MVIP phosphoramidite monomer. The exemplary structure of sense strand or antisense strand 5'MVIP phosphoramidite monomer is as follows:

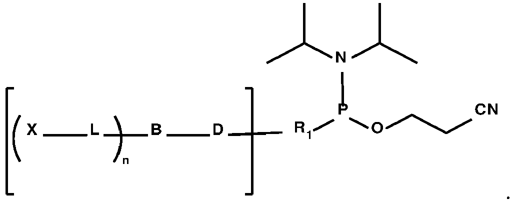

In some embodiments, the sense strand or antisense strand 5'MVIP phosphoramidite monomer preferably has the following structure:

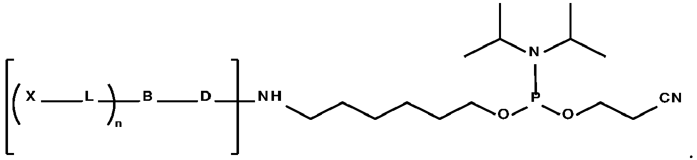

When n in the general formula is 1-4, the linker B part of the above monomer is branched for 1 to 4 times respectively to obtain the corresponding monomer compound. With the help of the above monomer compound, the liver targeting specific ligand X is introduced to the 5' end of the sense or antisense strand through solid-phase synthesis.

In some embodiments, the transition point R₁ is -NH(CH₂)ₓCH₂O-, where x can be an integer of 3-12, preferably an integer of 4-6.

In some embodiments, the 5'MVIP phosphoramidite monomer has the structure selected from the following structures:

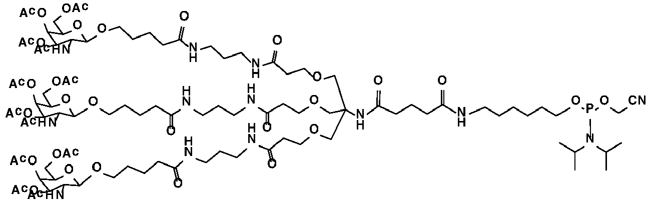

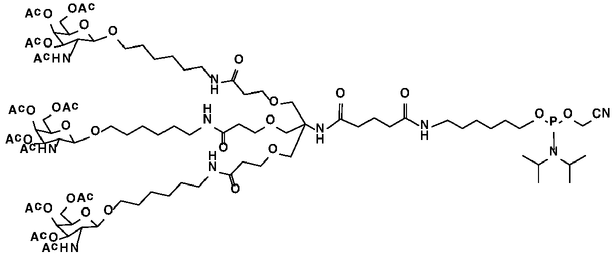

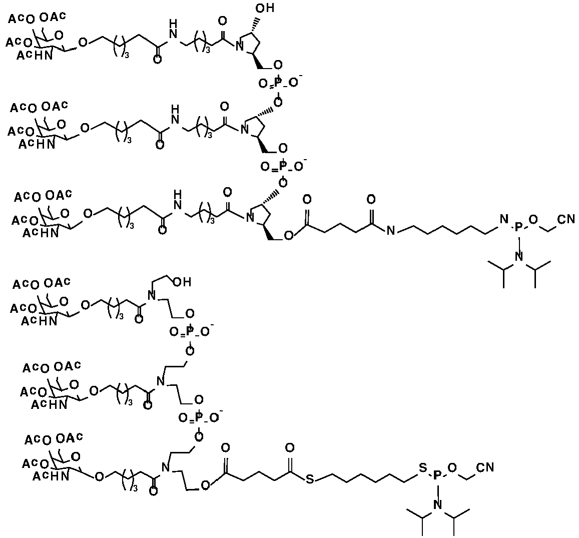

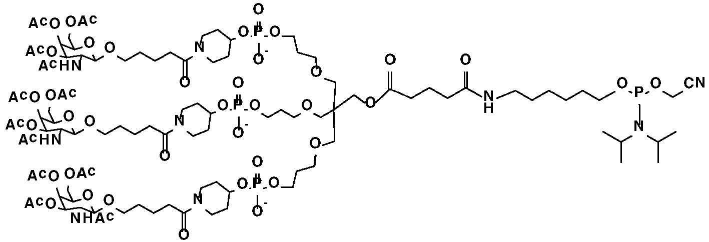

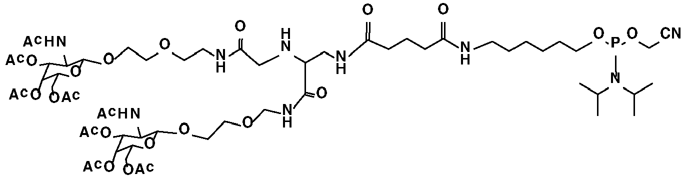

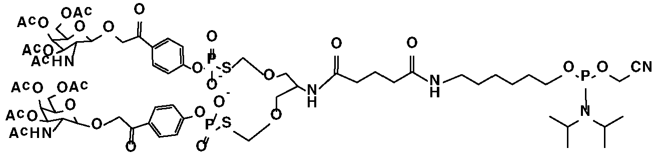

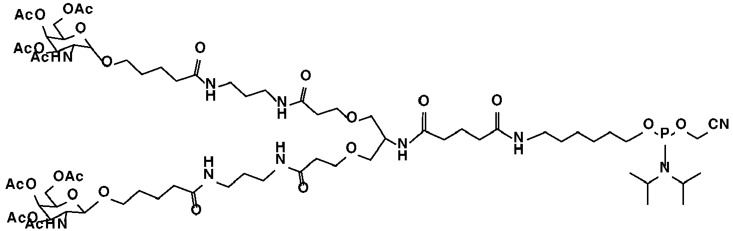

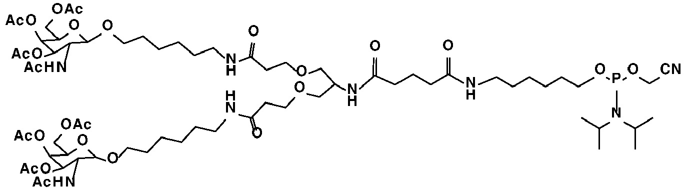

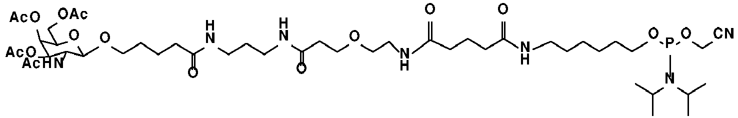

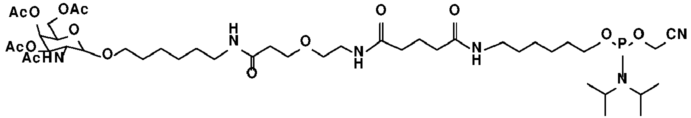

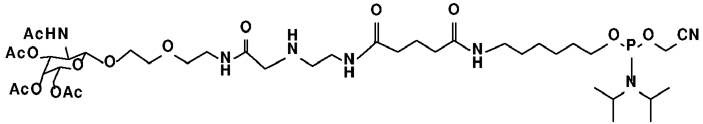

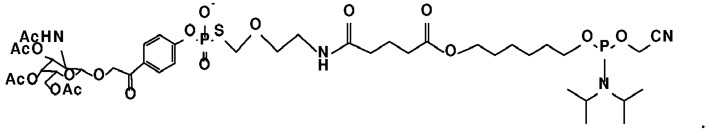

In some embodiments, the R₂ is a heterocyclic or carbocyclic structure containing N, S or O:

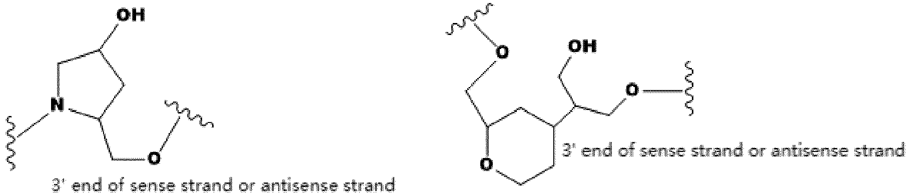

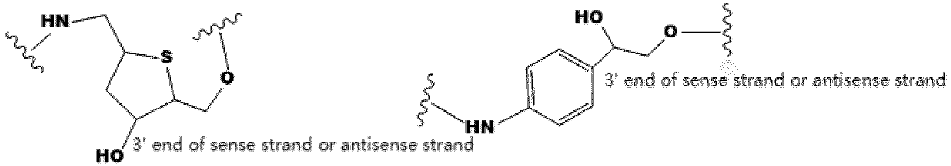

In some embodiments, the R₂ is -NH(CH₂)ₓ₁CH(OH)(CH₂)ₓ₂CH₂O-, where x1 is any integer of 1-4 and x2 is any integer of 0-4.

The R₂ described in this application is through succinic anhydride and -NH-, -S-, or -O- in the R₂ structure to form esters or amides, and at the same time, it is conjugated with -NH- in blank solid support to form 3'MVIP solid support, and then 3'MVIP is introduced into the 3' end of the sense strand or antisense strand by phosphoramidite solid-phase synthesis method.

In some embodiments, the heterocycle in the R₂ structure is a pyrrole ring or a piperidine ring, which is connected with the linking chain D of 3'MVIP through the nitrogen heteroatom in the ring. The exemplary structure of 3'MVIP solid support is as follows:

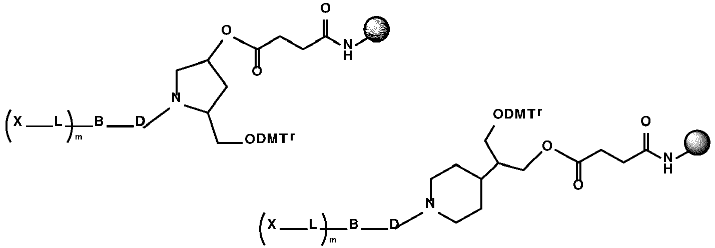

When m in the general formula is 1-4, the linker B part in the monomer above is branched for 1 to 4 times respectively to obtain the corresponding solid support.

In some embodiments, R₂ is -B₄(CH₂)ₓ₁CH(OH)(CH₂)ₓ₂CH₂B-, where x1 is an integer of 1-4, x2 is an integer of 0-4, and B₄ and B₅ are -NH-, -S-, or -O-, respectively.

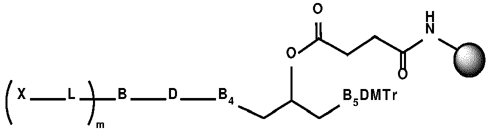

When m in the general formula is 1-4, the linker B part in the monomer above is branched for 1 to 4 times respectively to obtain the corresponding solid support.

In some embodiments, R₂ is -NHCH₂CH(OH)CH₂O-. The exemplary structure of the introduced 3 'MVIP solid support is as follows:

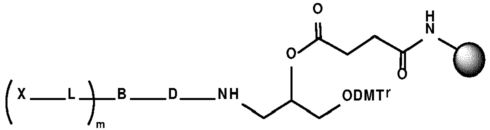

When m in the general formula is 1-4, the linker B part in the monomer above is branched for 1 to 4 times respectively to obtain the corresponding solid support.

In some embodiments, the 3'MVIP solid support has a structure as follows:

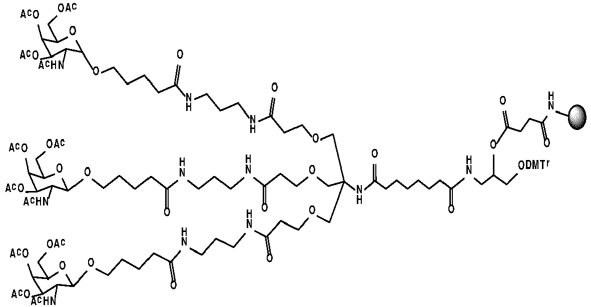

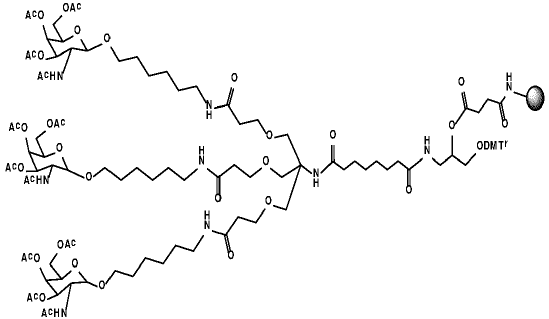

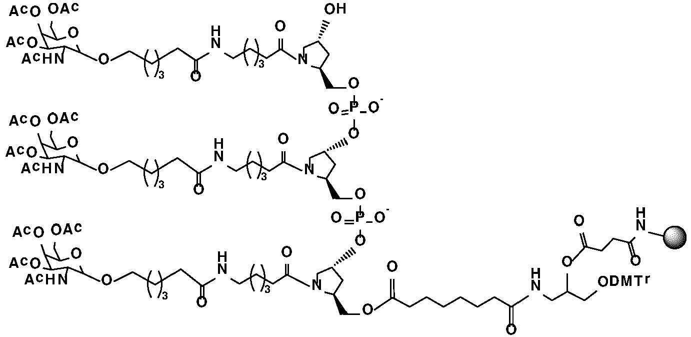

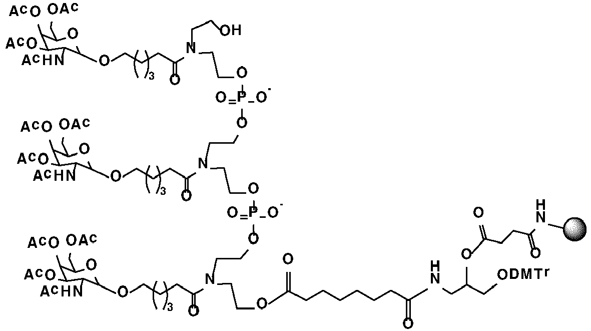

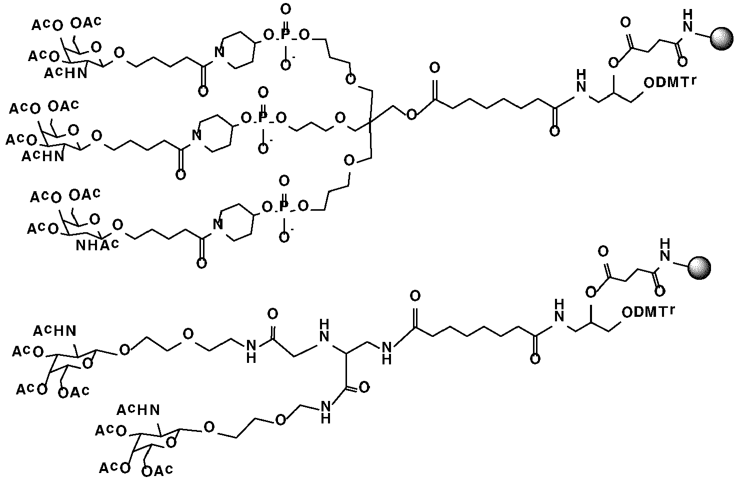

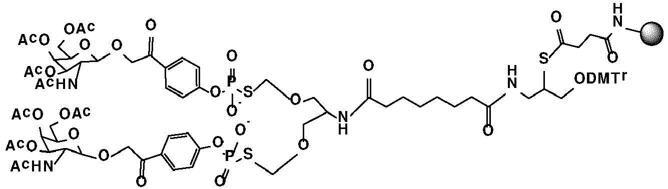

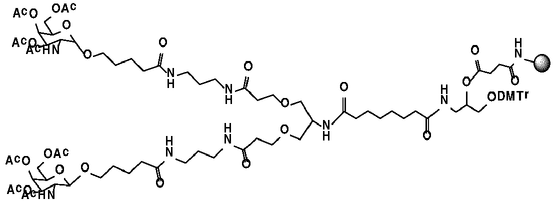

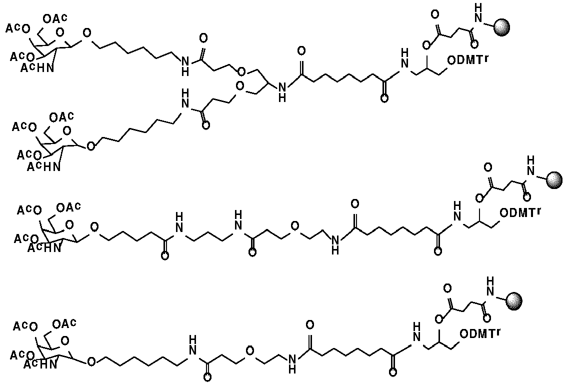

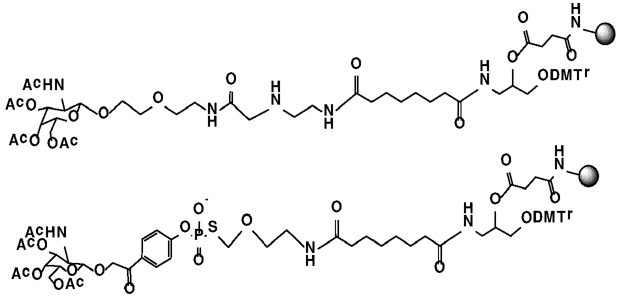

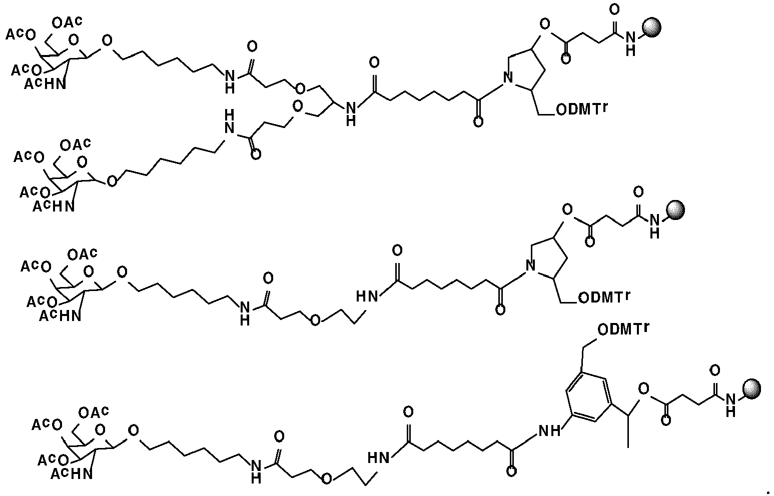

In some embodiments, the liver targeting specific ligand X is selected from structures used to enhance the uptake of RNAi agents by hepatocytes, which can be lipids, steroids, vitamins, sugars, proteins, peptides, polyamines and peptide mimetic structures. In the RNAi agent provided by this application, the liver targeting specific ligand X introduced into the end of the sense strand or antisense strand of the RNAi agent can be the same or different. For example, in terms of characteristics, some can enhance liver targeting, some can be the regulatory structure of the pharmacokinetics of the RNAi agent in vivo, and some can be the structure with dissolution activity in vivo. In some embodiments, the liver targeting specific ligand X is selected from one or more monosaccharides and derivatives thereof in the following structures.

In some embodiments, the monosaccharide is selected from one or more of the following structures: mannose, galactose, D-arabinose, glucose, fructose, xylose, glucosamine, ribose. The monosaccharide derivatives are selected from mannose derivatives, galactose derivatives, glucose derivatives, ribose derivatives, and other derivatives.

In some embodiments, the targeting unit X is selected from galactose, galactosamine, N-acetylgalactosamine and their derivatives, and its general structural formula is as follows: 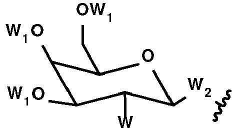 wherein, W₁ is a hydrogen or hydroxyl protecting group, which can be the same or different; W is -OH, -NHCOOH or -NHCO(CH₂)qCH₃, where q is an integer of 0-4; W₂ is -NH-, O, S or C.

In some embodiments, the targeting unit X is N-acetylgalactosamine and its derivatives.

In some embodiments, the targeting unit X is selected from the following structures: 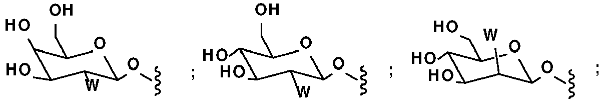 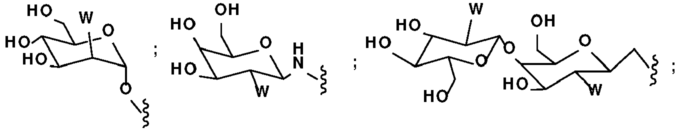 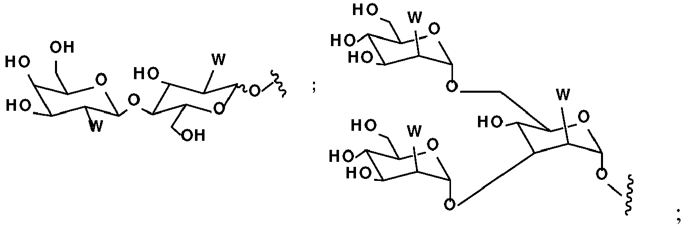 where W is selected from one or two of -OH, -NHCOOH or -NHCO(CH₂)_{q}CH₃, where q is an integer of 0-4.

In some embodiments, the liver targeting specific ligand X may be the same or different in the same 5'MVIP or 3'MVIP structure.

In some embodiments, the X between 5'MVIP and 3'MVIP can be the same or different.

In some embodiments, L is a C₄-C₁₈ straight chain containing -NH-, -C(=O)-, -O-, -S-, amido, phosphoryl, thiophosphoryl, C₄-C₁₀ aliphatic carbocyclyl, phenyl, or a combination of these groups.

In some embodiments, the L also has a side chain of hydroxyethyl or carboxylic acids.

In some embodiments, the L is C₇-C₁₈ straight chain containing amido group or six-membered aliphatic carbocyclyl group.

In some embodiments, the L is selected from one or more of the following structures:

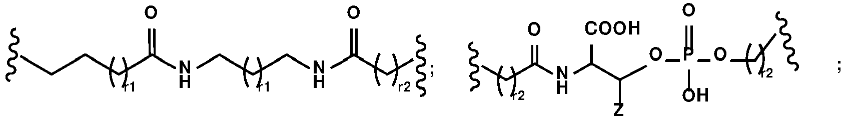

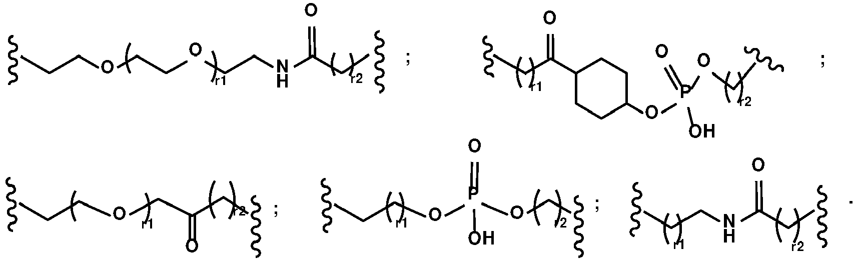

Where r1 is any integer from 1 to 12, r2 is any integer from 0 to 20, and Z is H, alkyl or amido group.

In some embodiments, the structure of the B is related to the amount of X that can be introduced. The B contains -NH-, C, O, S, amido, phosphoryl, thiophosphoryl. When n or m is 1, it is a straight chain, and when n or m is 2, 3, or 4, the number of branches is 2, 3, or 4, respectively.

In some embodiments, the B is selected from the following structures: 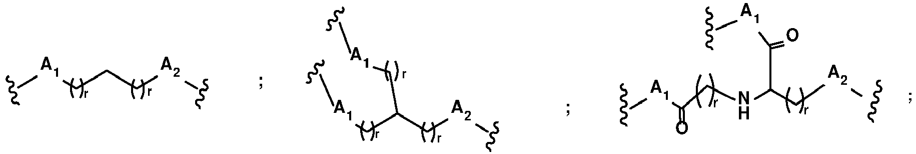 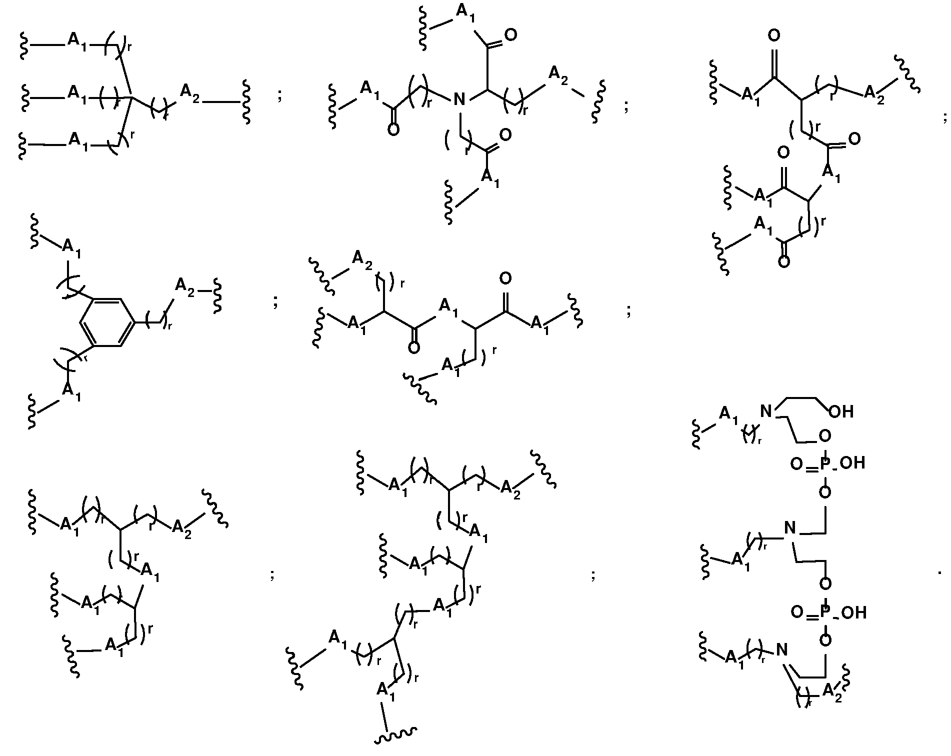 wherein A₁ and A₂ are each independently C, O, S, -NH-, carbonyl, amido, phosphoryl or thiophosphoryl, and r is an integer of 0-4.

In some embodiments, the B is selected from the following structure: 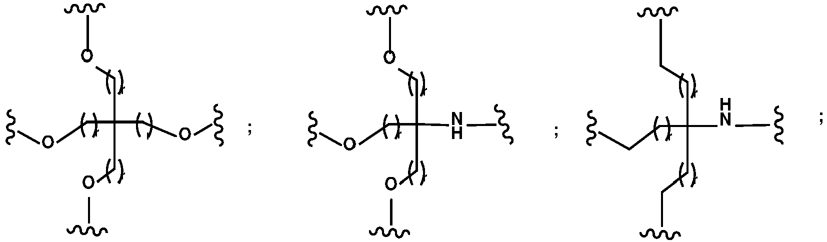 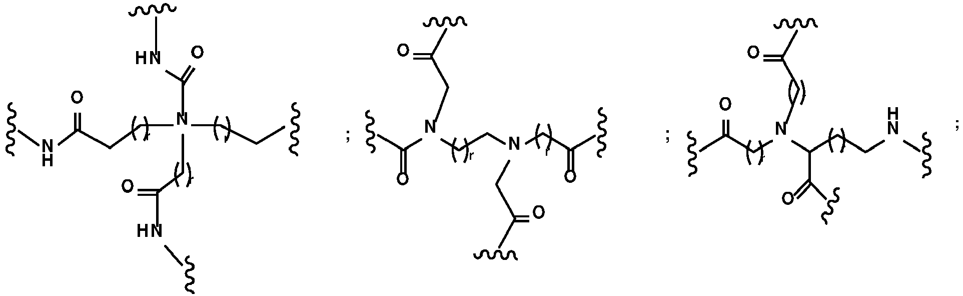 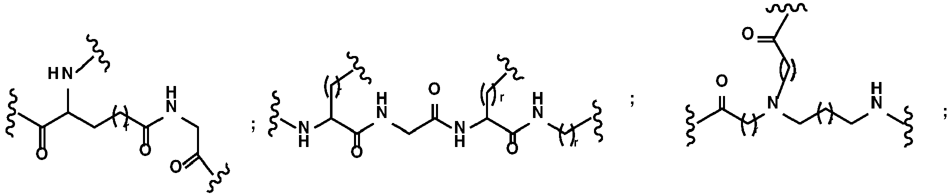 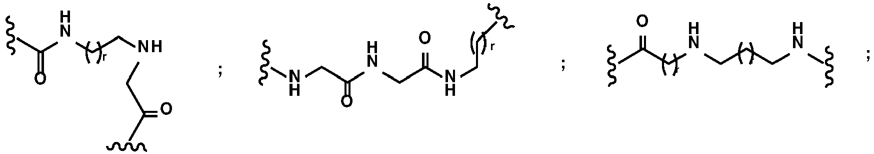 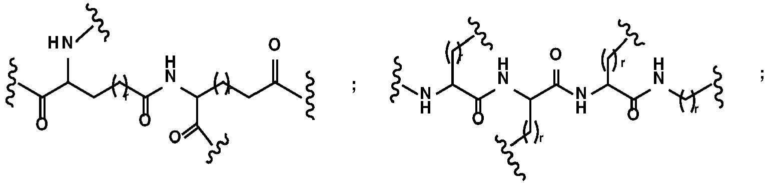 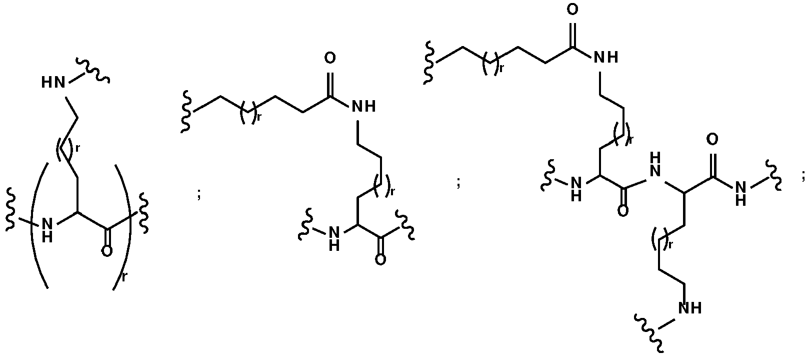 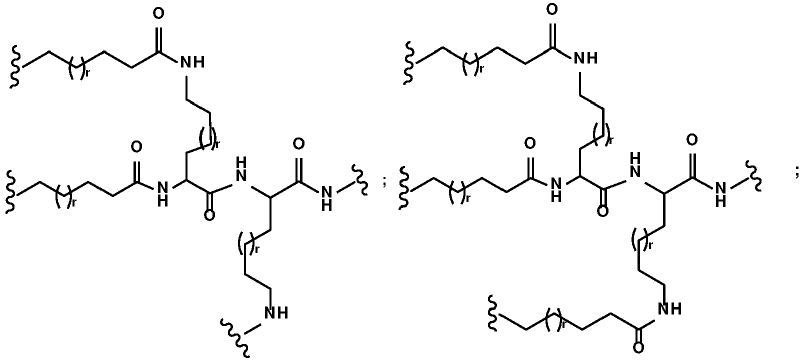 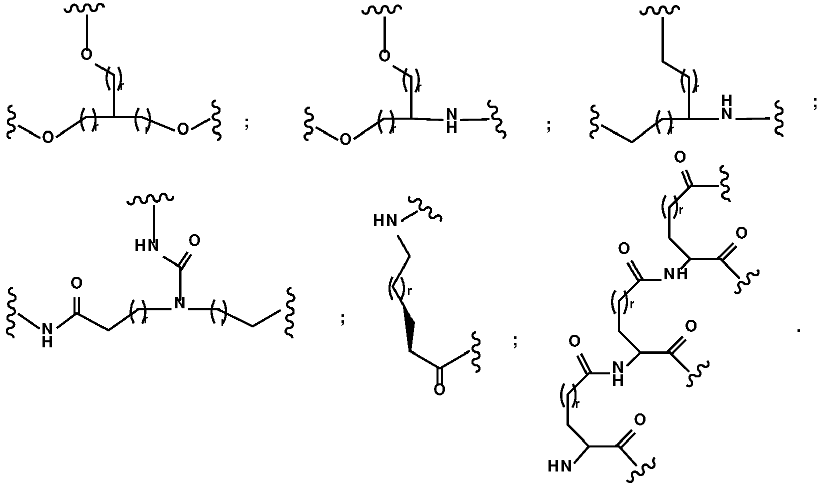 wherein r is any integer from 0 to 4.

In some embodiments, the B is selected from the following structures:

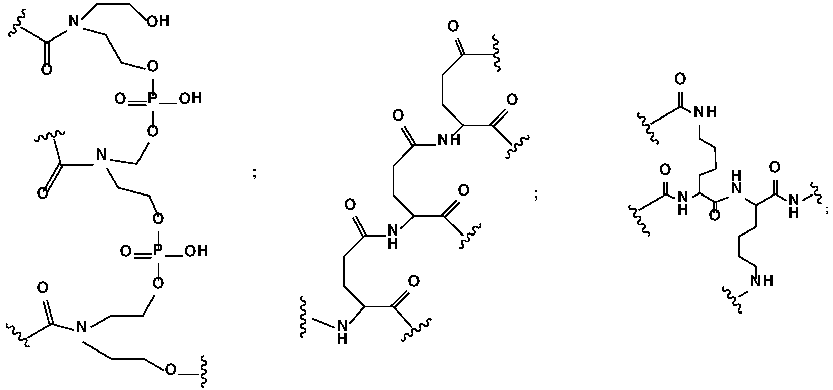

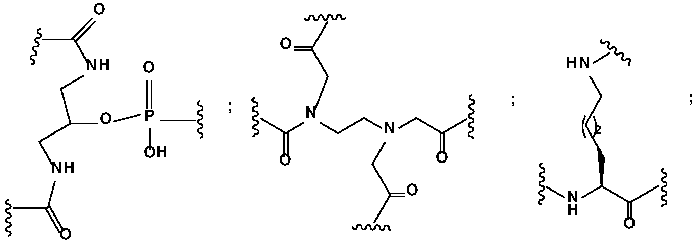

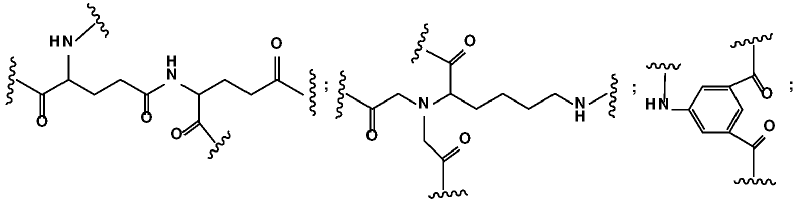

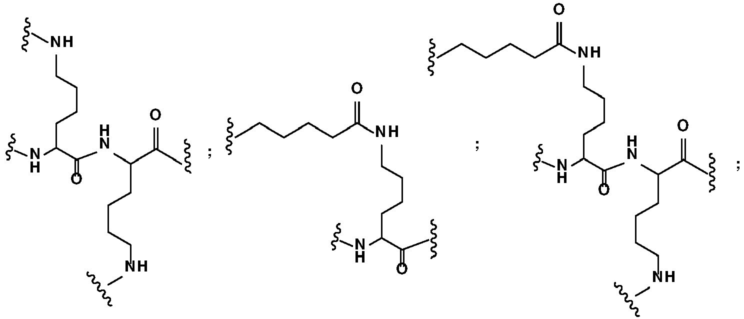

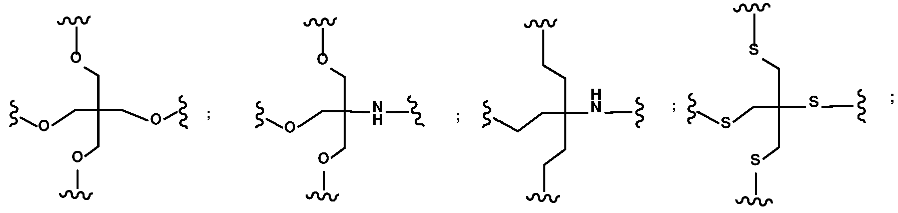

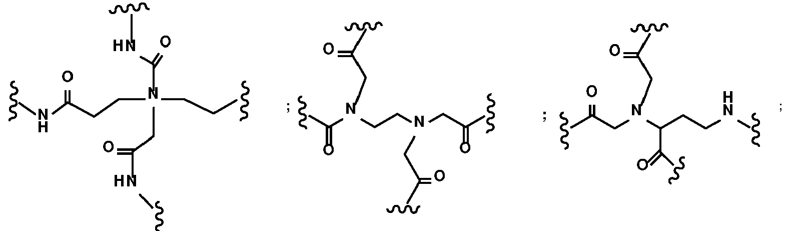

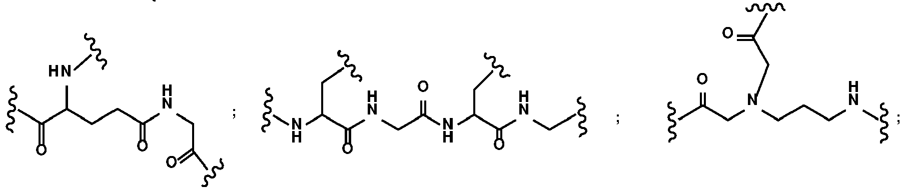

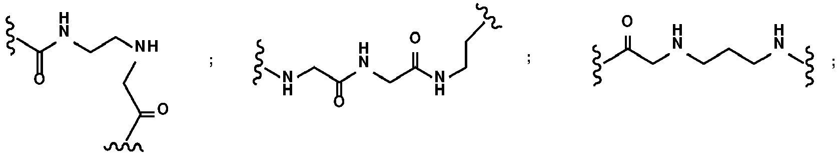

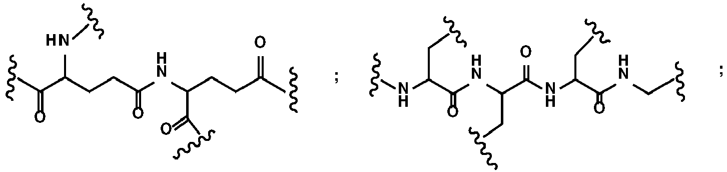

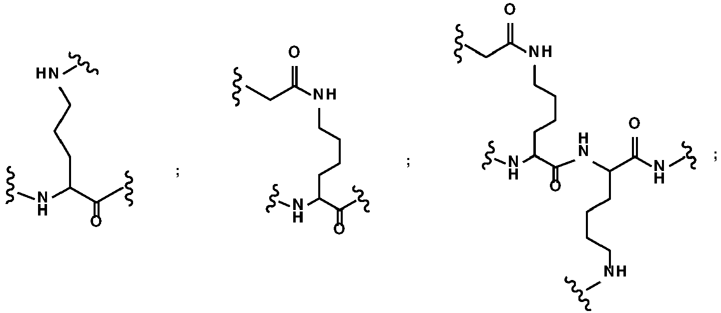

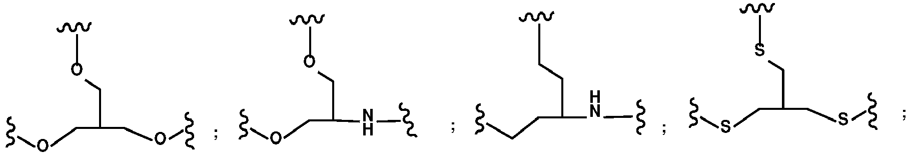

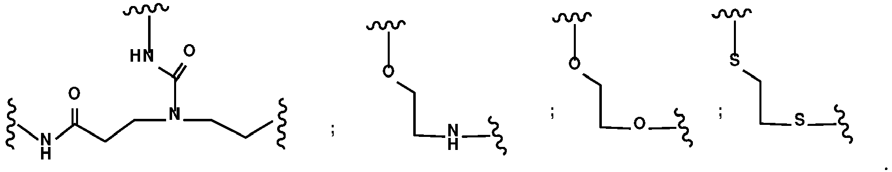

In some embodiments, the B is selected from the following structures:

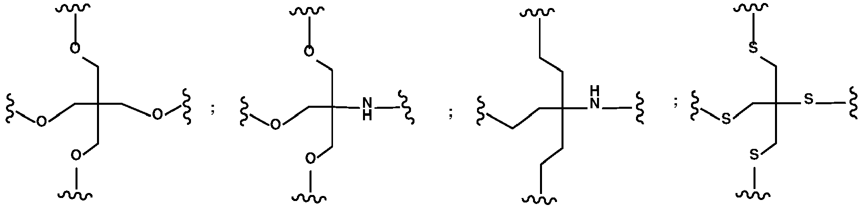

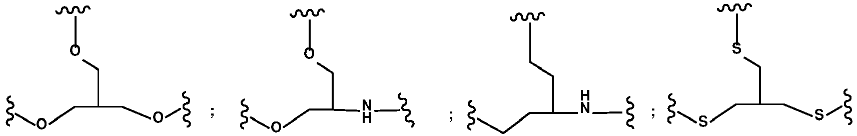

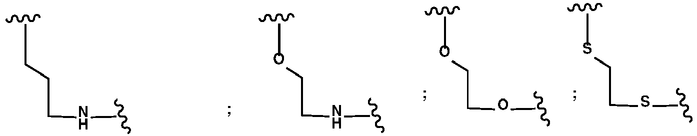

In some embodiments, the D is a C₃-C₁₈ straight chain containing -NH-, C=O, O, S, amido, phosphoryl, thiophosphoryl, aromatic hydrocarbyl, C₄-C₁₀ aliphatic carbocyclyl, five - or six-membered heterocyclyl containing 1-3 nitrogen, or a combination of these groups.

In some embodiments, the D also has side chains of hydroxymethyl, methyl tert-butyl, methylphenoyl, and C₅-C₆ aliphatic cyclic groups.

In some embodiments, the D is a C₃-C₁₀ straight chain containing two C=O, six-membered aliphatic carbocyclyl or phenyl groups.

In some embodiments, the D is a C₃-C₁₀ straight chain containing two C=O.

In some embodiments, the D is selected from the following structures: 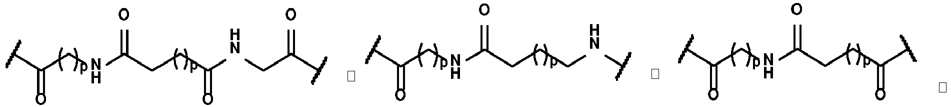 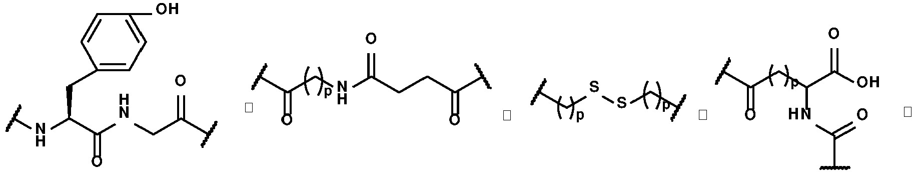 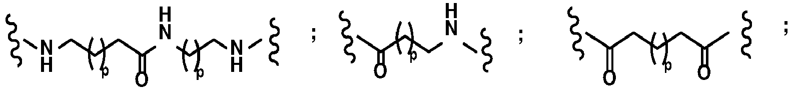 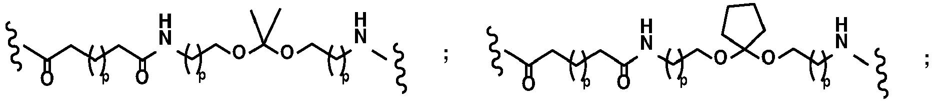 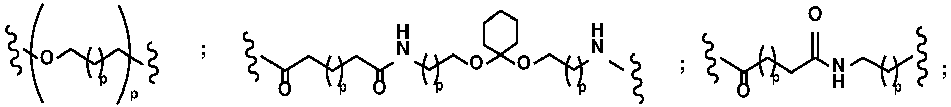 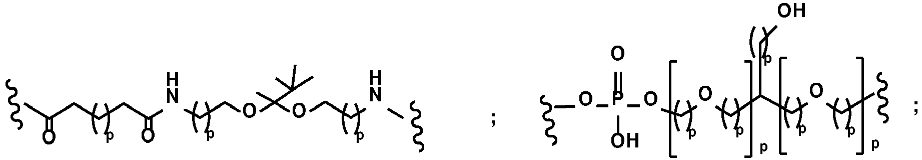 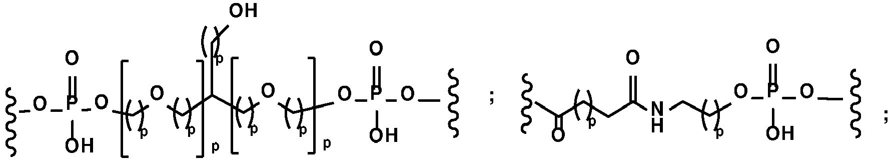 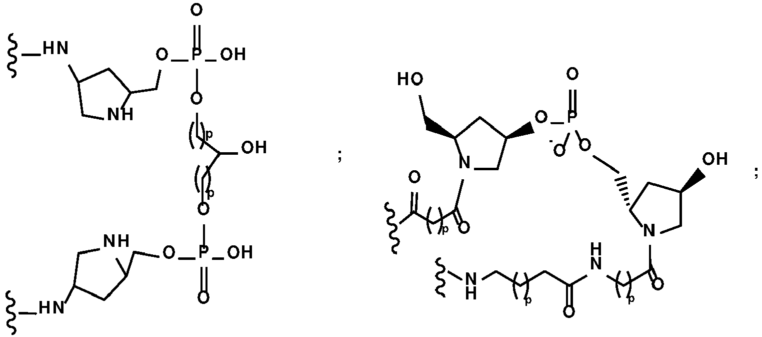 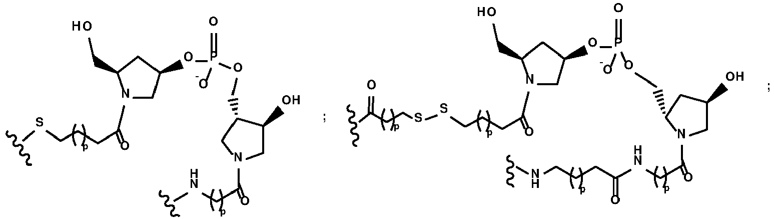 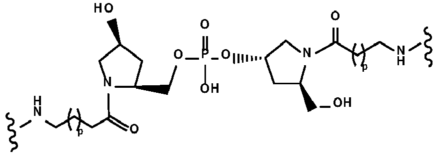 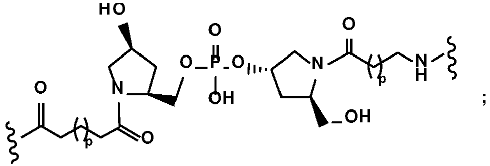 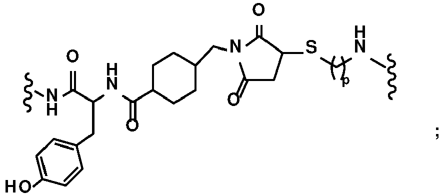 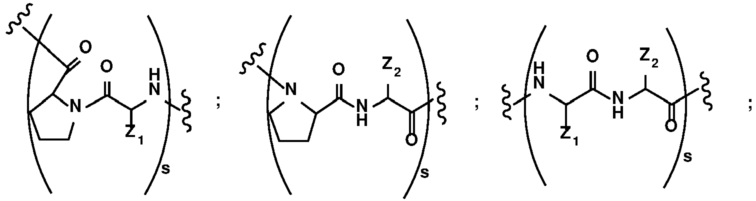 wherein, each p is independently any integer from 1 to 20; s is an integer of 2-13; Z₁ and Z₂ are the same or different substituents.

In some embodiments, the D is selected from the following structures:

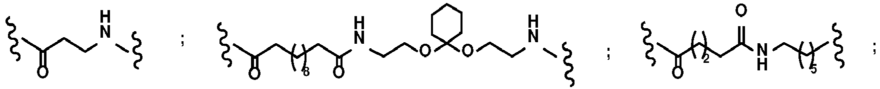

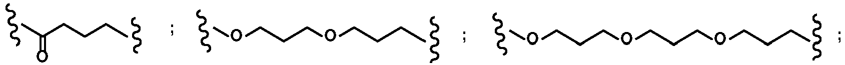

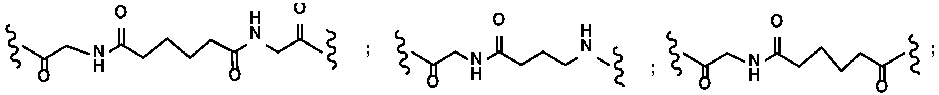

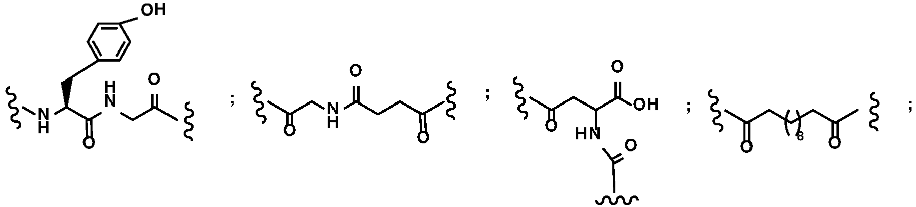

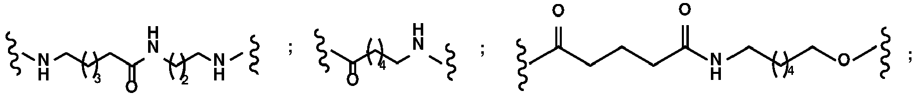

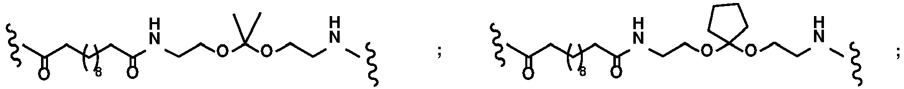

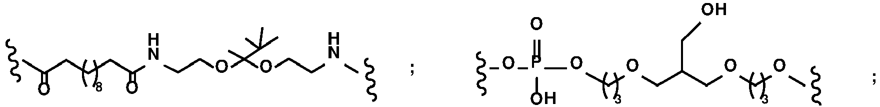

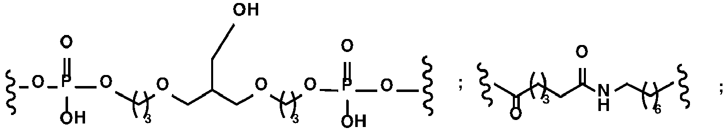

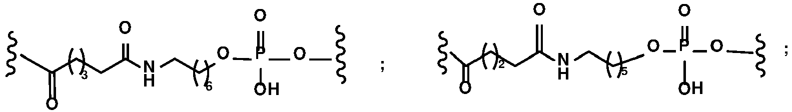

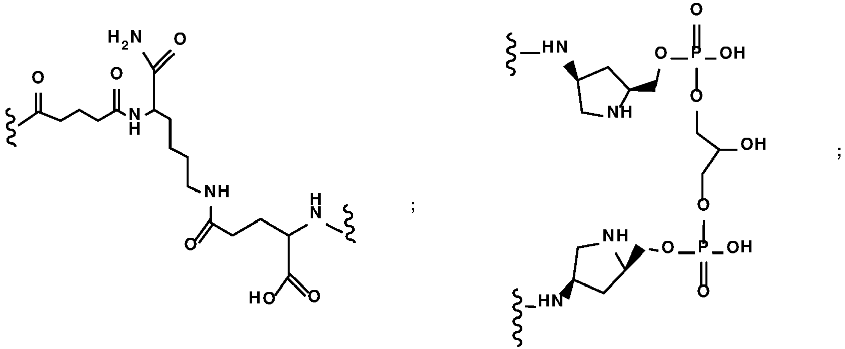

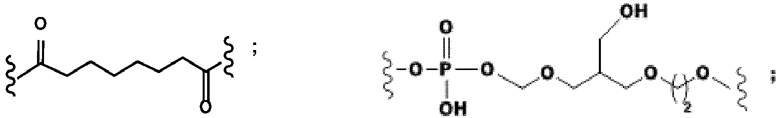

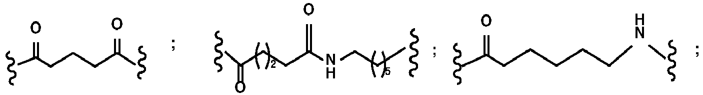

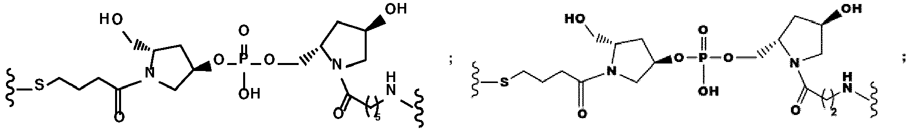

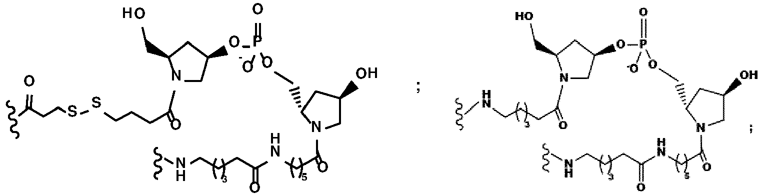

In some embodiments, the D is selected from the following structures:

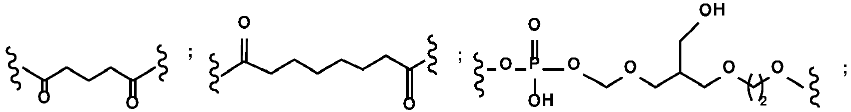

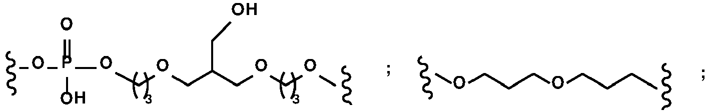

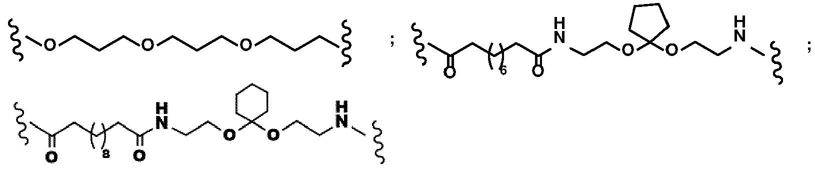

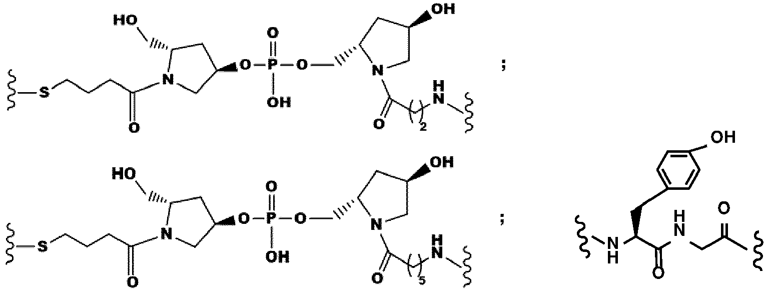

In some embodiments, **(X-L)ₙ-B-D-** in the 5'MVIP structure and **(X-L)ₘ-B-D-** in the 3'MVIP structure are selected from one or more of the following structures:

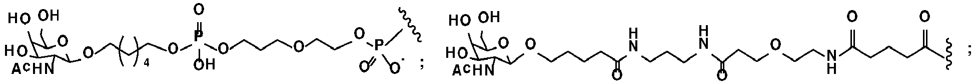

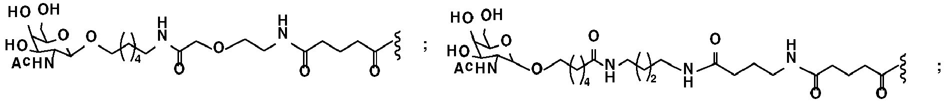

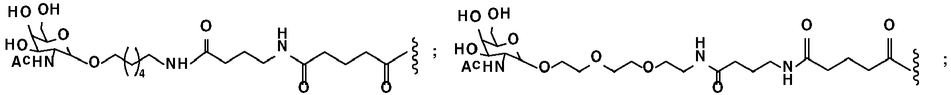

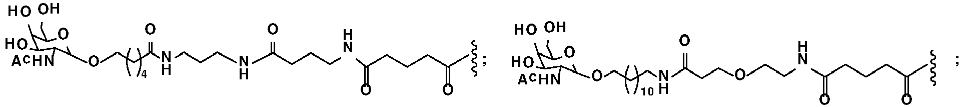

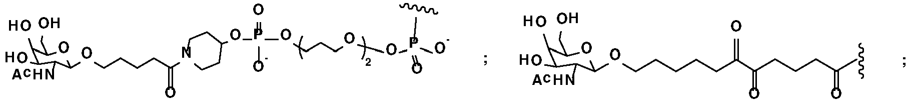

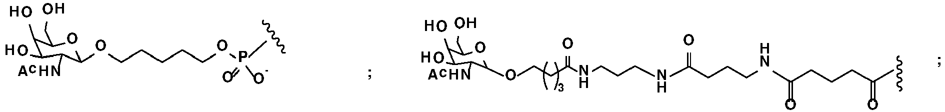

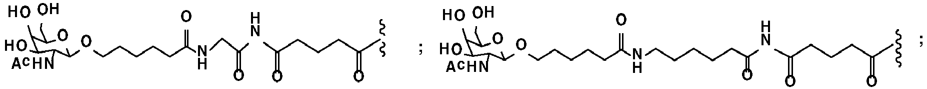

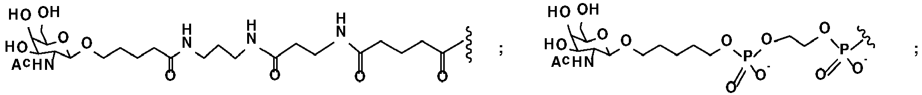

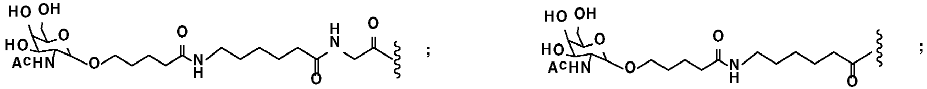

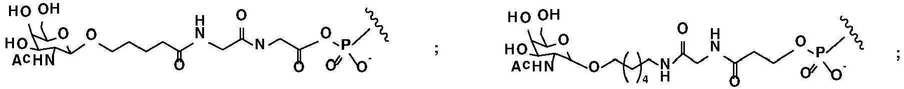

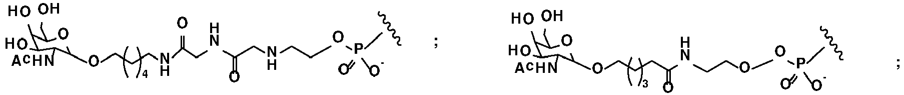

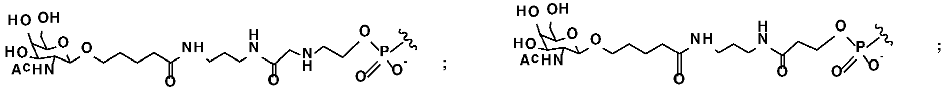

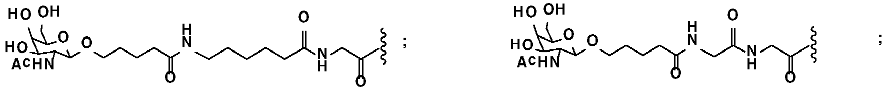

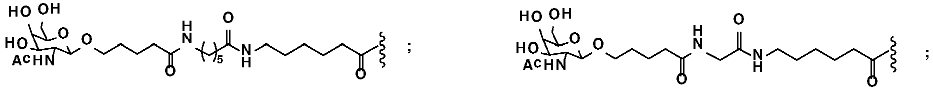

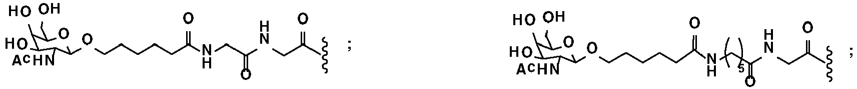

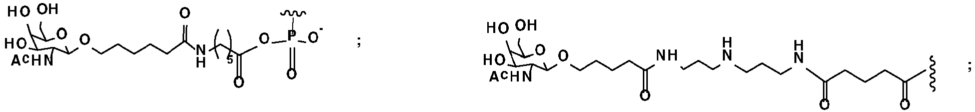

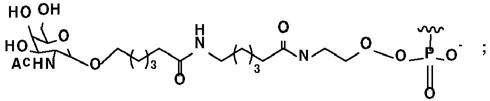

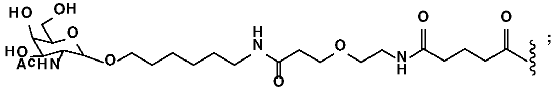

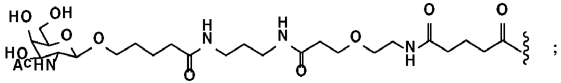

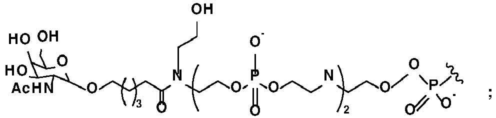

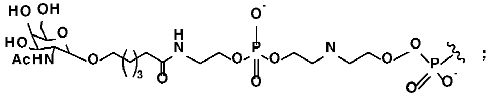

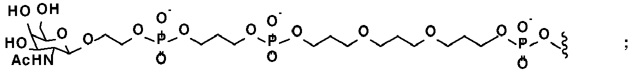

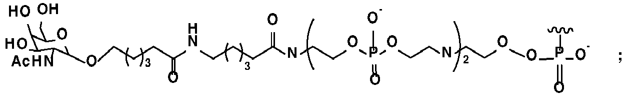

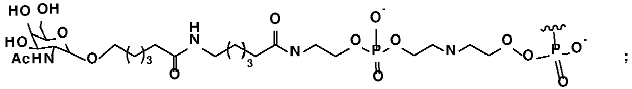

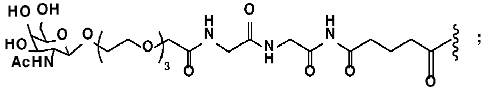

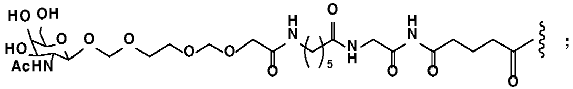

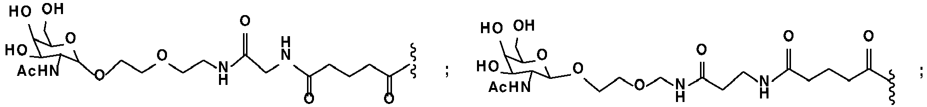

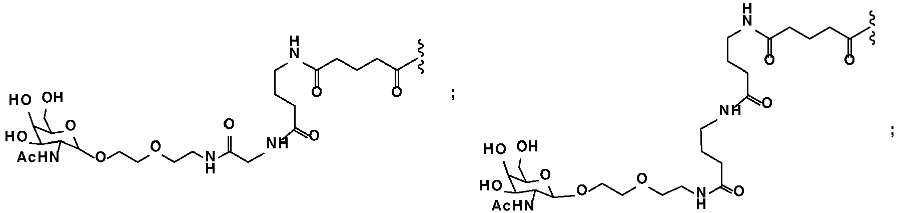

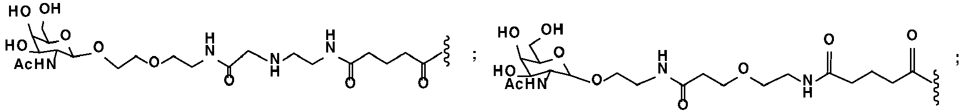

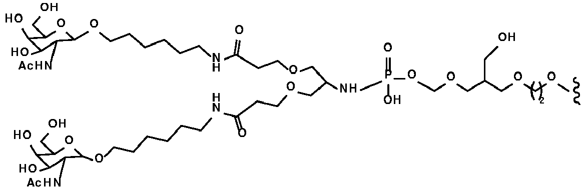

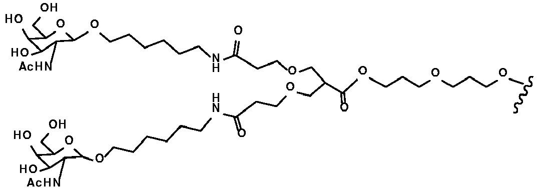

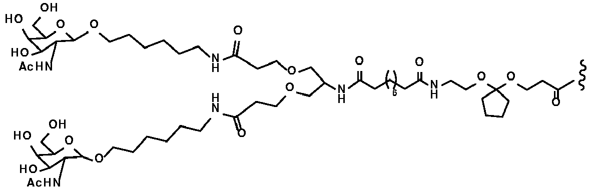

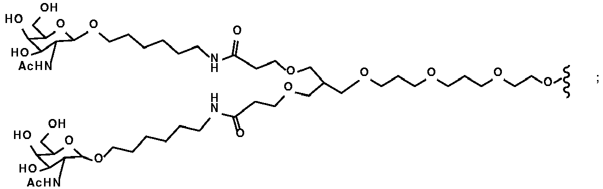

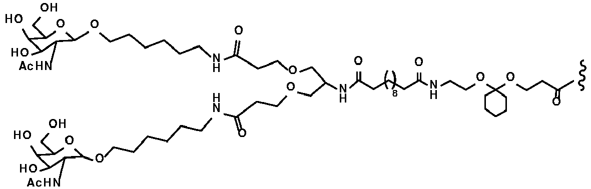

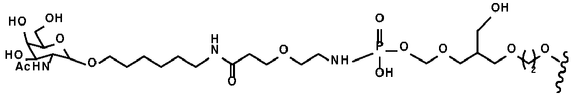

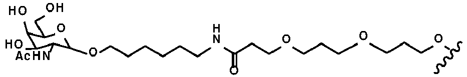

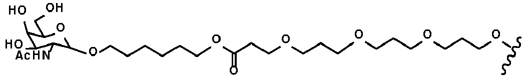

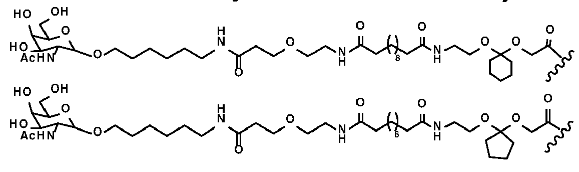

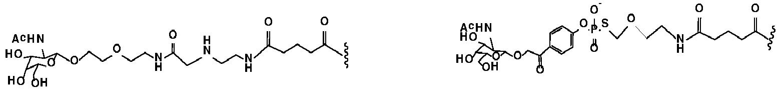

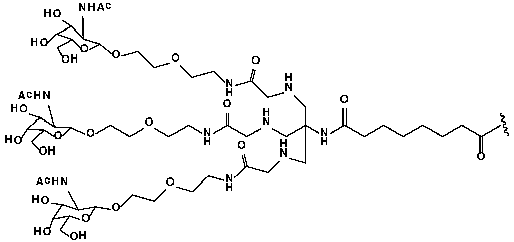

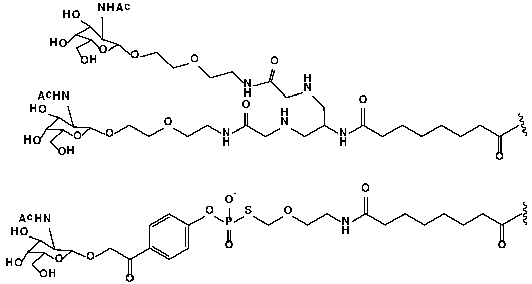

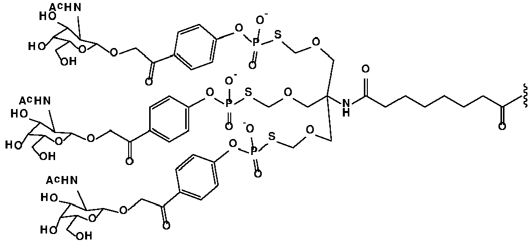

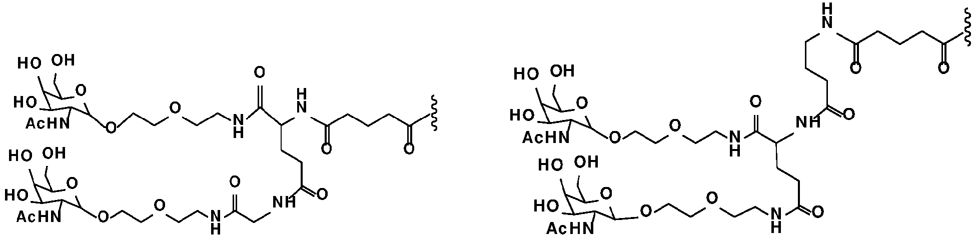

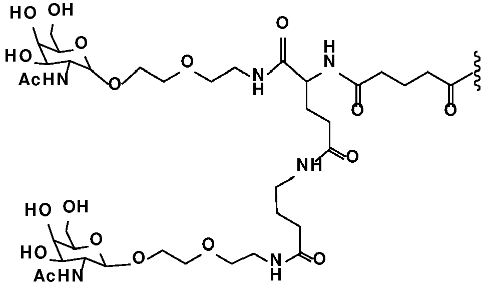

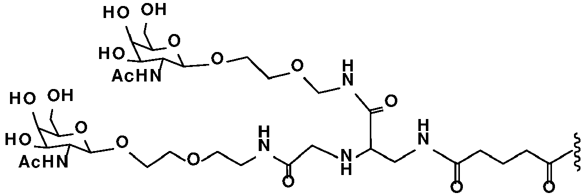

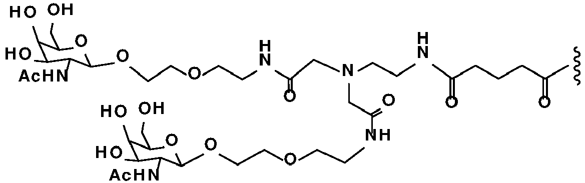

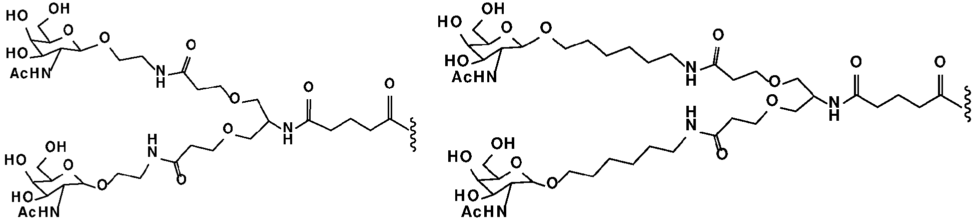

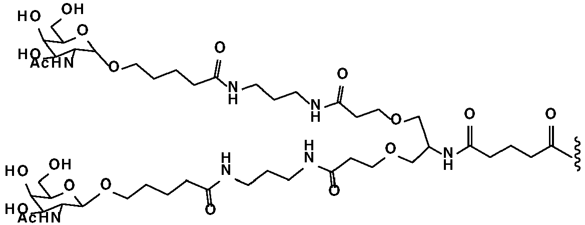

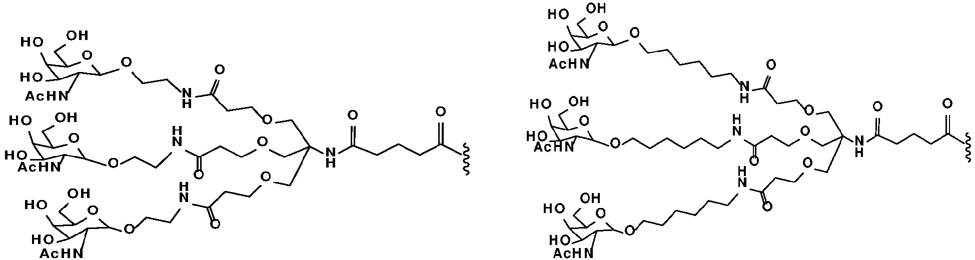

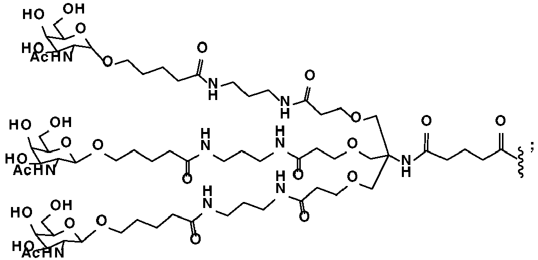

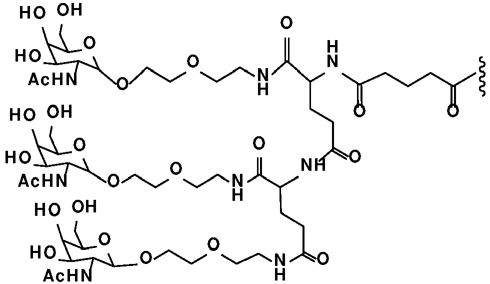

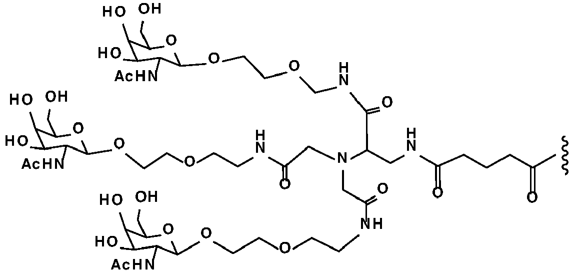

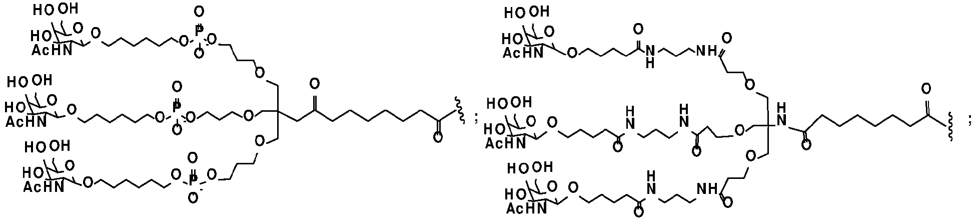

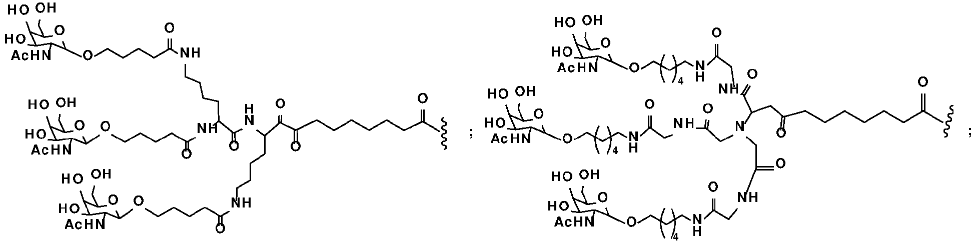

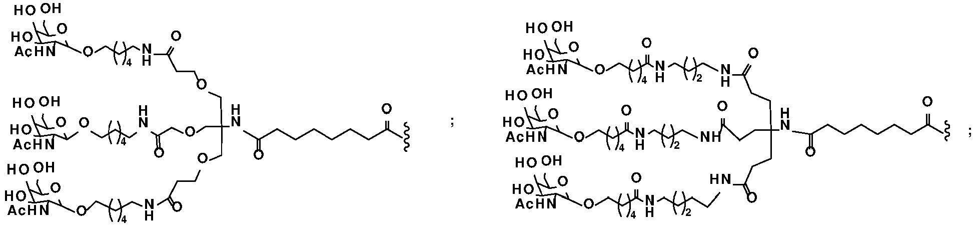

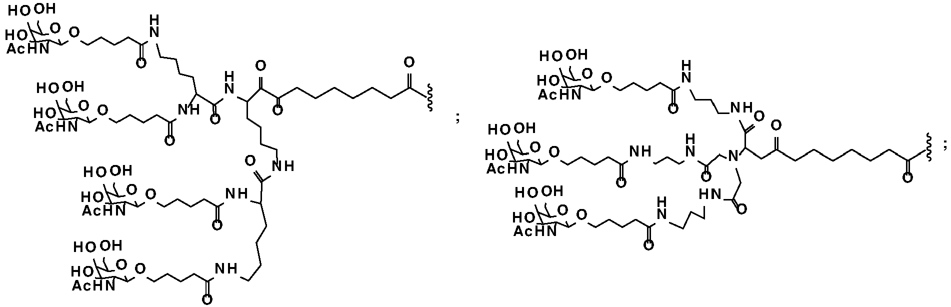

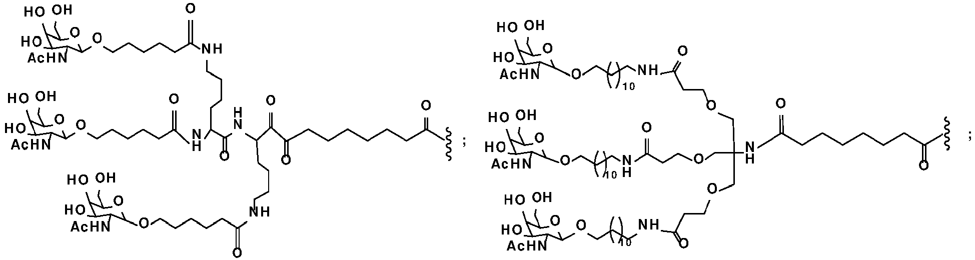

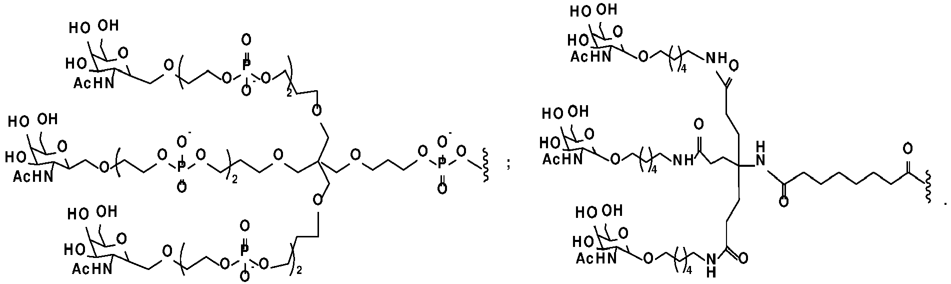

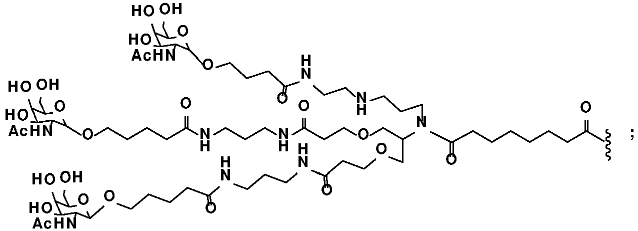

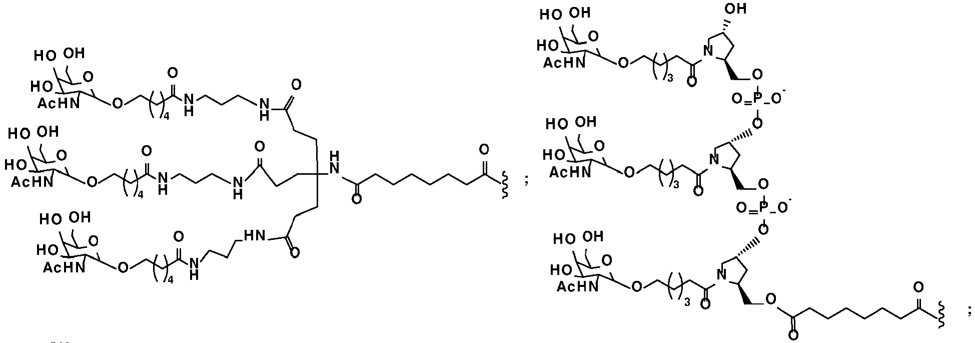

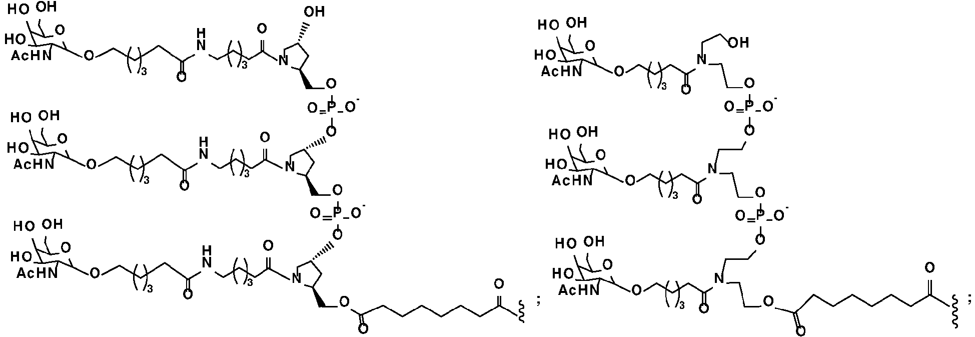

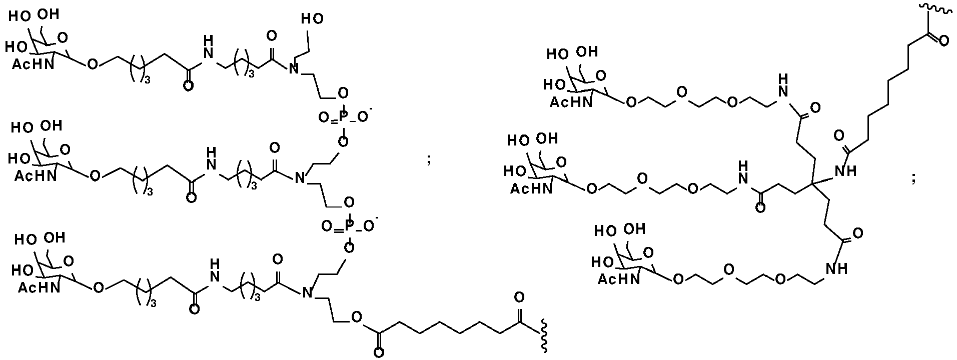

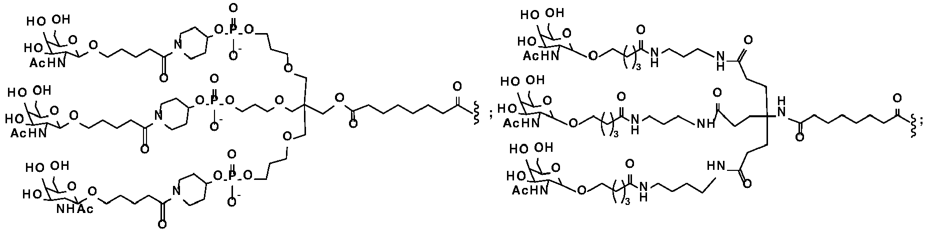

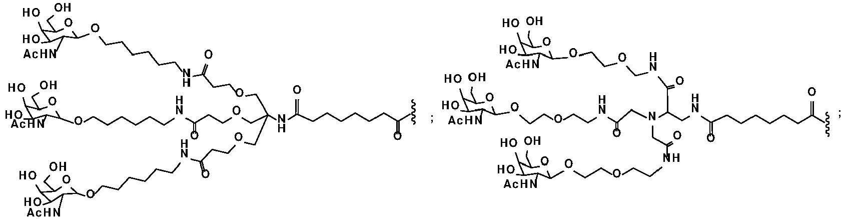

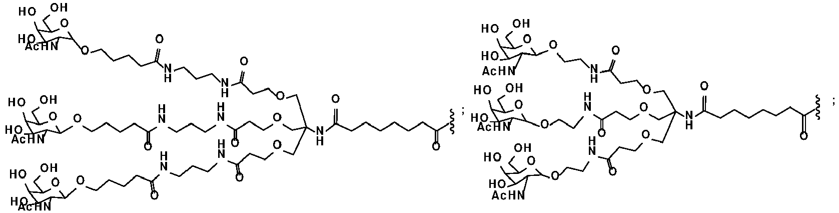

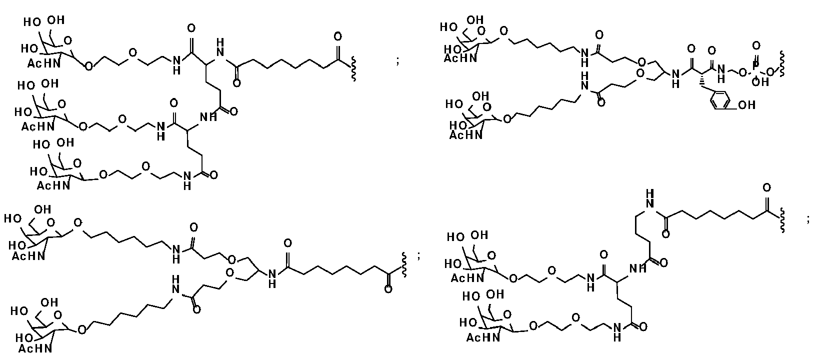

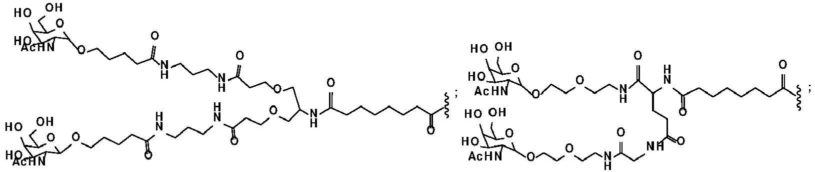

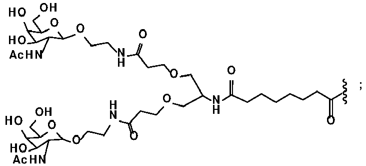

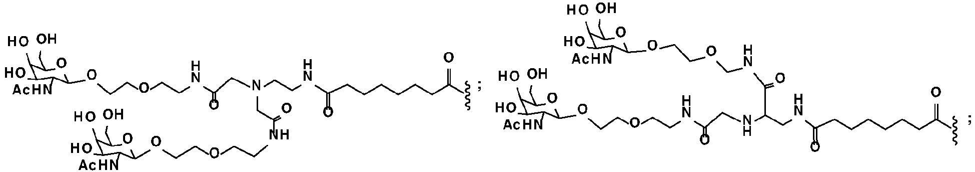

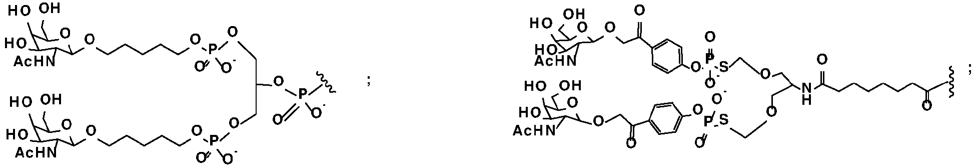

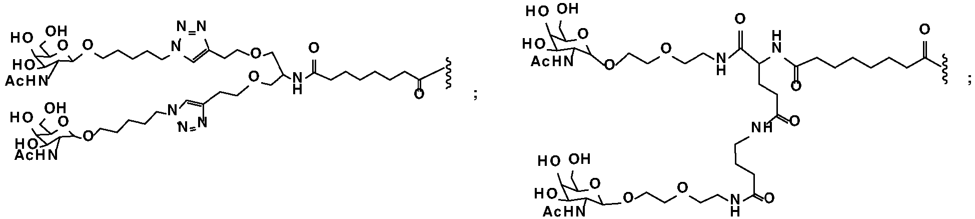

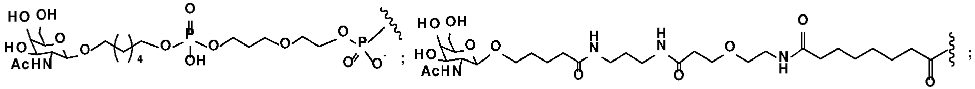

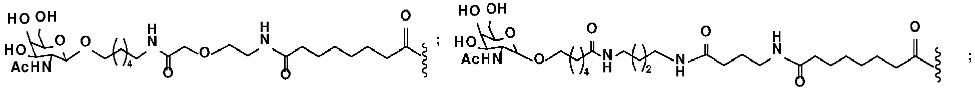

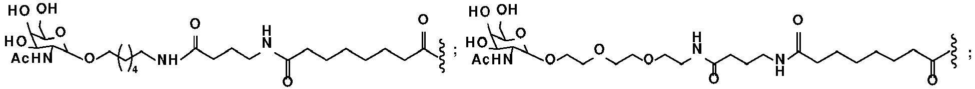

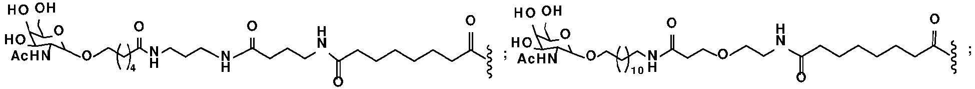

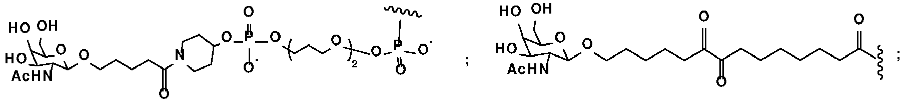

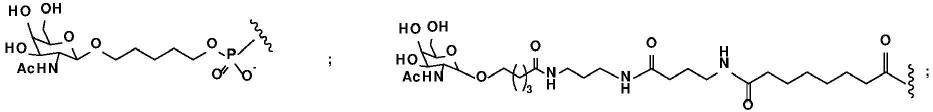

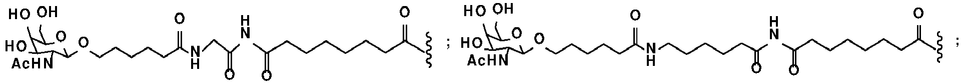

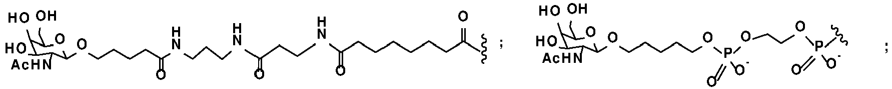

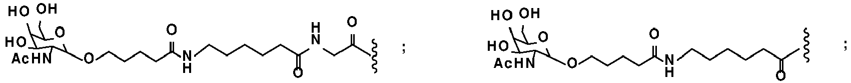

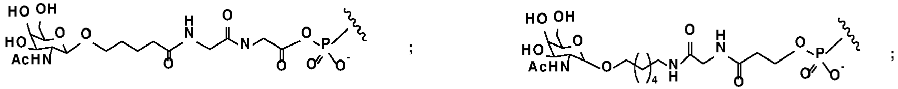

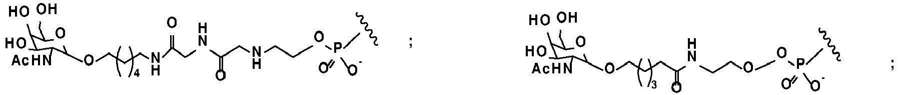

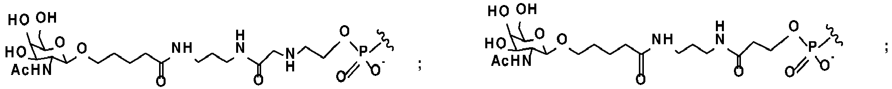

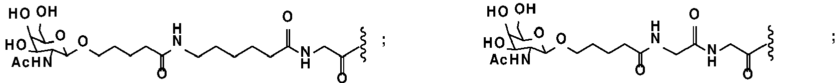

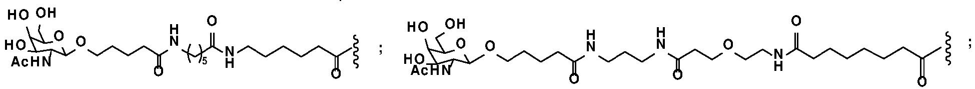

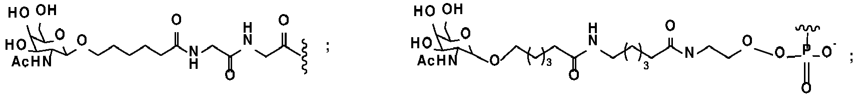

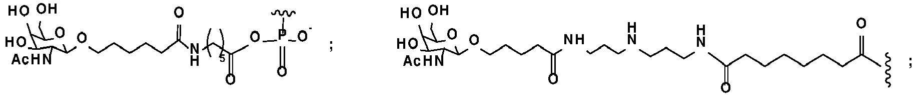

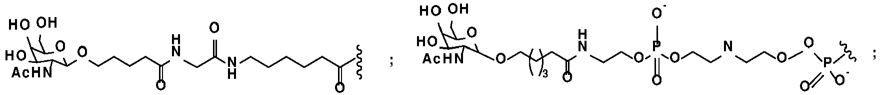

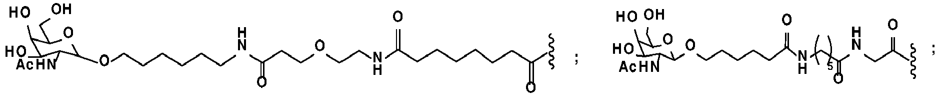

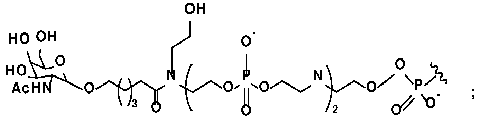

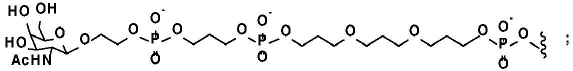

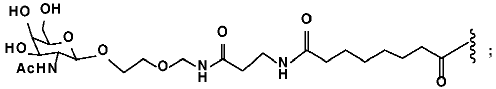

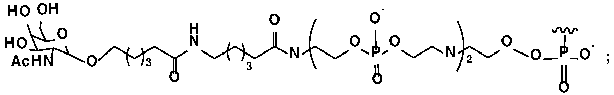

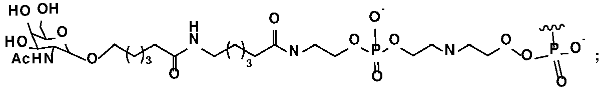

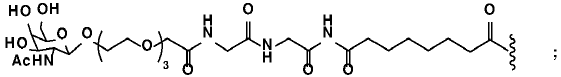

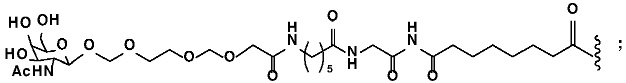

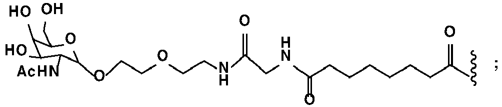

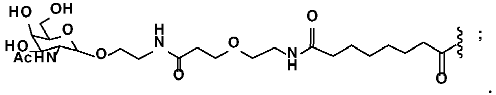

In some embodiments, the X, L, D, and B are the same or different within the respective 5 'MVIP and 3' MVIP or between the 5 'MVIP and 3' MVIP.

In some embodiments, **(X-L)ₙ-B-D-**in the 5'MVIP structure is selected from the structures shown in Table 8:

**Table 8 Structures of (X-L)ₙ-B-D- in 5'MVIP**

| Serial number | Code | Structural formula |
|---|---|---|
| 1 | 5'YICdd-01 | 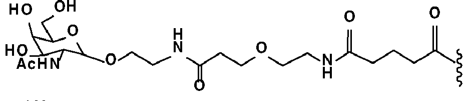 |
| 2 | 5'YICd-01 | 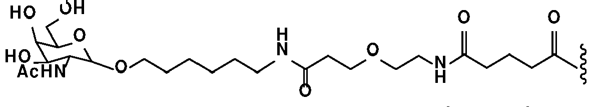 |
| 3 | 5'YICc-01 | 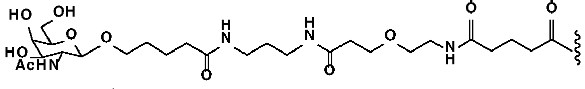 |
| 4 | 5'YICa-01 | 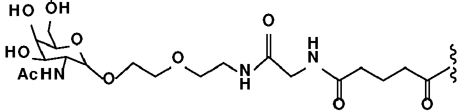 |
| 5 | 5'YICa-02 | 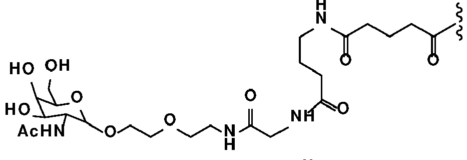 |
| 6 | 5'YICa-03 | 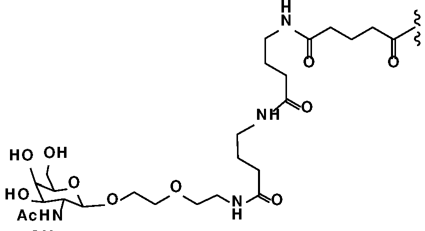 |
| 7 | 5'YICa-04 | 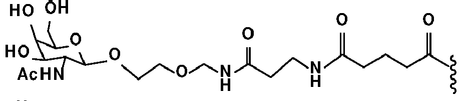 |
| 8 | 5'YICa-05 | 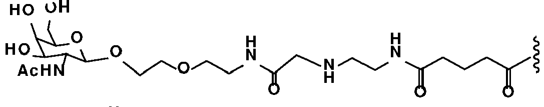 |
| 9 | 5'ERCa-01 | 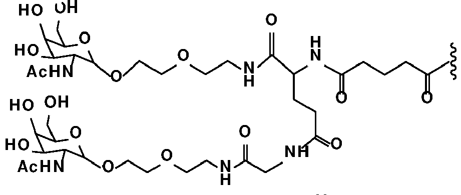 |
| 10 | 5'ERCa-02 | 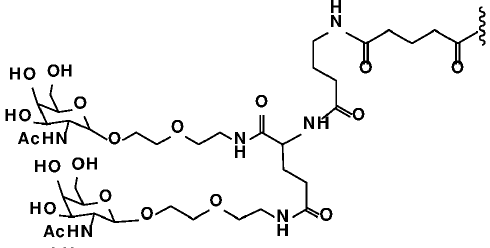 |
| 11 | 5'ERCa-03 | 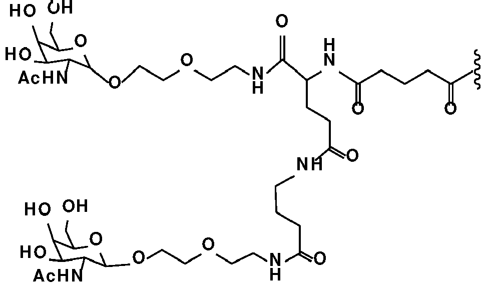 |
| 12 | 5'ERCa-04 | 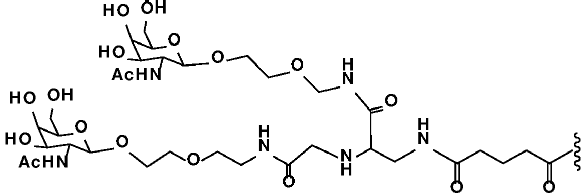 |
| 13 | 5'ERCa-05 | 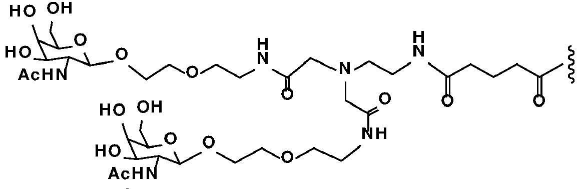 |
| 14 | 5'ERCdd-01 | 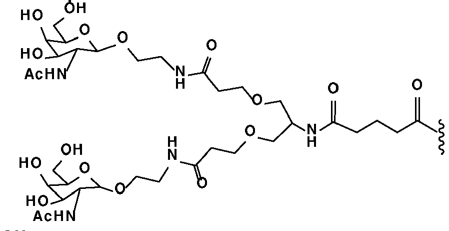 |
| 15 | 5'ERCd-01 | 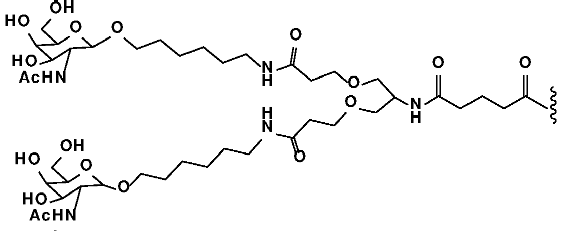 |
| 16 | 5'ERCc-01 | 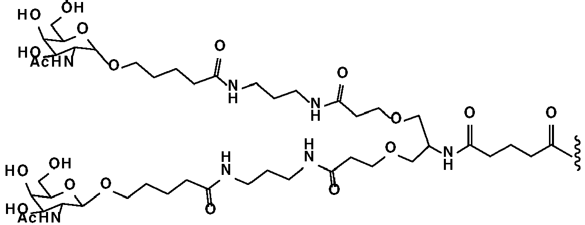 |
| 17 | 5'SANCdd-01 | 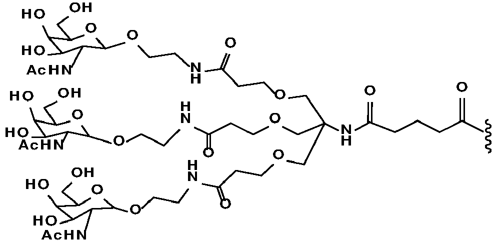 |
| 18 | 5'SANCd-01 | 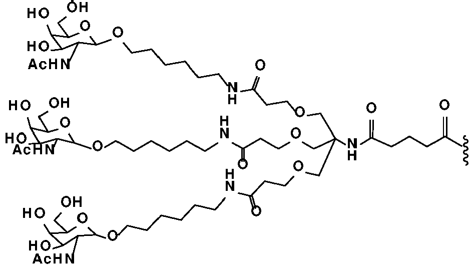 |
| 19 | 5'SANCc-01 | 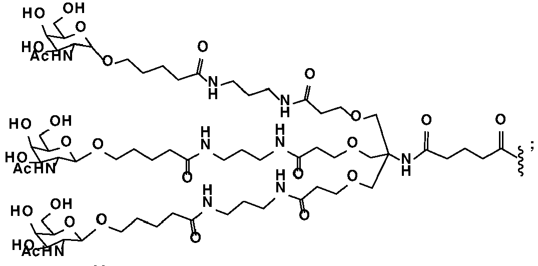 |
| 20 | 5'SANCa-01 | 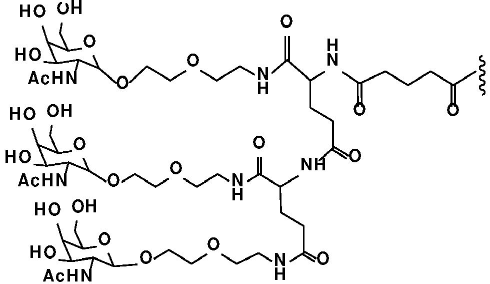 |
| 21 | 5'SANCa-02 | 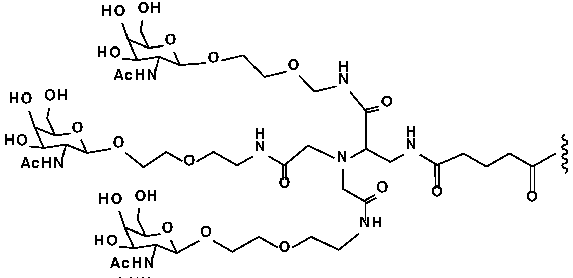 |
| 22 | 5'SANCa-03 | 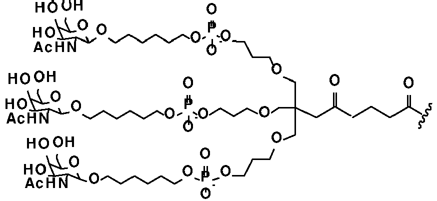 |

In some embodiments, 5'MVIP may not exist, and in this case m may be an integer of 2-4.

In some embodiments, **(X-L)ₘ-B-D-**in the 3'MVIP structure is selected from the structures shown in Table 9:

**Table 9 Structures of (X-L)ₘ-B-D- in 3'MVIP**

| Serial numb er | code | structure |
|---|---|---|
| 1 | 3'SANCdd-01 | 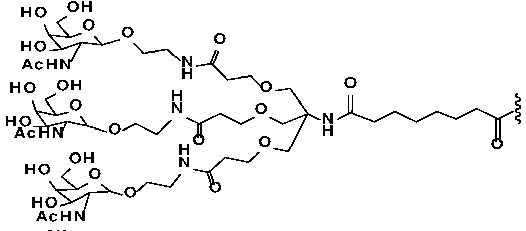 |
| 2 | 3'SANCd-01 | 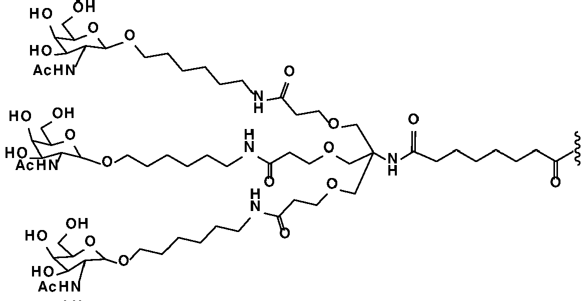 |
| 3 | 3'SANCc-01 | 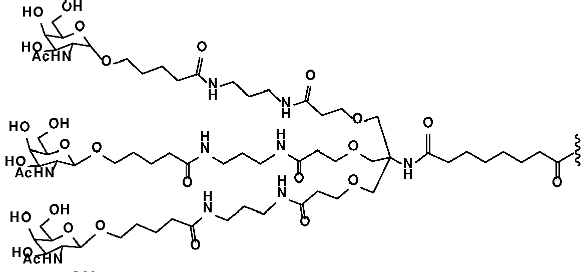 |
| 4 | 3'SANCa-01 | 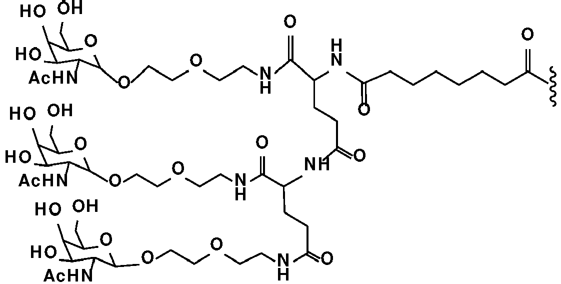 |
| 5 | 3'SANCa-02 | 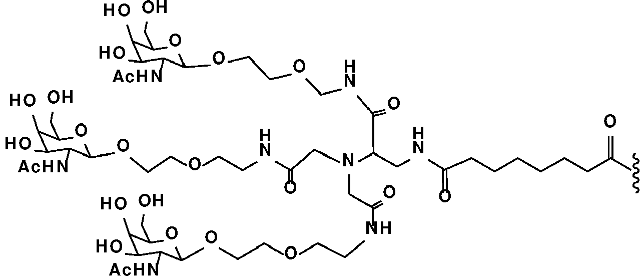 |
| 6 | 3'SANCa-03 | 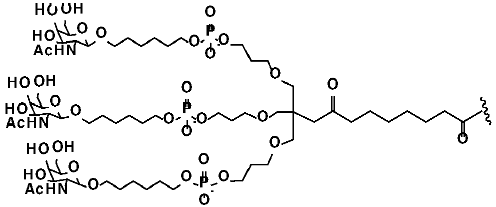 |
| 7 | 3'ERCdd-01 | 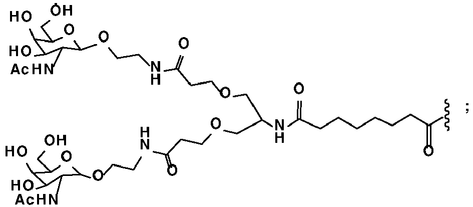 |
| 8 | 3'ERCd-01 | 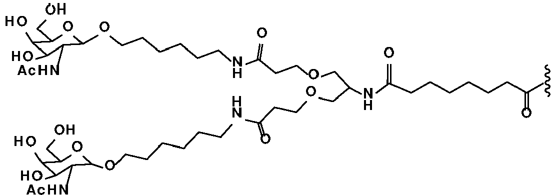 |
| 9 | 3'ERCc-01 | 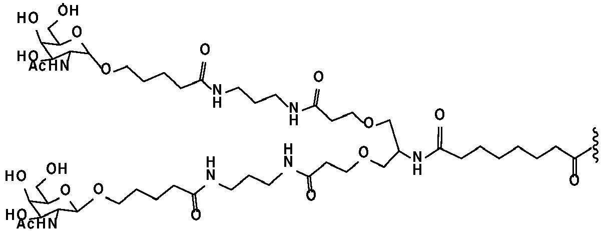 |
| 10 | 3'ERCa-01 | 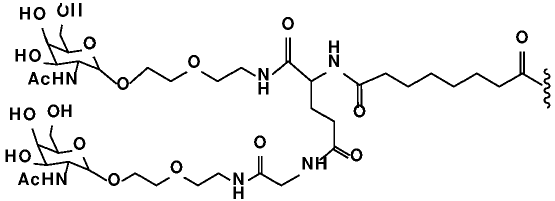 |
| 11 | 3'ERCa-02 | 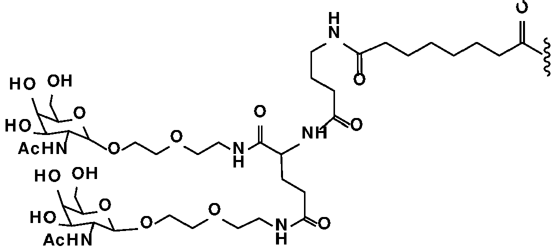 |
| 12 | 3'ERCa-03 | 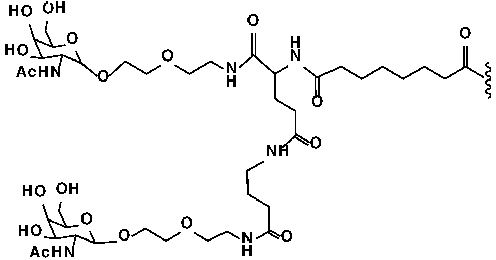 |
| 13 | 3'ERCa-04 | 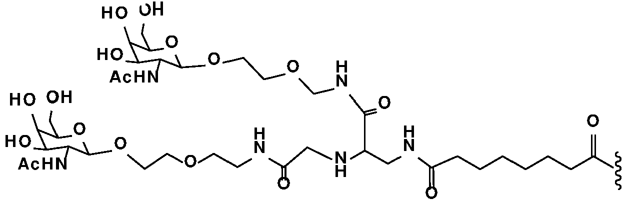 |
| 14 | 3'ERCa-05 | 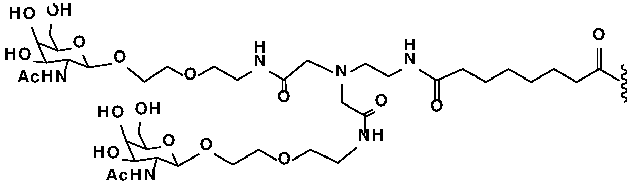 |
| 15 | 3'YICa-01 | 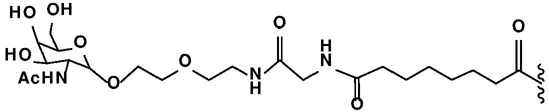 |
| 16 | 3'YICa-02 | 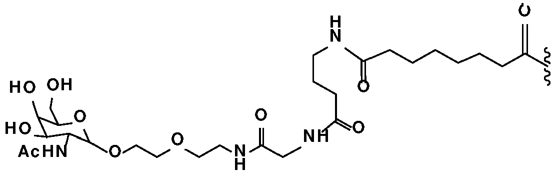 |
| 17 | 3'YICa-03 | 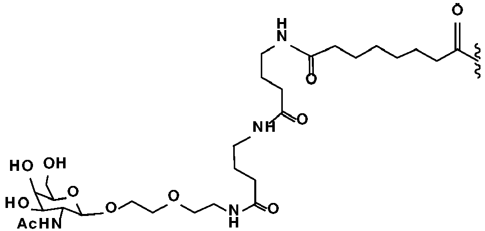 |
| 18 | 3'YICa-04 | 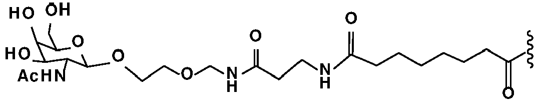 |
| 19 | 3'YICa-05 | 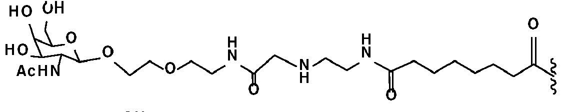 |
| 20 | 3'YICdd-01 | 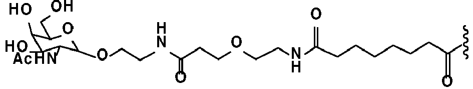 |
| 21 | 3'YICd-01 | 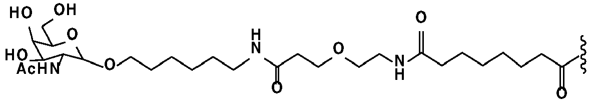 |
| 22 | 3'YICc-01 | 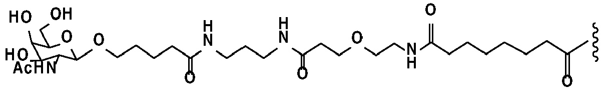 |

In some embodiments, the combination of R₁ and **(X-L)ₙ-B-D-** in the 5'MVIP ligand structure is shown in Table 10.

**Table 10 Combinations of R₁ and (X-L)ₙ-B-D- in 5 'MVIP**

| Seria l numb er | **(X-L)ₙ-B-D-**code | R₁ | 5'MVIP code |
|---|---|---|---|
| 1 | 5'YICd-01 | -NH(CH₂)₆O- | 5'MVIP01 |
| 2 | 5'YICc-01 | -NH(CH₂)₆O- | 5'MVIP02 |
| 3 | 5'YICa-01 | -O(CH₂)₆O- | 5'MVIP03 |
| 4 | 5'YICa-02 | -O(CH₂)₆O- | 5'MVIP04 |
| 5 | 5'YIC a-03 | -S(CH₂)₆O- | 5'MVIP05 |
| 6 | 5'YIC a-04 | -NH(CH₂)₆S- | 5'MVIP06 |
| 7 | 5'YICa-05 | -NH(CH₂)₈ O- | 5'MVIP07 |
| 8 | 5'YICr-06 | -NH(CH₂)₈O- | 5'MVIP08 |
| 9 | 5'ERCd-01 | -NH(CH₂)₆O- | 5'MVIP09 |
| 10 | 5'ERCc-01 | -NH(CH₂)₆O- | 5'MVIP10 |
| 11 | 5'ERCa-01 | -NH(CH₂)₅CH(CH₂CH₃)O- | 5'MVIP11 |
| 12 | 5'ERCa-02 | -O(CH₂)₆O- | 5'MVIP12 |

| Seria 1 numb er | **(X-L)ₙ-B-D-**code | R₁ | 5'MVIP code |
|---|---|---|---|
| 13 | 5'ERCa-03 | -S(CH₂)₆O- | 5'MVIP13 |
| 14 | 5'ERCa-04 | -O(CH₂)₆O- | 5'MVIP14 |
| 15 | 5'ERCa-05 | -O(CH₂)₆O- | 5'MVIP15 |
| 16 | 5'ERCr-06 | -S(CH₂)₄CH(CH₃)O- | 5'MVIP16 |
| 17 | 5'SANCd-01 | -NH(CH₂)₆O- | 5'MVIP17 |
| 18 | 5'SANCc-01 | -NH(CH₂)₆O- | 5'MVIP18 |
| 19 | 5'ERCd-01 | 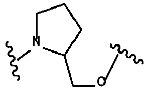 | 5'MVIP19 |
| 20 | 5'ERCd-01 | 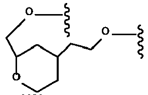 | 5'MVIP20 |
| 21 | 5'YICd-01 | 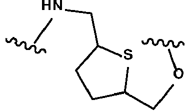 | 5'MVIP21 |
| 22 | 5'SANCd-01 | 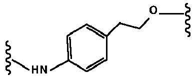 | 5'MVIP22 |

In some embodiments, 3'MVIP may not exist, and in this case n may be 2-4.

In some embodiments, the combination of R₂ and **(X-L)ₘ-B-D-** in the 3'MVIP ligand structure is shown in Table 11.

**Table 11 Combinations of R₂ and (X-L)ₘ-B-D- in 3'MVIP**

| Serial numbe r | **(X-L)m-B-D-** code | R₂ | 3'MVIP code |
|---|---|---|---|
| 1 | 3'YICd-01 | 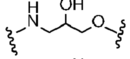 | 3'MVIP01 |
| 2 | 3'YICc-01 | 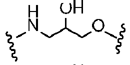 | 3'MVIP02 |
| 3 | 3'YICa-01 | 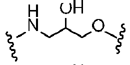 | 3'MVIP03 |
| 4 | 3'YICa-02 | 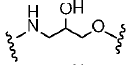 | 3'MVIP04 |
| 5 | 3'YIC a-03 | 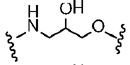 | 3'MVIP05 |
| 6 | 3'YIC a-04 | 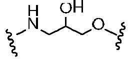 | 3'MVIP06 |
| 7 | 3'YICa-05 | 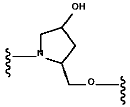 | 3'MVIP07 |
| 8 | 3'YICr-06 | 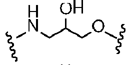 | 3'MVIP08 |
| 9 | 3'ERCd-01 | 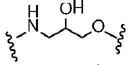 | 3'MVIP09 |
| 10 | 3'ERCc-01 | 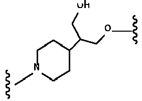 | 3'MVIP10 |
| 11 | 3'ERCa-01 | 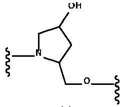 | 3'MVIP11 |
| 12 | 3'ERCa-02 | 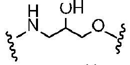 | 3'MVIP12 |
| 13 | 3'ERCa-03 | 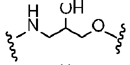 | 3'MVIP13 |
| 14 | 3'ERCa-04 | 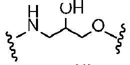 | 3'MVIP14 |
| 15 | 3'ERCa-05 | 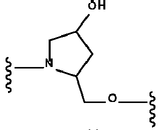 | 3'MVIP15 |
| 16 | 3'ERCr-06 | 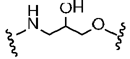 | 3'MVIP16 |
| 17 | 3'SANCd-01 | 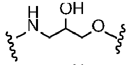 | 3'MVIP17 |
| 18 | 3'SANCc-01 | 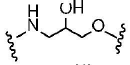 | 3'MVIP18 |
| 19 | 3'SANCa-01 | 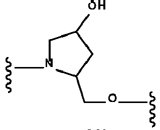 | 3'MVIP19 |
| 20 | 3'ERCd-01 | 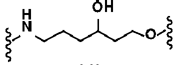 | 3'MVIP20 |
| 21 | 3'ERCd-01 | 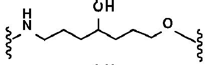 | 3'MVIP21 |
| 22 | 3'ERCd-01 | 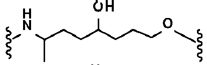 | 3'MVIP22 |
| 23 | 3'ERCd-01 | 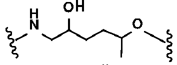 | 3'MVIP23 |
| 24 | 3'ERCd-01 | 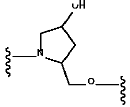 | 3'MVIP24 |
| 25 | 3'ERCd-01 | 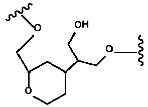 | 3'MVIP25 |
| 26 | 3'ERCd-01 | 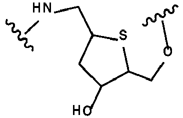 | 3'MVIP26 |
| 27 | 3'ERCd-01 | 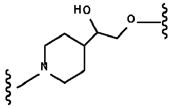 | 3'MVIP27 |

In some embodiments, the 5'MVIP is selected from any one of 5'MVIP01 to 5'MVIP22 in Table 10.

In some embodiments, the 5'MVIP is selected from:

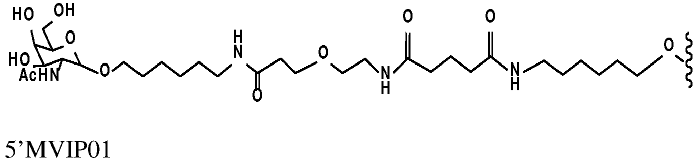

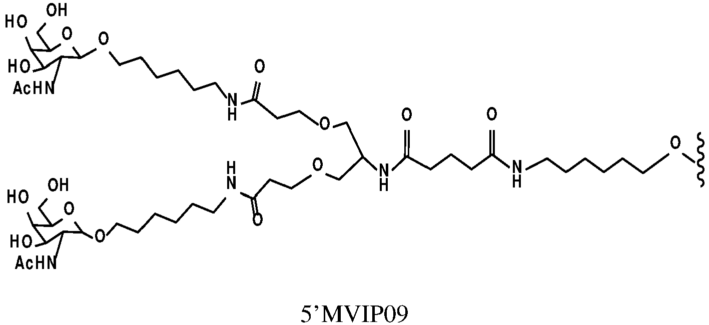

In some embodiments, the 3'MVIP is selected from any one of 3'MVIP01 to 3'MVIP27 in Table 11.

In some embodiments, the 3'MVIP is selected from:

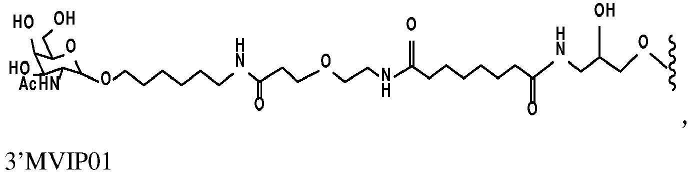

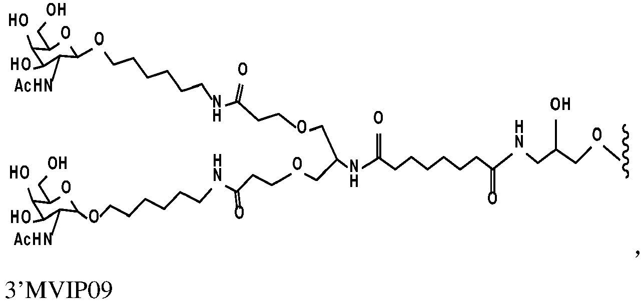

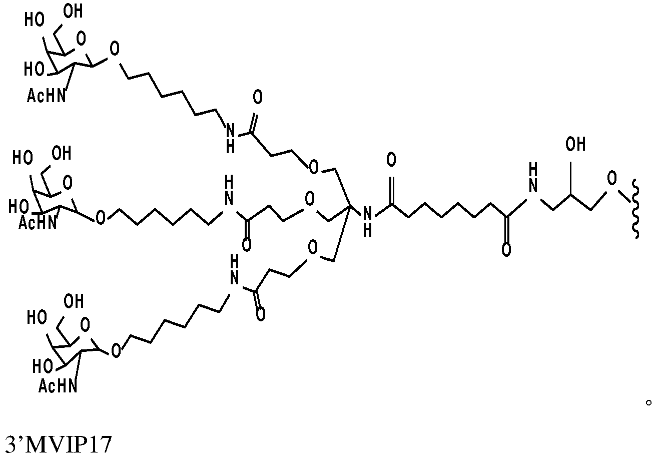

In some embodiments, by selecting different combinations of 5'MVIP and 3'MVIP in Table 12 to access different positions of the sense and/or antisense strands of the RNAi agent, including the terminal and intermediate positions of the sequence, the effect on AGT mRNA expression levels was examined.

**Table 12 Combinations of 5'MVIP and 3'MVIP**

| Se ria l nu m be r | 5'MVIP code | 5'MVIP structure | 3'MVIP code | 3'MVIP structure |
|---|---|---|---|---|
| 1 | 5'MVIP01 | 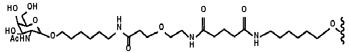 | 3'MVIP17 | 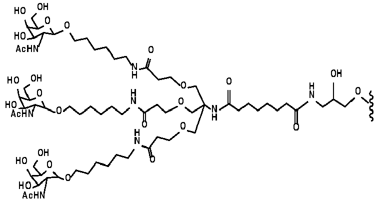 |
| 2 | 5'MVIP09 | 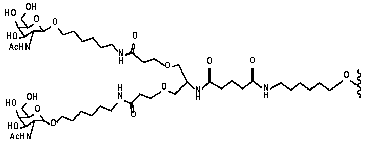 | 3'MVIP09 | 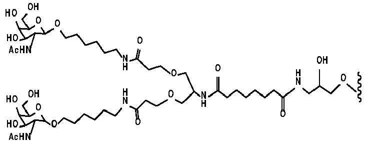 |
| 3 | 5'MVIP17 | 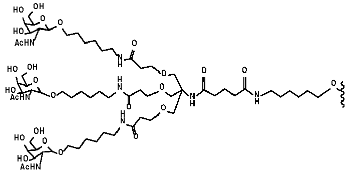 | 3'MVIP01 | 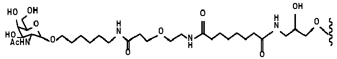 |
| 4 | 5'MVIP01 | 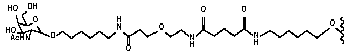 | 3'MVIP01 | 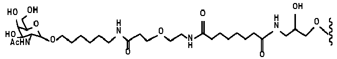 |
| 5 | 5'MVIP01 | 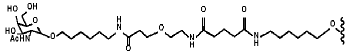 | 3' MVIP09 | 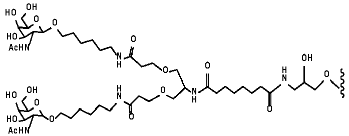 |
| 6 | 5'MVIP09 | 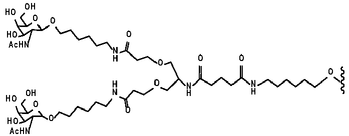 | 3'MVIP01 | 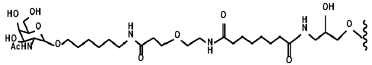 |

For example, the 3' end of the antisense strand sequence in Table 5 can be conjugated to the carrier structure 3'MVIP09. For example, the antisense strand in the RNAi agent can be selected from the sequences in the following Table 13.

**Table 13 Antisense strands conjugated to 3'MVIP**

| Single strand code | Antisense strand sequence 5'→ 3' |
|---|---|
| AS131 | UsUsUUGUfUUCACfAAfACAAGCsUsG-3'MVIP09 |
| AS132 | UsUsUAUUfACUAAfCAfCAAGGGsAsG-3'MVIP09 |
| AS133 | UsAsUACUfUUAAUfUUfUAAAACsCsC-3'MVIP09 |
| AS134 | UsAsCUUUfAAUUUfUAfAAACCCsAsA-3'MVIP09 |
| AS135 | UsUsGUUCfAAAAAfUCfACAAGCsAsU-3'MVIP09 |
| AS136 | UsUsAAUUfUUAAAfACfCCAAUUsUsU-3'MVIP09 |
| AS137 | AsUsACUUfUAAUUfUUfAAAACCsCsA-3'MVIP09 |
| AS138 | UsAsAAACfCCAAUfUUfUUGUUCsUsC-3'MVIP09 |
| AS139 | UsUsUUGCfAGCGAfCUfAGCACCsAsG-3'MVIP09 |
| AS140 | UsCsAAGCfUCAAAfAAfAAAUGCsUsG-3'MVIP09 |
| AS141 | UsUsUAAUfUUUAAfAAfCCCAAUsUsU-3'MVIP09 |
| AS142 | UsUsCAAAfAAUCAfCAfAGCAUCsUsG-3'MVIP09 |
| AS143 | UsAsAGCUfGUUGGfGUfAGACUCsUsG-3'MVIP09 |
| AS144 | UsUsCACAfAACAAfGCfUGGUCGsGsU-3'MVIP09 |
| AS145 | UsUsGUCCfUGGAUfGUfCACUCCsAsG-3'MVIP09 |
| AS146 | UsAsUUACfAGACAfCUfACACGGsAsG-3'MVIP09 |
| AS147 | UsUsUGUUfUCACAfAAfCAAGCUsGsG-3'MVIP09 |
| AS148 | UsUsUUGGfAACAGfUAfGUCCCGsCsG-3'MVIP09 |
| AS149 | AsAsGAAGfUUGGCfCAfGCAUsCsC-3'MVIP09 |
| AS150 | UsAsUACGfGAAGCfCCfAAGAsAsG-3'MVIP09 |
| AS151 | CsUsGUGCfAUGCCfAUfAUAUsAsC-3'MVIP09 |
| AS152 | CsAsUGGAfCCACGfCCfCCAUsAsG-3'MVIP09 |
| AS153 | AsAsAGACfAGCCGfUUfGGGGsAsG-3'MVIP09 |
| AS154 | UsCsUUGUfCCACCfCAfGAACsUsC-3'MVIP09 |
| AS155 | AsGsACCCfUCCACfCUfUGUCsCsA-3'MVIP09 |
| AS156 | AsGsUGAGfACCCUfCCfACCUsUsG-3'MVIP09 |
| AS157 | AsAsAGUGfAGACCfCUfCCACsCsU-3'MVIP09 |
| AS158 | GsUsUGAGfGGAGUfUUfUGCUsGsG-3'MVIP09 |
| AS159 | AsGsUUGAfGGGAGfUUfUUGCsUsG-3'MVIP09 |
| AS160 | UsCsCAGUfUGAGGfGAfGUUUsUsG-3'MVIP09 |
| AS161 | UsCsUUCAfUCCAGfUUfGAGGsGsA-3'MVIP09 |
| AS162 | UsUsCUUCfAUCCAfGUfUGAGsGsG-3'MVIP09 |
| AS163 | AsGsUUUCfUUCAUfCCfAGUUsGsA-3'MVIP09 |
| AS164 | UsUsGCUCfAAUUUfUUfGCAGsGsU-3'MVIP09 |
| AS165 | CsAsUUGCfUCAAUfUUfUUGCsAsG-3'MVIP09 |
| AS166 | UsCsAUUGfCUCAAfUUfUUUGsCsA-3'MVIP09 |
| AS167 | GsUsCAUUfGCUCAfAUfUUUUsGsC-3'MVIP09 |
| AS168 | UsGsUGGGfCUCUCfUCfUCAUsCsC-3'MVIP09 |
| AS169 | UsUsGAUCfAUACAfCAfGCAAsAsC-3'MVIP09 |
| AS170 | UsUsUGAUfCAUACfACfAGCAsAsA-3'MVIP09 |
| AS171 | AsAsAGGUfGGGAGfACfUGGGsGsG-3'MVIP09 |
| AS172 | CsAsUUAGfAAGAAfAAfGGUGsGsG-3'MVIP09 |
| AS173 | UsCsAUUAfGAAGAfAAfAGGUsGsG-3'MVIP09 |
| AS174 | CsUsCAUUfAGAAGfAAfAAGGsUsG-3'MVIP09 |
| AS175 | UsCsGGUUfGGAAUfUCfUUUUsUsG-3'MVIP09 |
| AS176 | AsAsACAAfGCUGGfUCfGGUUsGsG-3'MVIP09 |
| AS177 | UsCsACAAfACAAGfCUfGGUCsGsG-3'MVIP09 |
| AS178 | UsUsCACAfAACAAfGCfUGGUsCsG-3'MVIP09 |
| AS179 | UsUsUCACfAAACAfAGfCUGGsUsC-3'MVIP09 |
| AS180 | UsUsUUGUfUUCACfAAfACAAsGsC-3'MVIP09 |
| AS181 | UsUsUUUGfUUUCAfCAfAACAsAsG-3'MVIP09 |
| AS182 | UsUsUUUUfGUUUCfACfAAACsAsA-3'MVIP09 |
| AS183 | AsCsUUUUfUUGUUfUCfACAAsAsC-3'MVIP09 |
| AS184 | AsCsACUUfUUUUGfUUfUCACsAsA-3'MVIP09 |
| AS185 | AsAsAAGGfGAACAfCUfUUUUsUsG-3'MVIP09 |
| AS186 | UsCsUCAAfCUUGAfAAfAGGGsAsA-3'MVIP09 |
| AS187 | UsGsUUCUfCAACUfUGfAAAAsGsG-3'MVIP09 |
| AS188 | AsAsCCCAfAUUUUfUGfUUCUsCsA-3'MVIP09 |
| AS189 | UsAsAAACfCCAAUfUUfUUGUsUsC-3'MVIP09 |
| AS190 | UsUsUUAAfAACCCfAAfUUUUsUsG-3'MVIP09 |
| AS191 | AsUsUCAAfGACACfUAfAAUAsCsA-3'MVIP09 |
| AS192 | UsCsUUACfAUUCAfAGfACACsUsA-3'MVIP09 |
| AS193 | UsCsAUGUfUCUUAfCAfUUCAsAsG-3'MVIP09 |
| AS194 | GsUsCAUGfUUCUUfACfAUUCsAsA-3'MVIP09 |
| AS195 | AsUsCUGUfGGAAAfAAfACUAsAsG-3'MVIP09 |
| AS196 | AsAsAUCAfCAAGCfAUfCUGUsGsG-3'MVIP09 |
| AS197 | AsAsAAAUfCACAAfGCfAUCUsGsU-3'MVIP09 |
| AS198 | CsGsGACAfAAUCAfGCfGAUGUsGsU-3'MVIP09 |
| AS199 | CsCsAAAAfAGAAUfUCfCAAUUsGsA-3'MVIP09 |
| AS200 | AsCsCGACfCAGCUfUGfUUUGUsGsA-3'MVIP09 |
| AS201 | AsGsCGCGfGGACUfACfUGUUCsCsA-3'MVIP09 |
| AS202 | GsCsGCGGfGACUAfCUfGUUCCsAsA-3'MVIP09 |
| AS203 | AsAsCCGAfCCAGCfUUfGUUUGsUsG-3'MVIP09 |
| AS204 | UsGsUUCCfCUUUUfCAfAGUUGsAsG-3'MVIP09 |
| AS205 | UsCsCCUUfUUCAAfGUfUGAGAsAsC-3'MVIP09 |
| AS206 | UsAsUACUfCUCAUfUGfUGGAUGsAsC-3'MVIP09 |

In some embodiments, the antisense strand of the RNAi agent in this application differs from each sequence in Table 13 by one, two or three nucleotides.

For example, the 5' end of the sense strand sequence in Table 6 can be conjugated to the carrier structure 5'MVIP09. For example, the sense strand in the RNAi agent can be selected from the sequences in the following Table 14.

**Table 14 Sense strands conjugated to 5'MVIP**

| Single strand code | Sense strand sequence 5'→ 3' |
|---|---|
| S131 | 5'MVIP09-GsCsUUfGUfUfUfGUGAAACAAAAsAsA |
| S132 | 5'MVIP09-CsCsCUfUGfUfGfUUAGUAAUAAAsCsG |
| S133 | 5'MVIP09-GsUsUUfUAfAfAfAUUAAAGUAUAsCsA |
| S134 | 5'MVIP09-GsGsGUfUUfUfAfAAAUUAAAGUAsUsA |
| S135 | 5'MVIP09-GsCsUUfGUfGfAfUUUUUGAACAAsUsA |
| S136 | 5'MVIP09-AsAsUUfGGfGfUfUUUAAAAUUAAsAsG |
| S137 | 5'MVIP09-GsGsUUfUUfAfAfAAUUAAAGUAUsAsC |
| S138 | 5'MVIP09-GsAsACfAAfAfAfAUUGGGUUUUAsAsA |
| S139 | 5'MVIP09-GsGsUGfCUfAfGfUCGCUGCAAAAsCsU |
| S140 | 5'MVIP09-GsCsAUfUUfUfUfUUUGAGCUUGAsAsG |
| S141 | 5'MVIP09-AsUsUGfGGfUfUfUUAAAAUUAAAsGsU |
| S142 | 5'MVIP09-GsAsUGfCUfUfGfUGAUUUUUGAAsCsA |
| S143 | 5'MVIP09-GsAsGUfCUfAfCfCCAACAGCUUAsAsC |
| S144 | 5'MVIP09-CsGsACfCAfGfCfUUGUUUGUGAAsAsC |
| S145 | 5'MVIP09-GsGsAGfUGfAfCfAUCCAGGACAAsCsU |
| S146 | 5'MVIP09-CsCsGUfGUfAfGfUGUCUGUAAUAsCsC |
| S147 | 5'MVIP09-AsGsCUfUGfUfUfUGUGAAACAAAsAsA |
| S148 | 5'MVIP09-CsGsGGfACfUfAfCUGUUCCAAAAsAsG |
| S149 | 5'MVIP09-GsGsAUfGCfUfGfGCCAACUUCsUsU |
| S150 | 5'MVIP09-CsUsUCfUUfGfGfGCUUCCGUAsUsA |
| S151 | 5'MVIP09-GsUsAUfAUfAfUfGGCAUGCACsAsG |
| S152 | 5'MVIP09-CsUsAUfGGfGfGfCGUGGUCCAsUsG |
| S153 | 5'MVIP09-CsUsCCfCCfAfAfCGGCUGUCUsUsU |
| S154 | 5'MVIP09-GsCsUGfUGfAfCfAGGAUGGAAsGsA |
| S155 | 5'MVIP09-UsGsGAfCAfAfGfGUGGAGGGUsCsU |
| S156 | 5'MVIP09-CsAsAGfGUfGfGfAGGGUCUCAsCsU |
| S157 | 5'MVIP09-AsGsGUfGGfAfGfGGUCUCACUsUsU |
| S158 | 5'MVIP09-CsCsAGfCAfAfAfACUCCCUCAsAsC |
| S159 | 5'MVIP09-CsAsGCfAAfAfAfCUCCCUCAAsCsU |
| S160 | 5'MVIP09-CsAsAAfACfUfCfCCUCAACUGsGsA |
| S161 | 5'MVIP09-UsCsCCfUCfAfAfCUGGAUGAAsGsA |
| S162 | 5'MVIP09-CsCsCUfCAfAfCfUGGAUGAAGsAsA |
| S163 | 5'MVIP09-UsCsAAfCUfGfGfAUGAAGAAAsCsU |
| S164 | 5'MVIP09-AsCsCUfGCfAfAfAAAUUGAGCsAsA |
| S165 | 5'MVIP09-CsUsGCfAAfAfAfAUUGAGCAAsUsG |
| S166 | 5'MVIP09-UsGsCAfAAfAfAfUUGAGCAAUsGsA |
| S167 | 5'MVIP09-GsCsAAfAAfAfUfUGAGCAAUGsAsC |
| S168 | 5'MVIP09-GsGsAUfGAfGfAfGAGAGCCCAsCsA |
| S169 | 5'MVIP09-GsUsUUfGCfUfGfUGUAUGAUCsAsA |
| S170 | 5'MVIP09-UsUsUGfCUfGfUfGUAUGAUCAsAsA |
| S171 | 5'MVIP09-CsCsCCfCAfGfUfCUCCCACCUsUsU |
| S172 | 5'MVIP09-CsCsCAfCCfUfUfUUCUUCUAAsUsG |
| S173 | 5'MVIP09-CsCsACfCUfUfUfUCUUCUAAUsGsA |
| S174 | 5'MVIP09-CsAsCCfUUfUfUfCUUCUAAUGsAsG |
| S175 | 5'MVIP09-CsAsAAfAAfGfAfAUUCCAACCsGsA |
| S176 | 5'MVIP09-CsCsAAfCCfGfAfCCAGCUUGUsUsU |
| S177 | 5'MVIP09-CsCsGAfCCfAfGfCUUGUUUGUsGsA |
| S178 | 5'MVIP09-CsGsACfCAfGfCfUUGUUUGUGsAsA |
| S179 | 5'MVIP09-GsAsCCfAGfCfUfUGUUUGUGAsAsA |
| S180 | 5'MVIP09-GsCsUUfGUfUfUfGUGAAACAAsAsA |
| S181 | 5'MVIP09-CsUsUGfUUfUfGfUGAAACAAAsAsA |
| S182 | 5'MVIP09-UsUsGUfUUfGfUfGAAACAAAAsAsA |
| S183 | 5'MVIP09-GsUsUUfGUfGfAfAACAAAAAAsGsU |
| S184 | 5'MVIP09-UsUsGUfGAfAfAfCAAAAAAGUsGsU |
| S185 | 5'MVIP09-CsAsAAfAAfAfGfUGUUCCCUUsUsU |
| S186 | 5'MVIP09-UsUsCCfCUfUfUfUCAAGUUGAsGsA |
| S187 | 5'MVIP09-CsCsUUfUUfCfAfAGUUGAGAAsCsA |
| S188 | 5'MVIP09-UsGsAGfAAfCfAfAAAAUUGGGsUsU |
| S189 | 5'MVIP09-GsAsACfAAfAfAfAUUGGGUUUsUsA |
| S190 | 5'MVIP09-CsAsAAfAAfUfUfGGGUUUUAAsAsA |
| S191 | 5'MVIP09-UsGsUAfUUfUfAfGUGUCUUGAsAsU |
| S192 | 5'MVIP09-UsAsGUfGUfCfUfUGAAUGUAAsGsA |
| S193 | 5'MVIP09-CsUsUGfAAfUfGfUAAGAACAUsGsA |
| S194 | 5'MVIP09-UsUsGAfAUfGfUfAAGAACAUGsAsC |
| S195 | 5'MVIP09-CsUsUAfGUfUfUfUUUCCACAGsAsU |
| S196 | 5'MVIP09-CsCsACfAGfAfUfGCUUGUGAUsUsU |
| S197 | 5'MVIP09-AsCsAGfAUfGfCfUUGUGAUUUsUsU |
| S198 | 5'MVIP09-AsCsACfAUfCfGfCUGAUUUGUCsCsG |
| S199 | 5'MVIP09-UsCsGGfUUfGfGfAAUUCUUUUUsGsG |
| S200 | 5'MVIP09-UsCsACfAAfAfCfAAGCUGGUCGsGsU |
| S201 | 5'MVIP09-UsGsGAfACfAfGfUAGUCCCGCGsCsU |
| S202 | 5'MVIP09-UsUsGGfAAfCfAfGUAGUCCCGCsGsC |
| S203 | 5'MVIP09-CsAsCAfAAfCfAfAGCUGGUCGGsUsU |
| S204 | 5'MVIP09-CsUsCAfACfUfUfGAAAAGGGAAsCsA |
| S205 | 5'MVIP09-GsUsUCfUCfAfAfCUUGAAAAGGsGsA |
| S206 | 5'MVIP09-GsUsCAfUCfCfAfCAAUGAGAGUAsCsA |

In some embodiments, the sense strand of the RNAi agent in this application differs from each sequence in Table 14 by one, two or three nucleotides.

AGT is mainly expressed in the liver, and its expression is limited to humans and non primates. In some in vivo embodiments, monkeys are the preferred model for preclinical studies. In some in vivo embodiments, the RNAi agent is selected from the sequences in Table 15.

**Table 15 RNAi agents containing 5'MVIP09/3'MVIP09 combination**

| Singl e stran d code | Sense strand sequence 5'→ 3' | Single strand code | Antisense strand sequence 5'→ 3' | Double strand code |
|---|---|---|---|---|
| S131 | 5'MVIP09-GsCsUUfGUfUfUfGUGAAACAAA AsAsA | AS131 | UsUsUUGUfUUCACfAAfACAAGCs UsG-3'MVIP09 | Kylo-09-DS113 |
| S132 | 5'MVIP09-CsCsCUfUGfUfGfUUAGUAAUAA AsCsG | AS132 | UsUsUAUUfACUAAfCAfCAAGGGs AsG-3'MVIP09 | Kylo-09-DS114 |
| S133 | 5'MVIP09-GsUsUUfUAfAfAfAUUAAAGUA UAsCsA | AS133 | UsAsUACUfUUAAUfUUfUAAAACs CsC-3'MVIP09 | Kylo-09-DS115 |
| S134 | 5'MVIP09-GsGsGUfUUfUfAfAAAUUAAAG UAsUsA | AS 134 | UsAsCUUUfAAUUUfUAfAAACCCs AsA-3'MVIP09 | Kylo-09-DS116 |
| S135 | 5'MVIP09-GsCsUUfGUfGfAfUUUUUGAACA AsUsA | AS 135 | UsUsGUUCfAAAAAfUCfACAAGCs AsU-3'MVIP09 | Kylo-09-DS117 |
| S136 | 5'MVIP09-AsAsUUfGGfGfUfUUUAAAAUU AAsAsG | AS136 | UsUsAAUUfUUAAAfACfCCAAUUs UsU-3'MVIP09 | Kylo-09-DS118 |
| S137 | 5'MVIP09-GsGsUUfUUfAfAfAAUUAAAGU AUsAsC | AS 137 | AsUsACUUfUAAUUfUUfAAAACCs CsA-3'MVIP09 | Kylo-09-DS119 |
| S138 | 5'MVIP09-GsAsACfAAfAfAfAUUGGGUUU UAsAsA | AS138 | UsAsAAACfCCAAUfUUfUUGUUCs UsC-3'MVIP09 | Kylo-09-DS120 |
| S139 | 5'MVIP09-GsGsUGfCUfAfGfUCGCUGCAAA AsCsU | AS139 | UsUsUUGCfAGCGAfCUfAGCACCs AsG -3'MVIP09 | Kylo-09-DS121 |
| S140 | 5'MVIP09-GsCsAUfUUfUfUfUUUGAGCUUG AsAsG | AS 140 | UsCsAAGCfUCAAAfAAfAAAUGCs UsG-3'MVIP09 | Kylo-09-DS122 |
| S141 | 5'MVIP09-AsUsUGfGGfUfUfUUAAAAUUA AAsGsU | AS141 | UsUsUAAUfUUUAAfAAfCCCAAUs UsU-3'MVIP09 | Kylo-09-DS123 |
| S142 | 5'MVIP09-GsAsUGfCUfUfGfUGAUUUUUG AAsCsA | AS 142 | UsUsCAAAfAAUCAfCAfAGCAUCs UsG-3'MVIP09 | Kylo-09-DS124 |
| S143 | 5'MVIP09-GsAsGUfCUfAfCfCCAACAGCUU AsAsC | AS 143 | UsAsAGCUfGUUGGfGUfAGACUCs UsG-3'MVIP09 | Kylo-09-DS125 |
| S144 | 5'MVIP09-CsGsACfCAfGfCfUUGUUUGUGA AsAsC | AS 144 | UsUsCACAfAACAAfGCfUGGUCGs GsU-3'MVIP09 | Kylo-09-DS126 |
| S145 | 5'MVIP09-GsGsAGfUGfAfCfAUCCAGGACA AsCsU | AS145 | UsUsGUCCfUGGAUfGUfCACUCCs AsG-3'MVIP09 | Kylo-09-DS127 |
| S146 | 5'MVIP09-CsCsGUfGUfAfGfUGUCUGUAAU AsCsC | AS 146 | UsAsUUACfAGACAfCUfACACGGs AsG-3'MVIP09 | Kylo-09-DS 128 |
| S147 | 5'MVIP09-AsGsCUfUGfUfUfUGUGAAACAA AsAsA | AS 147 | UsUsUGUUfUCACAfAAfCAAGCUs GsG-3'MVIP09 | Kylo-09-DS129 |
| S148 | 5'MVIP09-CsGsGGfACfUfAfCUGUUCCAAA AsAsG | AS148 | UsUsUUGGfAACAGfUAfGUCCCGs CsG-3'MVIP09 | Kylo-09-DS130 |

In some embodiments, the sense and antisense strands of the RNAi agent in this application differ from each sequence in Table 15 by one, two or three nucleotides.

In some embodiments, the 5' end and / or 3' end of the antisense strand UsCsAAGCfUCAAAfAAfAAAUGCsUsG (SEQ ID NO: 118) of the RNAi agent are connected to 5'MVIP and / or 3'MVIP with different structures. The antisense strand is selected from Table 16 below:

**Table 16 5'MVIP and / or 3'MVIP conjugated antisense strand**

| Single strand code | Antisense strand sequence 5'→ 3' |
|---|---|
| AS140 | UsCsAAGCfUCAAAfAAfAAAUGCsUsG-3'MVIP09 |
| AS207 | 5' MVIP17-UsCsAAGCfUCAAAfAAfAAAUGCsUsG |
| AS208 | UsCsAAGCfUCAAAfAAfAAAUGCsUsG -3' MVIP01 |
| AS209 | UsCsAAGCfUCAAAfAAfAAAUGCsUsG -3' MVIP17 |
| AS210 | 5' MVIP01-UsCsAAGCfUCAAAfAAfAAAUGCsUsG -3' MVIP01 |
| AS211 | 5' MVIP09-UsCsAAGCfUCAAAfAAfAAAUGCsUsG -3' MVIP09 |
| AS212 | 5' MVIP17-UsCsAAGCfUCAAAfAAfAAAUGCsUsG -3' MVIP17 |
| AS213 | 5' MVIP01-UsCsAAGCfUCAAAfAAfAAAUGCsUsG -3' MVIP17 |
| AS214 | 5' MVIP17-UsCsAAGCfUCAAAfAAfAAAUGCsUsG -3' MVIP01 |
| AS215 | 5' MVIP01-UsCsAAGCfUCAAAfAAfAAAUGCsUsG -3' MVIP09 |
| AS216 | 5' MVIP09-UsCsAAGCfUCAAAfAAfAAAUGCsUsG -3' MVIP01 |
| AS217 | 5' MVIP09-UsCsAAGCfUCAAAfAAfAAAUGCsUsG -3' MVIP17 |
| AS218 | 5' MVIP17-UsCsAAGCfUCAAAfAAfAAAUGCsUsG -3' MVIP09 |
| AS219 | 5' MVIP12-UsCsAAGCfUCAAAfAAfAAAUGCsUsG |
| AS220 | UsCsAAGCfUCAAAfAAfAAAUGCsUsG-3' MVIP19 |
| AS221 | 5'MVIP16- UsCsAAGCfUCAAAfAAfAAAUGCsUsG -3'MVIP16 |
| AS222 | UsCsAAGCfUCAAAfAAfAAAUGCsUsG-3' MVIP17 |
| AS223 | UsCsAAGCfUCAAAfAAfAAAUGCsUsG-3' MVIP18 |
| AS224 | 5' MVIP03-UsCsAAGCfUCAAAfAAfAAAUGCsUsG |
| AS225 | 5' MVIP08-UsCsAAGCfUCAAAfAAfAAAUGCsUsG |
| AS226 | 5'MVIP16-UsCsAAGCfUCAAAfAAfAAAUGCsUsG |
| AS227 | 5'MVIP13-UsCsAAGCfUCAAAfAAfAAAUGCsUsG-3'MVIP06 |
| AS228 | 5'MVIP04-UsCsAAGCfUCAAAfAAfAAAUGCsUsG-3'MVIP06 |
| AS229 | 5' MVIP11-UsCsAAGCfUCAAAfAAfAAAUGCsUsG |
| AS230 | 5' MVIP11-UsCsAAGCfUCAAAfAAfAAAUGCsUsG-3' MVIP14 |
| AS231 | 5' MVIP15-UsCsAAGCfUCAAAfAAfAAAUGCsUsG |
| AS232 | 5' MVIP02-UsCsAAGCfUCAAAfAAfAAAUGCsUsG |
| AS233 | 5' MVIP05-UsCsAAGCfUCAAAfAAfAAAUGCsUsG |
| AS234 | 5' MVIP06-UsCsAAGCfUCAAAfAAfAAAUGCsUsG |
| AS235 | 5' MVIP07-UsCsAAGCfUCAAAfAAfAAAUGCsUsG |
| AS236 | 5' MVIP10- UsCsAAGCfUCAAAfAAfAAAUGCsUsG |
| AS237 | 5' MVIP14- UsCsAAGCfUCAAAfAAfAAAUGCsUsG |
| AS238 | 5' MVIP18- UsCsAAGCfUCAAAfAAfAAAUGCsUsG |
| AS239 | UsCsAAGCfUCAAAfAAfAAAUGCsUsG -3' MVIP02 |
| AS240 | UsCsAAGCfUCAAAfAAfAAAUGCsUsG-3' MVIP03 |
| AS241 | UsCsAAGCfUCAAAfAAfAAAUGCsUsG -3' MVIP04 |
| AS242 | 5' MVIP04-UsCsAAGCfUCAAAfAAfAAAUGCsUsG-3' MVIP04 |
| AS243 | 5' MVIP03-UsCsAAGCfUCAAAfAAfAAAUGCsUsG -3' MVIP19 |
| AS244 | 5' MVIP18- UsCsAAGCfUCAAAfAAfAAAUGCsUsG-3' MVIP18 |
| AS245 | 5' MVIP08- UsCsAAGCfUCAAAfAAfAAAUGCsUsG-3' MVIP18 |
| AS246 | UsCsAAGCfUCAAAfAAfAAAUGCsUsG -3' MVIP05 |
| AS247 | UsCsAAGCfUCAAAfAAfAAAUGCsUsG -3' MVIP07 |
| AS248 | UsCsAAGCfUCAAAfAAfAAAUGCsUsG -3' MVIP10 |
| AS249 | UsCsAAGCfUCAAAfAAfAAAUGCsUsG -3' MVIP11 |
| AS250 | 5' MVIP11- UsCsAAGCfUCAAAfAAfAAAUGCsUsG-3' MVIP11 |
| AS251 | 5' MVIP15- UsCsAAGCfUCAAAfAAfAAAUGCsUsG-3' MVIP15 |
| AS252 | UsCsAAGCfUCAAAfAAfAAAUGCsUsG -3' MVIP06 |
| AS253 | UsCsAAGCfUCAAAfAAfAAAUGCsUsG -3' MVIP08 |
| AS254 | UsCsAAGCfUCAAAfAAfAAAUGCsUsG -3' MVIP12 |
| AS255 | UsCsAAGCfUCAAAfAAfAAAUGCsUsG -3' MVIP13 |
| AS256 | UsCsAAGCfUCAAAfAAfAAAUGCsUsG -3' MVIP14 |
| AS257 | UsCsAAGCfUCAAAfAAfAAAUGCsUsG -3' MVIP15 |
| AS258 | UsCsAAGCfUCAAAfAAfAAAUGCsUsG -3' MVIP16 |
| AS259 | UsCsAAGCfUCAAAfAAfAAAUGCsUsG -3' MVIP24 |
| AS260 | UsCsAAGCfUCAAAfAAfAAAUGCsUsG -3' MVIP27 |
| AS261 | 5' MVIP19-UsCsAAGCfUCAAAfAAfAAAUGCsUsG |
| AS262 | 5' MVIP20-UsCsAAGCfUCAAAfAAfAAAUGCsUsG |
| AS263 | 5' MVIP21-UsCsAAGCfUCAAAfAAfAAAUGCsUsG |
| AS264 | 5' MVIP22-UsCsAAGCfUCAAAfAAfAAAUGCsUsG |
| AS265 | 5' MVIP01-UsCsAAGCfUCAAAfAAfAAAUGCsUsG |
| AS266 | 5' MVIP09-UsCsAAGCfUCAAAfAAfAAAUGCsUsG |

In some embodiments, the antisense strand of the RNAi agent in this application differs from each sequence in Table 16 by one, two or three nucleotides.

In some embodiments, the 5' end and / or 3' end of the sense strand GsCsAUfUUfUfUfUUUGAGCUUGAsAsG (SEQ ID NO: 194) of the RNAi agent are connected to 5'MVIP and / or 3'MVIP with different structures, and the sense strand is selected from table 17 below.

**Table 17 5'MVIP and / or 3'MVIP conjugated sense strand**

| Single strand code | Sense strand sequence 5'→ 3' |
|---|---|
| S140 | 5'MVIP09-GsCsAUfUUfUfUfUUUGAGCUUGAsAsG |
| S207 | GsCsAUfUUfUfUfUUUGAGCUUGAsAsG - 3'MVIP17 |
| S208 | GsCsAUfUUfUfUfUUUGAGCUUGAsAsG -3'MVIP09 |
| S209 | GsCsAUfUUfUfUfUUUGAGCUUGAsAsG-3'MVIP01 |
| S210 | 5'MVIP17- GsCsAUfUUfUfUfUUUGAGCUUGAsAsG |
| S211 | 5'MVIP01- GsCsAUfUUfUfUfUUUGAGCUUGAsAsG |
| S212 | 5'MVIP01-GsCsAUfUUfUfUfUUUGAGCUUGAsAsG -3'MVIP01 |
| S213 | 5'MVIP09-GsCsAUfUUfUfUfUUUGAGCUUGAsAsG -3'MVIP09 |
| S214 | 5'MVIP17-GsCsAUfUUfUfUfUUUGAGCUUGAsAsG -3'MVIP17 |
| S215 | 5'MVIP01-GsCsAUfUUfUfUfUUUGAGCUUGAsAsG -3'MVIP17 |
| S216 | 5'MVIP17-GsCsAUfUUfUfUfUUUGAGCUUGAsAsG -3'MVIP01 |
| S217 | 5'MVIP01-GsCsAUfUUfUfUfUUUGAGCUUGAsAsG -3'MVIP09 |
| S218 | 5'MVIP09-GsCsAUfUUfUfUfUUUGAGCUUGAsAsG -3'MVIP01 |
| S219 | 5'MVIP09-GsCsAUfUUfUfUfUUUGAGCUUGAsAsG -3'MVIP17 |
| S220 | 5'MVIP17-GsCsAUfUUfUfUfUUUGAGCUUGAsAsG -3'MVIP09 |
| S221 | 5'MVIP12-GsCsAUfUUfUfUfUUUGAGCUUGAsAsG |
| S222 | GsCsAUfUUfUfUfUUUGAGCUUGAsAsG -3'MVIP19 |
| S223 | 5'MVIP16-GsCsAUfUUfUfUfUUUGAGCUUGAsAsG -3'MVIP16 |
| S224 | GsCsAUfUUfUfUfUUUGAGCUUGAsAsG -3'MVIP17 |
| S225 | GsCsAUfUUfUfUfUUUGAGCUUGAsAsG -3'MVIP18 |
| S226 | 5' MVIP03-GsCsAUfUUfUfUfUUUGAGCUUGAsAsG |
| S227 | 5' MVIP08-GsCsAUfUUfUfUfUUUGAGCUUGAsAsG |
| S228 | 5' MVIP16-GsCsAUfUUfUfUfUUUGAGCUUGAsAsG |
| S229 | 5'MVIP13-GsCsAUfUUfUfUfUUUGAGCUUGAsAsG -3'MVIP06 |
| S230 | 5'MVIP04-GsCsAUfUUfUfUfUUUGAGCUUGAsAsG -3'MVIP06 |
| S231 | 5'MVIP11-GsCsAUfUUfUfUfUUUGAGCUUGAsAsG |
| S232 | 5'MVIP11-GsCsAUfUUfUfUfUUUGAGCUUGAsAsG -3'MVIP14 |
| S233 | 5'MVIP15-GsCsAUfUUfUfUfUUUGAGCUUGAsAsG |
| S234 | 5'MVIP02-GsCsAUfUUfUfUfUUUGAGCUUGAsAsG |
| S235 | 5'MVIP05-GsCsAUfUUfUfUfUUUGAGCUUGAsAsG |
| S236 | 5'MVIP06-GsCsAUfUUfUfUfUUUGAGCUUGAsAsG |
| S237 | 5'MVIP07-GsCsAUfUUfUfUfUUUGAGCUUGAsAsG |
| S238 | 5'MVIP10-GsCsAUfUUfUfUfUUUGAGCUUGAsAsG |
| S239 | 5'MVIP14-GsCsAUfUUfUfUfUUUGAGCUUGAsAsG |
| S240 | 5'MVIP18-GsCsAUfUUfUfUfUUUGAGCUUGAsAsG |
| S241 | GsCsAUfUUfUfUfUUUGAGCUUGAsAsG -3' MVIP02 |
| S242 | GsCsAUfUUfUfUfUUUGAGCUUGAsAsG -3' MVIP03 |
| S243 | GsCsAUfUUfUfUfUUUGAGCUUGAsAsG -3' MVIP04 |
| S244 | 5' MVIP04-GsCsAUfUUfUfUfUUUGAGCUUGAsAsG -3' MVIP04 |
| S245 | 5' MVIP03-GsCsAUfUUfUfUfUUUGAGCUUGAsAsG -3' MVIP19 |
| S246 | 5' MVIP18-GsCsAUfUUfUfUfUUUGAGCUUGAsAsG -3' MVIP18 |
| S247 | 5' MVIP08-GsCsAUfUUfUfUfUUUGAGCUUGAsAsG -3' MVIP18 |
| S248 | GsCsAUfUUfUfUfUUUGAGCUUGAsAsG -3' MVIP05 |
| S249 | GsCsAUfUUfUfUfUUUGAGCUUGAsAsG -3' MVIP07 |
| S250 | GsCsAUfUUfUfUfUUUGAGCUUGAsAsG -3' MVIP10 |
| S251 | GsCsAUfUUfUfUfUUUGAGCUUGAsAsG -3' MVIP11 |
| S252 | 5' MVIP11-GsCsAUfUUfUfUfUUUGAGCUUGAsAsG -3' MVIP11 |
| S253 | 5' MVIP15-GsCsAUfUUfUfUfUUUGAGCUUGAsAsG -3' MVIP15 |
| S254 | GsCsAUfUUfUfUfUUUGAGCUUGAsAsG -3' MVIP06 |
| S255 | GsCsAUfUUfUfUfUUUGAGCUUGAsAsG -3' MVIP08 |
| S256 | GsCsAUfUUfUfUfUUUGAGCUUGAsAsG -3' MVIP12 |
| S257 | GsCsAUfUUfUfUfUUUGAGCUUGAsAsG -3' MVIP13 |
| S258 | GsCsAUfUUfUfUfUUUGAGCUUGAsAsG -3' MVIP14 |
| S259 | GsCsAUfUUfUfUfUUUGAGCUUGAsAsG -3' MVIP15 |
| S260 | GsCsAUfUUfUfUfUUUGAGCUUGAsAsG -3' MVIP16 |
| S261 | 5' MVIP19-GsCsAUfUUfUfUfUUUGAGCUUGAsAsG |
| S262 | 5' MVIP20--GsCsAUfUUfUfUfUUUGAGCUUGAsAsG |
| S263 | 5' MVIP21--GsCsAUfUUfUfUfUUUGAGCUUGAsAsG |
| S264 | 5' MVIP22--GsCsAUfUUfUfUfUUUGAGCUUGAsAsG |

In some embodiments, the sense strand of the RNAi agent in this application differs from each sequence in Table 17 by one, two or three nucleotides.

In some embodiments, the RNAi agents described in this application are formed by randomly pairing a sense strand in Table 17 or a sequence that differs from any of sense strands in Table 17 by one, two, or three nucleotides with an antisense strand in Table 16 or a sequence that differs from any of sense strands in Table 16 by one, two, or three nucleotides.

In some embodiments, the RNAi agent in this application is synthesized by pairing and annealing of the antisense strand in Table 16 and the sense strand in Table 17, as shown in Table 18. The n+m of the RNAi agents are 2, 3, 4, 5 and 6, respectively. The positions of 5'MVIP and / or 3'MVIP conjugating include the 5' end and / or 3' end of antisense strand, the 5' end and / or 3' end of sense strand, the 5' end of antisense strand and 3' end of sense strand, and the 5' end of sense strand and 3' end of antisense strand. Where n+m=2, 3, 4,5 and 6.

**Table 18 RNAi agents containing a combination of 5'MVIP and 3'MVIP**

| Singl e strand code | Carrier structure | Single strand code | Carrier structure | Double strand code | n+m | The conjugation position of carrier structure and siRNA(S:AS) |
|---|---|---|---|---|---|---|
| S211 | 5'MVIP01 | AS208 | 3' MVIP01 | Kylo-09-DS131 | 2 | n:m |
| S263 | 5'MVIP21 | AS208 | 3' MVIP01 | Kylo-09-DS132 | 2 | n:m |
| S211 | 5'MVIP01 | AS247 | 3' MVIP07 | Kylo-09-DS133 | 2 | n:m |
| S212 | 5'MVIP01/3' MVIP01 | AS64 | / | Kylo-09-DS134 | 2 | nm:/ |
| S209 | 3'MVIP01 | AS265 | 5'MVIP01 | Kylo-09-DS135 | 2 | m:n |
| S249 | 3' MVIP07 | AS263 | 5'MVIP21 | Kylo-09-DS136 | 2 | m:n |
| S211 | 5'MVIP01 | AS 140 | 3'MVIP09 | Kylo-09-DS137 | 3 | n:m |
| S227 | 5' MVIP08 | AS257 | 3'MVIP15 | Kylo-09-DS138 | 3 | n:m |
| S140 | 5'MVIP09 | AS208 | 3' MVIP01 | Kylo-09-DS139 | 3 | n:m |
| S261 | 5' MVIP19 | AS247 | 3' MVIP07 | Kylo-09-DS140 | 3 | n:m |
| S217 | 5'MVIP01/3' MVIP09 | AS64 | / | Kylo-09-DS141 | 3 | nm:/ |
| S218 | 5'MVIP09/3' MVIP01 | AS64 | / | Kylo-09-DS142 | 3 | nm:/ |
| S208 | 3'MVIP09 | AS265 | 5'MVIP01 | Kylo-09-DS143 | 3 | m:n |
| S251 | 3'MVIP11 | AS234 | 5'MVIP06 | Kylo-09-DS144 | 3 | m:n |
| S209 | 3'MVIP01 | AS262 | 5' MVIP20 | Kylo-09-DS145 | 3 | m:n |
| S249 | 3' MVIP07 | AS261 | 5' MVIP19 | Kylo-09-DS146 | 3 | m:n |
| S211 | 5'MVIP01 | AS209 | 3' MVIP17 | Kylo-09-DS147 | 4 | n:m |
| S263 | 5' MVIP21 | AS209 | 3' MVIP17 | Kylo-09-DS148 | 4 | n:m |
| S210 | 5'MVIP17 | AS208 | 3' MVIP01 | Kylo-09-DS149 | 4 | n:m |
| S264 | 5' MVIP22 | AS247 | 3' MVIP07 | Kylo-09-DS150 | 4 | n:m |
| S140 | 5'MVIP09 | AS 140 | 3'MVIP09 | Kylo-09-DS122 | 4 | n:m |
| S262 | 5' MVIP20 | AS248 | 3' MVIP10 | Kylo-09-DS151 | 4 | n:m |
| S261 | 5' MVIP19 | AS257 | 3'MVIP15 | Kylo-09-DS152 | 4 | n:m |
| S238 | 5' MVIP10 | AS254 | 3'MVIP12 | Kylo-09-DS153 | 4 | n:m |
| S213 | 5'MVIP09/3' MVIP09 | AS64 | / | Kylo-09-DS154 | 4 | nm:/ |
| S208 | 3'MVIP09 | AS266 | 5' MVIP09 | Kylo-09-DS155 | 4 | m:n |
| S250 | 3'MVIP10 | AS262 | 5' MVIP20 | Kylo-09-DS156 | 4 | m:n |
| S209 | 3'MVIP01 | AS207 | 5' MVIP17 | Kylo-09-DS157 | 4 | m:n |
| S241 | 3'MVIP02 | AS238 | 5' MVIP18 | Kylo-09-DS158 | 4 | m:n |
| S207 | 3'MVIP17 | AS265 | 5' MVIP01 | Kylo-09-DS159 | 4 | m:n |
| S225 | 3'MVIP18 | AS263 | 5' MVIP21 | Kylo-09-DS160 | 4 | m:n |
| S140 | 5'MVIP09 | AS209 | 3' MVIP17 | Kylo-09-DS161 | 5 | n:m |
| S262 | 5'MVIP20 | AS220 | 3' MVIP19 | Kylo-09-DS162 | 5 | n:m |
| S210 | 5'MVIP17 | AS 140 | 3'MVIP09 | Kylo-09-DS163 | 5 | n:m |
| S264 | 5'MVIP22 | AS249 | 3'MVIP11 | Kylo-09-DS164 | 5 | n:m |
| S219 | 5'MVIP09/3' MVIP17 | AS64 | / | Kylo-09-DS165 | 5 | nm:/ |
| S220 | 5'MVIP17/3' MVIP09 | AS64 | / | Kylo-09-DS166 | 5 | nm:/ |
| S207 | 3'MVIP17 | AS262 | 5' MVIP20 | Kylo-09-DS167 | 5 | m:n |
| S222 | 3'MVIP19 | AS226 | 5' MVIP16 | Kylo-09-DS168 | 5 | m:n |
| S208 | 3'MVIP09 | AS207 | 5' MVIP17 | Kylo-09-DS169 | 5 | m:n |
| S259 | 3' MVIP15 | AS207 | 5' MVIP17 | Kylo-09-DS170 | 5 | m:n |
| S210 | 5'MVIP17 | AS209 | 3' MVIP17 | Kylo-09-DS171 | 6 | n:m |
| S264 | 5' MVIP22 | AS220 | 3' MVIP19 | Kylo-09-DS172 | 6 | n:m |
| S214 | 5'MVIP17/3' MVIP17 | AS64 | / | Kylo-09-DS173 | 6 | nm:/ |
| S207 | 3'MVIP17 | AS207 | 5' MVIP17 | Kylo-09-DS174 | 6 | m:n |
| S225 | 3'MVIP18 | AS264 | 5' MVIP22 | Kylo-09-DS175 | 6 | m:n |

In some embodiments, the RNAi agent or pharmaceutically acceptable salt thereof in this application is preferably prepared or synthesized in the form of sodium salt and triethylamine salt or other pharmaceutically acceptable salt.

In some embodiments, the RNAi agent or pharmaceutically acceptable salt thereof is more preferably sodium salt or triethylamine salt.

### Pharmaceutical composition

The present invention also includes a pharmaceutical composition comprising the RNAi agent or pharmaceutically acceptable salt thereof of the present invention.

In one embodiment, a pharmaceutical composition comprising the RNAi agent of the present invention and pharmaceutically acceptable pharmaceutical adjuvants is provided herein. The pharmaceutical composition containing the RNAi agent can be used to prevent and / or treat AGT related disorders, such as hypertension. Such pharmaceutical compositions are formulated according to the mode of delivery. An embodiment is to formulate as a composition for systemic administration in parenteral delivery, such as, subcutaneous (SC), intramuscular (IM), or intravenous (IV) delivery. The pharmaceutical composition of the present invention can be administered at a dose sufficient to inhibit AGT gene expression.

Pharmaceutically acceptable "adjuvants" or "excipients" are pharmaceutically acceptable solvents, suspensions or any other pharmaceutically inert vehicles used to deliver one or more nucleic acids to animals. The excipients may be liquid or solid and are selected taking account of the intended mode of administration to provide the required volume, consistency, etc. when combined with nucleic acids and other components in a given pharmaceutical composition. RNAi agents can be delivered in a manner that targets specific tissues (e.g., hepatocytes).

In some embodiments, the pharmaceutical composition further comprises a delivery vehicle (such as nanoparticles, dendrimers, polymers, liposomes, or cation delivery systems).

In some embodiments, the delivery vehicle includes a liposome.

In some embodiments, the delivery vehicle includes a nanoliposome capable of forming liposome-nucleic acid nanoparticles with nucleic acid molecules.

In some embodiments, the delivery vehicle includes the amphoteric lipids compound M10C1.

The pharmaceutical composition of the present invention includes (but is not limited to) solutions, emulsions, and preparations containing liposomes. These compositions can be produced from a variety of components, including (but not limited to) pre-formed liquids, self-emulsifying solids, and self-emulsifying semisolids. Preparations include those targeting the liver. The pharmaceutical preparations of the present invention that can conveniently exist in a unit dosage form can be prepared according to the conventional technology known to the pharmaceutical industry. Such technologies include the steps of combining active ingredients with pharmaceutical adjuvants or excipients.

### Purpose

In yet another aspect, the present invention provides a method for reducing AGT mRNA or protein expression in a cell or tissue, which includes contacting the cell or tissue with an effective amount of the aforementioned RNAi agent or pharmaceutically acceptable salt thereof that inhibits AGT gene expression, and / or the aforementioned pharmaceutical composition.

The cell suitable for treatment using the method of the invention can be any cell expressing AGT gene, such as liver cell, brain cell, gallbladder cell, heart cell or kidney cell, but preferably liver cell. The cell suitable for the method of the invention can be mammalian cell.When in contact with cell expressing AGT gene, RNAi agent inhibits the expression of AGT gene (for example, human, primate, non-primate or rat AGT gene) by at least about 50%. For example, it can be determined by PCR or methods based on branched DNA (bDNA), or methods based on protein, such as immunofluorescence, Western blotting or flow cytometry.

In some embodiments, the tissue is liver tissue.

In some embodiments, the cell and tissue are ex vivo.

In some embodiments, the cell and tissue are within a subject.

The term "inhibition" used herein may be used interchangeably with "reduction", "decrease", "silence", "downregulation", "repression" and other similar terms, and includes any level of inhibition. The expression of AGT gene can be evaluated according to the level or level change of any variable related to AGT gene expression, for example, AGT mRNA level or AGT protein level. Such level can be analyzed in individual cell or cell populations, including, for example, samples derived from subjects. Inhibition can be evaluated by the decrease in absolute or relative levels of one or more variables associated with AGT expression compared with control levels. The control level can be any type of control level adopted in the art, for example, the baseline level before dosing or the level measured from similar subjects, cells or samples that have never been treated or received control treatment (e.g., only buffer control or inactive agent control).

The inhibition of AGT gene expression can be represented by a decrease in the amount of mRNA expressed by the first cell or cell population that inhibit AGT gene expression (such cells may be, for example, present in a sample derived from a subject) in which the AGT gene is transcribed and treated (for example, by contacting one or more cells with the RNAi agent of the present invention, or by administering the RNAi agent of the present invention to a subject in which the cell is present) compared to a second cell or cell population (control cells not treated with RNAi agents or RNAi agents targeting the gene of interest) that is essentially the same as that first cell or cell population but not so treated. In a preferred embodiment, inhibition is evaluated in a cell line that highly expresses AGT by the method provided in Example 2 using an appropriate concentration of siRNA, and the mRNA level in the interfered cells is expressed as a percentage of the mRNA levels in the uninterfered control cells.

In other embodiments, the inhibition of AGT gene expression can be evaluated by the reduction of parameters functionally related to AGT gene expression, such as AGT protein levels in the blood or serum of subjects. AGT gene silencing can be determined in any AGT expressing cell (endogenous or exogenous from the expression constructs) and by any assay known in the art.

The inhibition of AGT protein expression can be manifested by the reduction of AGT protein levels expressed by cells or cell populations or subject samples (e.g., protein levels in blood samples derived from subjects). As mentioned above, for the evaluation of mRNA inhibition, the inhibition of protein expression levels of treated cells or cell populations can be similarly shown as a percentage of protein levels of control cells or cell populations, or changes in protein levels in subject samples (e.g., blood or serum derived from it).

The cells, cell populations or subject samples in control group that can be used to evaluate AGT gene inhibition include cells, cell populations or subject samples that are not in contact with the RNAi agent of this invention. For example, control cells, cell populations, or subject samples may be derived from a single subject (e.g., human or animal subjects) or an appropriately matched group controls before treatment with RNAi agents.

AGT mRNA levels expressed by cells or cell populations can be determined using any method known in the art for evaluating mRNA expression. For example, qRT-PCR evaluates the reduction of gene expression. The reduction in protein production can be evaluated by any method known in the art, for example, ELISA. In some embodiments, the liver biopsy sample is used as tissue material to monitor the reduction of AGT gene or protein expression. In other embodiments, blood samples are used as subject samples for monitoring the reduction of AGT proteins expression.

In yet another aspect, the present invention provides the use of the aforementioned RNAi agent or pharmaceutically acceptable salt thereof for inhibiting AGT gene expression, or the aforementioned pharmaceutical composition in the preparation of drugs for preventing and / or treating diseases or conditions or reducing the risk of diseases or conditions.

In some embodiments, the disease or condition includes AGT related disease or condition.

In some embodiments, the disease or condition is selected from: hypertension, high blood pressure, borderline hypertension, primary hypertension, secondary hypertension, isolated systolic or diastolic hypertension, pregnancy related hypertension, diabetic hypertension, treatment-resistant hypertension, refractory hypertension, paroxysmal hypertension, renovascular hypertension, Goldblatt's hypertension, hypertension associated with low plasma renin activity or plasma renin concentration, ocular hypertension, glaucoma, pulmonary hypertension, portal hypertension, systemic venous hypertension, systolic hypertension, and unstable hypertension; Hypertensive heart disease, hypertensive nephropathy, atherosclerosis, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, heart failure, myocardial infarction, angina, stroke, renal disease, renal failure, systemic sclerosis, intrauterine growth retardation (IUGR), fetal growth restriction, obesity, hepatic steatosis / fatty liver, non-alcoholic steatohepatitis (NASH), non-alcoholic fatty liver disease (NAFLD); Glucose intolerance, type 2 diabetes mellitus (non-insulin dependent diabetes mellitus), and metabolic syndrome.

In yet another aspect, the present invention provides a method for preventing and / or treating diseases or conditions, which includes administering an effective amount of the aforementioned RNAi agent or a pharmaceutically acceptable salt thereof that inhibits AGT gene expression, and/or the aforementioned pharmaceutical composition to a subject in need.

The in vivo method of the present invention may comprise administering to a subject a composition comprising an RNAi agent, wherein the RNAi agent comprises a nucleotide sequence complementary to at least a portion of the RNA transcript of the AGT gene of a mammal receiving the administration of the RNAi agent. The composition may be administered in any manner known in the art, including (but not limited to): oral, intraperitoneal or parenteral routes, including intracranial (e.g., intraventricular, intraparenchymal, and intrathecal), intravenous, intramuscular, subcutaneous, transdermal, airway (aerosol), nasal, rectal, and local (including buccal and sublingual) administration. In some embodiments, the composition is administered by intravenous infusion or injection. In some embodiments, the composition is administered by subcutaneous injection. In some embodiments, the composition is administered by intramuscular injection.

The RNAi agent of the invention can also be administered as a "free RNAi agent". Free RNAi agents are administered in the absence of a pharmaceutical composition. Naked RNAi agents can be in a suitable buffer. The buffer may contain acetate, citrate, prolamin, carbonate, or phosphate, or any combination thereof. In one embodiment, the buffer is phosphate buffered saline (PBS). The pH and osmotic pressure of the buffer containing RNAi agents can be adjusted to be suitable for administration to subjects.

Alternatively, the RNAi agent of the present invention may be administered as a pharmaceutical composition, such as a liposome preparation.

The pharmaceutical composition of the present invention can be administered at a dosage sufficient to inhibit AGT gene expression. Generally, the appropriate dosage of the RNAi agent of the present invention is in the range of about 0.001 to about 200.0mg per kilogram of recipient body weight per day, and is usually in the range of about 1 to 50mg per kilogram of body weight per day. Generally, the suitable dosage of the RNAi agent of the present invention is in the range of about 0.1mg/kg to about 5.0mg/kg, for example, in the range of about 0.3mg/kg to about 3.0mg/kg.

In one embodiment, the method includes administering the composition described herein such that the target AGT gene expression is reduced for, such as approximately 1, 2, 3, 4, 5, 6, 1-6, 1-3, or 3-6 months per dose. In some embodiments, the composition is administered every 3-6 months.

In some embodiments, after the initial treatment regimen, treatment is administered at less frequency. The repeated dosage regimen may include regular administration of therapeutic amounts of RNAi agents, such as once a month to once a year. In some embodiments, RNAi agents are administered approximately once a month to approximately once every three months, or approximately once every three months to approximately once every six months.

After the initial treatment regimen, treatment can be administered at a lower frequency. The duration of treatment can be determined according to the severity of the disease.

In other embodiments, a single dose of the pharmaceutical composition may be long-acting, such that the dose is administered at intervals of no more than 1, 2, 3, or 4 months. In some embodiments of the present application, a single dose of the pharmaceutical composition of the present application is administered approximately once a month. In other embodiments of the application, a single dose of the pharmaceutical composition of the application is administered quarterly (i.e. about every 3 months). In other embodiments of the application, a single dose of the pharmaceutical composition of the application is administered twice a year (i.e., about once every 6 months).

The person skilled in the art should understand that certain factors can affect the dosage and duration of administration required to effectively treat the subject, including (but not limited to): the mutation existing in the subject, previous treatment, the general health or age and presence of other diseases of the subject. In addition, treating a subject with a prophylactically and / or therapeutically effective amount of the composition as needed may include a single treatment or a series of treatments.

In some embodiments, it further includes determining AGT levels in samples from the subject.

For example, it further includes determining the AGT protein level in the blood sample, serum sample or urine sample from the subject.

In some embodiments, it further includes administering an additional therapeutic agent for the treatment of hypertension to the subject.

For example, the additional therapeutic agent can be selected from diuretics, angiotensin converting enzyme (ACE) inhibitors, angiotensin II receptor antagonists, βblockers, vasodilators, calcium channel blockers, aldosterone antagonists, α2-agonists, renin inhibitors, α-blockers, peripheral-acting adrenergics agents, selective D1 receptor partial agonists, nonselective α-adrenergic antagonists, synthetic steroidal antimineralocorticoid agents, angiotensin receptor-neprilysin inhibitors (ARNi), sacubitril / valsartan; Or endothelin receptor antagonist (ERA), sitasentan, ambrisentan, atrasentan, BQ-123, zipotentan, bosentan, macitentan, and tizosentan; a combination of any of the above therapeutic agents; and hypertension therapeutic agents formulated as pharmaceutical compositions.

In some embodiments, the additional therapeutic agent includes an angiotensin II receptor antagonist.

For example, the angiotensin II receptor antagonist may be selected from the group consisting of losartan, valsartan, olmesartan, eprosartan, and azilsartan.

In yet another aspect, the present invention provides a cell containing the aforementioned RNAi agent or pharmaceutically acceptable salt thereof for inhibiting AGT gene expression.

In yet another aspect, the present invention provides a kit, which contains the aforementioned RNAi agent or pharmaceutically acceptable salt thereof for inhibiting AGT gene expression, or the aforementioned pharmaceutical composition.

### Specifically, the application also discloses the following embodiments:

1. An RNAi agent having a structure containing carrier structure and interfering nucleic acid and represented by formula IIIa, IIIb or IIIc or pharmaceutically acceptable salt thereof.: 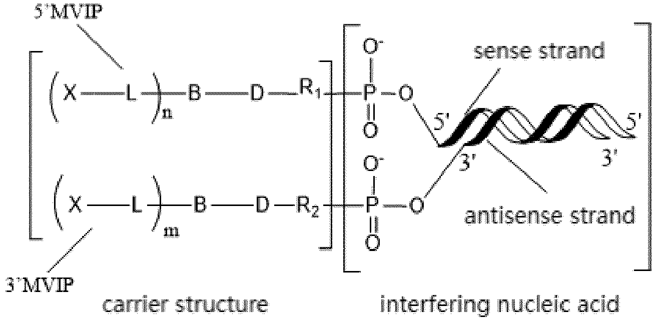 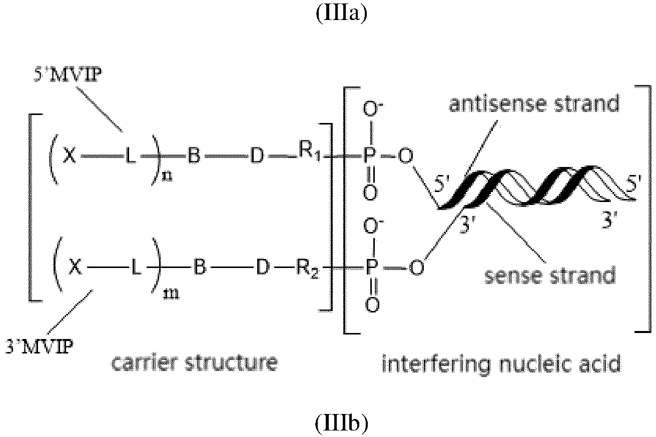 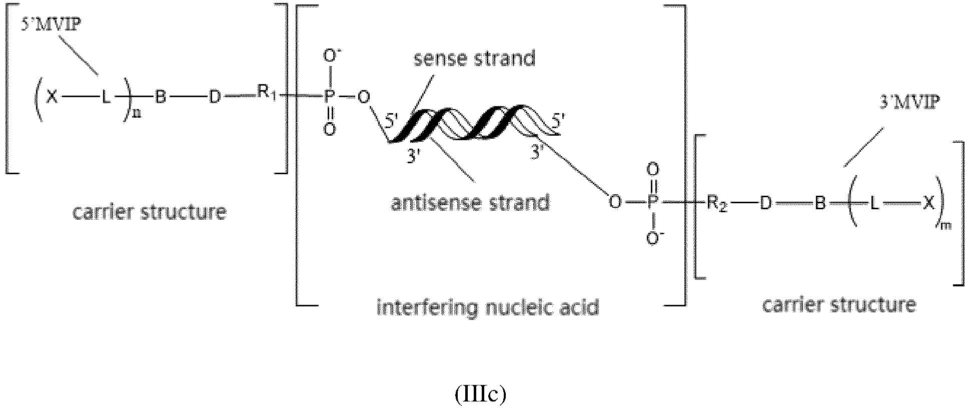 wherein,
   the interfering nucleic acid targets AGT gene, which includes an antisense strand and a sense strand;
   the carrier structure includes 5'MVIP (5'MultiValent Import Platform) and 3'MVIP (3'MultiValent Import Platform);
   the 5 'MVIP is composed of transition point R₁, linking chain D, linker B, branched chain L and liver targeting specific ligand X, the 3'MVIP is composed of transition point R₂, linking chain D, linker B, branched chain L and liver targeting specific ligand X, the 5'MVIP is connected with the 5' end of sense strand or the 5' end of antisense strand through transition point R₁, the 3'MVIP is connected with the 3' end of sense strand or the 3' end of antisense strand through transition point R₂, n and m are each independently any integer from 0 to 4.
2. The RNAi agent or pharmaceutically acceptable salt thereof according to embodiment 1, wherein the interfering nucleic acid includes siRNA or miRNA.
3. The RNAi agent or pharmaceutically acceptable salt thereof according to any one of embodiments 1-2, wherein n+m is an integer of 2 to 6, preferably n+m=2, 3 or 4, more preferably 4.
4. The RNAi agent or pharmaceutically acceptable salt thereof according to any one of embodiments 1-3, wherein R₁ is a heterocyclic or carbocyclic structure containing N, S or O: 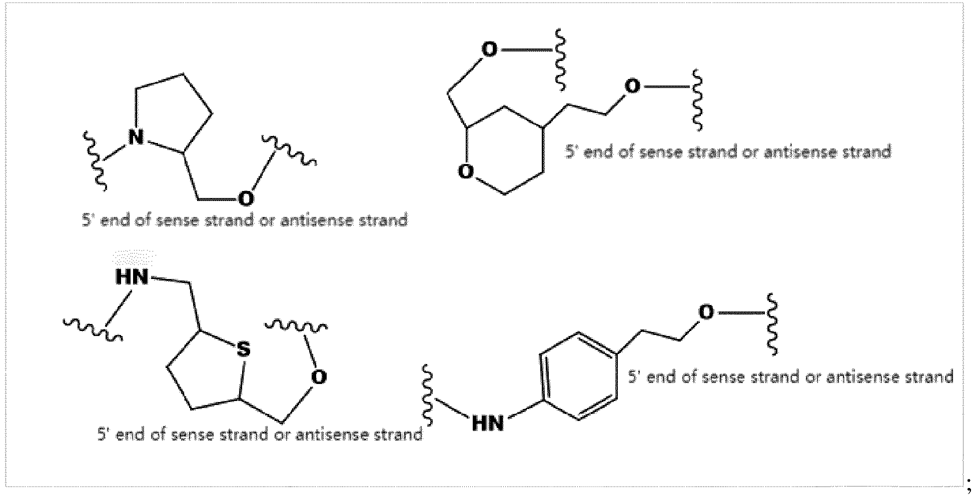 alternatively, R₁ is -NH(CH₂)ₓCH₂O-, wherein x is any integer from 3 to 12, preferably any integer from 4 to 6.
5. The RNAi agent or pharmaceutically acceptable salt thereof according to any one of embodiments 1-4, wherein the R₂ is a heterocyclic or carbocyclic structure containing N, S or O: 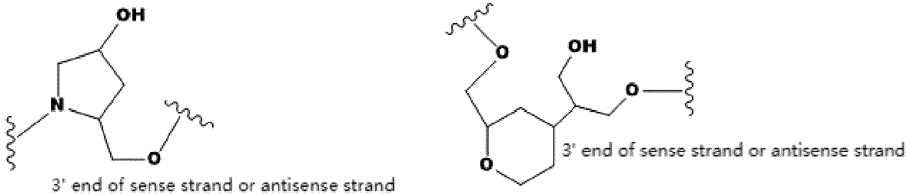 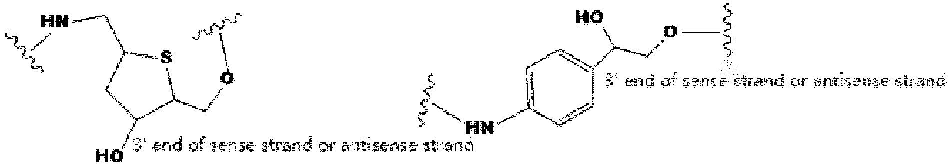 ; alternatively, the R₂ is -NH(CH₂)ₓ₁CH(OH)(CH₂)ₓ₂CH₂O-, where x1 is any integer of 1-4 and x2 is any integer of 0-4.
6. The RNAi agent or pharmaceutically acceptable salt thereof according to any one of embodiments 1-5, wherein the X is selected from the structure used to enhance the uptake of RNAi agent by hepatocytes.
7. The RNAi agent or pharmaceutically acceptable salt thereof according to any one of embodiments 1-6, wherein the X is selected from monosaccharide and derivatives thereof.
8. The RNAi agent or pharmaceutically acceptable salt thereof according to any one of embodiments 1-7, wherein the X is N-acetylgalactosamine and derivatives thereof.
9. The RNAi agent or pharmaceutically acceptable salt thereof according to any one of embodiments 1-8, wherein the X is selected from the following structures: 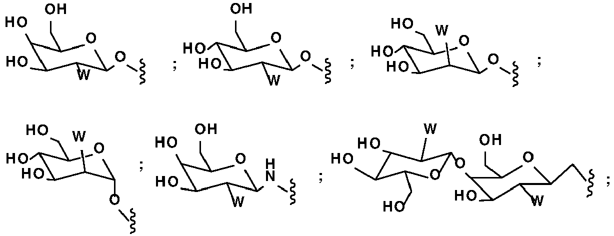 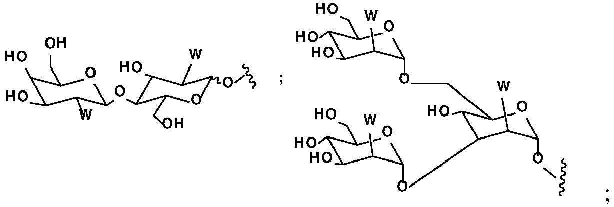 wherein W is selected from one or two of -OH, -NHCOO or -NHCO(CH₂)_{q}CH₃, wherein q is an integer of 0-4.
10. The RNAi agent or pharmaceutically acceptable salt thereof according to any one of embodiments 1-9, wherein the L is selected from one or more of the following structures: 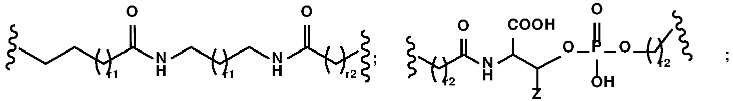 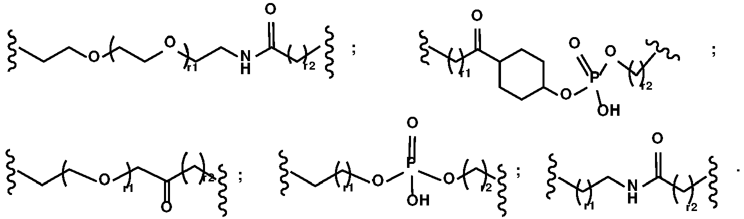 wherein r1 is any integer from 1 to 12, r2 is any integer from 0 to 20, and Z is H, alkyl or amido group.
11. The RNAi agent or pharmaceutically acceptable salt thereof according to any one of embodiments 1-10, wherein the B is selected from the following structures: 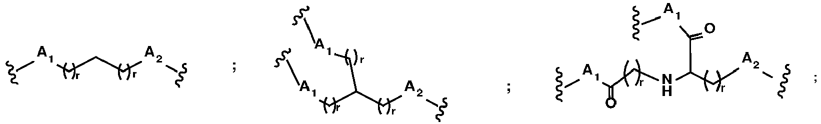 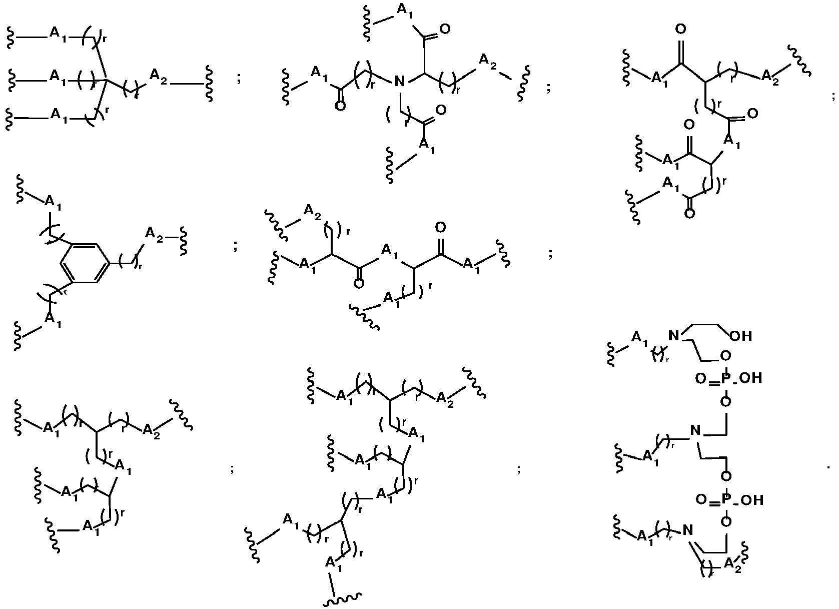 wherein A₁ and A₂ are each independently C, O, S, -NH-, carbonyl, amido, phosphoryl or thiophosphoryl, and r is an integer of 0-4.
12. The RNAi agent or pharmaceutically acceptable salt thereof according to any one of embodiments 1-11, wherein the D is selected from the following structures: 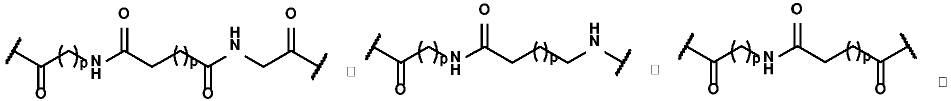 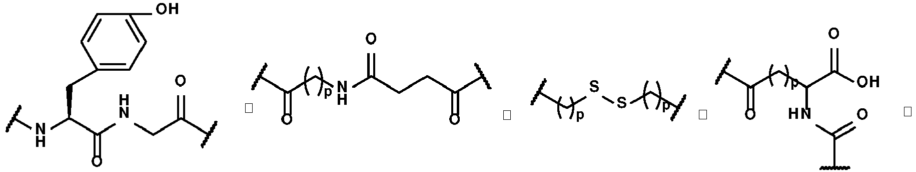 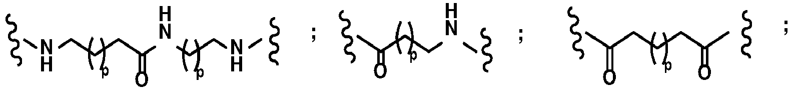 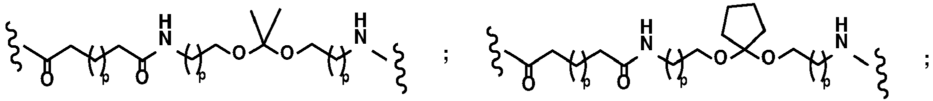 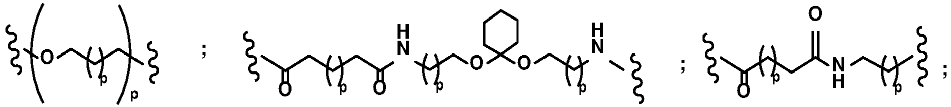 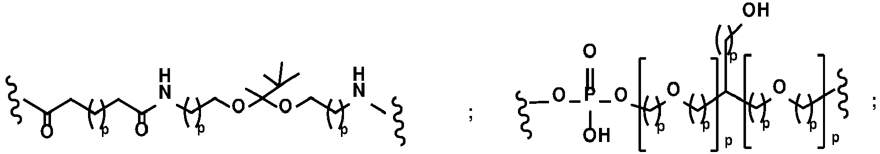 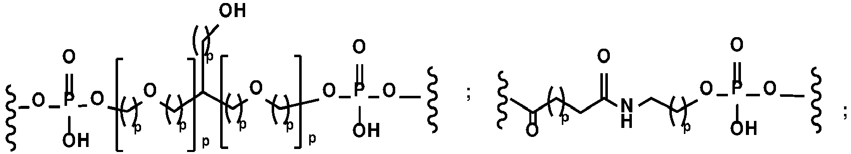 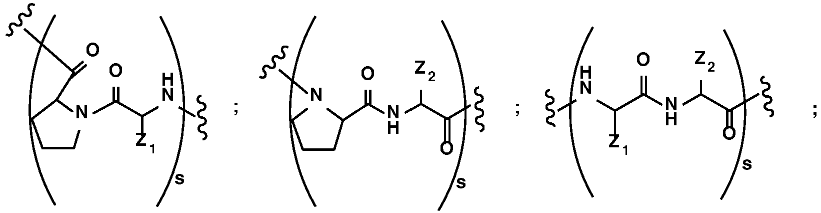 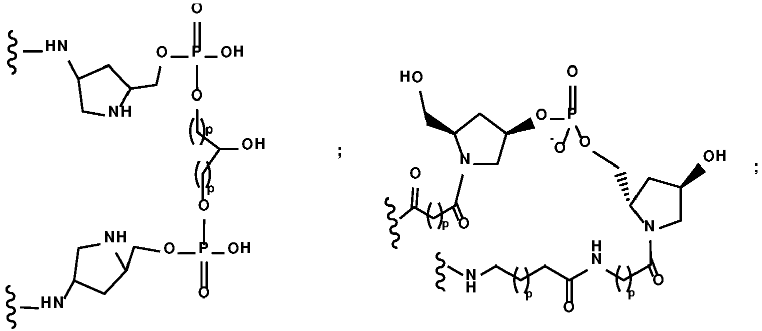 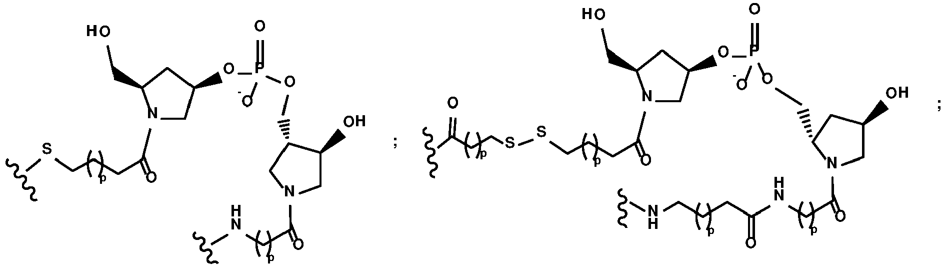 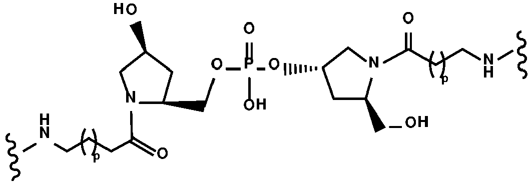 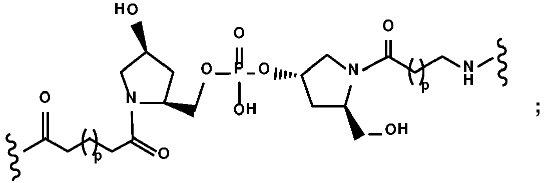 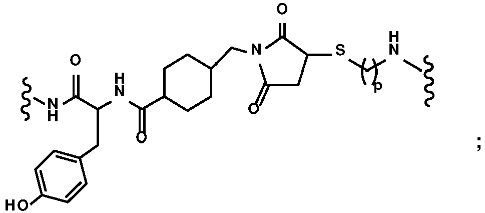 where, each p is any integer of 1-20 independently; s is an integer of 2-13; Z₁ and Z₂ are the same or different substituents.
13. The RNAi agent or pharmaceutically acceptable salt thereof according to any one of embodiments 1-12, wherein **(X-L)ₙ-B-D-** in the 5'MVIP structure and **(X-L)ₘ-B-D-** in the 3'MVIP structure are selected from one or more of the following structures: 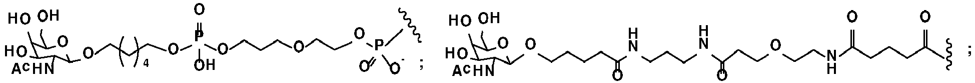 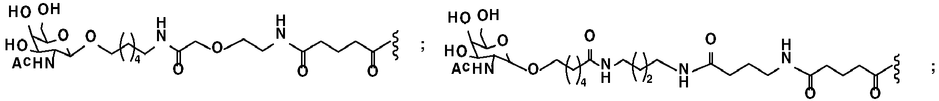 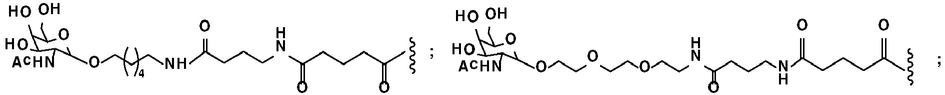 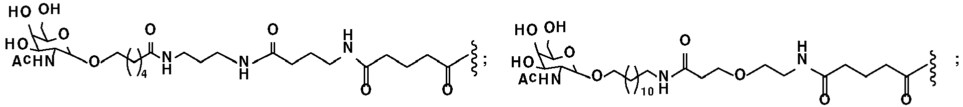 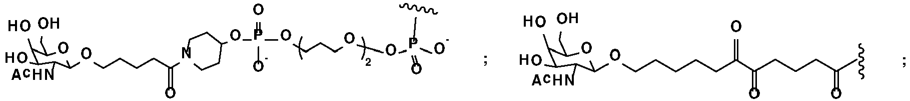 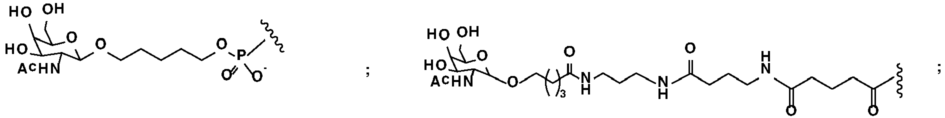 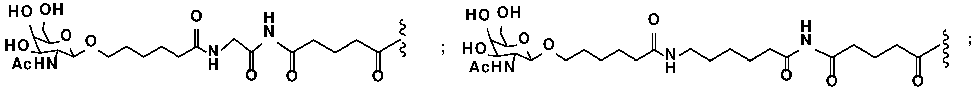 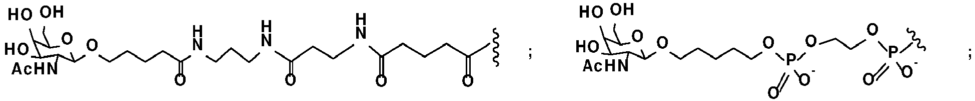 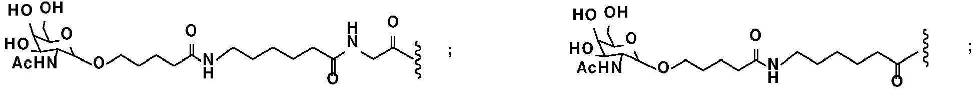 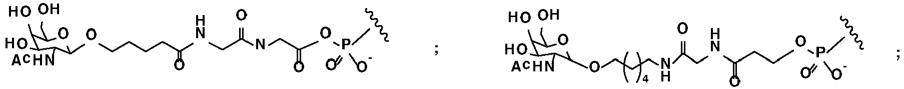 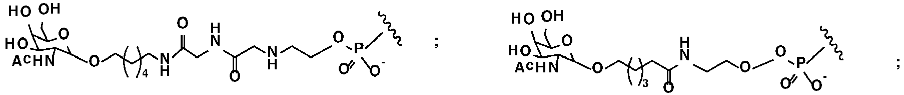 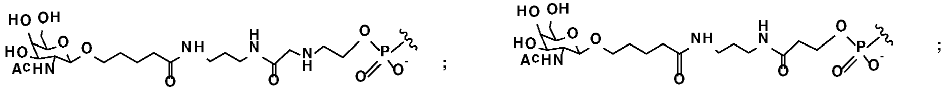 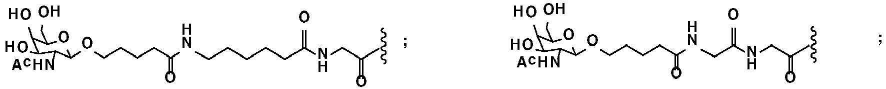 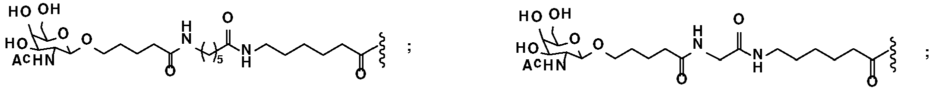 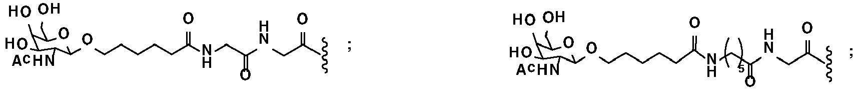 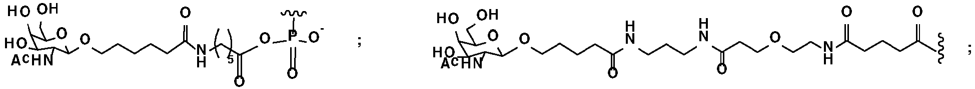 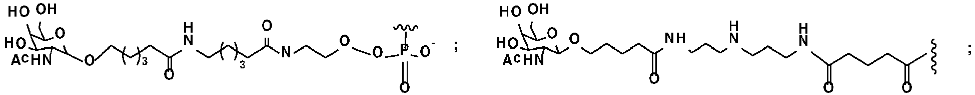 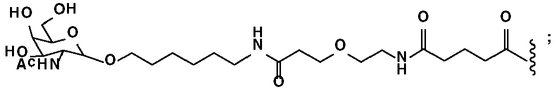 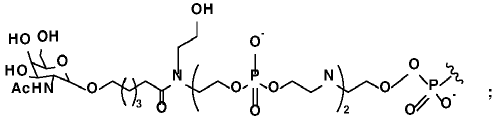 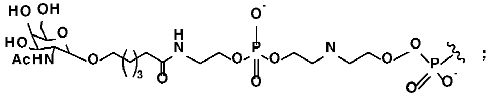 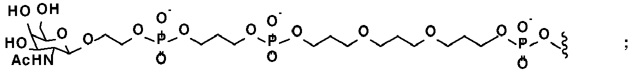 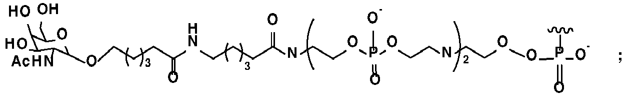 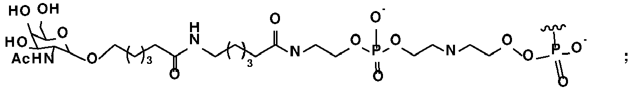 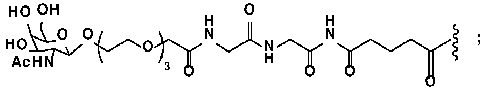 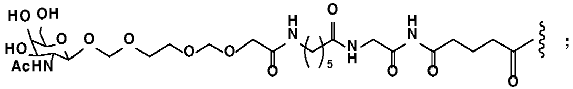 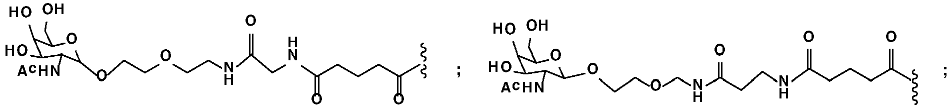 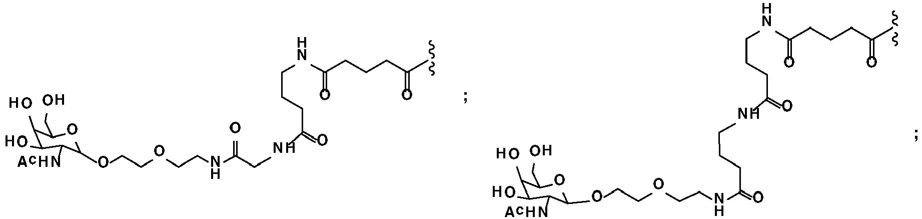 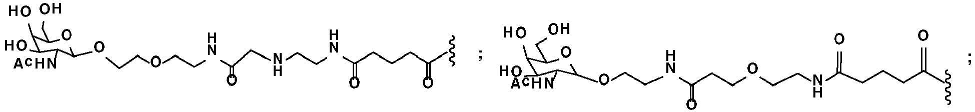 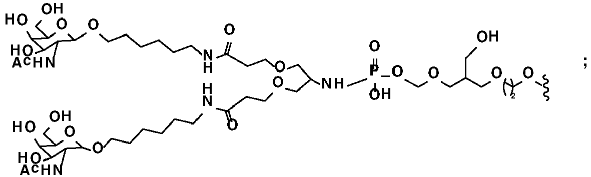 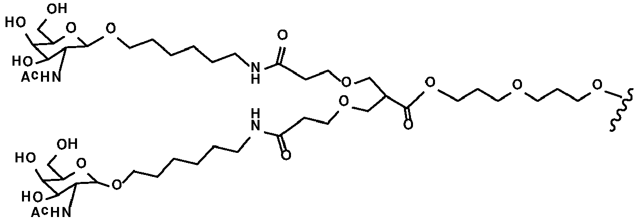 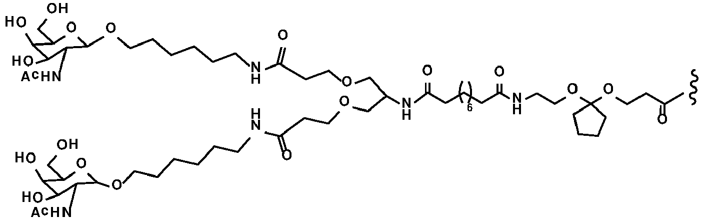 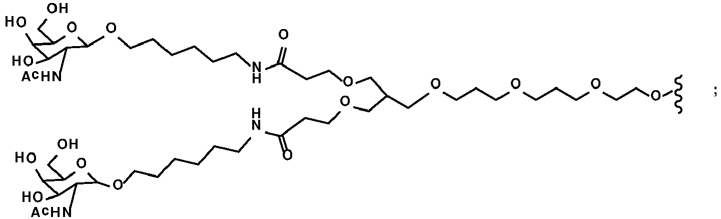 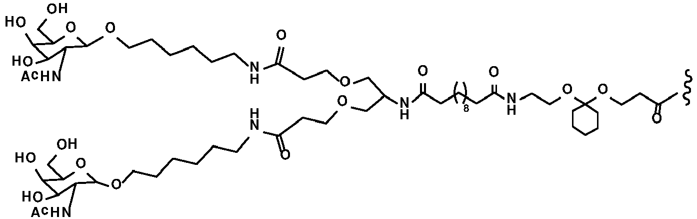 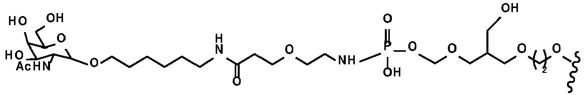 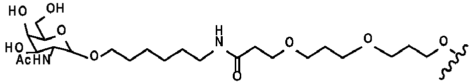 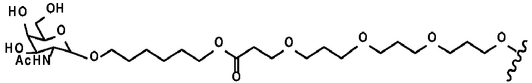 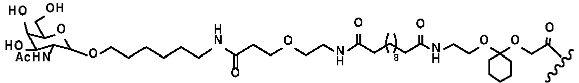 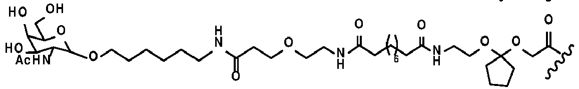 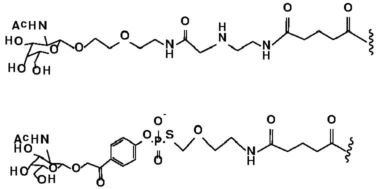 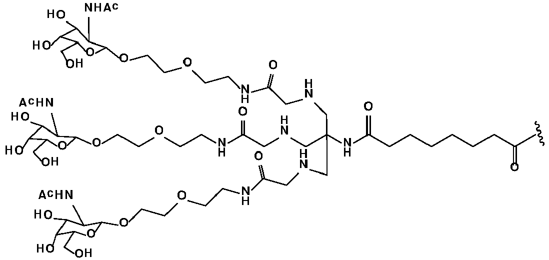 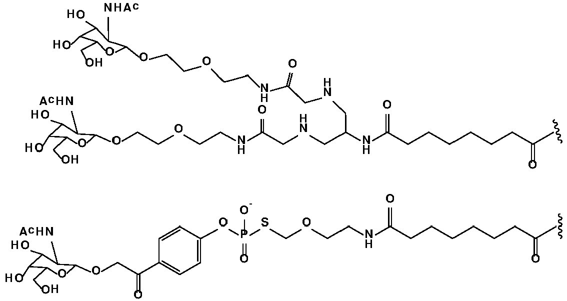 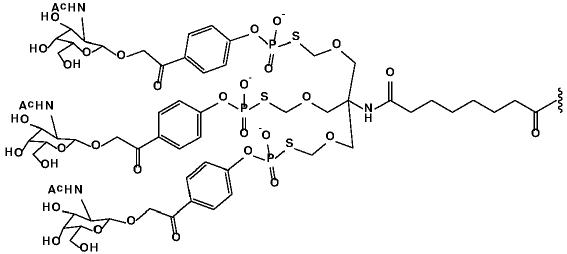 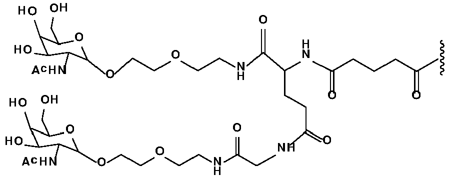 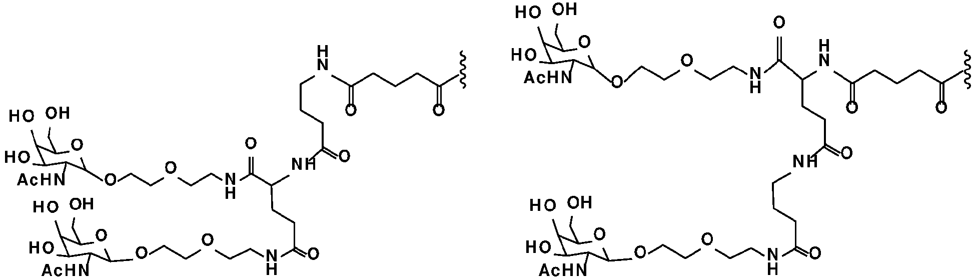 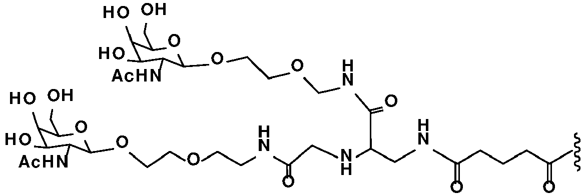 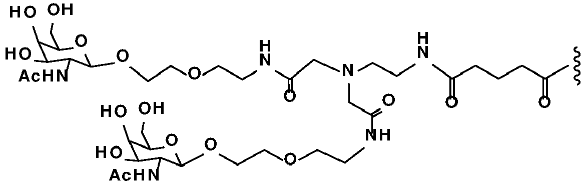 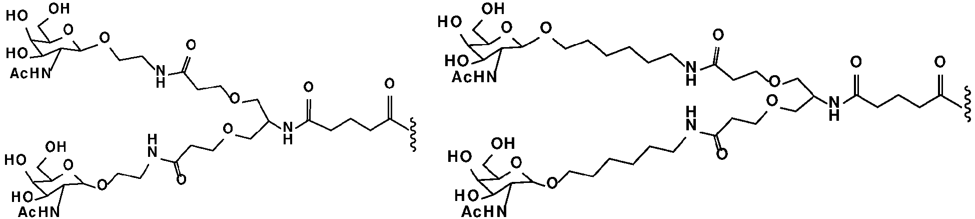 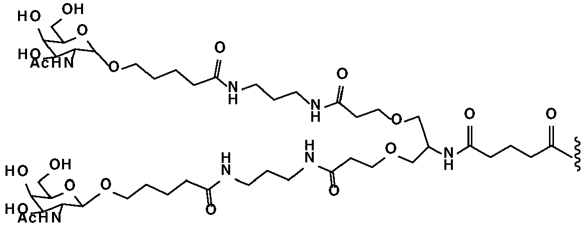 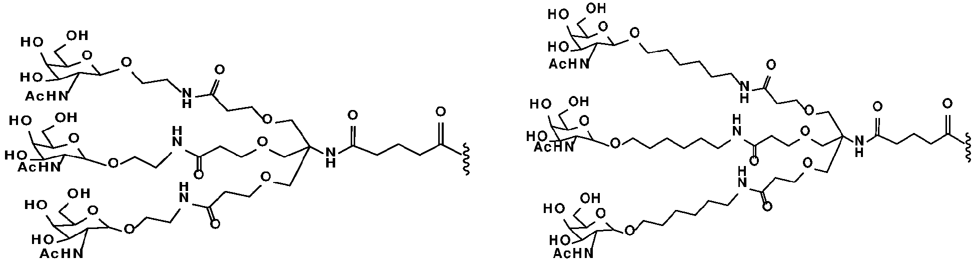 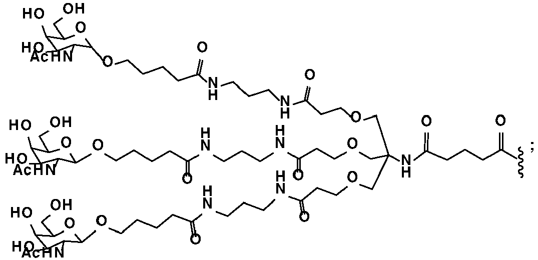 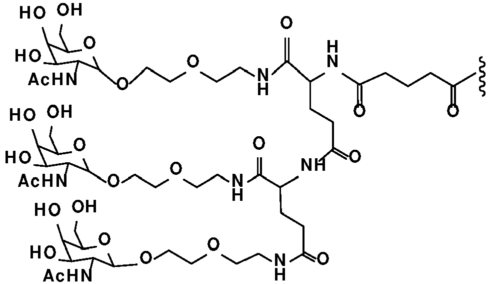 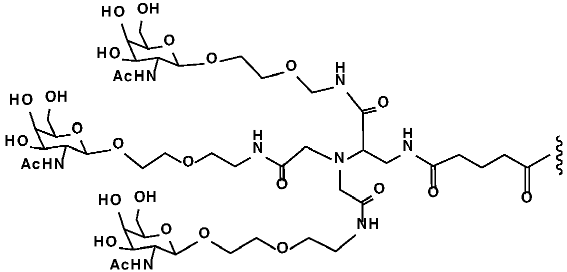 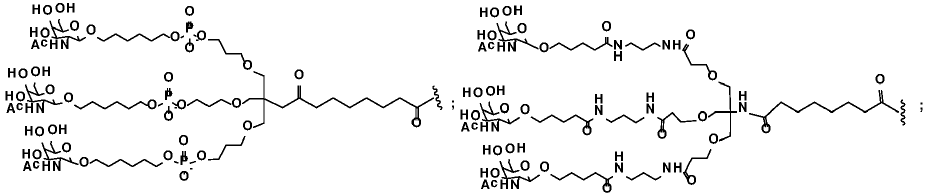 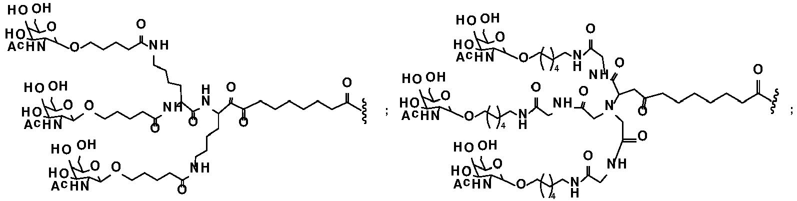 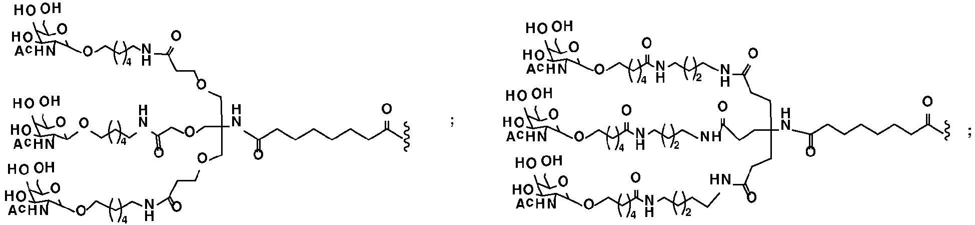 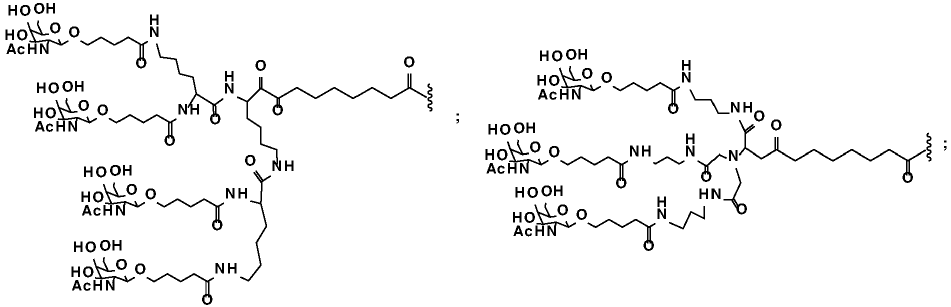 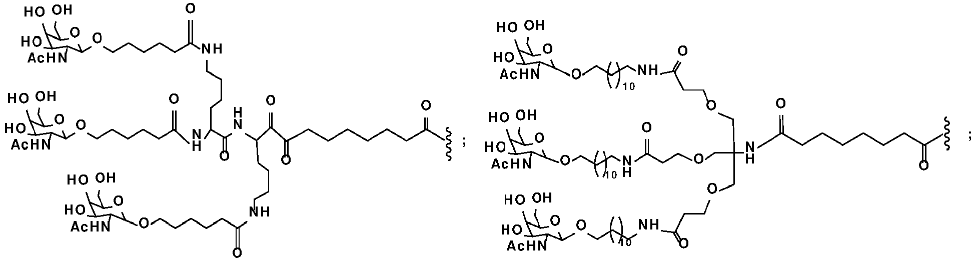 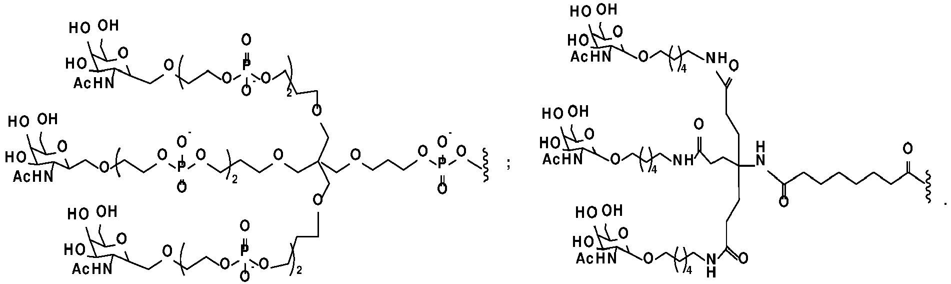 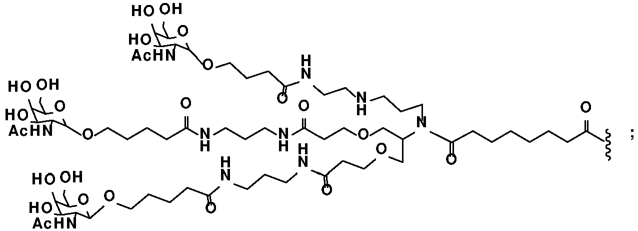 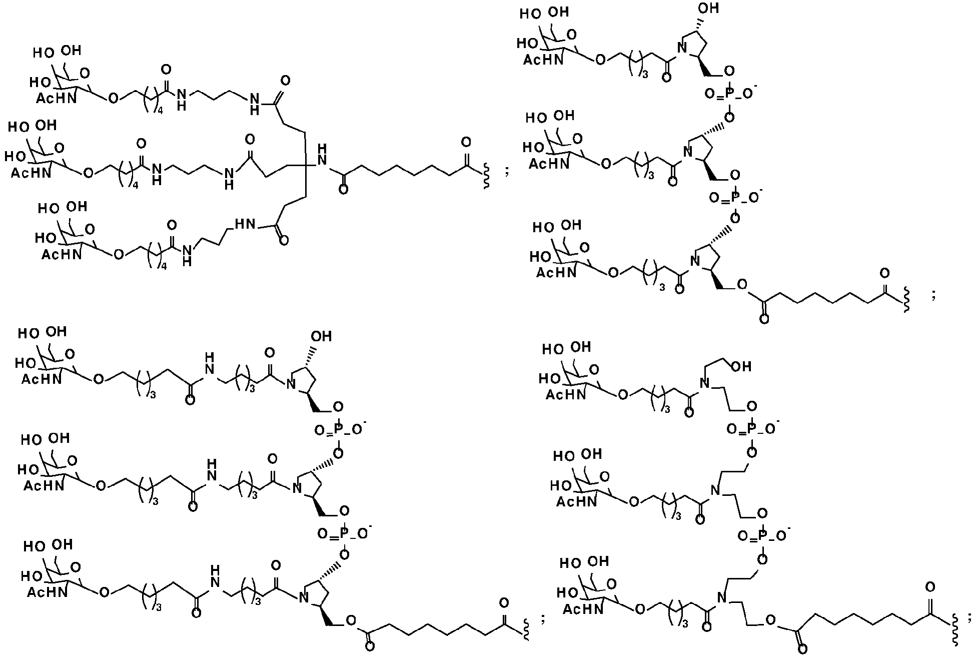 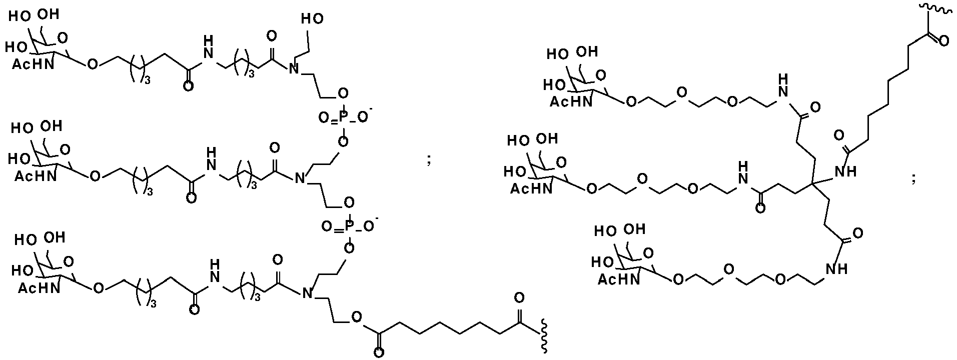 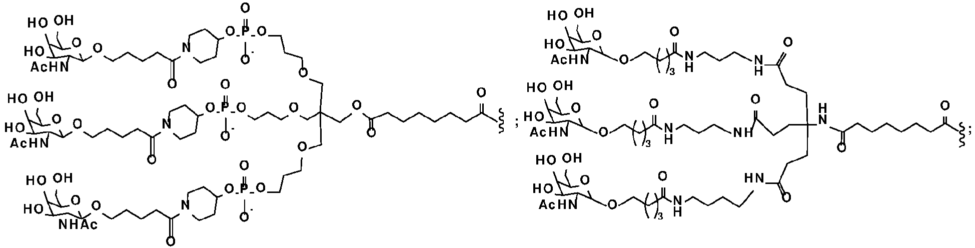 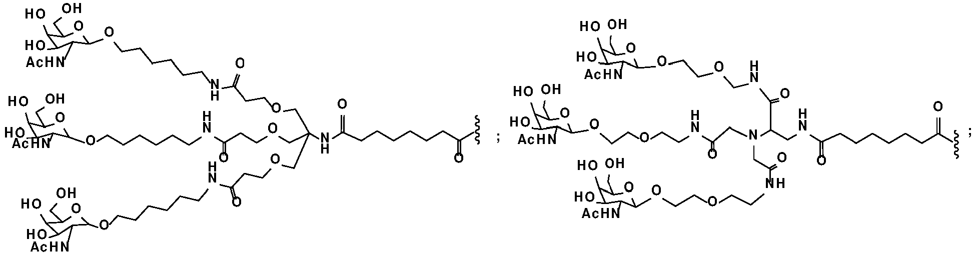 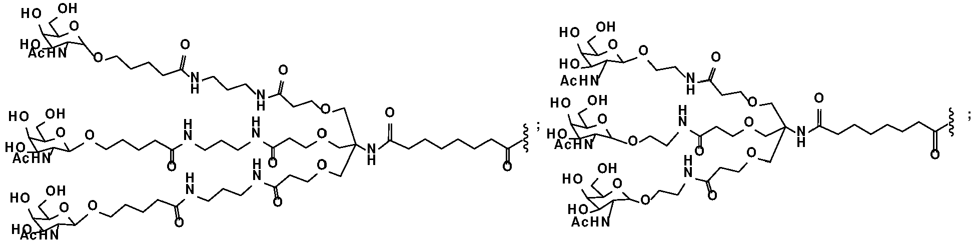 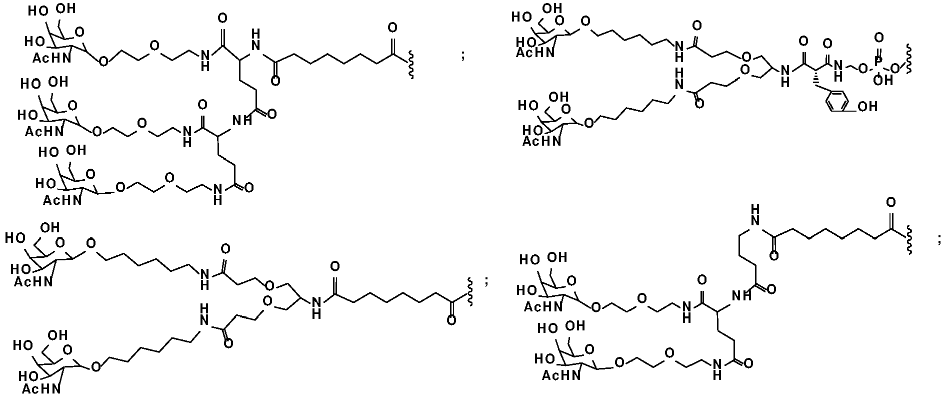 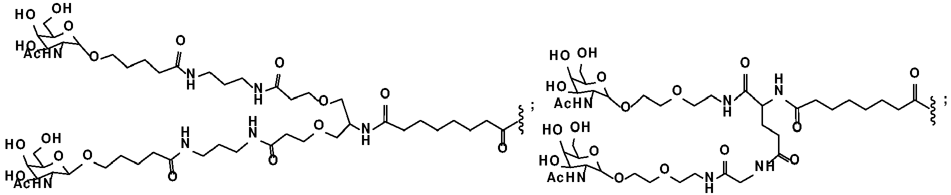 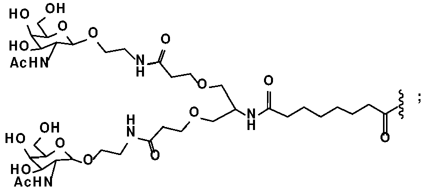 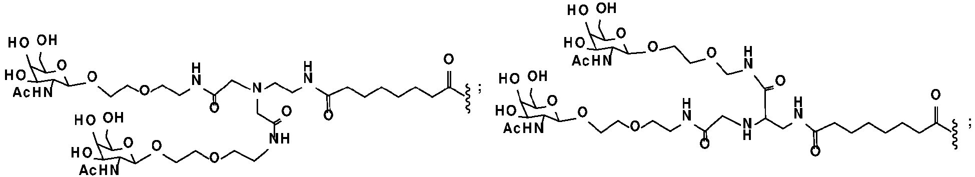 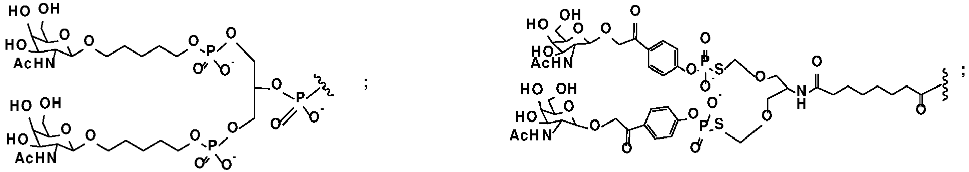 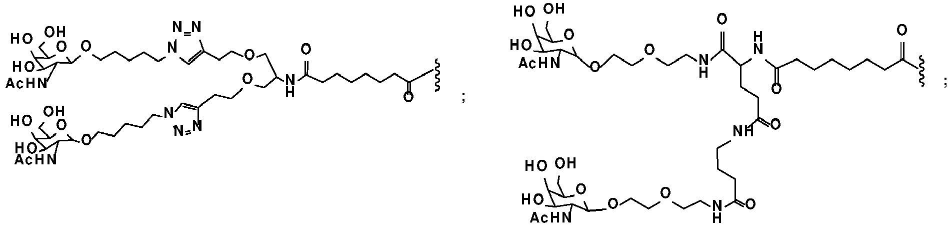 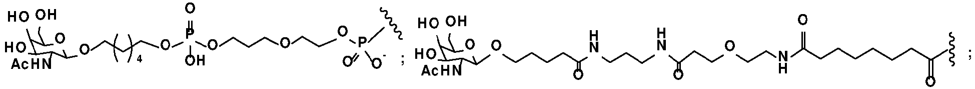 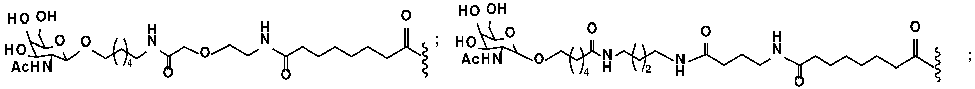 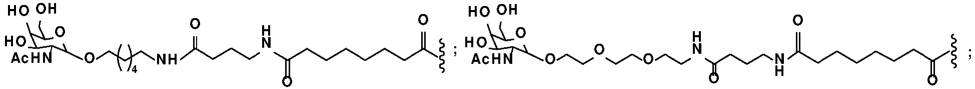 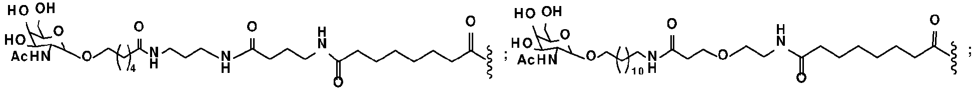 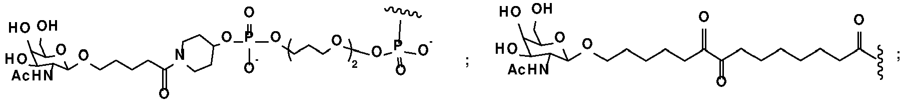 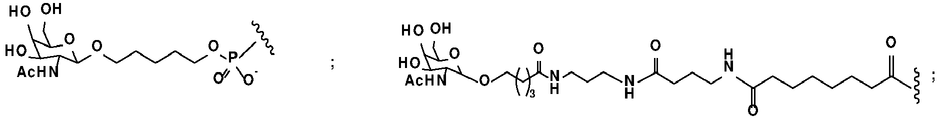 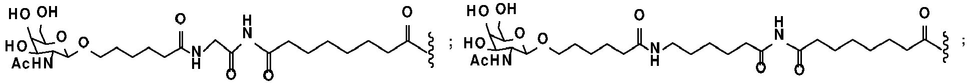 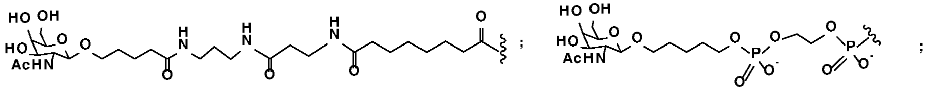 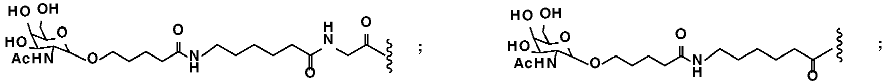 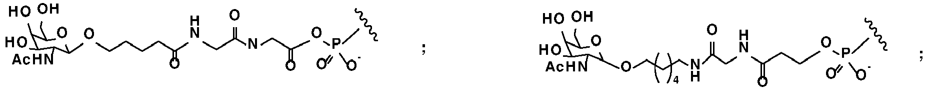 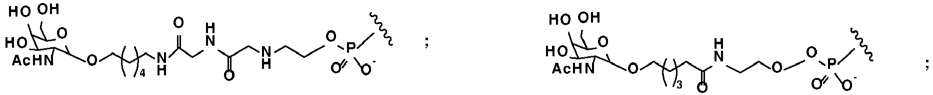 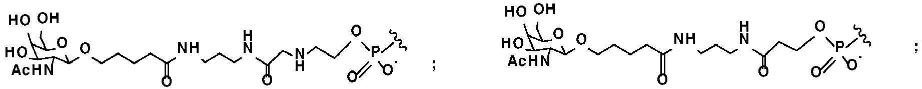 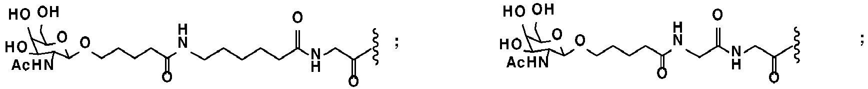 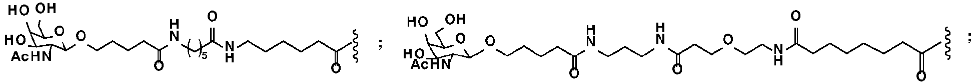 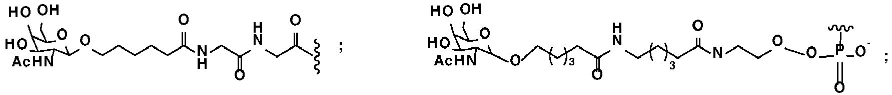 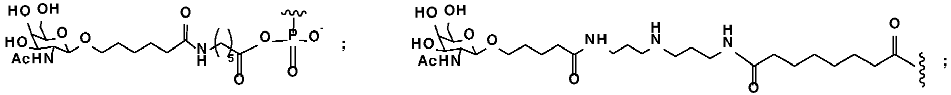 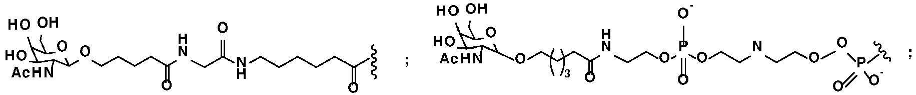 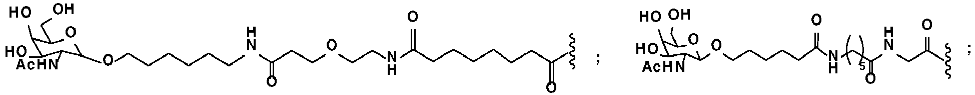 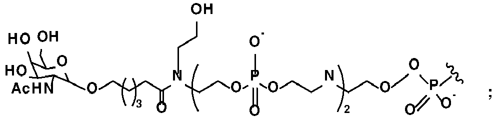 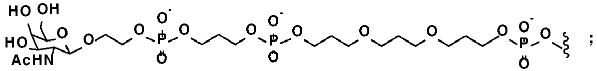 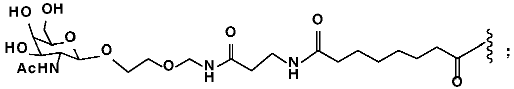 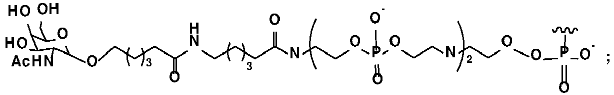 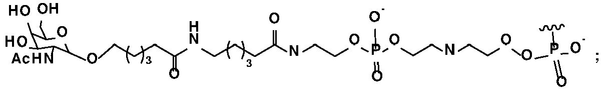 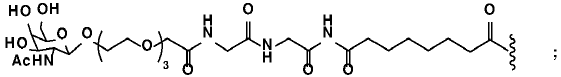 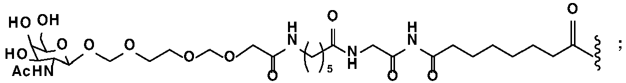 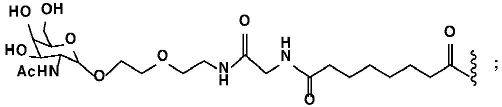 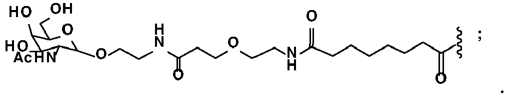
14. The RNAi agent or pharmaceutically acceptable salt thereof according to any one of embodiments 1-13, wherein the X, L, D and B are the same or different within the respective 5'MVIP and 3'MVIP or between the 5'MVIP and 3'MVIP.
15. The RNAi agent or pharmaceutically acceptable salt thereof according to any one of embodiments 1-14, wherein the 5'MVIP is selected from any one of 5'MVIP01 to 5'MVIP22 in Table 10.
16. The RNAi agent or pharmaceutically acceptable salt thereof according to any one of embodiments 1-15, wherein the 3'MVIP is selected from any one of 3'MVIP01 to 3'MVIP27 in Table 11.
17. The RNAi agent or pharmaceutically acceptable salt according to any one of embodiments 1-16, wherein the combination of sense strand 5'MVIP and antisense strand 3'MVIP is 5'MVIP01/3'MVIP01, 5'MVIP01/3'MVIP17 or 5'MVIP09/3'MVIP09; or the combination of sense strand 5'MVIP and sense strand 3'MVIP is 5'MVIP01/3'MVIP09 or 5'MVIP09/3'MVIP01.
18. The RNAi agent or pharmaceutically acceptable salt thereof according to any one of embodiments 1-17, wherein the complementary region formed by the antisense strand and the sense strand comprises at least 12 consecutive nucleotides, wherein the sense strand comprises at least 12 consecutive nucleotides with the starting positions of 1854-1874, 1907-1927, 1895-1915, 1352-1372, 1903-1923, 2019-2039, 1853-1873 and 1818-1838 in the AGT mRNA NM_001382817.3 or sequences that differ by no more than 3 nucleotides from any of them, or at least 12 consecutive nucleotides with the starting positions of 1822-1842, 1875-1895, 1863-1883, 1320-1340, 1871-1891, 1987-2007, 1821-1841 and 1786-1806 in NM_001384479.1 or sequences that differ by no more than 3 nucleotides from any of them.
19. The RNAi agent or pharmaceutically acceptable salt thereof according to any one of embodiments 1-18, wherein at least about 80% of the bases are complementary between the sense strand and antisense strand.
20. The RNAi agent or pharmaceutically acceptable salt thereof according to any one of embodiments 1-19, wherein the sense strand and antisense strand are each 15-30 nucleotides independently, preferably 17-25 nucleotides, more preferably 19-23 nucleotides.
21. The RNAi agent or pharmaceutically acceptable salt according to any one of embodiments 1-20, wherein the sense strand has substantial homology with any one of SEQ ID NO: 1, SEQ ID NO: 7, SEQ ID NO: 8, SEQ ID NO: 10, SEQ ID NO: 11, SEQ ID NO: 12, SEQ ID NO: 17 and SEQ ID NO: 18 or a sequence that differs by no more than 3 nucleotides from any of them.
22. The RNAi agent or pharmaceutically acceptable salt according to any one of embodiments 1-21, wherein the antisense strand comprises any one of SEQ ID NO: 19, SEQ ID NO: 25, SEQ ID NO: 26, SEQ ID NO: 28, SEQ ID NO: 29, SEQ ID NO: 30, SEQ ID NO: 35 and SEQ ID NO: 36 or a sequence that differs by no more than 3 nucleotides from any of them.
23. The RNAi agent or pharmaceutically acceptable salt thereof according to any one of embodiments 1-22, wherein the sense strand comprises any one of SEQ ID NO: 37, SEQ ID NO: 43, SEQ ID NO: 44, SEQ ID NO: 46, SEQ ID NO: 47, SEQ ID NO: 48, SEQ ID NO: 53 and SEQ ID NO: 54 or a sequence that differs by no more than 3 nucleotides from any of them.
24. The RNAi agent or pharmaceutically acceptable salt according to any one of embodiments 1-23, wherein the antisense strand comprises any one of SEQ ID NO: 55, SEQ ID NO: 61, SEQ ID NO: 62, SEQ ID NO: 64, SEQ ID NO: 65, SEQ ID NO: 66, SEQ ID NO: 71 and SEQ ID NO: 72 or a sequence that differs by no more than 3 nucleotides from any of them.
25. The RNAi agent or pharmaceutically acceptable salt thereof according to any one of embodiments 1-24, wherein one or more nucleotides on the sense strand and / or antisense strand are modified to form modified nucleotides.
26. The RNAi agent or pharmaceutically acceptable salt thereof according to embodiment 25, wherein the modified nucleotides are selected from: deoxyribonucleotides, nucleotide mimics, abasic nucleotides, 2'-modified nucleotides, 3'to 3' linkage (inverted) nucleotides, nucleotides containing non-natural bases, bridged nucleotides, peptide nucleic acids (PNAs), unlocked nucleobase analogues, locked nucleotides, 3'-O-methoxy (2' inter-nucleoside linkage) nucleotides, 2'-Fluoro arabinose nucleotide, 5'-methyl /2'-Fluoro-nucleotide, morpholino nucleotide, vinyl phosphonate deoxyribonucleotide, nucleotides containing vinyl phosphonate and nucleotides containing cyclopropyl phosphonate.
27. The RNAi agent or pharmaceutically acceptable salt thereof according to any one of embodiments 1-26, wherein 2' positions of part or all of the nucleotide glycosyls of the sense strand and antisense strand are fluorine or methoxy.
28. The RNAi agent or pharmaceutically acceptable salt thereof according to any one of embodiments 1-27, wherein at least two consecutive phosphorothioate linkages exist among three consecutive nucleotides at the end of the sense strand and / or the end of the antisense strand.
29. The RNAi agent or pharmaceutically acceptable salt according to any one of embodiments 1-28, wherein the 2' positions of nucleotide glycosyls at positions 7, 12 and 14 from the 5' end of the antisense strand are fluorine, the 2' positions of the remaining nucleotide glycosyls of the antisense strand are methoxy, and there are at least two consecutive phosphorothioate linkages among the three consecutive nucleotides at the end of the antisense strand; the 2' positions of nucleotide glycosyls at positions 5, 7, 8 and 9 from the 5' end of the sense strand are fluorine, and the 2' positions of the remaining nucleotide glycosyls of the sense strand are methoxy; and there are at least two consecutive phosphorothioate linkages among the three consecutive nucleotides at the end of the sense strand.
30. The RNAi agent or pharmaceutically acceptable salt according to any one of embodiments 1-29, wherein the antisense strand comprises any one of SEQ ID NO: 109, SEQ ID NO: 115, SEQ ID NO: 116, SEQ ID NO: 118, SEQ ID NO: 119, SEQ ID NO: 120, SEQ ID NO: 125 and SEQ ID NO: 126 or a sequence that differs by no more than 3 nucleotides from any of them.
31. The RNAi agent or pharmaceutically acceptable salt according to any one of embodiments 1-30, wherein the sense strand comprises any one of SEQ ID NO: 185, SEQ ID NO: 191, SEQ ID NO: 192, SEQ ID NO: 194, SEQ ID NO: 195, SEQ ID NO: 196, SEQ ID NO: 201 and SEQ ID NO: 202 or a sequence that differs by no more than 3 nucleotides from any of them.
32. The RNAi agent or pharmaceutically acceptable salt thereof according to any one of embodiments 1-31, wherein the interfering nucleic acid includes any one of Kylo-09-DS01 to Kylo-09-DS112.
33. An RNAi agent or pharmaceutically acceptable salt thereof for inhibiting AGT gene expression, comprising an antisense strand, wherein the antisense strand comprises at least 12 consecutive nucleotides that are substantially complementary to the nucleotides selected from the corresponding positions of the following sequences or a sequence that differ by no more than 3 nucleotides from it: at least 12 consecutive nucleotides with starting positions of 1854-1874, 1907-1927, 1895-1915, 1352-1372, 1903-1923, 2019-2039, 1853-1873 and 1818-1838 in AGT mRNA NM_001382817.3 or a sequence that differs from the above sequence by no more than 3 nucleotides, or at least 12 consecutive nucleotides with starting positions of 1822-1842, 1875-1895, 1863-1883, 1320-1340, 1871-1891, 1987-2007, 1821-1841, and 1786-1806 in NM_001384479.1 or a sequence that differs from the above sequence by no more than 3 nucleotides.
34. The RNAi agent or pharmaceutically acceptable salt thereof for inhibiting AGT gene expression according to embodiment 33, wherein the antisense strand has a length of 15-30 nucleotides, preferably 17-25 nucleotides, more preferably 19-23 nucleotides.
35. The RNAi agent or pharmaceutically acceptable salt thereof for inhibiting AGT gene expression according to any one of embodiments 33-34, wherein the antisense strand comprises any one of the following nucleotide sequences: SEQ ID NO: 19, SEQ ID NO: 25, SEQ ID NO: 26, SEQ ID NO: 28, SEQ ID NO: 29, SEQ ID NO: 30, SEQ ID NO: 35 and SEQ ID NO: 36 or a sequence that differs by no more than 3 nucleotides from any of them.
36. The RNAi agent or pharmaceutically acceptable salt thereof for inhibiting AGT gene expression according to any one of embodiments 33-35, wherein the RNAi agent includes a single stranded or double stranded nucleic acid molecule.
37. The RNAi agent or pharmaceutically acceptable salt thereof for inhibiting AGT gene expression according to any one of embodiments 33-36, wherein the RNAi agent includes siRNA or miRNA.
38. The RNAi agent or pharmaceutically acceptable salt thereof for inhibiting AGT gene expression according to any one of embodiments 33-37, which further comprises a sense strand, wherein the complementary region formed by the antisense strand and the sense strand comprises at least 12 consecutive nucleotides.
39. The RNAi agent or pharmaceutically acceptable salt thereof for inhibiting AGT gene expression according to any one of embodiments 33-38, wherein the complementary region comprises 12-25 consecutive nucleotide base pairs.
40. The RNAi agent or pharmaceutically acceptable salt thereof for inhibiting AGT gene expression according to any one of embodiments 33-39, wherein at least about 80% of bases are complementary between the sense strand and the antisense strand.
41. The RNAi agent or pharmaceutically acceptable salt thereof for inhibiting AGT gene expression according to any one of embodiments 33-40, wherein the sense strand comprises at least 12 consecutive nucleotides with the starting positions of 1854-1874, 1907-1927, 1895-1915, 1352-1372, 1903-1923, 2019-2039, 1853-1873 and 1818-1838 in the AGT mRNA NM_001382817.3 or a sequence that differs from the above sequence by no more than 3 nucleotides, or at least 12 consecutive nucleotides with the starting positions of 1822-1842, 1875-1895, 1863-1883, 1320-1340, 1871-1891, 1987-2007, 1821-1841 and 1786-1806 in NM_001384479.1 or a sequence that differs from the above sequence by no more than 3 nucleotides.
42. The RNAi agent or pharmaceutically acceptable salt thereof for inhibiting AGT gene expression according to any one of embodiments 33-41, wherein the sense strand has a length of 15-30 nucleotides, preferably 17-25 nucleotides, more preferably 19-23 nucleotides.
43. The RNAi agent or pharmaceutically acceptable salt thereof for inhibiting AGT gene expression according to any one of embodiments 33-43, wherein the sense strand has substantial homology with any one of SEQ ID NO: 1, SEQ ID NO: 7, SEQ ID NO: 8, SEQ ID NO: 10, SEQ ID NO: 11, SEQ ID NO: 12, SEQ ID NO: 17 and SEQ ID NO: 18 or a sequence that differs by no more than 3 nucleotides from any of them..
44. The RNAi agent or pharmaceutically acceptable salt thereof for inhibiting AGT gene expression according to any one of embodiments 33-43, wherein the sense strand has substantial homology with any one of SEQ ID NO: 1, SEQ ID NO: 7, SEQ ID NO: 8, SEQ ID NO: 10, SEQ ID NO: 11, SEQ ID NO: 12, SEQ ID NO: 17 and SEQ ID NO: 18 or a sequence that differs by no more than 3 nucleotides from any of them, and the antisense strand comprises any one of SEQ ID NO: 19, SEQ ID NO: 25, SEQ ID NO: 26, SEQ ID NO: 28, SEQ ID NO: 29, SEQ ID NO: 30, SEQ ID NO: 35 and SEQ ID NO: 36 or a sequence that differs by no more than 3 nucleotides from any of them.
45. The RNAi agent or pharmaceutically acceptable salt thereof for inhibiting AGT gene expression according to any one of embodiments 33-44, wherein the antisense strand comprises any one of SEQ ID NO: 55, SEQ ID NO: 61, SEQ ID NO: 62, SEQ ID NO: 64, SEQ ID NO: 65, SEQ ID NO: 66, SEQ ID NO: 71 and SEQ ID NO: 72 or a sequence that differs by no more than 3 nucleotides from any of them.
46. The RNAi agent or pharmaceutically acceptable salt thereof for inhibiting AGT gene expression according to any one of embodiments 33-45, wherein the sense strand comprises any one of SEQ ID NO: 37, SEQ ID NO: 43, SEQ ID NO: 44, SEQ ID NO: 46, SEQ ID NO: 47, SEQ ID NO: 48, SEQ ID NO: 53 and SEQ ID NO: 54 or a sequence that differs by no more than 3 nucleotides from any of them.
47. The RNAi agent or pharmaceutically acceptable salt thereof for inhibiting AGT gene expression according to any one of embodiments 33-46, wherein the antisense strand comprises any one of SEQ ID NO: 55, SEQ ID NO: 61, SEQ ID NO: 62, SEQ ID NO: 64, SEQ ID NO: 65, SEQ ID NO: 66, SEQ ID NO: 71 and SEQ ID NO: 72 or a sequence that differs by no more than 3 nucleotides from any of them, and the sense strand comprises any one of SEQ ID NO: 37, SEQ ID NO: 43, SEQ ID NO: 44, SEQ ID NO: 46, SEQ ID NO: 47, SEQ ID NO: 48, SEQ ID NO: 53 and SEQ ID NO: 54 or a sequence that differs by no more than 3 nucleotides from any of them.
48. The RNAi agent or pharmaceutically acceptable salt thereof for inhibiting AGT gene expression according to any one of embodiments 33-47, wherein one or more nucleotides on the sense strand and / or antisense strand are modified to form modified nucleotides.
49. The RNAi agent or pharmaceutically acceptable salt thereof for inhibiting AGT gene expression according to embodiment 48, wherein the modified nucleotide is selected from: deoxyribonucleotide, nucleotide mimics, abasic nucleotide, 2'- modified nucleotide, 3' to 3 '- linkage (inverted) nucleotide, nucleotide containing unnatural base, bridged nucleotide, peptide nucleic acid (PNA), unlocked nucleobase analogue, locked nucleotide 3'-O-methoxy (2' inter-nucleoside linkage) nucleotide, 2'-Fluoro-arabinose nucleotide, 5'-methyl /2'-Fluoro nucleotide, morpholino nucleotide, vinyl phosphonate deoxyribonucleotide, nucleotide containing vinyl phosphonate and nucleotide containing cyclopropyl phosphonate.
50. The RNAi agent or pharmaceutically acceptable salt thereof for inhibiting AGT gene expression according to embodiment 49, wherein the 2'- modified nucleotide includes: 2'-O-methyl nucleotide, 2'-deoxy-2'-fluoronucleotide, 2'-deoxynucleotide, 2'-methoxyethyl nucleotide, 2'-amino nucleotide and / or 2'-alkyl nucleotide.
51. The RNAi agent or pharmaceutically acceptable salt thereof for inhibiting AGT gene expression according to any one of embodiments 33-50, wherein the substituents at 2' positions of part or all of the nucleotide glycosyls of the sense strand and antisense strand are fluorine or methoxy.
52. The RNAi agent or pharmaceutically acceptable salt thereof for inhibiting AGT gene expression according to any one of embodiments 33-51, wherein at least two consecutive phosphorothioate linkages exist among three consecutive nucleotides at the end of the sense strand and / or the end of the antisense strand.
53. The RNAi agent or pharmaceutically acceptable salt thereof for inhibiting AGT gene expression according to any one of embodiments 33-52, wherein the 2' positions of nucleotide glycosyls at positions 7, 12 and 14 starting from the 5' end of the antisense strand are fluorine, the 2' positions of the remaining nucleotide glycosyls of the antisense strand are methoxy, and there are at least two consecutive phosphorothioate linkages among three consecutive nucleotides at the end of the antisense strand; the 2' positions of nucleotide glycosyls at positions 5, 7, 8 and 9 from the 5' end of the sense strand are fluorine, and the 2'positions of the remaining nucleotide glycosyls of the sense strand are methoxy; and there are at least two consecutive phosphorothioate linkages among three consecutive nucleotides at the end of the sense strand.
54. The RNAi agent or pharmaceutically acceptable salt thereof for inhibiting AGT gene expression according to any one of embodiments 33-53, wherein the antisense strand comprises any one of SEQ ID NO: 109, SEQ ID NO: 115, SEQ ID NO: 116, SEQ ID NO: 118, SEQ ID NO: 119, SEQ ID NO: 120, SEQ ID NO: 125 and SEQ ID NO: 126 or a sequence that differs by no more than 3 nucleotides from any of them.
55. The RNAi agent or pharmaceutically acceptable salt thereof for inhibiting AGT gene expression according to any one of embodiments 33-54, wherein the sense strand comprises any one of SEQ ID NO: 185, SEQ ID NO: 191, SEQ ID NO: 192, SEQ ID NO: 194, SEQ ID NO: 195, SEQ ID NO: 196, SEQ ID NO: 201 and SEQ ID NO: 202 or a sequence that differs by no more than 3 nucleotides from any of them.
56. The RNAi agent or pharmaceutically acceptable salt thereof for inhibiting AGT gene expression according to any one of embodiments 33-55, wherein the interfering nucleic acid includes any one or more of Kylo-09-DS01, Kylo-09-DS07, Kylo-09-DS08, Kylo-09-DS10, Kylo-09-DS 11, Kylo-09-DS 12, Kylo-09-DS 17, Kylo-09-DS 18, Kylo-09-DS37~Kylo-09-DS54.
57. The RNAi agent or pharmaceutically acceptable salt thereof for inhibiting AGT gene expression according to any one of embodiments 33-56 further comprising a ligand, which is conjugated to the sense strand and / or antisense strand through a carrier structure.
58. The RNAi agent or pharmaceutically acceptable salt thereof for inhibiting AGT gene expression according to any one of embodiments 33-57, wherein the ligand comprises a targeting ligand.
59. The RNAi agent or pharmaceutically acceptable salt thereof for inhibiting AGT gene expression according to any one of embodiments 33-58, wherein the targeting ligand is conjugated to the 5' end and / or 3' end of the antisense strand through a carrier structure.
60. The RNAi agent or pharmaceutically acceptable salt thereof for inhibiting AGT gene expression according to any one of embodiments 33-59, wherein the carrier structure is bound to the 5' end and / or 3' end of the sense strand.
61. The RNAi agent or pharmaceutically acceptable salt thereof for inhibiting AGT gene expression according to any one of embodiments 57-60, wherein the carrier structure includes 5'MVIP and 3'MVIP, wherein the 5'MVIP is conjugated at the 5' end of the sense strand and / or antisense strand, the 3'MVIP is conjugated at the 3' end of the antisense strand and / or sense strand, the structure of the 5'MVIP is shown in formula I, and the structure of the 3'MVIP is shown in formula II,

   **(X-L)ₙ-B-D-R₁⁻,** I

   **(X-L)ₘ-B-D-R₂⁻**, II

   wherein,
   X is a targeting specific ligand;
   L is a branched chain;
   B is a linker;
   D is a linking chain;
   R₁ and R₂ are transition points;
   the 5'MVIP is connected with the 5' end of the sense strand or the 5 'end of the antisense strand through the transition point R₁, and the 3'MVIP is connected with the 3' end of the sense strand or the 3' end of the antisense strand through the transition point R₂, n and m are each independently any integer from 0 to 4.
62. The RNAi agent or pharmaceutically acceptable salt thereof for inhibiting AGT gene expression according to embodiment 61, wherein n+m is an integer of 2 to 6, preferably n+m=2, 3 or 4, more preferably 4.
63. The RNAi agent or pharmaceutically acceptable salt thereof for inhibiting AGT gene expression according to any one of embodiments 61-62, wherein the R₁ or R₂ is connected with the sense strand or antisense strand through phosphate or modified phosphate, preferably through phosphate or phosphorothioate.
64. The RNAi agent or pharmaceutically acceptable salt thereof for inhibiting AGT gene expression according to any one of embodiments 61-63, wherein R₁ is a heterocyclic or carbocyclic structure containing N, S or O: 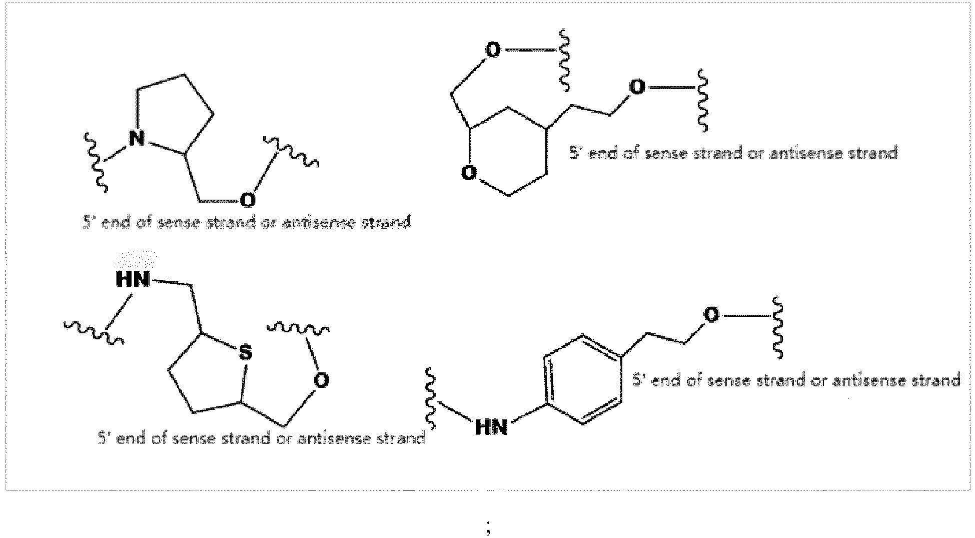 alternatively, R₁ is -NH(CH₂)ₓCH₂O-, wherein x is any integer from 3 to 12, preferably any integer from 4 to 6.
65. The RNAi agent or pharmaceutically acceptable salt thereof for inhibiting AGT gene expression according to any one of embodiments 61-64, wherein the R₂ is a heterocyclic or carbocyclic structure containing N, S or O: 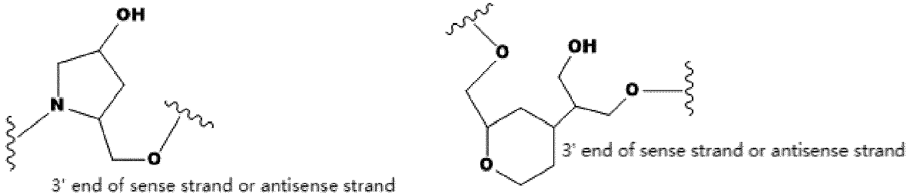 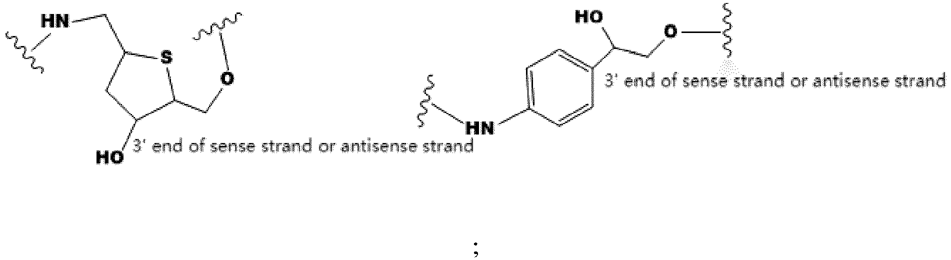 alternatively, the R₂ is -NH(CH₂)ₓ₁CH(OH)(CH₂)ₓ₂CH₂O-, wherein x1 is any integer of 1-4 and x2 is any integer of 0-4.
66. The RNAi agent or pharmaceutically acceptable salt thereof for inhibiting AGT gene expression according to any one of embodiments 61-65, wherein the X is a targeting ligand selected from a structure used to enhance the uptake of RNAi agent by hepatocytes.
67. The RNAi agent or pharmaceutically acceptable salt thereof for inhibiting AGT gene expression according to any one of embodiments 61-66, wherein the X is selected from monosaccharide and derivatives thereof.
68. The RNAi agent or pharmaceutically acceptable salt thereof for inhibiting AGT gene expression according to any one of embodiments 61-67, wherein the X is selected from galactose, galactosamine, N-acetylgalactosamine and derivatives thereof.
69. The RNAi agent or pharmaceutically acceptable salt thereof for inhibiting AGT gene expression according to any one of embodiments 61-68, wherein the X is N-acetylgalactosamine and derivatives thereof.
70. The RNAi agent or pharmaceutically acceptable salt thereof for inhibiting AGT gene expression according to any one of embodiments 61-69, wherein the X is selected from the following structures: 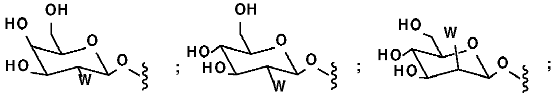 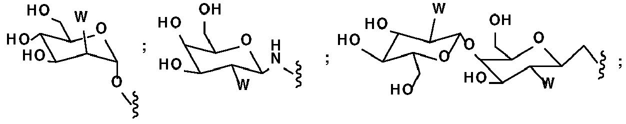 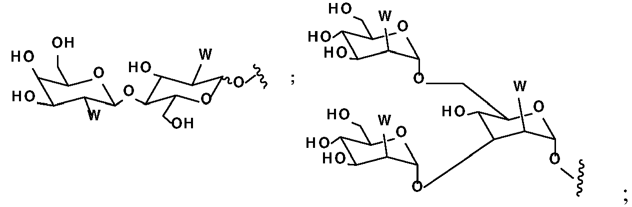 wherein W is selected from one or two of -OH, -NHCOOH or -NHCO(CH₂)_{q}CH₃, wherein q is an integer of 0-4.
71. The RNAi agent or pharmaceutically acceptable salt thereof for inhibiting AGT gene expression according to any one of embodiments 61-70, wherein the L is selected from one or more of the following structures: 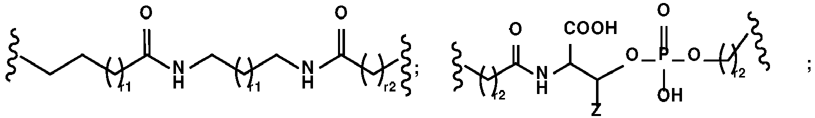 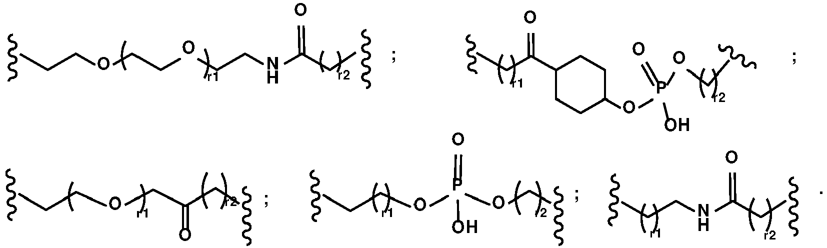 wherein r1 is any integer from 1 to 12, r2 is any integer from 0 to 20, and Z is H, alkyl or amido group.
72. The RNAi agent or pharmaceutically acceptable salt thereof for inhibiting AGT gene expression according to any one of embodiments 61-71, wherein the B is selected from the following structures: 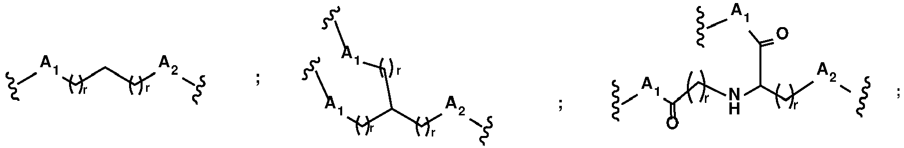 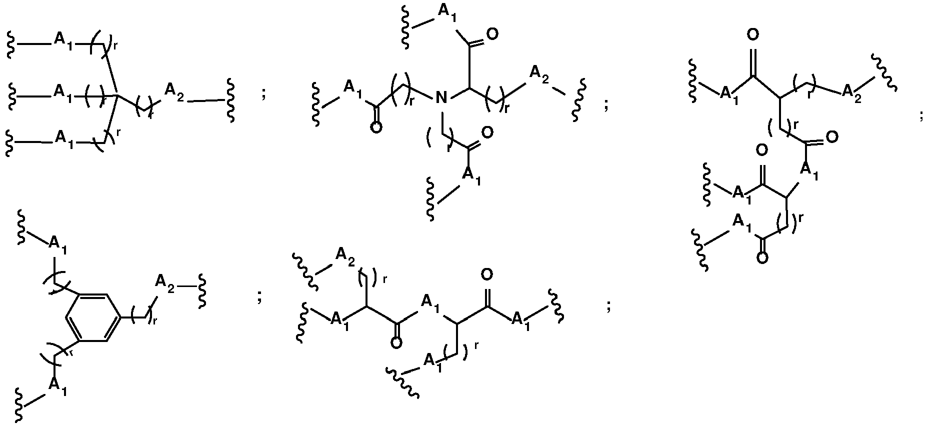 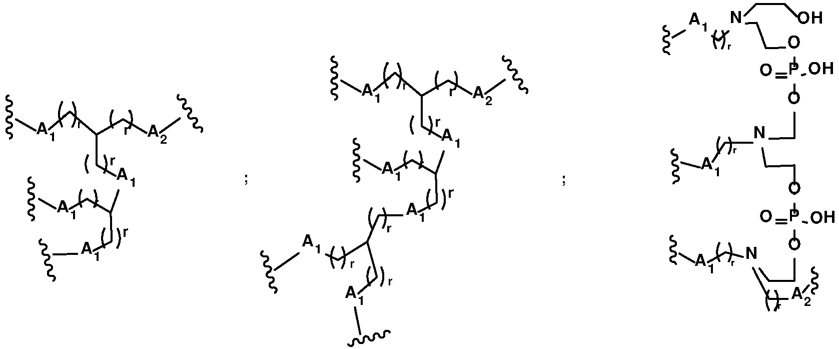 wherein A₁ and A₂ are each independently C, O, S, -NH-, carbonyl, amido, phosphoryl or thiophosphoryl, and r is an integer of 0-4.
73. The RNAi agent or pharmaceutically acceptable salt thereof for inhibiting AGT gene expression according to any one of embodiments 61-72, the D is selected from the following structure: 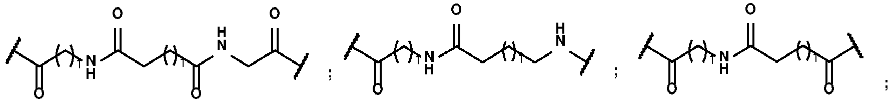 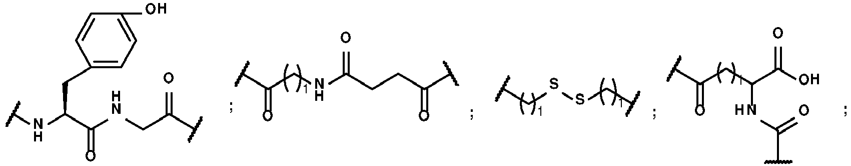 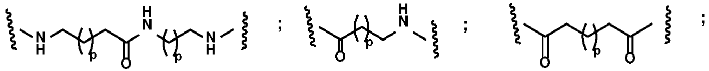 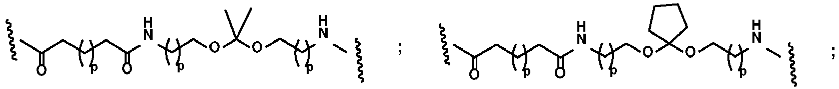 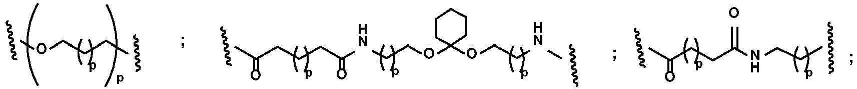 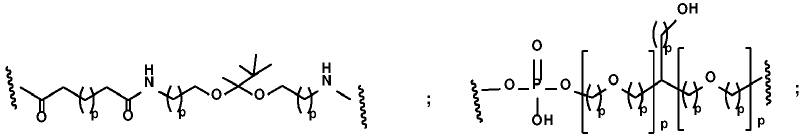 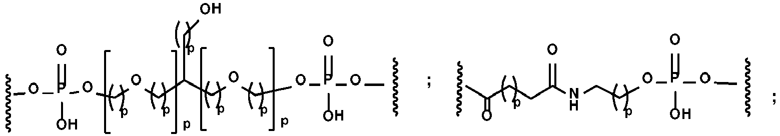 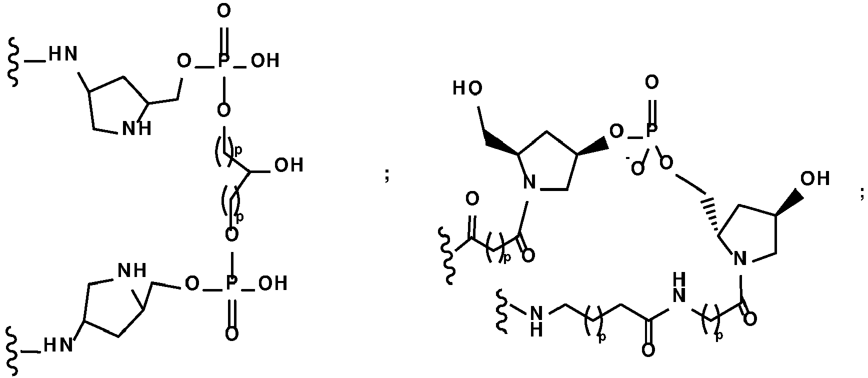 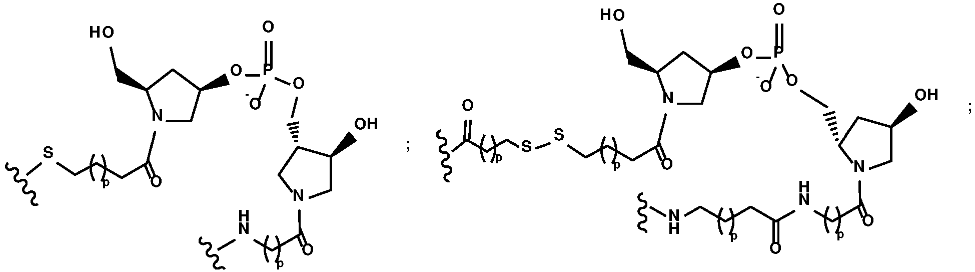 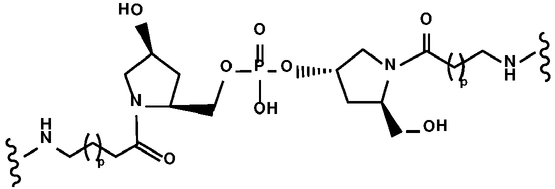 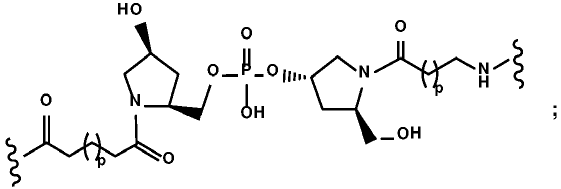 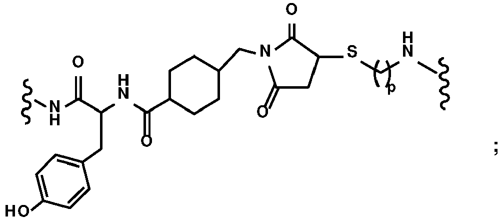 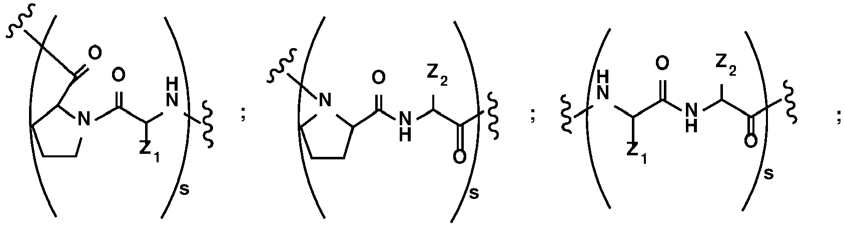 Where, each p is an arbitrary integer of 1-20 independently; s is an integer of 2-13; Z₁ and Z₂ are the same or different substituents.
74. The RNAi agent or pharmaceutically acceptable salt for inhibiting AGT gene expression according to any one of embodiments 61-73, the 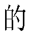 **(X-L)n-B-D-** 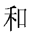 3'MVIP 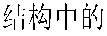 **(X-L)m-B-D-** 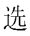 5'MVIP structure and the 3'MVIP structure are selected from one or more of the following structures: 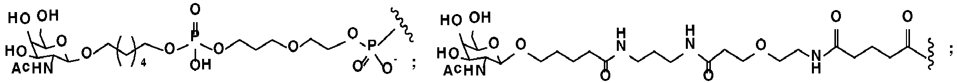 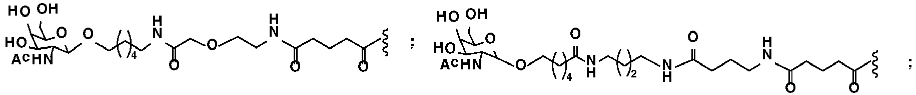 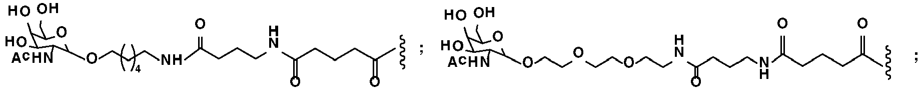 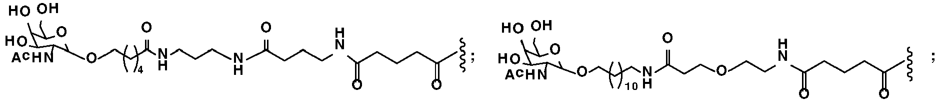 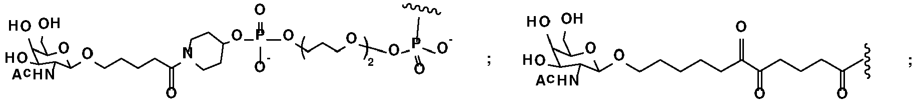 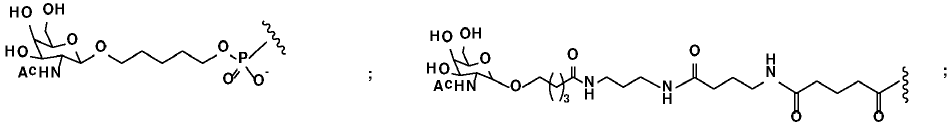 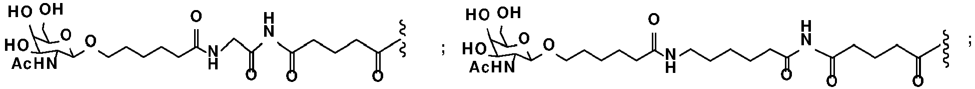 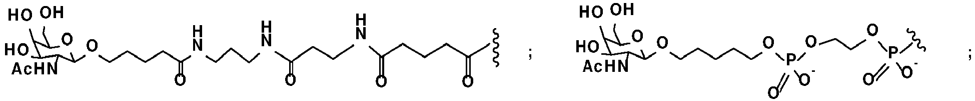 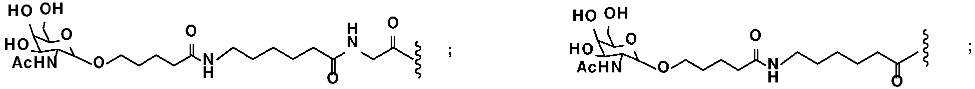 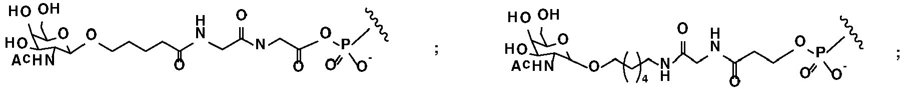 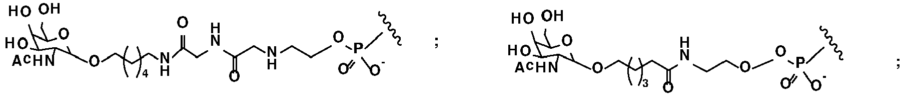 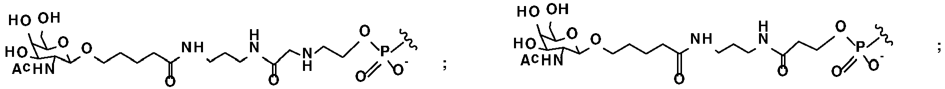 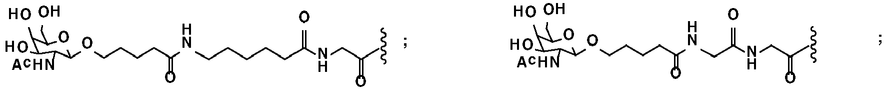 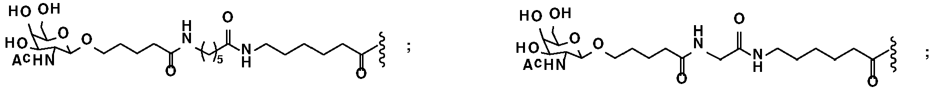 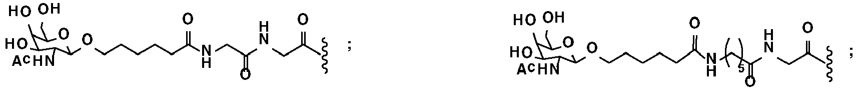 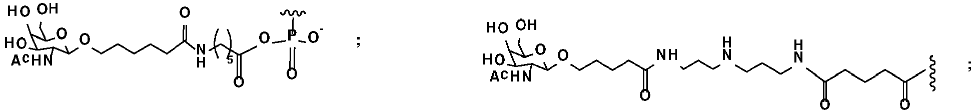 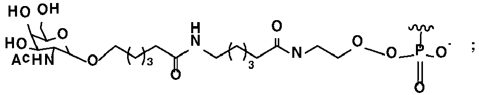 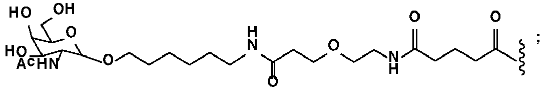 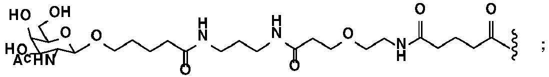 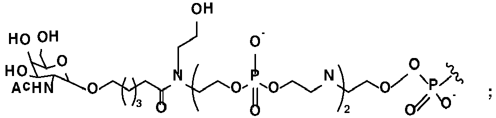 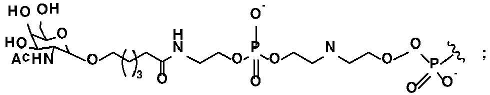 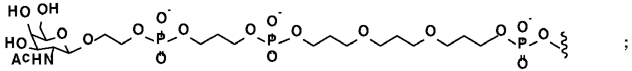 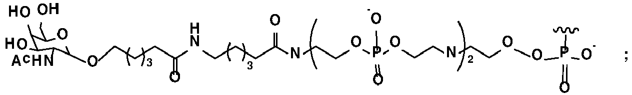 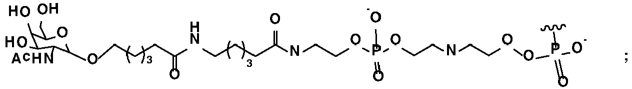 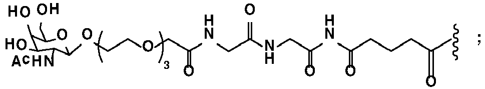 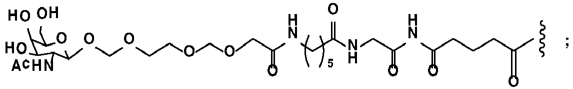 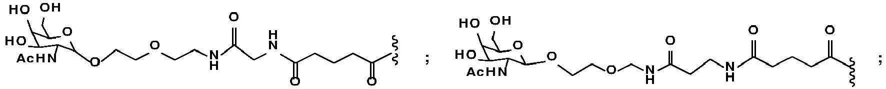 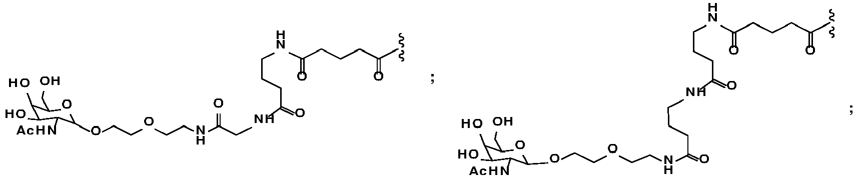 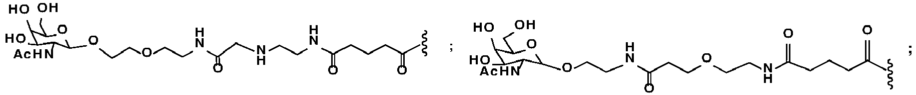 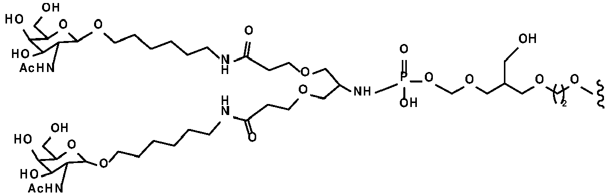 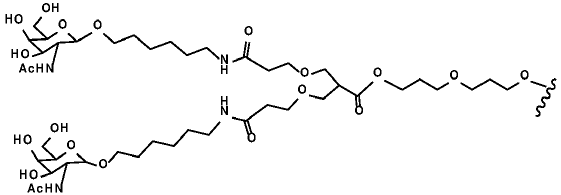 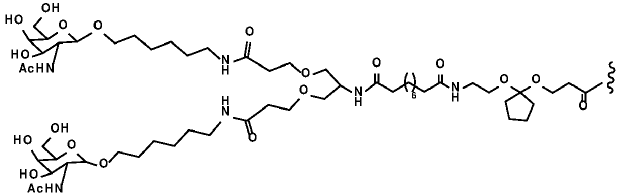 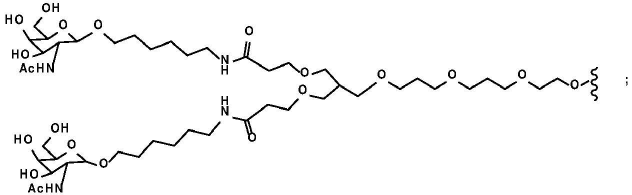 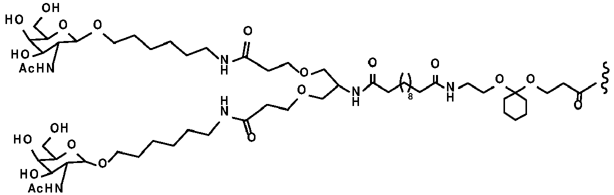 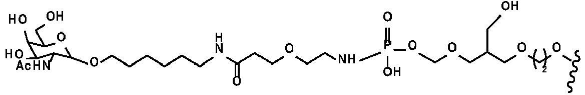 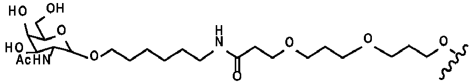 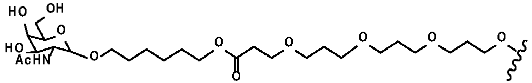 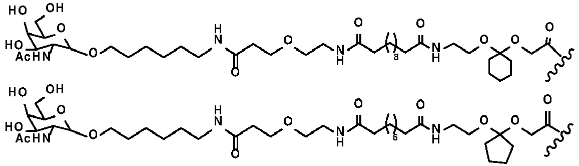 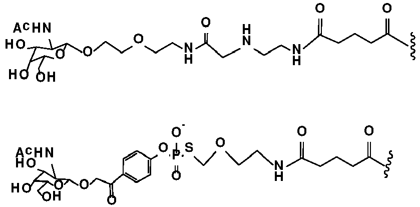 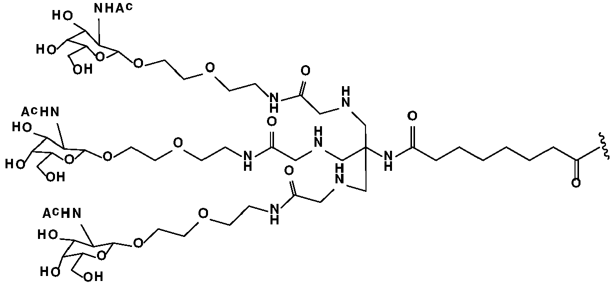 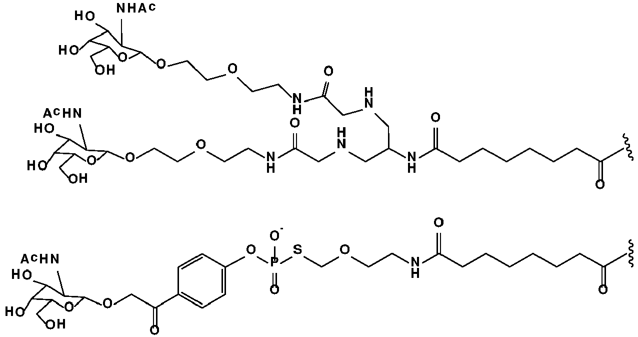 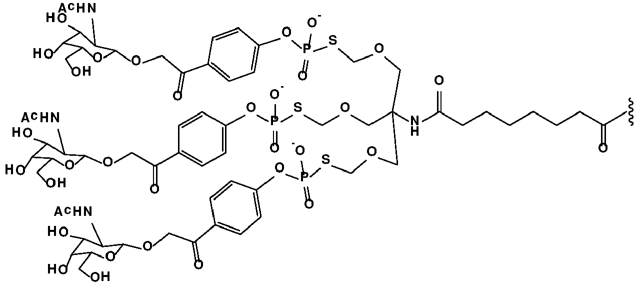 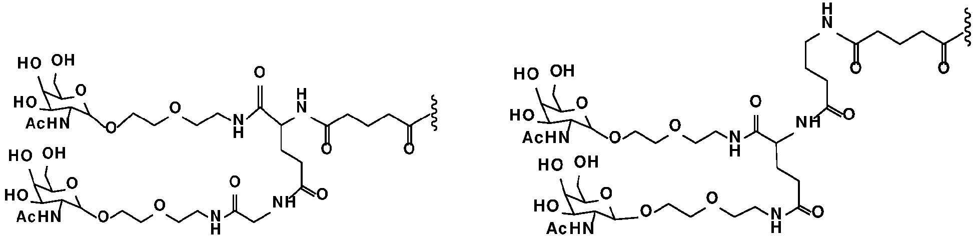 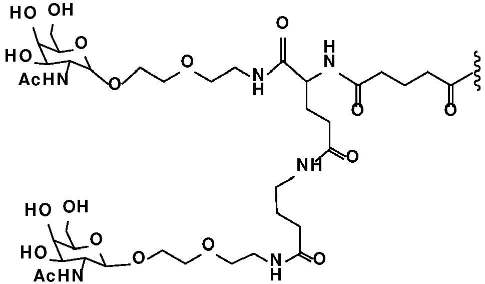 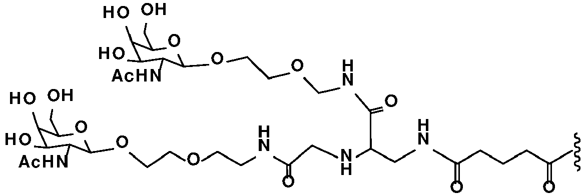 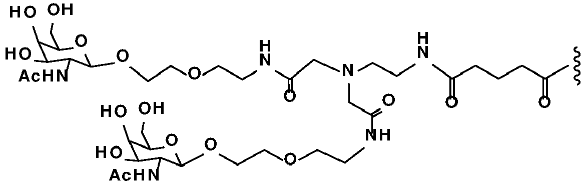 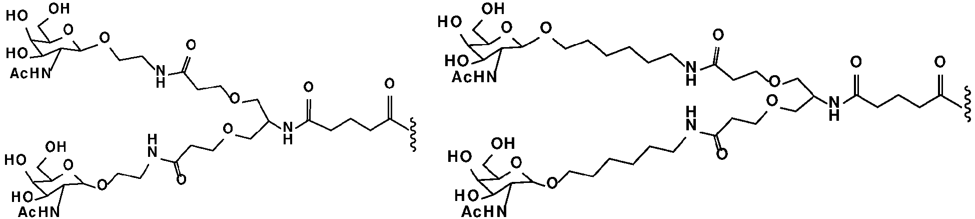 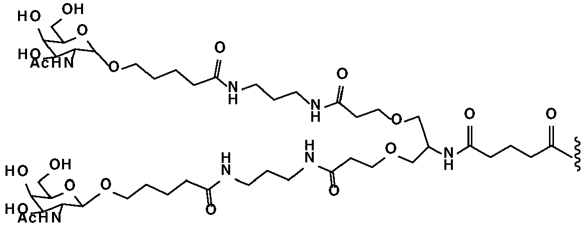 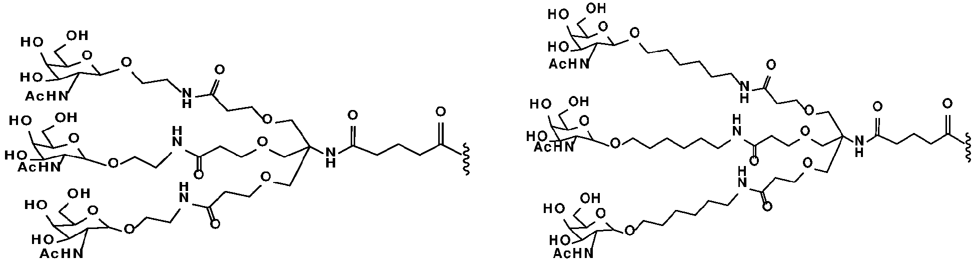 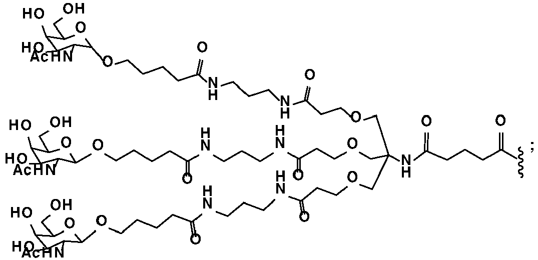 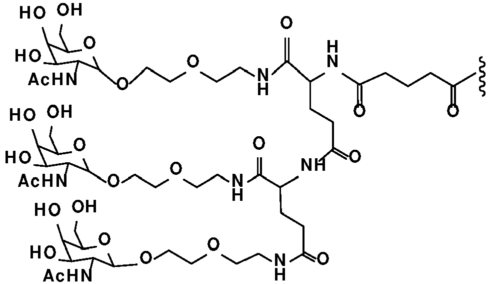 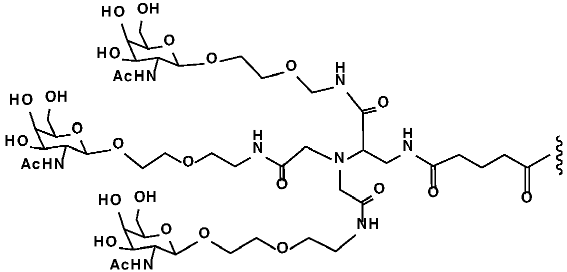 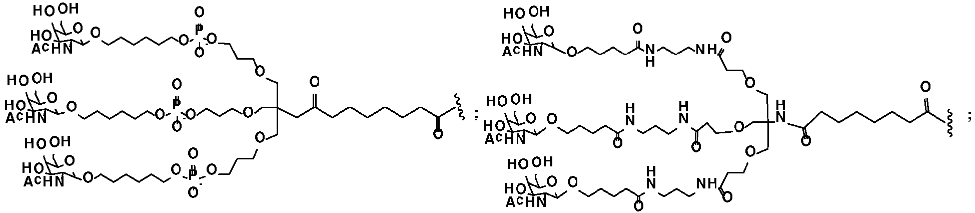 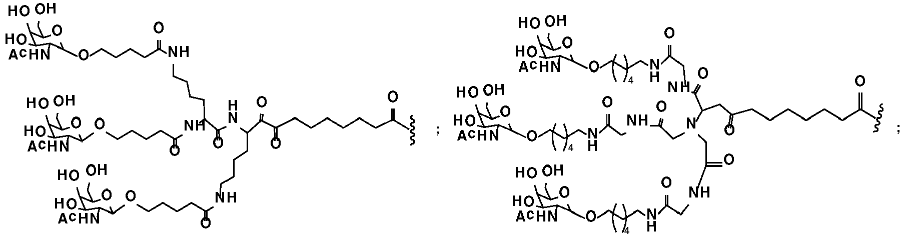 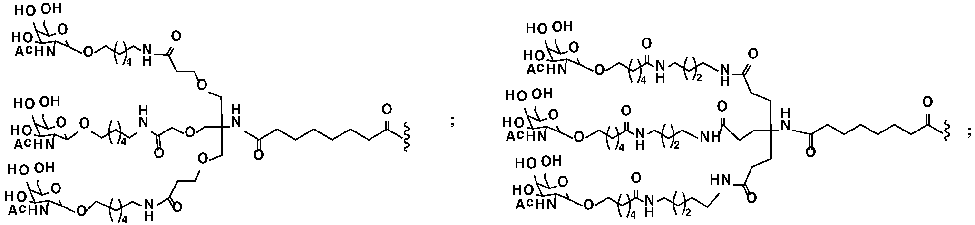 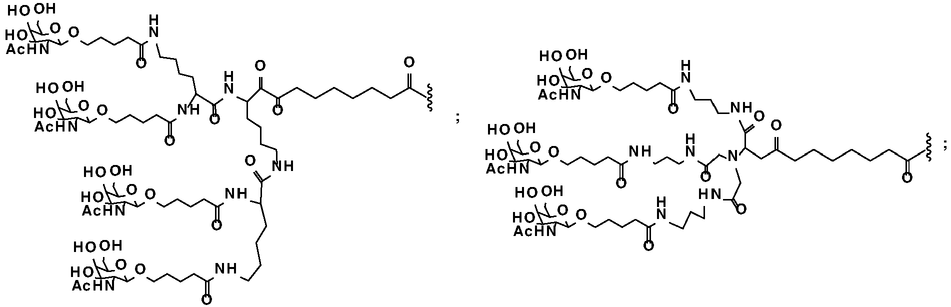 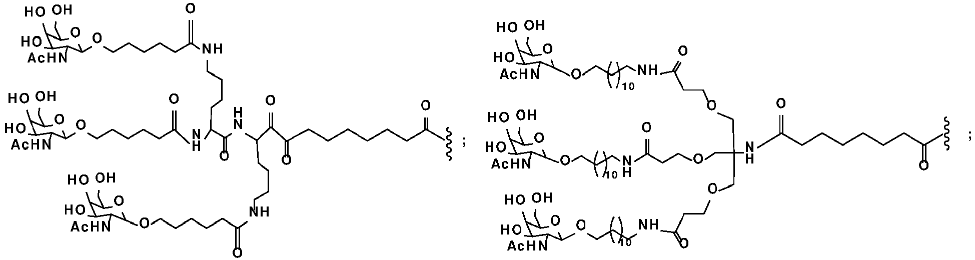 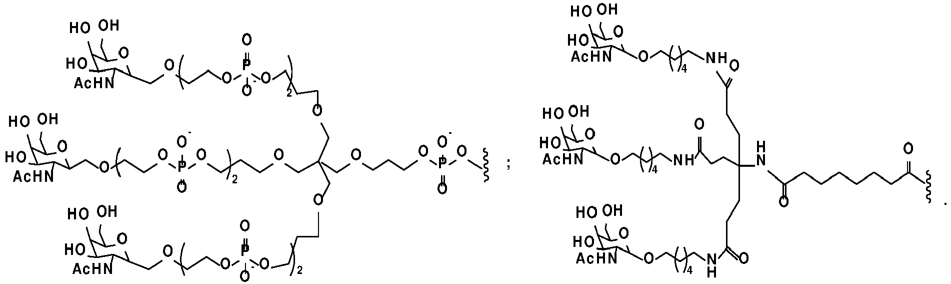 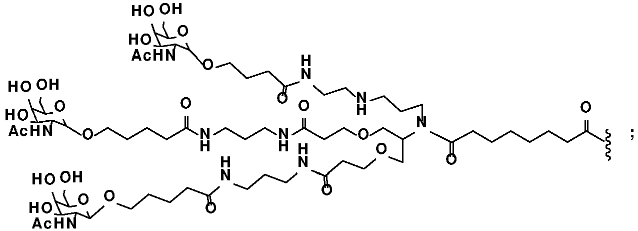 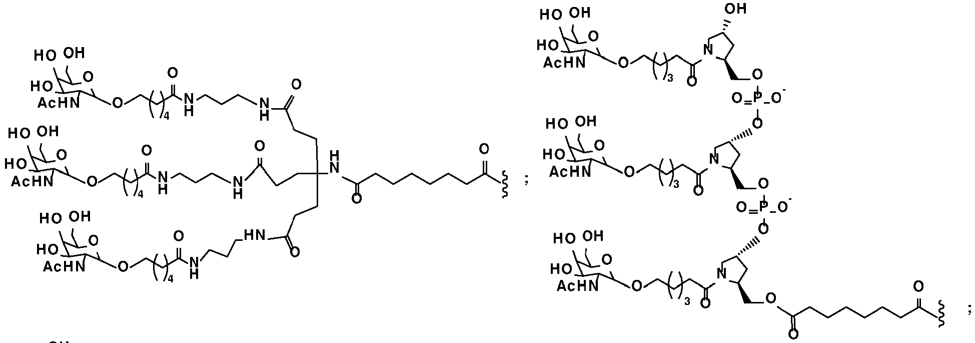 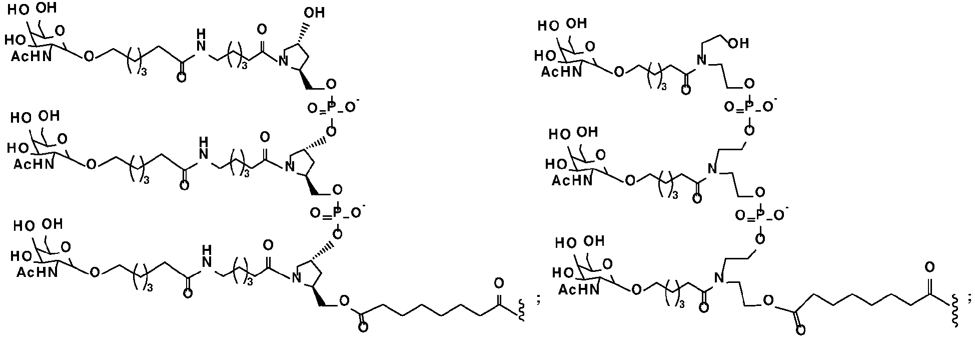 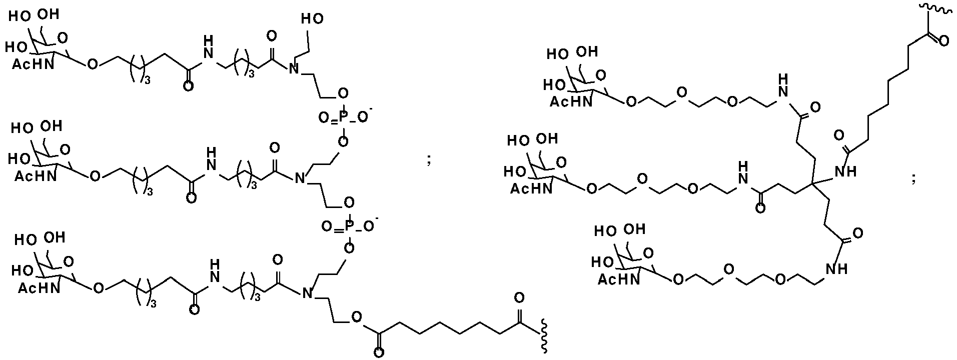 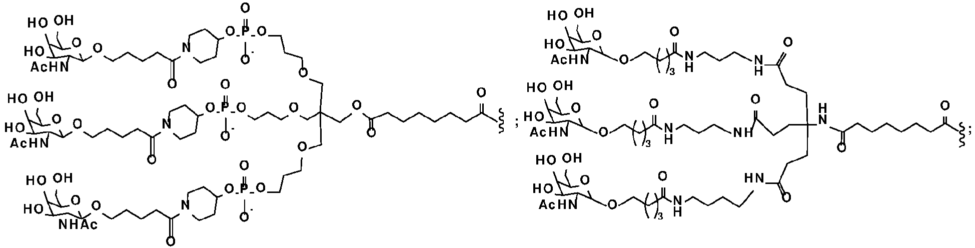 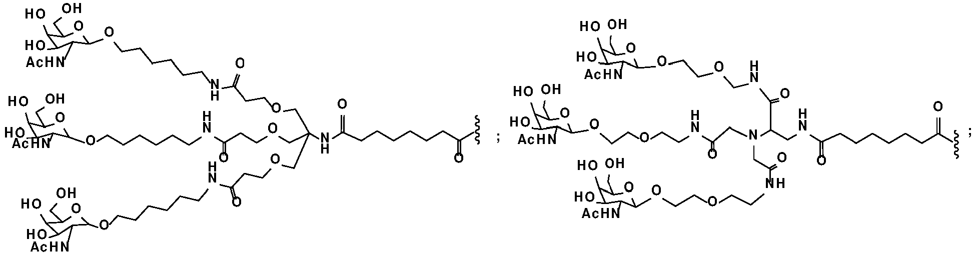 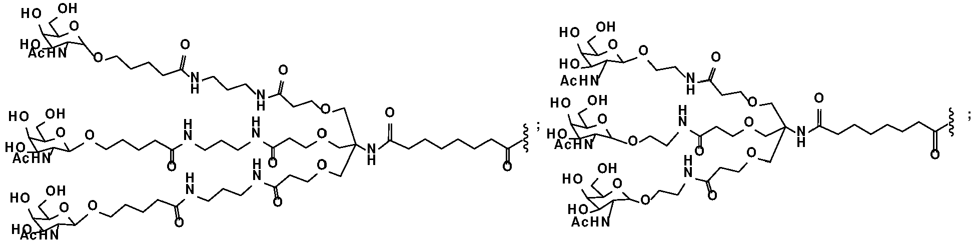 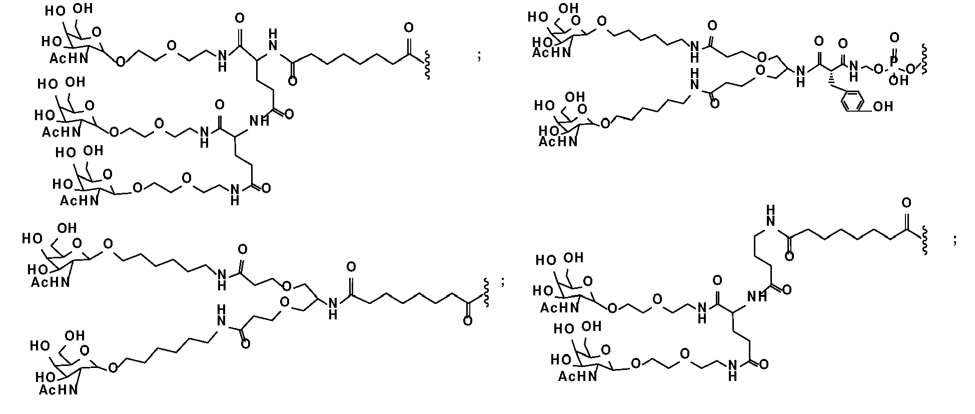 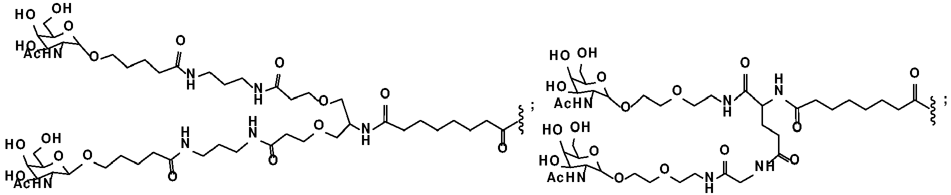 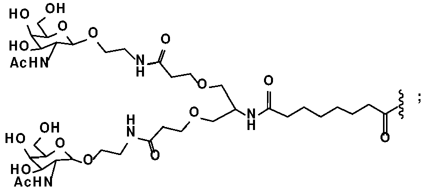 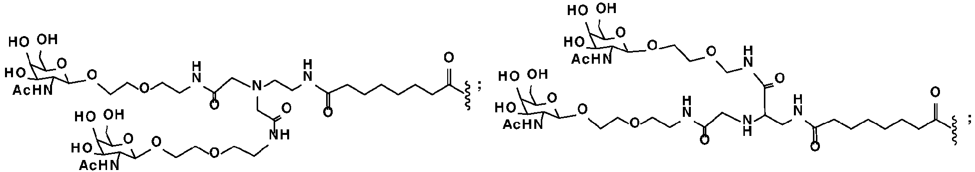 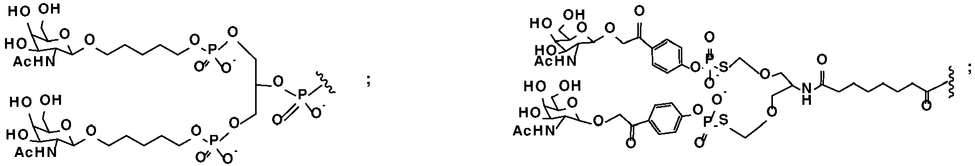 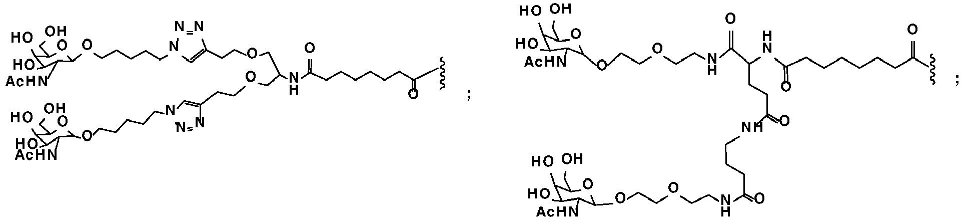 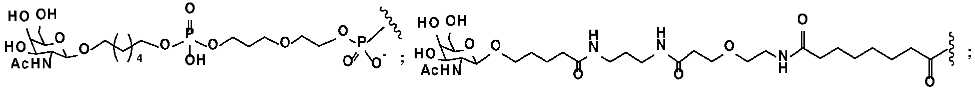 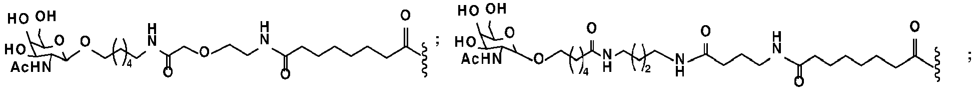 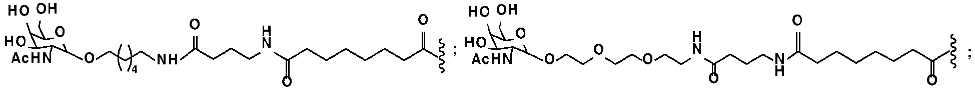 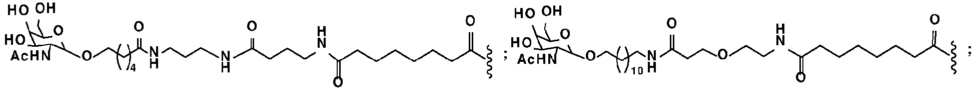 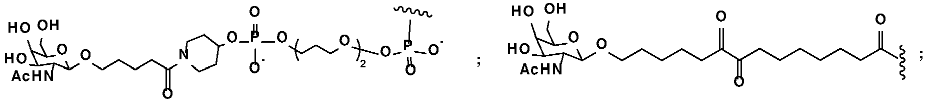 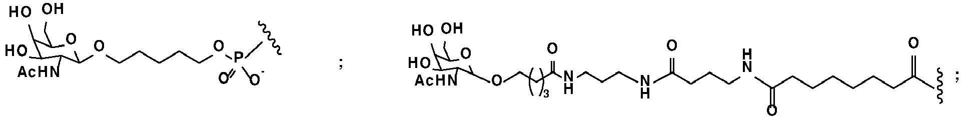 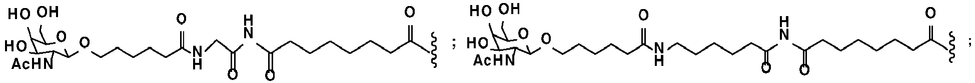 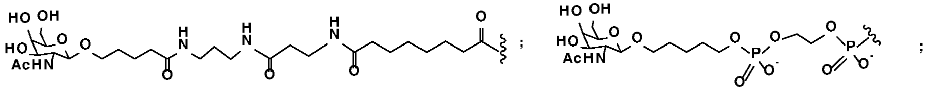 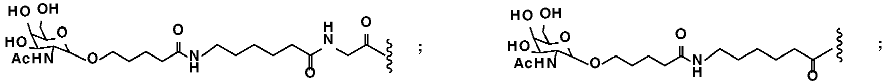 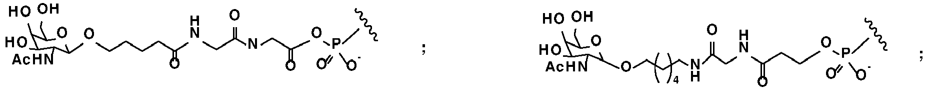 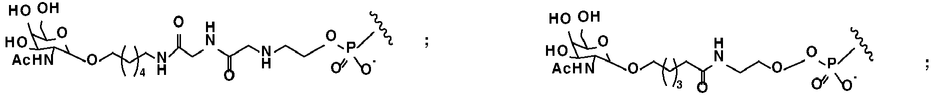 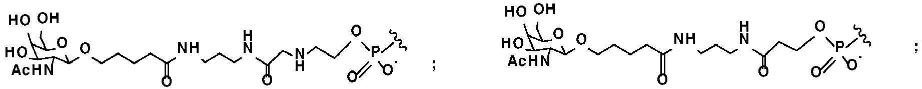 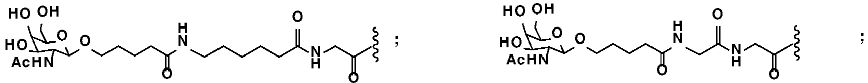 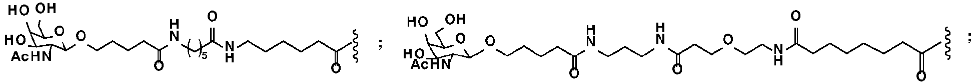 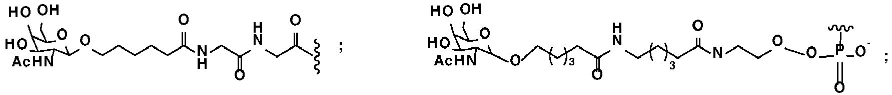 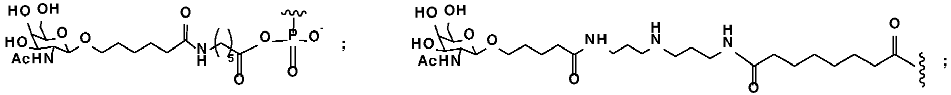 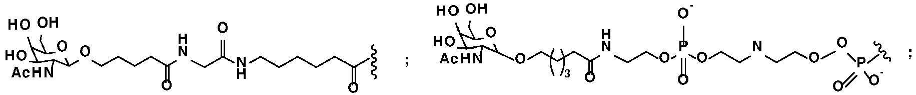 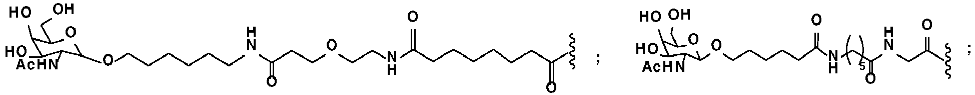 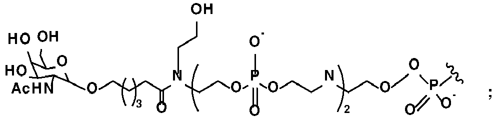 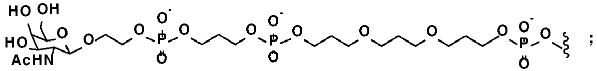 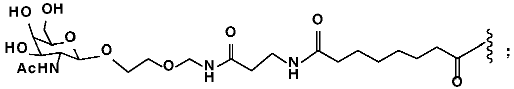 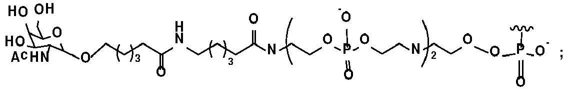 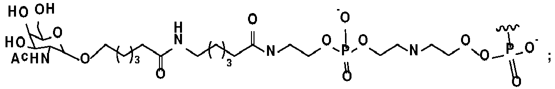 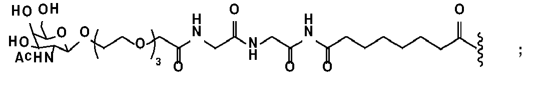 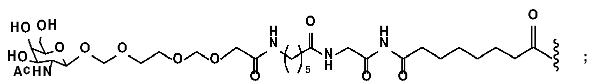 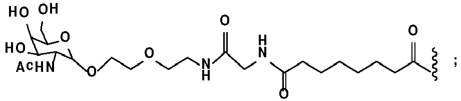 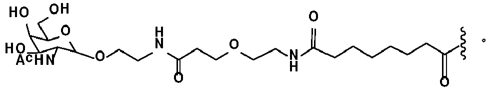
75. The RNAi agent or pharmaceutically acceptable salt thereof for inhibiting AGT gene expression according to any one of embodiments 61-74, wherein the X, L, D, and B are the same or different within the respective 5'MVIP and 3'MVIP or between the 5'MVIP and 3'MVIP.
76. The RNAi agent or pharmaceutically acceptable salt thereof for inhibiting AGT gene expression according to any one of embodiments 61-75, wherein the 5'MVIP is selected from any one of 5'MVIP01 to 5'MVIP22 in Table 10.
77. The RNAi agent or pharmaceutically acceptable salt thereof for inhibiting AGT gene expression according to any one of embodiments 61-76, wherein the 3'MVIP is selected from any one of 3'MVIP01 to 3'MVIP27 in Table 11.
78. The RNAi agent or pharmaceutically acceptable salt thereof for inhibiting AGT gene expression according to any one of embodiments 61-77, wherein the combination of 5'MVIP of the sense strand and 3'MVIP of the antisense strand is 5'MVIP01/3'MVIP01, 5'MVIP01/3'MVIP17 or 5'MVIP09/3'MVIP09; or the combination of the 5'MVIP of the sense strand and the 3'MVIP of the sense strand is 5'MVIP01/3'MVIP09 or 5'MVIP09/3'MVIP01.
79. The RNAi agent or pharmaceutically acceptable salt thereof for inhibiting AGT gene expression according to any one of embodiments 33-78, wherein the antisense strand includes any one or more of AS 131, AS 137, AS 138, AS 140, AS 141, AS 142, AS 147 and AS 148.
80. The RNAi agent or pharmaceutically acceptable salt thereof for inhibiting AGT gene expression according to one of embodiments 33-79, wherein the sense strand includes any one or more of S131, S137, S138, S140, S141, S142, S147 and S148.
81. The RNAi agent or pharmaceutically acceptable salt thereof for inhibiting AGT gene expression according to any one of embodiments 33-80, wherein the RNAi agent or pharmaceutically acceptable salt thereof is one or more of Kylo-09-DS113, Kylo-09-DS119, Kylo-09-DS120, Kylo-09-DS 122, Kylo-09-DS 123, Kylo-09-DS 124, Kylo-09-DS 129 and Kylo-09-DS 130 in Table 15.
82. The RNAi agent or pharmaceutically acceptable salt thereof for inhibiting AGT gene expression according to any one of embodiments 33-81, wherein the antisense strand is selected from any one or more of AS 140, AS207- AS266.
83. The RNAi agent or pharmaceutically acceptable salt thereof for inhibiting AGT gene expression according to any one of embodiments 33-82, wherein the sense strand is selected from any one or more of S 140, S207- S264.
84. The RNAi agent or pharmaceutically acceptable salt thereof for inhibiting AGT gene expression according to any one of embodiments 33-83, wherein the antisense strand is selected from any one or more of AS140, AS207SEQ ID NO: 413- AS266, and the sense strand is selected from any one or more of S140, S207- S264.
85. The RNAi agent or pharmaceutically acceptable salt thereof for inhibiting AGT gene expression according to any one of embodiments 33-84 including any one or more of Kylo-09-DS 122, Kylo-09-DS131, Kylo-09-DS141, Kylo-09-DS 142 and Kylo-09-DS 147 in Table 18.
86. A cell comprising RNAi agent or pharmaceutically acceptable salt thereof according to any one of embodiments 1-32, or RNAi agent or pharmaceutically acceptable salt thereof for inhibiting AGT gene expression according to any one of embodiments 33-85.
87. A pharmaceutical composition comprising an RNAi agent or a pharmaceutically acceptable salt thereof according to any one of embodiments 1-32, or an RNAi agent or a pharmaceutically acceptable salt thereof for inhibiting AGT gene expression according to any one of embodiments 33-85, and optionally a pharmaceutically acceptable excipient, vehicle, and / or diluent.
88. The pharmaceutical composition according to embodiment 87, further comprising a delivery vehicle.
89. The pharmaceutical composition according to embodiment 88, wherein the delivery vehicle comprises a liposome.
90. The pharmaceutical composition according to embodiment 89, wherein the delivery vehicle comprises a nanoliposome.
91. A method for reducing AGT mRNA or protein expression in a cell or tissue including contacting the cell or tissue with an effective amount of the RNAi agent or pharmaceutically acceptable salt thereof according to any one of embodiments 1-32, the RNAi agent or pharmaceutically acceptable salt thereof for inhibiting AGT gene expression according to any one of embodiments 33-85, and / or the pharmaceutical composition according to any one of embodiments 87-90.
92. The method according to embodiment 91, wherein the cell is a hepatocyte.
93. The method according to embodiment 91, wherein the tissue is liver tissue.
94. The method according to embodiment 91, wherein the cell and tissue are ex vivo.
95. The method according to embodiment 91, wherein the cell and tissue are within a subject.
96. Use of the RNAi agent or pharmaceutically acceptable salt thereof according to any one of embodiments 1-32, the RNAi agent or pharmaceutically acceptable salt thereof for inhibiting AGT gene expression according to any one of embodiments 33-85, or the pharmaceutical composition according to any one of embodiments 87-90 in the preparation of a drug, which is used to prevent and / or treat a disease or condition or reduce the risk of a disease or condition.
97. The use according to embodiment 96, wherein the disease or condition includes a disease or condition related to AGT.
98. The use according to embodiment 96, wherein the disease or condition is selected from: hypertension, high blood pressure, borderline hypertension, primary hypertension, secondary hypertension, isolated systolic or diastolic hypertension, pregnancy related hypertension, diabetic hypertension, treatment-resistant hypertension, refractory hypertension, paroxysmal hypertension, renovascular hypertension, Goldblatt's hypertension, hypertension associated with low plasma renin activity or plasma renin concentration, ocular hypertension, glaucoma, pulmonary hypertension, portal hypertension, systemic venous hypertension, systolic hypertension, unstable hypertension; hypertensive heart disease, hypertensive nephropathy, atherosclerosis, arteriosclerosis, angiopathy, diabetic nephropathy, diabetic retinopathy, chronic heart failure, cardiomyopathy, diabetic cardiomyopathy, glomerulosclerosis, aortic coarctation, aortic aneurysm, ventricular fibrosis, heart failure, myocardial infarction, angina, stroke, renal disease, renal failure, systemic sclerosis, intrauterine growth retardation (IUGR), fetal growth restriction, obesity, hepatic steatosis / fatty liver, non-alcoholic steatohepatitis (NASH), non-alcoholic fatty liver disease (NAFLD); glucose intolerance, type 2 diabetes mellitus (non-insulin dependent diabetes mellitus), and metabolic syndrome.
99. A method for preventing and / or treating a disease or condition including administering an effective amount of the RNAi agent or pharmaceutically acceptable salt thereof according to any one of embodiments 1-32, the RNAi agent or pharmaceutically acceptable salt thereof for inhibiting AGT gene expression according to any one of embodiments 33-85, and / or the pharmaceutical composition according to any one of embodiments 87-90 to a subject in need thereof.
100. The method according to embodiment 99, wherein the administration includes applying by subcutaneous, intravenous, oral, rectal or intraperitoneal route to the subject.
101. The method according to embodiment 99, wherein the RNAi agent, the RNAi agent inhibiting AGT gene expression or the pharmaceutical composition is applied to the subject at a dosage of about 0.01mg/kg to about 50mg/kg.
102. The method according to embodiment 99, which further includes determining the AGT level in the sample from the subject.
103. The method according to embodiment 99, wherein the AGT level in the subject sample is the AGT protein level in blood sample, serum sample or urine sample.
104. The method according to embodiment 99, which further includes administering additional therapeutic agent for treating hypertension to the subject.
105. The method according to embodiment 104, wherein the additional therapeutic agent is selected from diuretics, angiotensin converting enzyme (ACE) inhibitors, angiotensin II receptor antagonists, β- blockers, vasodilators, calcium channel blockers, aldosterone antagonists, α2-agonists, renin inhibitors, α-blockers, peripheral-acting adrenergic agents, selective D1 receptor partial agonists, nonselective α-adrenergic antagonists, synthetic steroidal anti-mineralocorticoid agents, angiotensin receptor-neprilysin inhibitors (ARNi), sacubitril / valsartan; or endothelin receptor antagonist (ERA), sitasentan, ambrisentan, altrasentan, BQ-123, zipotentan, bosentan, macitentan, and tizosentan; a combination of any of the above therapeutic agents; and hypertension therapeutic agents formulated into pharmaceutical combinations.
106. The method according to embodiment 104, wherein the additional therapeutic agent includes an angiotensin II receptor antagonist.
107. The method according to embodiment 106, wherein the angiotensin II receptor antagonist is selected from losartan, valsartan, olmesartan, eprosartan and azilsartan.
108. A kit comprising an RNAi agent or a pharmaceutically acceptable salt thereof according to any one of embodiments 1-32, an RNAi agent or a pharmaceutically acceptable salt thereof for inhibiting AGT gene expression according to any one of embodiments 33-85, or a pharmaceutical composition according to any one of embodiments 87-90.

Without being bound by any theory, the examples in the following are only for the purpose of explaining the RNAi agent, preparation method and use of the invention, and are not used to limit the scope of the invention of the application.

### Examples

### Description:

The name of DMSO is dimethyl sulfoxide;
The name of DMF is N, N-dimethylformamide;
The name of HOBt is 1-hydroxybenzotriazole;
The name of HBTU is O-benzotriazole-tetramethylurea hexafluorophosphate;
The name of DIPEA (DIEA) is N, N-diisopropylethylamine;
The name of DCM is dichloromethane;
The name of DMAP is 4-dimethylaminopyridine;
The name of DMT-CL is 4,4'- dimethoxytriphenylchloromethane;
The name of MEOH is methanol;
The name of TBTU is O-benzotriazole-N, N, N', N'- tetramethylurea tetrafluoroboric acid;
The name of 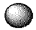 is solid-phase support, such as macroporous aminomethyl resin (resin).

### Example 1: solid-phase phosphoramidite method for siRNA synthesis

The sense strand in Table 1 and the antisense strand in Table 2 were synthesized according to the standard solid-phase phosphoramidite method, the sense strand and the corresponding antisense strand were annealed to obtain siRNA.

The basic steps of the solid-phase phosphoramidite method include:
1) Deprotection: removing theSolid Support hydroxyl protecting group (DMTr) of starting monomer;
2) Conjugation: adding the first phosphoramidite monomer to conjugate in the 3' to 5' direction;
3) Oxidation: oxidizing the resulting nucleoside phosphite to a more stable nucleoside phosphate (that is, trivalent phosphorus is oxidized to pentavalent phosphorus);
4) Blocking: adding cap to the unreacted 5 '-OH of the nucleotide sequence in the previous step to prevent further reaction; repeating the above steps until the last phosphoramidite monomer is introduced; then cleaving the ester bond between Solid Support and the starting monomer with methylamine aqueous solution and ammonia water, and removing the protective groups on each base and phosphate on the resulting nucleotide sequence; after separation and purification by HPLC, filtering to sterilize and lyophilizing to obtain the corresponding sense strand or antisense strand.

### Description of annealing process:

The sense strand and antisense strand lyophilized powders were respectively redissolved and mixed in equal molar ratio, and an appropriate amount of water for injection was added, and an appropriate amount of TRIS buffer solution was added. Shaked gently for about 1 ~ 2min to mix the solution evenly. Heated a water bath to 92 °C - 95 °C and placed the above reaction solution in the water bath to heat for 3min ~ 5min, shaked gently to heat the solution evenly. Let it to cool naturally to room temperature. Colorless or yellowish transparent liquid was obtained. Took samples for testing to measure concentration. The RNAi agent described in this invention was produced by obtaining sense and antisense strands through the solid-phase phosphoramidite method respectively, and the sense strand and the corresponding antisense strand were annealed to obtain the final product.

### Example 2 Experiment of RNAi agent to inhibit AGT gene expression in vitro

Took the RNAi agent (Kylo-09-DS01~ Kylo-09-DS18 in Table 3) prepared by the method described in example 1. The aqueous solution of RNAi agent and organic solution of DOTMA were mixed to form water-insoluble precipitate, which was dissolved in chloroform after separation and drying, and further mixed with chloroform solution of other lipid, including M10C1 and PEG600-cholesterol. The mixture was evaporated and dried overnight by vacuum centrifugation to obtain nanolipid-encapsulated RNAi agent, in which the weight ratios of DOTMA, M10C1 and PEG600-cholesterol to RNAi agent were 1-1.6, 1.5-2.5 and 2.5-3.5.

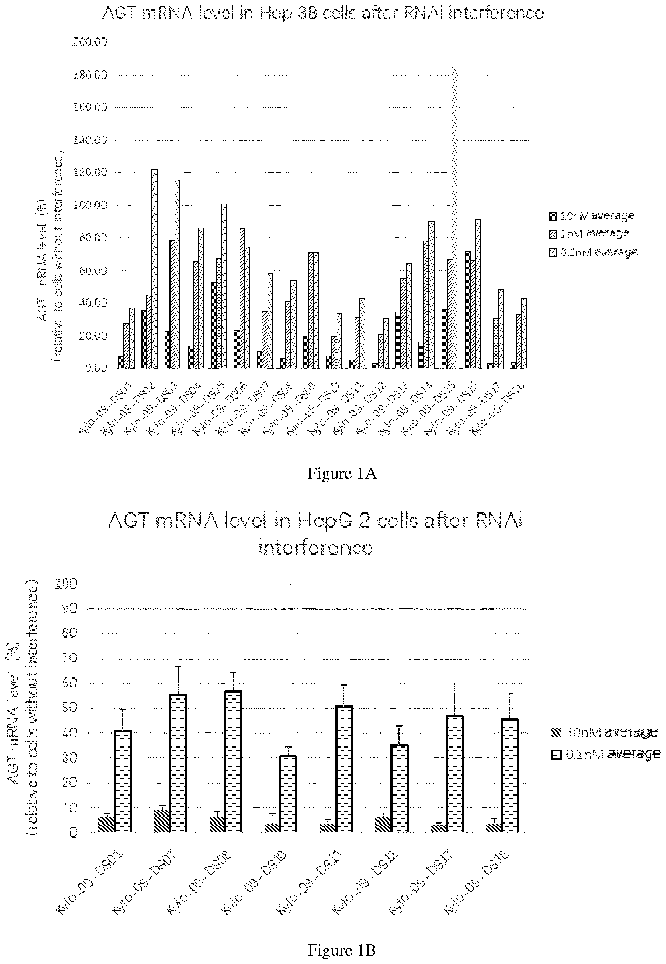

DMEM containing 10% fetal bovine serum was used to prepare the nanolipid-encapsulated RNAi agent (RNAi agent in Table 3:Kylo-09-DS01~Kylo-09-DS18) sample solution with corresponding concentration. Hep3B cells were seeded at a density of 10⁵ cells. After incubation for 24h in DMEM medium supplemented with 10% fetal bovine serum, 37°C, 5%CO₂, samples with different concentrations (10nM, 1nM, 0.1nM) were added for interference. After incubation for 72h, collected the cell samples, added 1 ml Ezol lysate to the collected cell samples and mixed well by a vortex shaker. Added 0.2 ml trichloromethane, shook vigorously for 10 s, and left at room temperature for 1 min. Centrifuged at 12000 × g for 15 min at 4 °C. Transferred the upper clear-water phase to another new RNase free centrifuge tube, and added an equal volume of 100% ethanol. Pipetted all of the sample and added it to a mini-spin centrifuge column with a 2 ml collection tube. Centrifuged at 8000 × g for 15 seconds at room temperature, and discarded the flow-through liquid. Transferred the remaining sample to a centrifuge column and repeated the previous step. Added 700 µl WB to the centrifuge column, gently closed the cover, centrifuged at 8000 × g, room temperature for 15 seconds, discarded the flow-through liquid. Repeated the previous step, and used 500 µ L WB to wash the centrifuge column twice. AGT mRNA levels were determined by QRT-PCR. Compared with the supernatant of Hep3B cells without interference, the relative percentages of AGT mRNA in the interference group were calibrated. The obtained test results were shown in Table 19 and Figure 1A.

**Table19 AGT mRNA level in Hep3B cells after RNAi interference**

| RNAi agent | mRNA levels in Hep3B cells (%) (relative to cells without interference) | | |
|---|---|---|---|
| | 10nM average | 1nM average | 0.1nM average |
| Kylo-09-DS01 | 7.1 | 27.4 | 37.1 |
| Kylo-09-DS02 | 35.5 | 45.2 | 122.1 |
| Kylo-09-DS03 | 22.6 | 78.5 | 115.4 |
| Kylo-09-DS04 | 13.8 | 65.4 | 86.1 |
| Kylo-09-DS05 | 52.7 | 67.3 | 101.0 |
| Kylo-09-DS06 | 23.1 | 85.7 | 74.3 |
| Kylo-09-DS07 | 10.0 | 35.2 | 58.1 |
| Kylo-09-DS08 | 6.2 | 40.9 | 54.5 |
| Kylo-09-DS09 | 20.0 | 71.1 | 70.8 |
| Kylo-09-DS10 | 7.4 | 19.2 | 33.4 |
| Kylo-09-DS11 | 4.9 | 31.3 | 42.5 |
| Kylo-09-DS12 | 3.0 | 20.9 | 30.3 |
| Kylo-09-DS13 | 34.5 | 55.4 | 64.4 |
| Kylo-09-DS14 | 16.2 | 78.3 | 90.5 |
| Kylo-09-DS15 | 36.1 | 67.0 | 185.1 |
| Kylo-09-DS16 | 71.9 | 66.6 | 91.3 |
| Kylo-09-DS17 | 2.9 | 30.5 | 48.0 |
| Kylo-09-DS18 | 3.6 | 32.9 | 42.5 |

The results of the experiment showed that the RNAi agents in Table 3 formed by annealing the sense strand in Table 1 and the antisense strand in Table 2, demonstrated different degrees of inhibition on the level of AGT mRNA expression in Hep3B cells at different concentrations. Among them, Kylo-09-DS01, Kylo-09-DS07, Kylo-09-DS08, Kylo-09-DS10, Kylo-09-DS11, Kylo-09-DS12, Kylo-09-DS17 and Kylo-09-DS18 showed significant dose dependence on the inhibition of AGT mRNA expression in Hep3B cells, with their corresponding sense strand sequences being SEQ ID NO.37, 43, 44, 46, 47, 48, 53 and 54 and corresponding antisense strand sequences being SEQ ID NO.55, 61, 62, 64, 65, 66, 71 and 72.

The selected Kylo-09-DS01, Kylo-09-DS07, Kylo-09-DS08, Kylo-09-DS10, Kylo-09-DS11, Kylo-09-DS12, Kylo-09-DS17 and Kylo-09-DS18 were used to observe the interference effect of RNAi agents at different concentrations (10nM, 0.1nM) on HepG2 cells by reference to the experimental operation of Hep3B cells, and the AGT mRNA levels were determined by QRT-PCR. Compared with the supernatant of HepG2 cells without interference, the relative percentages of AGT mRNA in the interference group were calibrated. The experiment results were shown in Table 20 and Figure 1B below.

**Table 20 AGT mRNA levels in HepG2 cells after RNAi interference**

| RNAi agent | mRNA levels in HepG2 cells (%) | | | |
|---|---|---|---|---|
| | (relative to cells without interference) | | | |
| | 10nM average | 10nM SD | 0.1nM average | 0.1nM SD |
| Kylo-09-DS01 | 6.9 | 0.9 | 40.9 | 8.7 |
| Kylo-09-DS07 | 9.7 | 1.1 | 55.7 | 11.2 |
| Kylo-09-DS08 | 6.8 | 2.2 | 56.8 | 7.9 |
| Kylo-09-DS10 | 3.8 | 3.9 | 31.0 | 3.5 |
| Kylo-09-DS11 | 4.1 | 1.3 | 50.9 | 8.5 |
| Kylo-09-DS12 | 6.8 | 1.8 | 35.1 | 7.9 |
| Kylo-09-DS17 | 3.4 | 0.6 | 46.8 | 13.5 |
| Kylo-09-DS18 | 4.1 | 1.5 | 45.5 | 10.8 |

### Example 3 Stability studies of modified siRNA in plasma

The RNAi agents involved in this example are selected from Table 7, and the parent chain is the sequence selected from example 2 (the sense strand sequences are SEQ ID NO.37, 43, 44, 46, 47, 48, 53 and 54 and the corresponding antisense strand sequences are SEQ ID NO.55, 61, 62, 64, 65, 66, 71 and 72). The 2' positions of nucleotides at positions 7, 12, and 14 from the 5' end of the antisense strand are fluorine and the 2' positions of the remaining nucleotides are methoxy, and the phosphate bond among at least three adjacent nucleotides at the end of the antisense strand can be thioated. The 2' positions of nucleotides at positions 5, 7, 8, and 9 from the 5' end of the sense strand are fluorine and the 2' positions of the remaining nucleotides are methoxy, and the phosphate bond among at least three adjacent nucleotides at the end of the antisense strand can be thiolated.

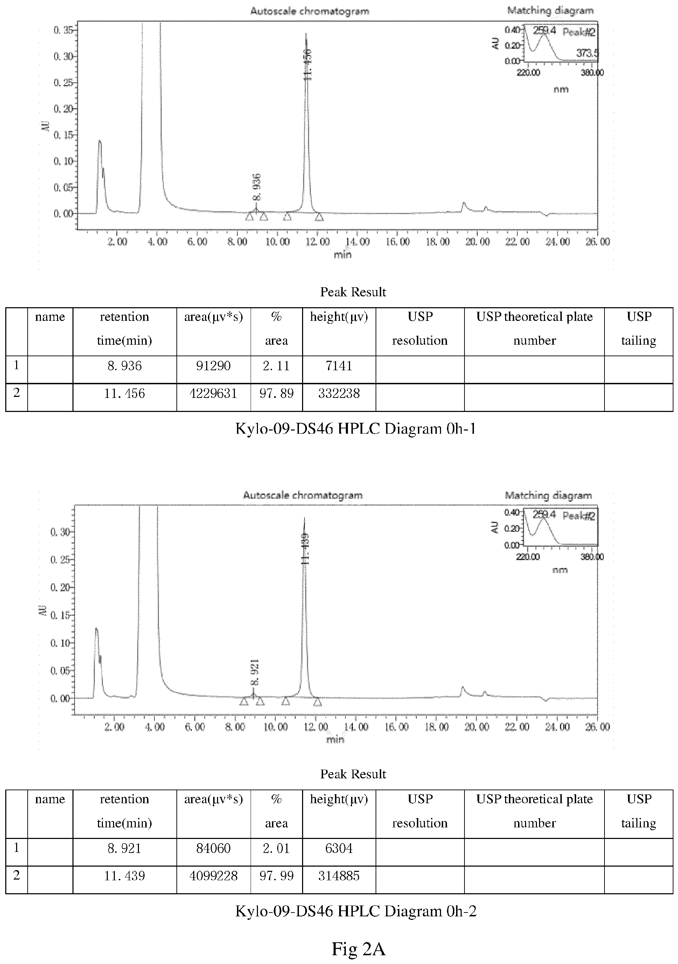

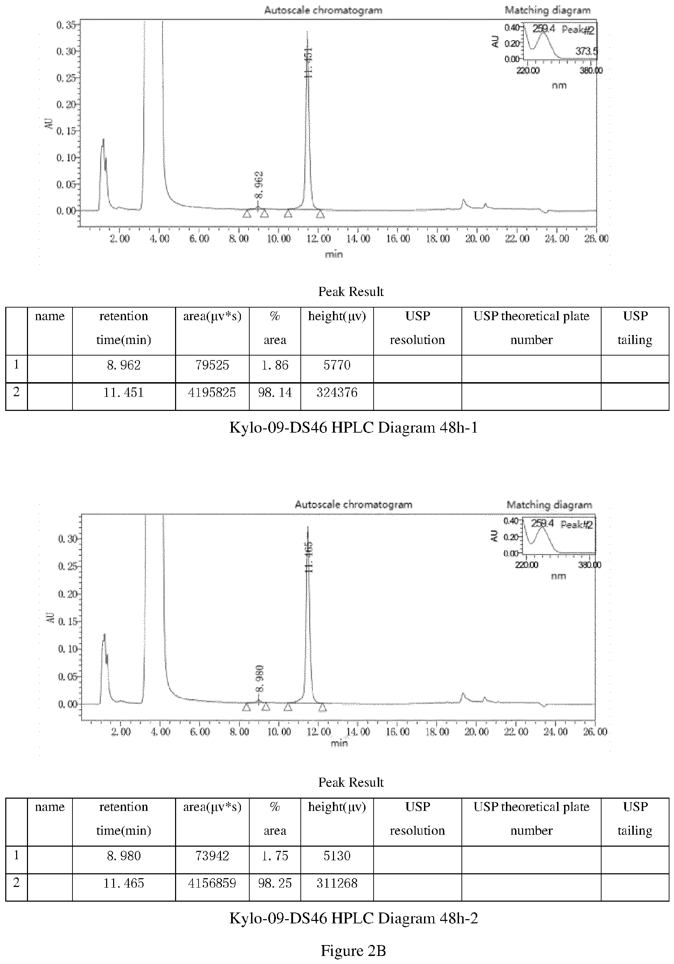

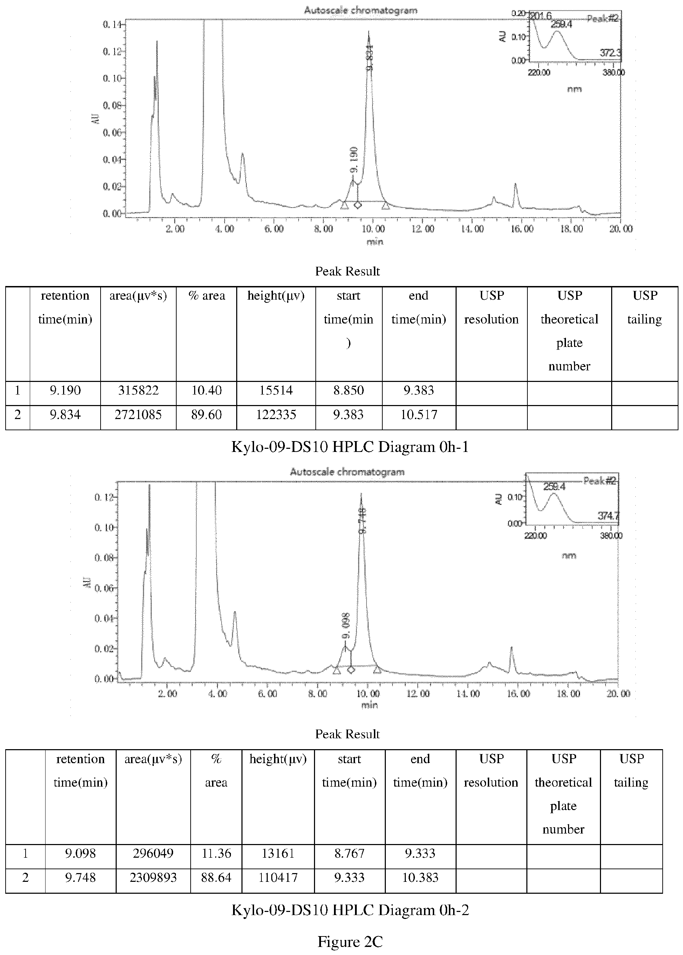

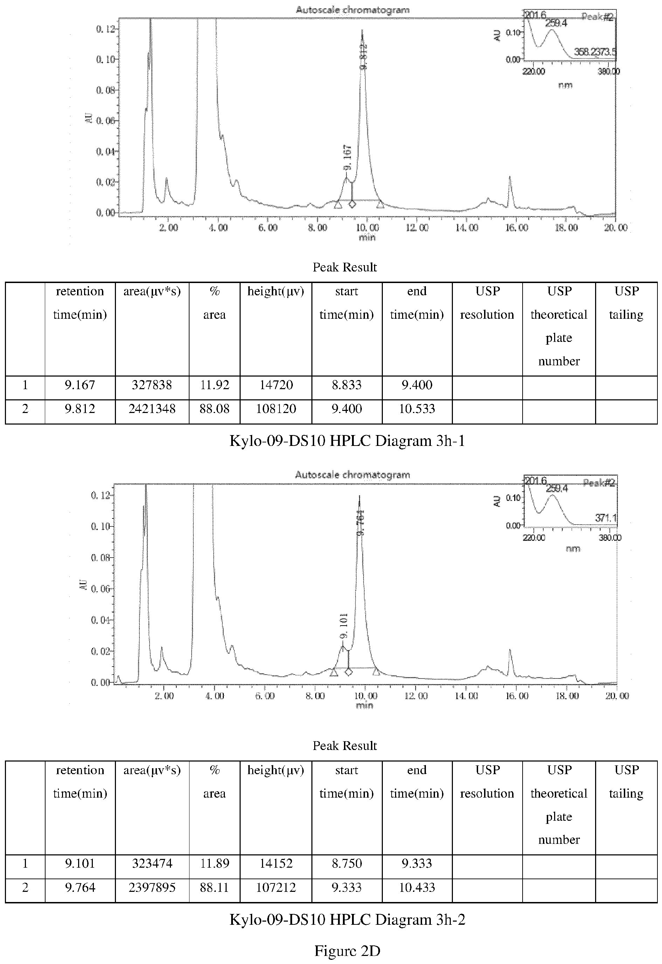

The purpose of this example is to verify that the above modification can enhance stability of RNAi agents in human serum. The experiment results are shown in Table 21 and Figure 2. Figure 2 shows the HPLC diagram of stability of RNAi Kylo-09-DS46 and its parent chain Kylo-09-DS 10 detected at different time periods.

**Table 21 The peak area ratio % of the full-length double strand relative to 0h in human serum for different durations of RNAi agents**

| RNAi agent | Peak area ratio % relative to 0h | | | | result |
|---|---|---|---|---|---|
| | 0h | 3h | 6h | - | |
| Kylo-09-DS01 | 100.0 | 85.0 | 61.6 | - | 3h stable |
| Kylo-09-DS07 | 100.0 | 84.3 | 59.9 | - | 3h unstable |
| Kylo-09-DS08 | 100.0 | 80.4 | 56.3 | - | 3h unstable |
| Kylo-09-DS10 | 100.0 | 98.8 | 65.9 | - | 3h stable |
| Kylo-09-DS11 | 100.0 | 90.5 | 63.4 | - | 3h stable |
| Kylo-09-DS12 | 100.0 | 82.3 | 52.1 | - | 3h unstable |
| Kylo-09-DS17 | 100.0 | 80.5 | 49.9 | - | 3h unstable |
| Kylo-09-DS18 | 100.0 | 81.9 | 46.6 | - | 3h unstable |

| **RNAi agent** | **0h** | **24h** | **48h** | **72h** | **result** |
|---|---|---|---|---|---|
| Kylo-09-DS37 | 100.0 | 100.6 | 84.3 | 64.8 | 24 h stable |
| Kylo-09-DS43 | 100.0 | 99.9 | 85.1 | 61.3 | 48h stable |
| Kylo-09-DS44 | 100.0 | 98.5 | 81.0 | 60.1 | 24h stable |
| Kylo-09-DS46 | 100.0 | 100.1 | 100.3 | 76.7 | 48h stable |
| Kylo-09-DS47 | 100.0 | 99.5 | 83.8 | 63.1 | 24h stable |
| Kylo-09-DS48 | 100.0 | 99.7 | 81.3 | 59.8 | 24h stable |
| Kylo-09-DS53 | 100.0 | 100.3 | 84.9 | 62.5 | 24h stable |
| Kylo-09-DS54 | 100.0 | 100.4 | 85.4 | 60.0 | 48h stable |

The above results show that, compared with the respective unmodified parent strands, the stability of RNAi agents in human serum can be effectively enhanced when the double-strand is modified in the following ways, and the enhancement effect of different sequence stability is different due to the existence of sequence specificity. The modification model is, the 2' positions of nucleotides at positions 7, 12 and 14 starting from the 5' end of the antisense strand are fluorine and the 2' positions of the remaining nucleotides are methoxy, and the phosphate bond among at least three adjacent nucleotides at the end of the antisense strand can be thiolated, and the 2' positions of nucleotides at positions 5, 7, 8 and 9 starting from the 5' end of the sense strand are fluorine and the 2' positions of the remaining nucleotides are methoxy, and the phosphate bond among at least three adjacent nucleotides at the end of the sense strand can be thiolated.

### Example 4 Synthesis of 5'MVIP and 3'MVIP compounds

When the 3'end of the sense strand or antisense strand of the RNAi agent of this application is conjugated with a carrier structure 3'MVIP, the Solid Support of 3'MVIP is used as the starting monomer for solid-phase synthesis. When the 5'-end of the sense strand or antisense strand of the RNAi agent of the application is conjugated with a carrier structure 5'MVIP, the 5'MVIP phosphoramidite monomer is used as the last monomer for solid-phase synthesis.

When the 3' end of the sense strand or antisense strand of the RNAi agent of this application is conjugated with 3'MVIP, the solid support of 3'MVIP is used as the starting monomer for solid-phase synthesis. The general formula of solid support of 3'MVIP is as follows:

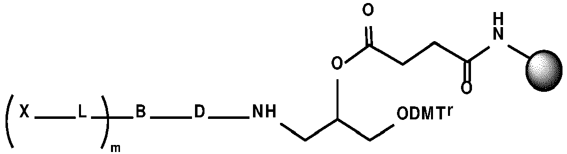

When m is 1-4, the linker B in the general formula is branched for 1 to 4 times respectively to obtain the corresponding Solid Support of 3'MVIP.

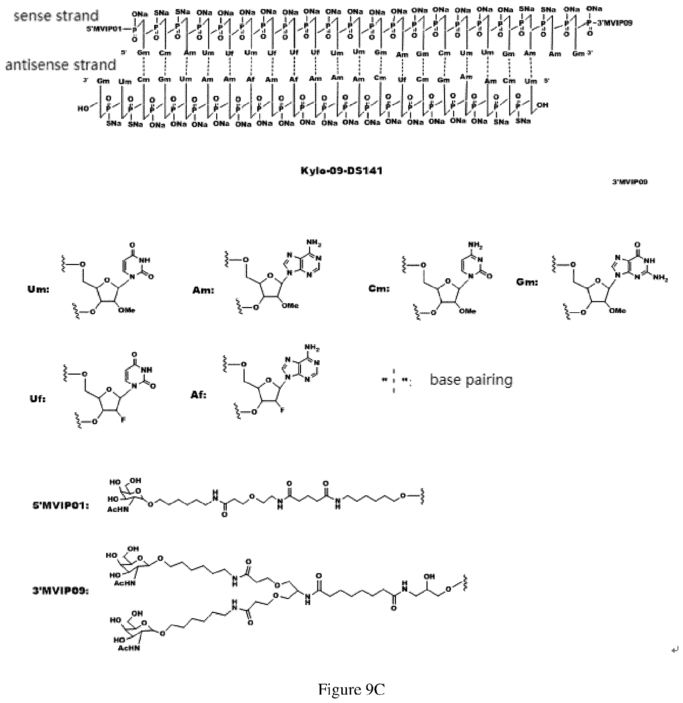

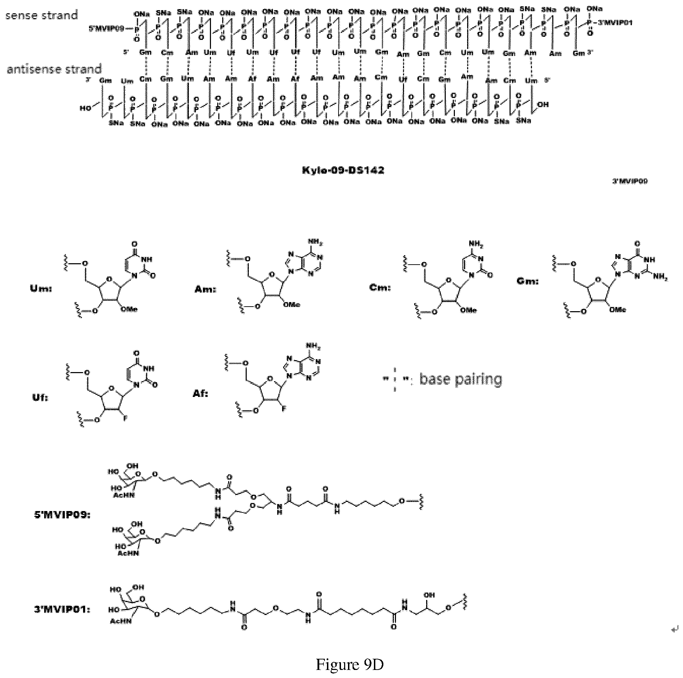

For example, when m is 1, the obtained Solid Support serves as the starting monomer for solid-phase synthesis of the sense strand of the RNAi agent Kylo-09-DS 142 (see Figure 9D for the structural formula) and the antisense strand of Kylo-09-DS 133; when m is 2, the obtained Solid Support is used as the starting monomer for solid-phase synthesis of the sense strand of the RNAi agent Kylo-09-DS 141 (see Figure 9C for the structural formula) and the antisense strand of Kylo-09-DS 122;

When m is 3, the obtained Solid Support is used as the starting monomer for solid-phase synthesis of the antisense strand of the RNAi agent Kylo-09-DS147.

When the 5'end of the sense strand or antisense strand of the RNAi agent of the application has 5'MVIP, the 5'MVIP phosphoramidite monomer is the last phosphoramidite monomer for solid-phase synthesis of the sense strand or antisense strand. The general formula of 5'MVIP phosphoramidite monomer is as follows:

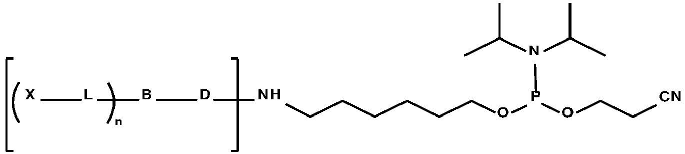

When n is 1-4, the linker B in the general formula is respectively branched for 1 to 4 times to obtain the corresponding 5'MVIP phosphoramidite monomer.

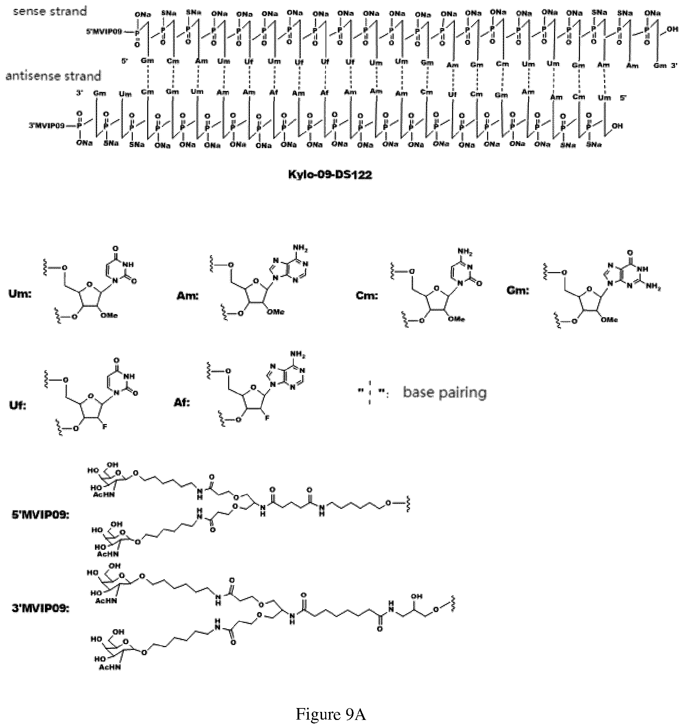

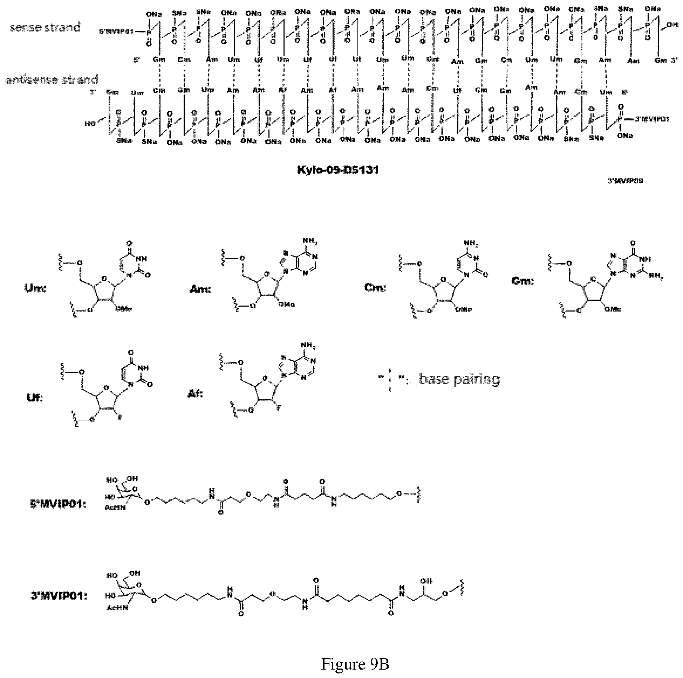

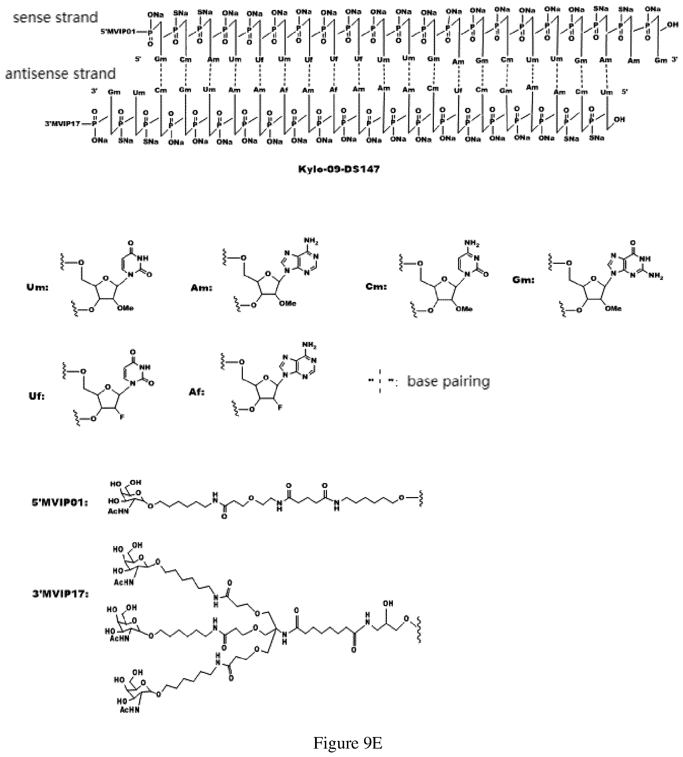

For example, when n is 1, the resulting 5'MVIP phosphoramidite monomer is used as the last monomer for solid-phase synthesis of the sense strand of RNAi agents Kylo-09-DS 141, Kylo-09-DS131 (structural formula see Figure 9B) and Kylo-09-DS147 (structural formula see Figure 9E); when n is 2, the resulting 5'MVIP phosphoramidite monomer is the last monomer for solid-phase synthesis of the sense strand of Kylo-09-DS 122 (see Figure 9A for the structural formula) and Kylo-09-DS 142.

When n equals 3, the resulting 5'MVIP phosphoramidite monomer with three ligands X can be used as the last monomer for solid-phase synthesis of sense or antisense strands.

Before the phosphoramidite solid-phase synthesis of the sense and antisense strands of these RNAi agents described in this application, the corresponding 3'MVIP Solid Support and 5'MVIP phosphoramidite monomers need to be chemically synthesized first.

This example describes the chemical synthesis process of 3'MVIP Solid Support and 5'MVIP phosphoramidite monomer of RNAi agent as follows:

### 4.1 Synthesis of Solid Support of 3'MVIP

### 4.1.1 Synthesis of Solid Support of 3'MVIP09

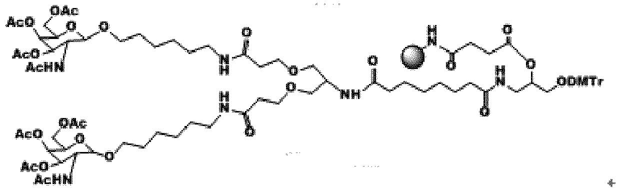

### Solid Support of 3'MVIP09

### Description of synthesis process:

### 4.1.1.1 Synthesis of ERC-01-c1

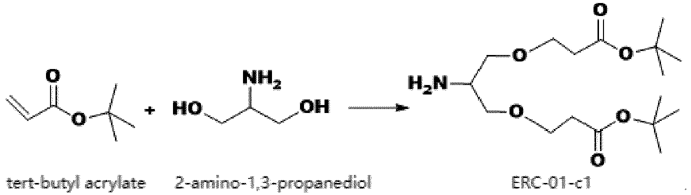

Weighed 2-amino-1,3-propanediol (5.0g, 54.9mmol) and added 50ml of DMSO, 5ml of sodium hydroxide solution (1g/ml), cooled it down to 0 °C, added tert-butyl acrylate (20ml, 137.8mol) dropwise for 2 hours, let it react at room temperature for 48h, added petroleum ether (100ml), washed twice with saturated salt water, and dried the organic layer. Passed it through a chromatography column (eluent: ethyl acetate: petroleum ether = 25%-75%), then added 0.05% triethylamine to the column, and obtained 6.2g of colorless oil.

### 4.1.1.2 Synthesis of ERC-01-c2

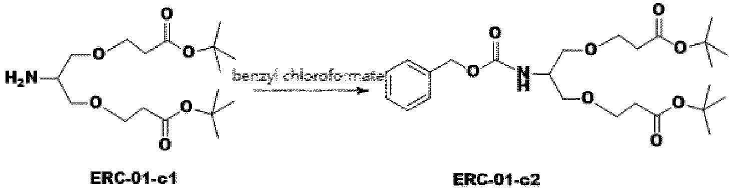

Weighed ERC-01-c2 (6.2g, 17.9mmol), added 50ml of dichloromethane, 23ml of sodium carbonate solution (25%), added benzyl chloroformate (8.2ml, 57.4mmol) dropwise at room temperature for 2 hours, let it react overnight at room temperature, washed with saturated salt water for three times, dried with anhydrous sodium sulfate, evaporated the solvent, passed it through a chromatography column (ethyl acetate: petroleum ether =5%-30%), and obtained 4.0g of oil.

### 4.1.1.3 Synthesis of ERC-01-c3

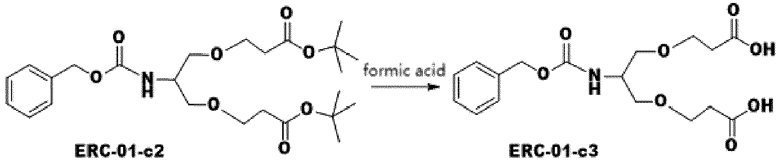

Weighed ERC-01-c2 (4.0g, 8.3mmol), added 12ml of formic acid, let it react overnight at room temperature, evaporated the solvent under reduced pressure to obtain 2.8g of product.

### 4.1.1.4 Synthesis of ERCd-01-c11

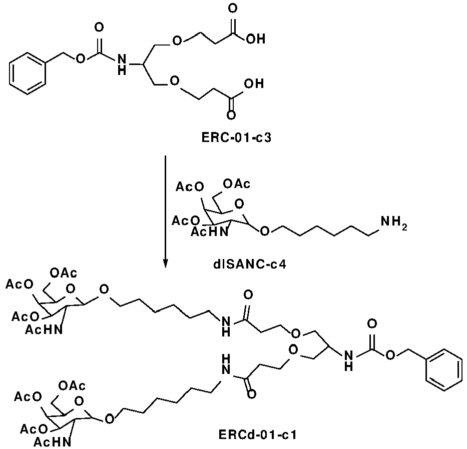

Added compounds ERC-01-c3 (1.11g, 3.0mmol) and dlSANC-c4 (3.6g, 8.04mmol) to DMF (60 ml), then added HOBt (2.24g) and HBTU (3.36g), and then slowly added DIEA (4.16ml). Stirred the reaction solution for 3 hours at room temperature. Then added water and extracted the aqueous layer with dichloromethane (2x10ml). Combined the organic layers, and then washed with saturated sodium bicarbonate (80 ml), water (2x60 ml), and saturated salt water (60ml). Dried with anhydrous sodium sulfate, evaporated to dryness under reduced pressure, and purified by silica gel column chromatography (eluent: 3-15% MeOH in DCM). Obtained light yellow solid 3.24g.

### 4.1.1.5 Synthesis of ERCd-01-c2

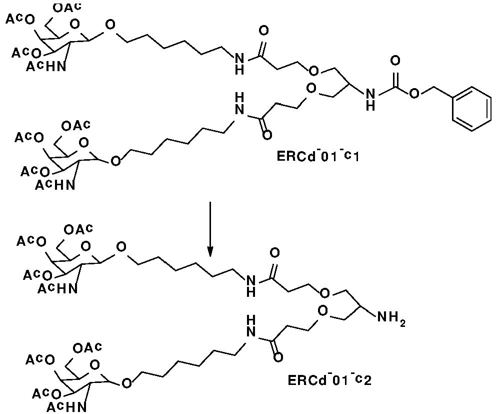

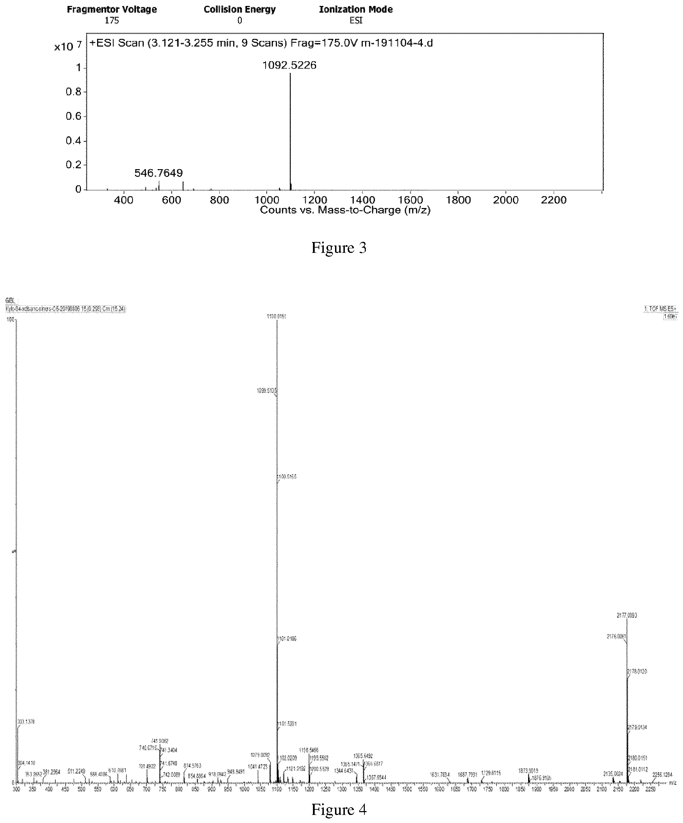

Dissolved ERCd-01-c1 (3.24g, 2.6mmol) in methanol (60ml), and added 10% palladium carbon (0.3g) and acetic acid (2.0ml). Then introduced hydrogen at atmospheric pressure to react overnight. The reaction solution was filtered with diatomite, and the filtrate was evaporated to dryness under reduced pressure to obtain 2.9g of oil ERCd-01-c2, its high-resolution mass spectrum was shown in Figure 3.

### 4.1.1.6 Synthesis of 3'MVIP09-c1

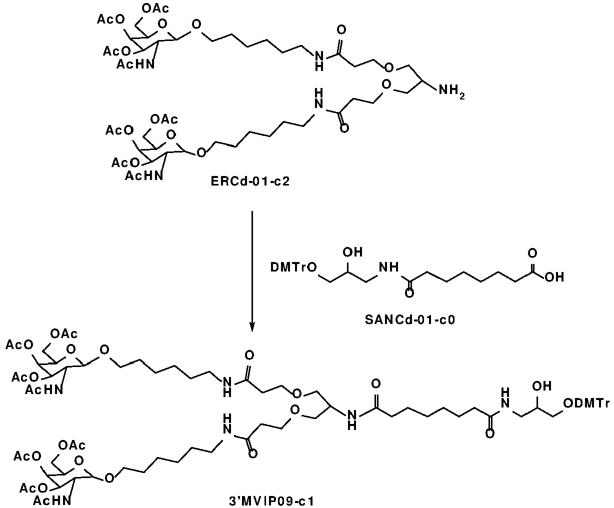

Successively added SANCd-01-c0 (0.824g, 1.5mmol) and ERCd-01-c2 (1.09g, 1.0mmol) into
a vial, then added 10ml of DCM, stirred until dissolved, then successively added TBTU (0.963g) and DIPEA (0.517g), let is react overnight, added water, extracted with DCM, washed the organic phase with saturated salt water, dried, filtered and concentrated, and finally purified by a silica gel column to obtain 1.3g of product.

### 4.1.1.7 Synthesis of 3'MVIP09-c2

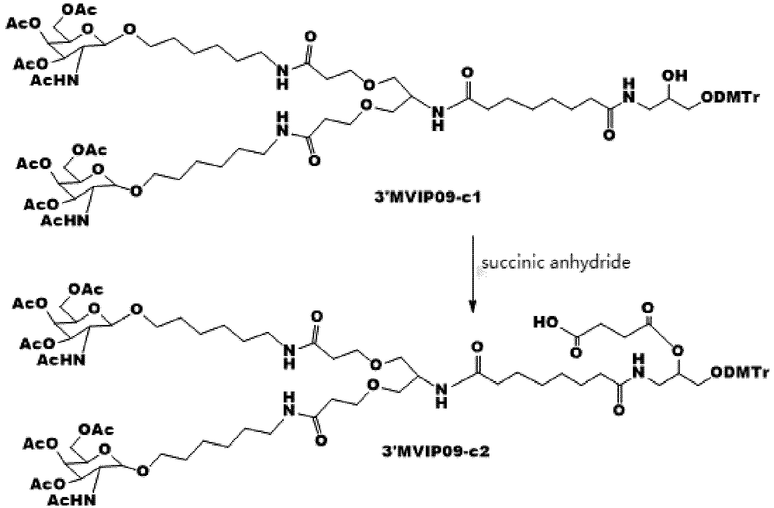

Added 3'MVIP09-c1 (1.62g, 1 µmol) and 10ml of DCM into a vial in sequence, stirred at room temperature until dissolved, then added DMAP (0.366g) and succinic anhydride (0.2g, 3 µmol) in sequence, stirred at room temperature to react. Concentrated DCM after the reaction is qualified by TLC analysis , added water, extracted with DCM, washed the organic phase with saturated salt water, dried the organic phase by anhydrous sodium sulfate, filtered and concentrated, and finally purified by a silica gel column to obtain 1.55g product.

### 4.1.1.8 Synthesis of Solid Support of 3'MVIP09

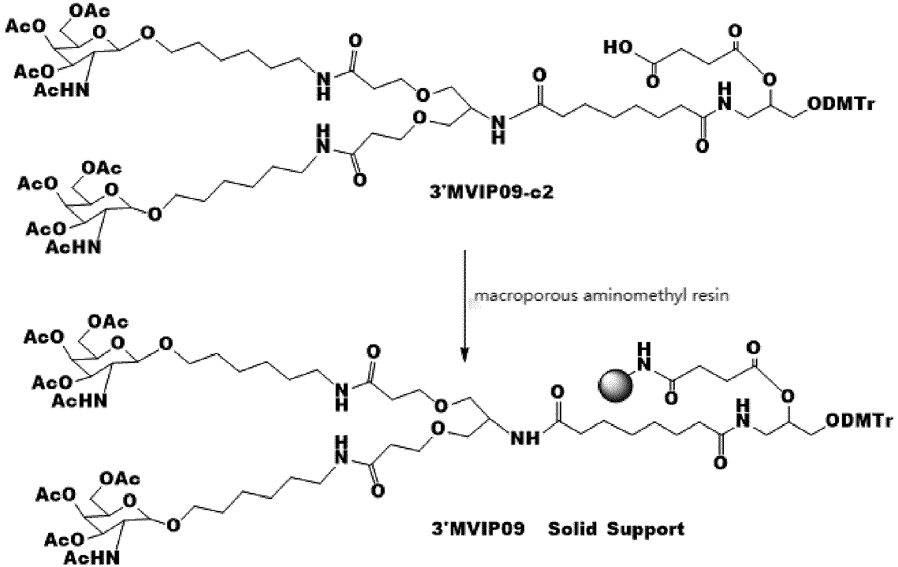

Added 3'MVIP09-c2 (0.86g, 0.5 µmol) and 10ml DMF into a vial in sequence, after dissolution, added HBTU (0.19g), DIPEA (0.194g) and macroporous aminomethyl resin (2.0g) in sequence, placed on the shaker for 24h, filtered, washed the resin with 10% methanol /DCM, and then used 25% acetic acid / pyridine for end capping, with a degree of substitution of 150 µmol/g.

### 4.1.2 Synthesis of Solid Support of 3'MVIP17

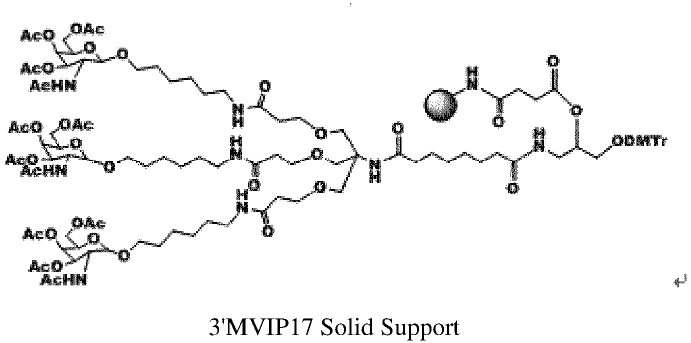

### 4.1.2.1 Synthesis of SANC-01-c1

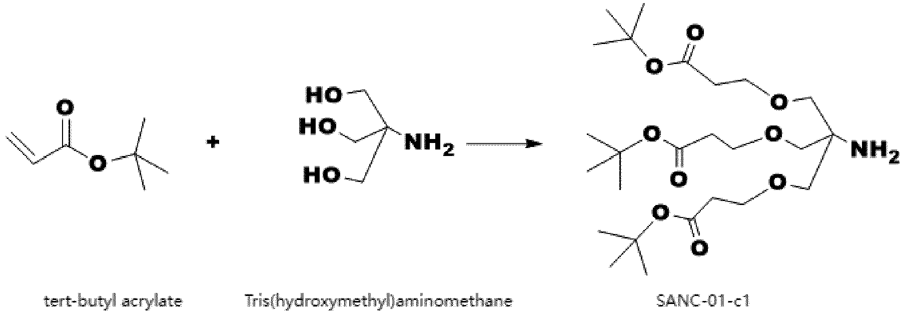

The synthesis steps are referred to 4.1.1.1 synthesis of ERC-01-c1.

### 4.1.2.2 Synthesis of SANC-01-c2

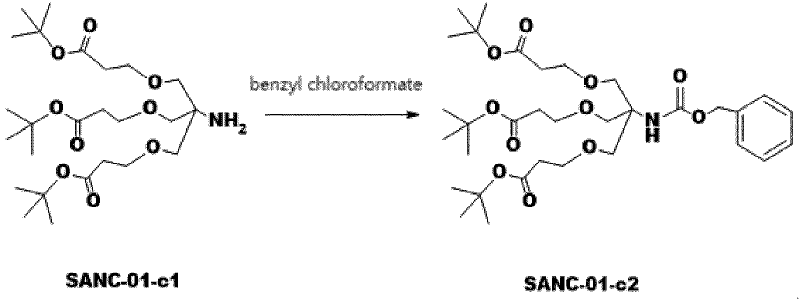

The synthesis steps are referred to 4.1.1.2 synthesis of ERC-01-c2.

### 4.1.2.3 Synthesis of SANC-01-c3

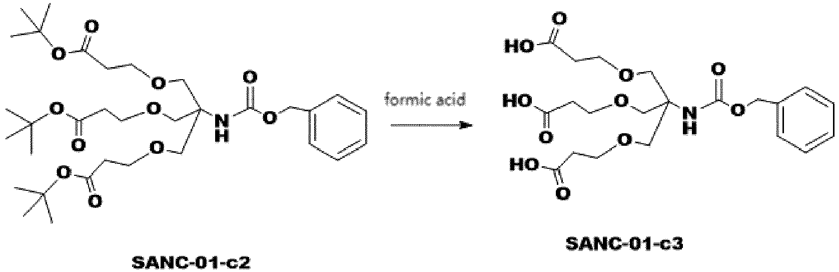

The synthesis steps are referred to 4.1.1.3 synthesis of ERC-01-c3.

### 4.1.2.4 Synthesis of SANCd-01-c1

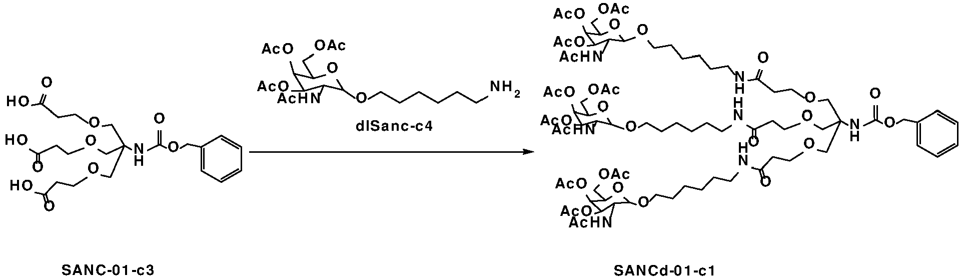

The synthesis steps are referred to 4.1.1.4 synthesis of ERCd-01-c1.

### 4.1.2.5 Synthesis of SANCd-01-c2

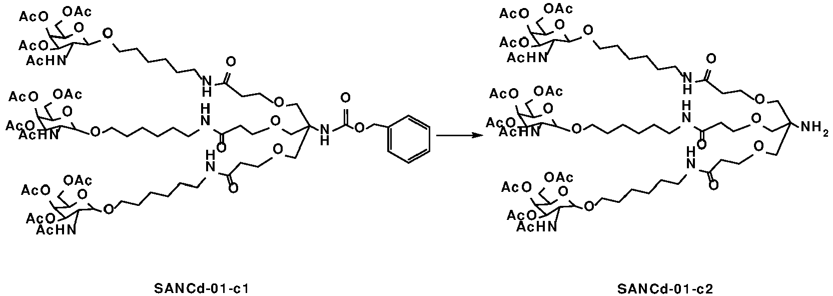

The synthesis steps are referred to 4.1.1.5 synthesis of ERCd-01-c2.

### 4.1.2.6 Synthesis of 3'MVIP17-c1

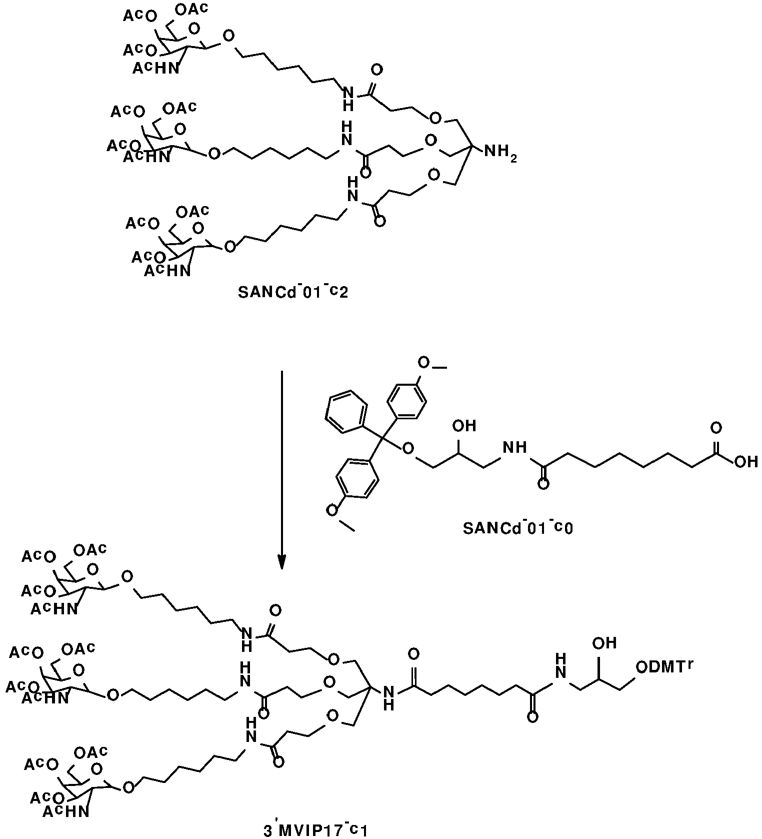

The synthesis steps are referred to 4.1.1.6 synthesis of 3'MVIP09-c1. The high-resolution mass spectrum of the synthesized 3'MVIP09-c1 is shown in Figure 4.

### 4.1.2.7 Synthesis of 3'MVIP17-c2

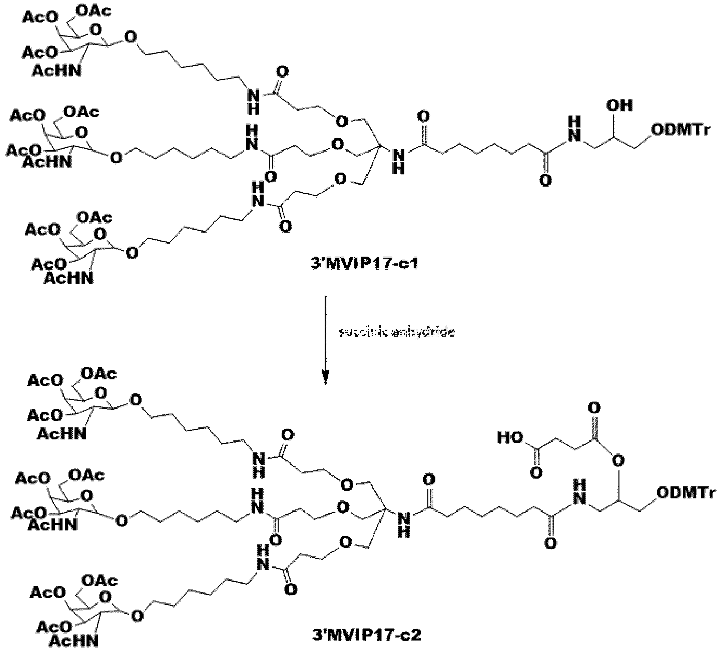

The synthesis steps are referred to 4.1.1.7 synthesis of 3'MVIP09-c2.

### 4.1.2.8 Synthesis of Solid Support of 3'MVIP17

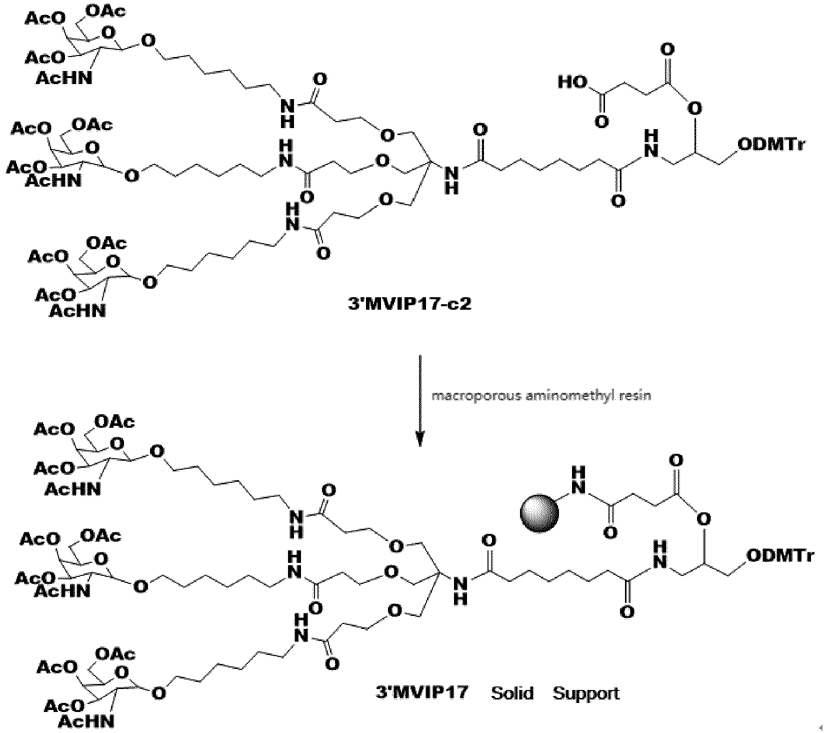

The synthesis steps are referred to 4.1.1.8 synthesis of Solid Support of 3'MVIP09. 4.1.3 Synthesis of Solid Support of 3'MVIP01:

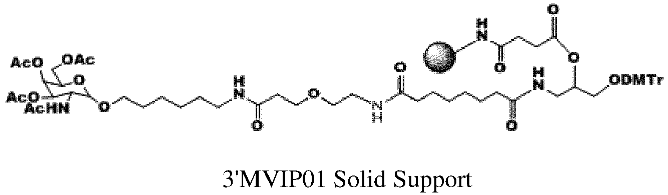

### Description of synthesis process:

### 4.1.3.1 Synthesis of 3'MVIP01-c1

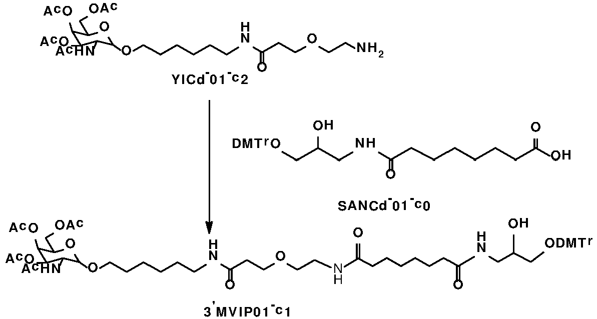

The synthesis steps are referred to 4.1.1.6 synthesis of 3'MVIP09-c1.

### 4.1.3.2 Synthesis of 3'MVIP01-c2

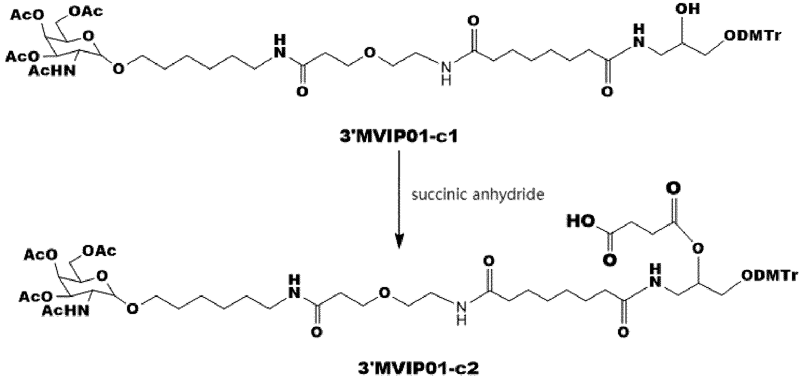

The synthesis steps are referred to 4.1.1.7 synthesis of 3'MVIP09-c2.

### 4.1.3.3 Synthesis of Solid Support of 3'MVIP01

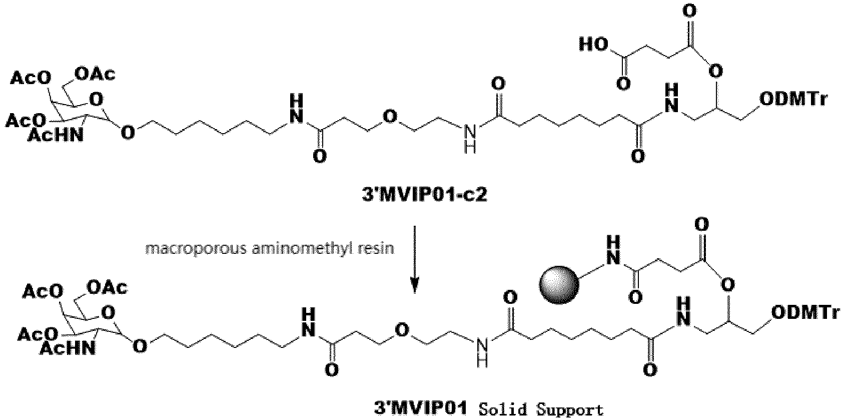

The synthesis steps are referred to 4.1.1.8 synthesis of Solid Support of 3'MVIP09.

### 4.2 Synthesis of 5 'MVIP phosphoramidite monomer

### 4.2.1 When n is 2, the synthesis of the 5'MVIP phosphoramidite monomer, which is the last monomer synthesized in the solid-phase synthesis of the Kylo-09-DS122 and Kylo-09-DS142 justice chains..

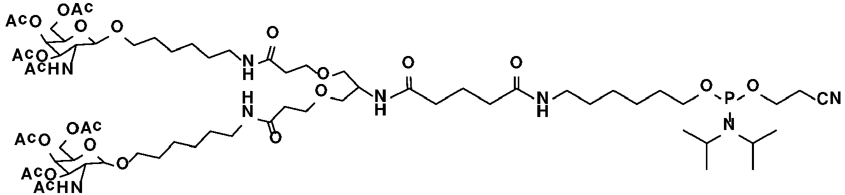

### 5'MVIP09 phosphoramidite monomer

### 4.2.1.1 Synthesis of 5 'MVIP09-ERCd-PFP-c1

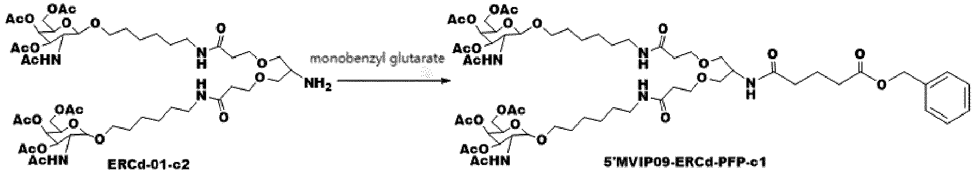

Weighed ERCd-01-c2 (2.18g, 2.0mmol) to dissolve in DMF (50ml), added monobenzyl glutarate (0.53g, 2.4mmol), DIPEA (0.78g) and TBTU (0.84g), stirred at room temperature overnight, added water to quench (50ml), extracted with DCM (30ml*3), washed with 10% citric acid (50ml*3), 50ml saturated sodium bicarbonate and 100ml pyridine, dried with anhydrous sodium sulfate, filtered, conducted rotary evaporation, and purified by a column to obtain the product 5'MVIP09-ERCd-PFP-c1 (2.15g).

### 4.2.1.2 Synthesis of 5'MVIP09-ERCd-PFP-c2

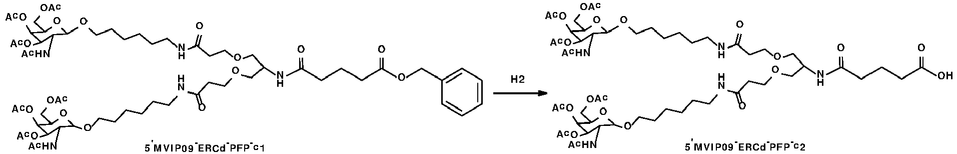

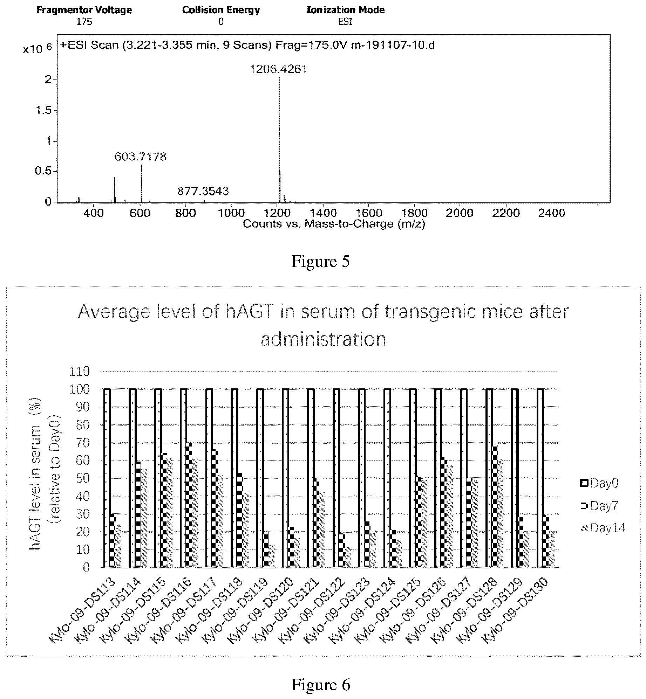

Weighed 5'MVIP09-ERCd-PFP-c1 (2.15g, 1.66mmol) and 10% palladium carbon (0.21g), added methanol (50ml), and hydrogenated overnight with stirring at room temperature, filtered palladium carbon with diatomite after the reaction, conducted rotary evaporation to obtain 5'MVIP09-ERCd-PFP-c2 crude product (1.9g), and its high-resolution mass spectrum is shown in Figure 5.

### 4.2.1.3 Synthesis of 5'MVIP09-ERCd-PFP

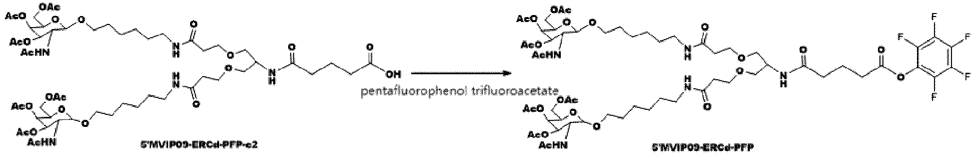

Weighed 5'MVIP09-ERCd-PFP-c2 crude product (1.9g, 1.58mmol) to dissolve in DCM (60ml), added DIPEA (1.33g), cooled, added pentafluorophenol trifluoroacetate (2.21g, 7.9mmol), let it react for 2h with stirring at room temperature, then conducted rotary evaporation, redissolved in DCM (60ml), washed with saturated sodium bicarbonate (30ml*3), 10% citric acid (30ml*1), saturated salt water (50ml* 1), dried with anhydrous sodium sulfate, filtered, conducted rotary evaporation to obtain 5'MVIP09-ERCd-PFP crude product (2.35g). After vacuum drying by pump, it was directly used for the next reaction without purification.

### 4.2.1.4 Synthesis of 5'MVIP09 phosphoramidite monomer-c1

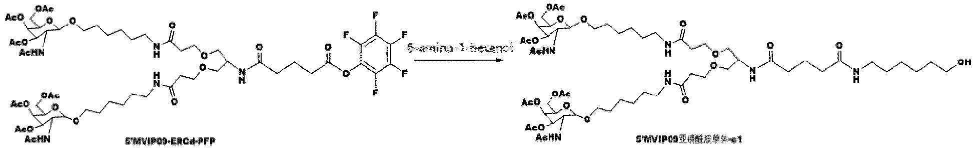

Dissolved 5'MVIP09-ERCd-PFP crude product (2.35g, 1.58mmol) in DCM (60ml), added DIPEA (0.82g, 6.32mmol), 6-amino-1-hexanol (0.37g, 3.16mmol), and let it react overnight with stirring at room temperature. Added 10% citric acid (30ml), extracted with DCM (30ml*3), washed with saturated salt water (50ml), dried with anhydrous sodium sulfate, filtered, conducted rotary evaporation, and purified by a column to obtain the product 5'MVIP09 monomer-c1 (1.73g).

### 4.2.1.5 5'MVIP09 phosphoramidite monomer

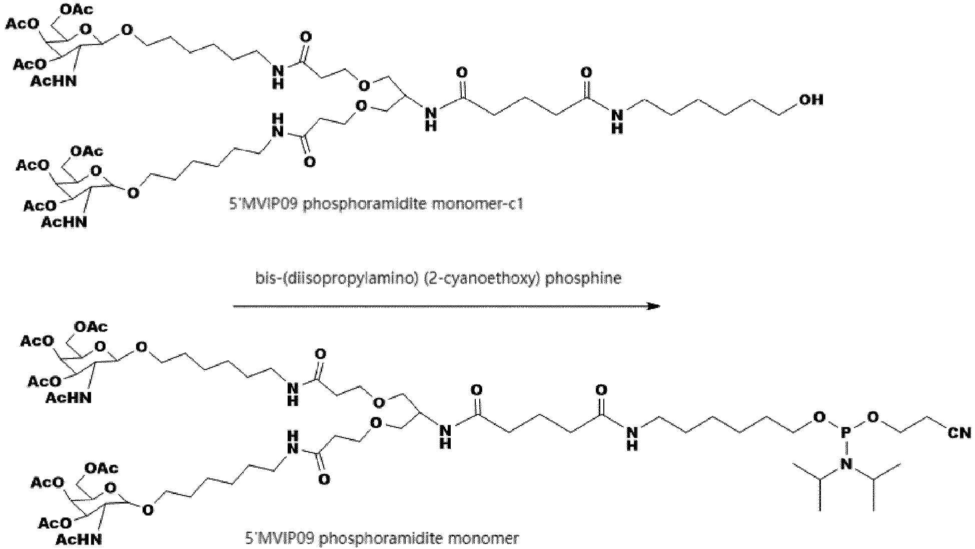

Weighed 5'MVIP09 phosphoramidite monomer-c1 (1.3g, 1.0mmol) to dissolve in acetonitrile (30ml), added diisopropylamine triazole (0.22g), added bis - (diisopropylamino) (2-cyanoethoxy) phosphine (0.36g, 1.2mmol) dropwise in an ice bath, let it react for 4h at room temperature, after the reaction is qualified by centralized control of HPLC, concentrated and purified by a column to obtain the product 5'MVIP09 monomer (1.2g).

When n is 1, the obtained 5'MVIP phosphoramidite monomer is used as the last monomer for the solid-phase synthesis of the sense strands of Kylo-09-DS 141, Kylo-09-DS 131, and Kylo-09-DS147, with the code 5'MVIP01:

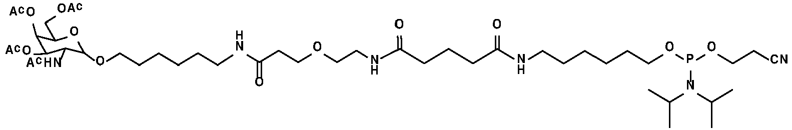

### 5'MVIP01 phosphoramidite monomer

Except for weighing YICd-01-c2 (1.12g, 2.0mmol) as the phosphoramidite monomer of 5'MVIP01, refer to 4.2.1.1.~ 4.2.1.5 for the remaining operations.

### Example 5 Synthesis of RNAi agents with different siRNA conjugated to 5' MVIP09/3' MVIP09

Description of synthesis of antisense strands in Table 13: Purged a reagent bottle with argon for at least 2 min. Added phosphoramidite monomer and acetonitrile into the reagent bottle in turn, tightened the bottle cap and shook until the solid is completely dissolved by visual inspection. Then added 3A molecular sieve and let it stand for more than 8 h for later use. Purged a reagent bottle with argon for at least 2 min. Added xanthane hydride and dried pyridine into the reagent bottle in turn, tightened the bottle cap, shook until the solid is completely dissolved by visual inspection, and stored temporarily for later use. Ensure to carry out the following operations at room temperature of 20-30 °C: weighed the 3'MVIP carrier, added it to a reagent bottle, then added acetonitrile, shook until mix evenly. Transferred the carrier to a synthesis column, and eluted the residual carrier in the reagent bottle with acetonitrile and transferred to the synthesis column. After elution, added acetonitrile to fill the synthetic column, and recorded the amount of acetonitrile used. Installed and fixed the synthetic column according to the instrument instructions.

Connected the monomer solution, CAP A, CAP B, oxidant, thioreagent, activator, decapping agent and acetonitrile prepared above to the pipeline corresponding to AKTA PILOT100, and ensure that the pipeline is inserted into the bottom of the reagent bottle.

After the synthesis method is set, the instrument is ready for all work, clicked Run to start the synthesis. Each detritylation peak area was recorded by online observation. During the synthesis process, added additional amount according to the actual amount of deprotection reagent used.

After the synthesis, purged the synthetic column with argon for ≥ 2 h, and unloaded the synthetic column according to the operating procedures. Transferred the solid supporter in the synthesis column to a reaction bottle, added methylamine aqueous solution and ammonia water, and put the reaction bottle into a shaker at 35 °C for 2-3 hours. Filtered the solution into a round bottom flask, washed the residual solid phase with 50% ethanol aqueous solution, filtered again and mixed with the previous filtrate, connected the round bottom flask with a rotary evaporator, set the water temperature at 50 °C and evaporated until there is no distillate, added ethanol into the round bottom flask, mixed welland evaporated again until there is no distillate. Repeated the operation until white powder appears at the bottom of the flask. Prepared the obtained white powder into a solution, purified by a reverse chromatography column, and sampled to detect OD260 and purity. Divided the purified antisense strand solution into vials and lyophilized for future use, and stored the product sealed in a -20°C refrigerator.

The synthesis process of sense strand in Table 14 is the same as that of antisense strand, in which the carrier loaded in the column is Universal carrier. Added DIPEA to the obtained intermediate to prepare a solution, added 5'MVIP phosphoramidite monomer, mixed well, and put the reaction bottle into a shaker at 35 °C for 2-3 hours.

Description of synthetic annealing process of RNAi agents in Table 15:
Took an antisense strand in Table 13, took a sense strand in Table 14 that is base paired with the antisense strand in Table 13, mixed them in the reaction bottle in an equimolar ratio of 1:1. Turned off the power of the water bath after 5 minutes of water bath at 95 °C, let it naturally cool down to below 40 °C. Added 3M sodium acetate aqueous solution to the solution containing double strands, mixed well, then added an appropriate volume of absolute ethanol, mixed well, and put the reaction solution into a -20 °C refrigerator for 45min. Set the high-speed freezing centrifuge to pre-cooling at 4 °C , put in the solution containing double strands after the temperature is reached, and started the centrifuge. Took out the centrifuged solution containing double strands, removed the supernatant, added ultrapure water to completely dissolve the solid, and took samples to detect OD260 and purity. Divided the purified solution containing double strands into vials and lyophilized for future use, and stored the product sealed in a -20 °C refrigerator.

### Example 6 Activity studies of RNAi agents with different siRNA conjugated to 5' MVIP09/3' MVIP09

Selected female AGT transgenic mice of appropriate age for evaluation experiments. The samples were the RNAi agents Kylo-09-DS113~Kylo-09-DS130 in Table 15. Administered 3mg/kg by subcutaneous injection on Day0; the dosing volumes were 100-200 µL. Collected blood on days 0, 7, 14, 21, 28 and 35 after administration, isolated serum and stored at -80 °C. The levels of hAGT in serum were determined by ELISA method. The test results are shown in Table 22 and Figure 6.

**Table 22 Average levels of hAGT in serum of transgenic mice after administration**

| RNAi agent | Average levels of hAGT in serum (%) (relative to day 0) | | |
|---|---|---|---|
| | Day0 | Day7 | Day14 |
| Kylo-09-DS113 | 100.0 | 30.2 | 24.0 |
| Kylo-09-DS114 | 100.0 | 59.3 | 55.1 |
| Kylo-09-DS115 | 100.0 | 64.2 | 61.1 |
| Kylo-09-DS116 | 100.0 | 69.8 | 62.2 |
| Kylo-09-DS117 | 100.0 | 66.4 | 51.7 |
| Kylo-09-DS118 | 100.0 | 52.8 | 41.9 |
| Kylo-09-DS119 | 100.0 | 19.9 | 12.3 |
| Kylo-09-DS120 | 100.0 | 22.6 | 16.3 |
| Kylo-09-DS121 | 100.0 | 49.8 | 42.3 |
| Kylo-09-DS122 | 100.0 | 19.1 | 11.5 |
| Kylo-09-DS123 | 100.0 | 25.7 | 20.6 |
| Kylo-09-DS124 | 100.0 | 21.3 | 15.1 |
| Kylo-09-DS125 | 100.0 | 51.4 | 48.9 |
| Kylo-09-DS126 | 100.0 | 62.1 | 57.1 |
| Kylo-09-DS127 | 100.0 | 49.8 | 48.6 |
| Kylo-09-DS128 | 100.0 | 68.4 | 60.5 |
| Kylo-09-DS129 | 100.0 | 28.6 | 20.0 |
| Kylo-09-DS130 | 100.0 | 29.3 | 19.8 |

The results showed that the RNAi agents Kylo-09-DS 113, 119, 120, 122, 123, 124, 129, and 130 obtained from the 5'MVIP09/3'MVIP09 conjugated sequences (selected from Example 2,the sense strand sequences of the above sequences were SEQ ID NO.37, 43, 44, 46, 47, 48, 53, and 54 and the corresponding antisense strand sequences were SEQ ID NO.55, 61, 62, 64, 65, 66, 71, and 72) still had significant in vivo activity and has good sustainability. This example verified that the 5'MVIP09/3'MVIP09 carrier structure could achieve safe delivery of siRNA and has significant effect.

### Example 7 Studies on the effect of conjugating different structures of 5' MVIP and 3' MVIP with the same siRNA on the activity of RNAi agents

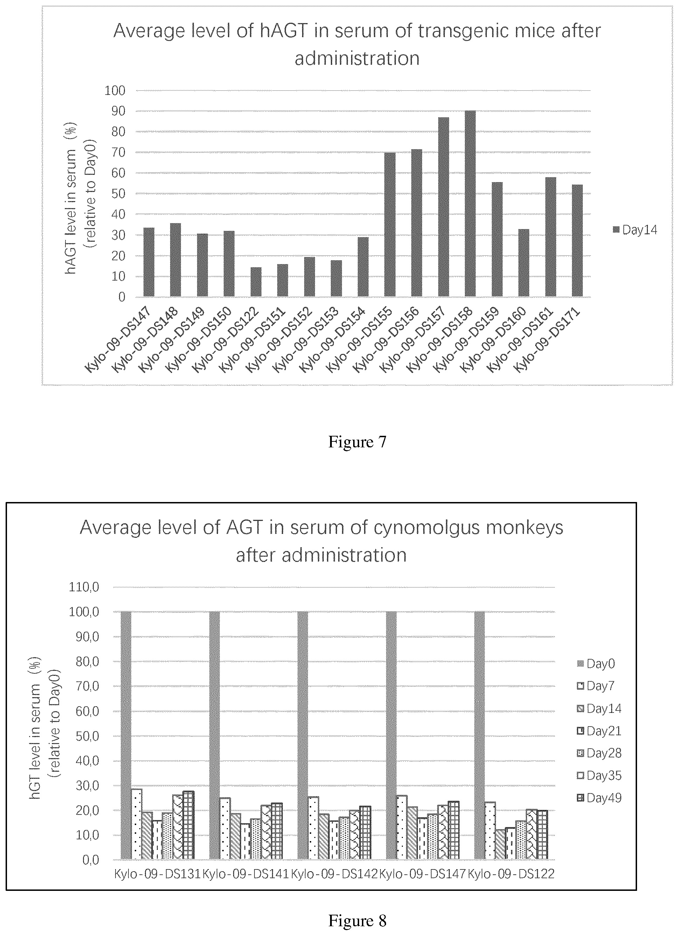

Synthesized the antisense strand in Table 16 and the sense strand in Table 17 according to the method described in Example 5, and synthesized the RNAi agent of this example Kylo-09-DS 122, Kylo-09-Ds147~160, Kylo-09-DS161 and Kylo-09-DS 171 in Table 18 by paired annealing. Selected female human AGT transgenic mice of appropriate age for evaluation experiment. Administered 3mg/kg by subcutaneous injection on Day0, the dosing volumes were 100-200 µL. Collected blood on Day 14 after administration, isolated serum, and the levels of hAGT in serum were determined by ELISA method. The test results are shown in Table 23 and Figure 7.

**Table 23 Average levels of hAGT in serum of transgenic mice**

| Single strand code | MVIP | Single strand code | MVIP | RNAi agent | Average levels on Dayl4(%) (Rel ative to day 0) |
|---|---|---|---|---|---|
| S211 | 5'MVIP01 | AS209 | 3' MVIP17 | Kylo-09-DS147 | 33.3 |
| S263 | 5' MVIP21 | AS209 | 3' MVIP17 | Kylo-09-DS148 | 35.6 |
| S210 | 5'MVIP17 | AS208 | 3' MVIP01 | Kylo-09-DS149 | 30.5 |
| S264 | 5' MVIP22 | AS247 | 3' MVIP07 | Kylo-09-DS150 | 31.9 |
| S140 | 5'MVIP09 | AS140 | 3'MVIP09 | Kylo-09-DS122 | 14.4 |
| S262 | 5' MVIP20 | AS248 | 3' MVIP10 | Kylo-09-DS151 | 15.8 |
| S261 | 5' MVIP19 | AS257 | 3'MVIP15 | Kylo-09-DS152 | 19.2 |
| S238 | 5' MVIP10 | AS254 | 3'MVIP12 | Kylo-09-DS153 | 17.8 |
| S213 | 5'MVIP09/ 3'MVIP09 | AS64 | / | Kylo-09-DS154 | 28.9 |
| S208 | 3'MVIP09 | AS266 | 5' MVIP09 | Kylo-09-DS155 | 69.7 |
| S250 | 3'MVIP10 | AS262 | 5' MVIP20 | Kylo-09-DS156 | 71.5 |
| S209 | 3'MVIP01 | AS207 | 5' MVIP17 | Kylo-09-DS157 | 86.9 |
| S241 | 3'MVIP02 | AS238 | 5' MVIP18 | Kylo-09-DS158 | 90.1 |
| S207 | 3'MVIP17 | AS265 | 5' MVIP01 | Kylo-09-DS159 | 55.4 |
| S225 | 3'MVIP18 | AS263 | 5' MVIP21 | Kylo-09-DS160 | 32.7 |
| S140 | 5'MVIP09 | AS209 | 3' MVIP17 | Kylo-09-DS161 | 57.8 |
| S210 | 5'MVIP17 | AS209 | 3' MVIP17 | Kylo-09-DS171 | 54.3 |

The above test results showed that the positions to which the carrier structure 5'MVIP and / or 3'MVIP is conjugated include the 5' end and / or 3' end of antisense strand, the 5' end and / or 3' end of sense strand, the 5' end of antisense strand and 3' end of sense strand, the 5' end and 3' end of sense strand. When various carrier structures are conjugated with the same AGT siRNA at different positions, the obtained RNAi agents have different inhibitory effects on the level of hAGT in transgenic mice. Among them, RNAi agents Kylo-09-DS122, 151, 152 and 153, which were obtained by annealing and pairing sense strands to which 5'MVIP09, 20, 19 and 10 were conjugated, and antisense strands to which 3'MVIP09, 10, 15 and 12 were correspondingly conjugated, with n and m are 2 respectively, were superior to other combinations in inhibiting hAGT of transgenic mice. RNAi agents Kylo-09-DS147, 148, 149 and 150, with n and m are different and n+m equal to 4 has secondary inhibitory effect. Among the RNAi agents obtained by conjugating carrier structure to the 5' end of antisense strand , the inhibition effect of Kylo-09-DS 160 obtained from the combination of 5'MVIP21 and 3'MVIP18 also reached 67.3%. Although Kylo-09-DS161 and Kylo-09-DS171 have n+m values of 5 and 6, respectively, they failed to show the advantage of having more branches in the inhibitory effect, which was presumed to be related to the specificity of the sequence or the steric hindrance of the introduced carrier structure. The test results showed that the RNAi agents obtained by combining 5'MVIP from Table 10 and/or 3'MVIP from Table 11 into a carrier structure had a certain inhibitory effect on the expression level of hAGT in transgenic mice.

### Example 8 Exploration and research on the efficacy of RNAi agents formed by the combination of 5'MVIP09/3'MVIP09 in cynomolgus monkeys

The purpose of this example is to study the effect of RNAi obtained by conjugating 5'MVIP and 3'MVIP with different structures to the same AGT siRNA in cynomolgus monkeys on inhibitory activity. Prepared the corresponding RNAi agent Kylo-09-DS 131, Kylo-09-DS 141, Kylo-09-DS 142, Kylo-09-DS 147 or Kylo-09-DS 122 according to the method described in Example 6, and selected 18 3-5-year-old male cynomolgus monkeys and randomly divided into control group (n=3) and administration group (n=3) according to body weight after the adaptive feeding. The dosage was 3mg/kg and the volume was 3ml/kg. The day of grouping and administration was defined as day0. Collected blood on Day 7, 14, 21, 28, 35, 42 and 49 after the first administration. After separating the plasma, determined the AGT level in serum by ELISA method. The test results are shown in Table 24 and Figure 8.

**Table 24 Average levels of AGT in serum of cynomolgus monkeys**

| **Sens e stra nd code** | **Carrier structure** | **Antisen se strand code** | **Carrier structure** | RNAi agent | Average levels of AGT (%) (relative to Day0) | | | | | | |
|---|---|---|---|---|---|---|---|---|---|---|---|
| | | | | | Day 0 | Day 7 | Day 14 | Day 21 | Day 28 | Day 35 | Day 49 |
| S211 | 5'MVIP01 | AS208 | 3' MVIP01 | Kylo-09-DS131 | 100.0 | 28.5 | 19.2 | 15.8 | 18.9 | 26.1 | 27.6 |
| S217 | 5'MVIP01/3'MV IP09 | AS64 | / | Kylo-09-DS141 | 100.0 | 24.9 | 18.6 | 14.5 | 16.5 | 21.9 | 22.8 |
| S218 | 5'MVIP09/3'MV IP01 | AS64 | / | Kylo-09-DS142 | 100.0 | 25.3 | 18.4 | 15.6 | 17.1 | 19.9 | 21.6 |
| S211 | 5'MVIP01 | AS209 | 3' MVIP17 | Kylo-09-DS147 | 100.0 | 25.9 | 21.3 | 16.8 | 18.4 | 22.0 | 23.5 |
| S140 | 5'MVIP09 | AS140 | 3'MVIP0 9 | Kylo-09-DS122 | 100.0 | 23.2 | 12.1 | 12.9 | 15.6 | 20.2 | 19.8 |

The experiment results showed that the combination of sense strand 5'MVIP and antisense strand 3'MVIP was 5'MVIP01/3'MVIP01, 5'MVIP01/3'MVIP17 or 5'MVIP09/3'MVIP09, or the combination of sense strand 5'MVIP and sense strand 3'MVIP was 5'MVIP01/3"MVIP09 or 5'MVIP09/3'MVIP01, both had significant effect on the inhibition of AGT levels in cynomolgus monkeys and had good sustainability.

## Claims

1. An RNAi agent comprising a carrier structure and an interfering nucleic acid in its structure, as shown in formula IIIa, IIIb or IIIc, or pharmaceutically acceptable salt thereof: 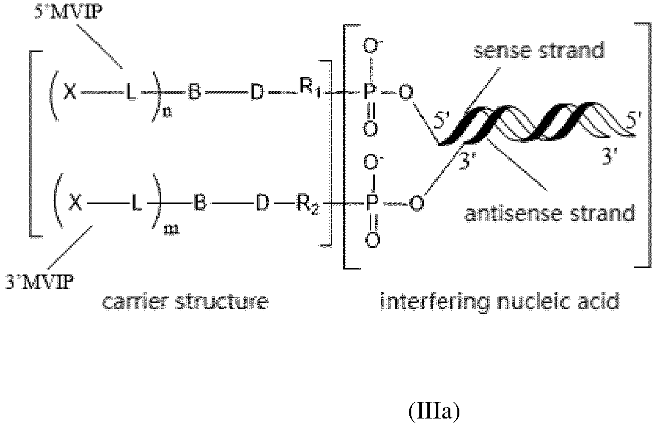 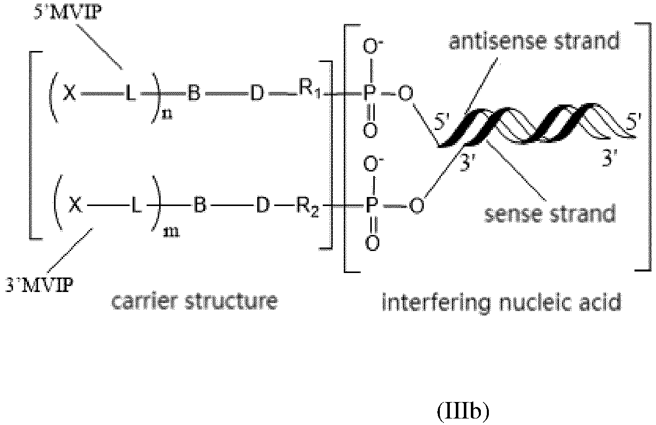 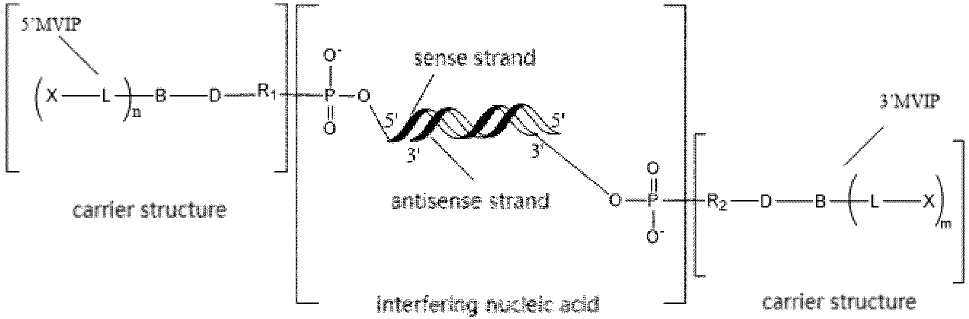 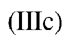 (IIIc) wherein,
the interfering nucleic acid targets an AGT gene, which includes antisense strand and sense strand; the carrier structure includes a 5'MVIP (5'MultiValent Import Platform) and / or a 3'MVIP (3' MultiValent Import Platform);
the 5'MVIP is composed of a transition point R₁, a linking chain D, a linker B, a branched chain L and a liver targeting specific ligand X, the 3'MVIP is composed of a transition point R₂, a linking chain D, a linker B, a branched chain L and a liver targeting specific ligand X, the 5'MVIP is connected with the 5' end of sense strand or the 5' end of antisense strand through the transition point R₁, the 3'MVIP is connected with the 3' end of sense strand or the 3' end of antisense strand through transition point R₂, n and m are each independently any integer from 0 to 4.

2. The RNAi agent or pharmaceutically acceptable salt thereof according to claim 1, wherein n+m is an integer of 2 to 6, preferably n+m=2, 3 or 4, more preferably 4.

3. The RNAi agent or pharmaceutically acceptable salt thereof according to any one of claims 1-2, wherein the 5'MVIP is selected from any one of 5'MVIP01 to 5'MVIP22 in Table 10, and / or the 3'MVIP is selected from any one of 3'MVIP01 to 3'MVIP27 in Table 11.

4. The RNAi agent or pharmaceutically acceptable salt thereof according to any one of claims 1-3, wherein the sense strand is essentially homologous to any one of SEQ ID NO: 1, SEQ ID NO: 7, SEQ ID NO: 8, SEQ ID NO: 10, SEQ ID NO: 11, SEQ ID NO: 12, SEQ ID NO: 17 and SEQ ID NO: 18 or a sequence that differs from any of the above by no more than 3 nucleotides.

5. The RNAi agent or pharmaceutically acceptable salt thereof according to any one of claims 1-4, wherein the antisense strand comprises any one of the following nucleotide sequences: SEQ ID NO: 19, SEQ ID NO: 25, SEQ ID NO: 26, SEQ ID NO: 28, SEQ ID NO: 29, SEQ ID NO: 30, SEQ ID NO: 35 and SEQ ID NO: 36 or a sequence that differs from any of the above by no more than 3 nucleotides.

6. The RNAi agent or pharmaceutically acceptable salt thereof according to any one of claims 1-5, wherein the sense strand comprises any one of SEQ ID NO: 37, SEQ ID NO: 43, SEQ ID NO: 44, SEQ ID NO: 46, SEQ ID NO: 47, SEQ ID NO: 48, SEQ ID NO: 53 and SEQ ID NO: 54 or a sequence that differs from any of the above by no more than 3 nucleotides, and the antisense strand comprises any one of SEQ ID NO: 55, SEQ ID NO: 61, SEQ ID NO: 62, SEQ ID NO: 64, SEQ ID NO: 65, SEQ ID NO: 66, SEQ ID NO: 71 and SEQ ID NO: 72 or a sequence that differs from any of the above by no more than 3 nucleotides.

7. The RNAi agent or pharmaceutically acceptable salt thereof according to any one of claims 1-6, wherein the interfering nucleic acid includes any one or more of Kylo-09-DS01, Kylo-09-DS07, Kylo-09-DS08, Kylo-09-DS10, Kylo-09-DS11, Kylo-09-DS12, Kylo-09-DS17, Kylo-09-DS18, Kylo-09-DS37- Kylo-09-DS54.

8. The RNAi agent or pharmaceutically acceptable salt thereof according to any one of claims 1-7, which includes any one or more of Kylo-09-DS 122, Kylo-09-DS131 to Kylo-09-DS 147 in table 18.

9. A pharmaceutical composition comprising the RNAi agent or pharmaceutically acceptable salt thereof according to any one of claims 1-8, and an optional pharmaceutically acceptable excipient, vehicle and / or diluent.

10. Use of the RNAi agent or pharmaceutically acceptable salt thereof according to any one of claims 1-8, or the pharmaceutical composition of claim 9 in the preparation of a drug used to prevent and/or treat a disease or condition or to reduce the risk of a disease or condition.
